(19) Europäisches Patentamt
European Patent Office
Office européen des brevets

(11) EP 1 276 491 B1

(12) **EUROPEAN PATENT SPECIFICATION**

(45) Date of publication and mention of the grant of the patent:
**20.09.2006 Bulletin 2006/38**

(21) Application number: **01924886.3**

(22) Date of filing: **09.04.2001**

(51) Int Cl.:
*A61K 38/00* (2006.01) *A61K 38/12* (2006.01)
*A61K 38/14* (2006.01) *C07K 17/00* (2006.01)
*C07K 9/00* (2006.01)

(86) International application number:
**PCT/US2001/011607**

(87) International publication number:
**WO 2001/076616 (18.10.2001 Gazette 2001/42)**

(54) **TAMANDARIN AND DIDEMNIN ANALOGS AND METHODS OF MAKING AND USING THEM**

TAMANDARIN UND DIDEMNIN ANALOGA UND METHODEN ZU DEREN HERSTELLUNG UND VERWENDUNG

ANALOGUES DE TAMANDARINE ET DE DIDEMNINE ET METHODES DE FABRICATION ET D'UTILISATION CORRESPONDANTES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **07.04.2000 US 545848**
**22.01.2001 US 767080**

(43) Date of publication of application:
**22.01.2003 Bulletin 2003/04**

(73) Proprietor: **THE TRUSTEES OF THE UNIVERSITY OF PENNSYLVANIA**
**Philadelphia, Pennsylvania 19104 (US)**

(72) Inventors:
- **JOULLIE, Madeleine, M.**
**Philadelphia, PA 19106 (US)**
- **LIANG, Bo**
**Drexel Hill, PA 19026 (US)**
- **DING, Xiaobin**
**Newark, DE 19711 (US)**

(74) Representative: **Grünecker, Kinkeldey, Stockmair & Schwanhäusser Anwaltssozietät**
**Maximilianstrasse 58**
**80538 München (DE)**

(56) References cited:

- **VERVOORT H ET AL: "TAMANDARINS A AND B: NEW CYTOTOXIC DEPSIPEPTIDES FROM A BRAZILIAN ASCIDIAN OF THE FAMILY DIDEMNIDAE" JOURNAL OF ORGANIC CHEMISTRY, AMERICAN CHEMICAL SOCIETY. EASTON, US, vol. 65, no. 3, January 2000 (2000-01), pages 782-792, XP002187653 ISSN: 0022-3263**
- **JOULLIE M M ET AL: "Total synthesis of (-)-tamandarin B" TETRAHEDRON LETTERS, ELSEVIER SCIENCE PUBLISHERS, AMSTERDAM, NL, vol. 41, no. 49, 2 December 2000 (2000-12-02), pages 9373-9376, XP004226174 ISSN: 0040-4039**
- **BO LIANG, MATTHEW D. VERA, MADELEINE M. JOULLI[1]: "Total Synthesis of [(2S)-Hiv2]Didemnin M" J. ORG. CHEM., vol. 65, 2000, pages 4762-4765, XP002286347**
- **LIANG ET AL.: 'The first total synthesis of (-)-tamandarin A' ORGANIC LETTERS vol. 1, no. 8, 1999, pages 1319 - 1322, XP002943722**
- **SAKAI ET AL.: 'Structure-activity relationships of the didemnins 1,2' JOURNAL OF MEDICAL CHEMISTRY vol. 39, no. 14, 1996, pages 2819 - 2834, XP002943723**
- **LI ET AL.: 'The didemnins: Biological properties, chemistry and total synthesis' STUDIES IN NATURAL PRODUCTS CHEMISTRY vol. 10, 1992, pages 241 - 302, XP002943724**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **GRUBB ET AL.: 'Didemnin B induces cell death by apoptosis: The fastest induction of apoptosis ever described' BIOCHEMICAL AND BIOPHYSICAL RESEARCH COMMUNICATIONS vol. 215, no. 3, 24 October 1995, pages 1130 - 1136, XP002943725**

## Description

### BACKGROUND OF THE INVENTION

Didemnin B is a macrocyclic depsipeptide isolated from a species of marine tunicate. Didemnin B exhibits potent anti-viral, immunosuppressive, and anti-tumor activities in vitro and in vivo, and was the first marine natural product to enter clinical testing against human cancers (Li et al., 1992, Studies in Natural Products Chemistry, 10:241-302; Sakai et al., 1996, J. Med. Chem. 39:2819-2834; Wipf, 1995, Chem. Rev. 95:2115-2134). Didemnin B is a didemnin, a family of compounds which potently inhibit protein synthesis and cell cycle progression, and induce more rapid apoptosis than any other natural products that has been isolated to date (Grubb et al., 1995, Biochem. Biophys, Res. Commun. 215: 1130-1136; Johnson et al., 1996, FEBS Lett. 383:1-5; Johnson et al., 1999, Immunol. Cell Biol. 77:242-248; Johnson et al., 1999, J. Cell. Biochem. 72:269-278). Other members of this family of compounds, including didemnin M and dehydrodidemnin B, exhibit cytotoxic and cytostatic effects as well.

Tamandarin A (also designated {(2S)Hiv$^2$}didemnin B) is a naturally occurring didemnin congener which has recently been isolated from a marine tunicate. Tamandarin A exhibits biological activity which is analogous to the activities exhibited didemnin B. For example, tamandarin A is a potent inhibitor of protein synthesis, cell growth, and tumorigenesis. Tamandarin A exhibits greater in vitro activity against pancreatic carcinoma than does didemnin B (Liang et al., 1999, Org. Lett. 1: 1319-1322). A significant limitation on use of tamandarin A, either for research or for practical applications, is the limited supply of tamandarin A that is available from natural sources and the difficulty and expense of isolating this product. A need exists for a method of synthesizing tamandarin A and other didemnin analogs (including dehydrodidemnin analogs).

Despite the potency of didemnin B in isolated studies, its clinical effectiveness is hampered by side effects associated with therapeutic doses of the compound. As with many anti-proliferative agents, didemnin B exhibits a relatively narrow therapeutic window. Although didemnin M and dehydrodidemnin B exhibit improved therapeutic potential, relative to didemnin B, a need still exists for anti-proliferative agents which exhibit less toxicity at a therapeutic dose (i.e., didemnin analogs having a greater therapeutic index).

The present invention satisfies the needs set forth above.

### BRIEF SUMMARY OF THE INVENTION

The invention relates to tamandarin and didemnin analogs that have a deoxo-proline residue or a dehydroproline residue in their structure. In one embodiment, the invention relates to a composition comprising a tamandarin analog having the structure of formula **I**

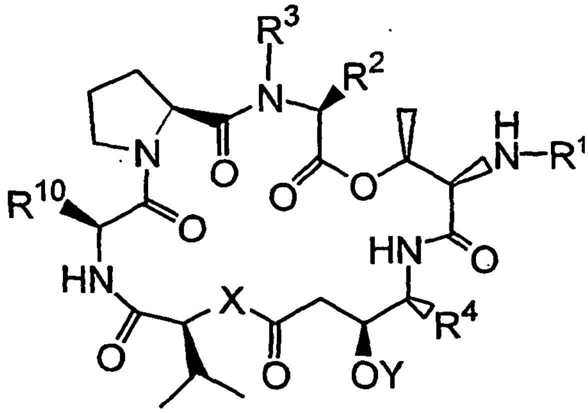

(I).

In formula **I**, $R^1$ is selected from the group consisting of

-H,
-(tert-butyloxycarbonyl),
-leucine,
-(N-methyl)leucine,
-(N-methyl)leucine-(a first fluorophore),
-(N-methyl)leucine-proline,

-(N-methyl)leucine-proline-lactate,
-(N-methyl)leucine-proline-pyruvate,
-(N-methyl)leucine-proline-lactate-(a first fluorophore),
-(N-methyl)leucine-proline-lactate-glutamine-pyroglutamate,
-(N-methyl)leucine-proline-lactate-glutamine-cyclopentanoate,
-(N-methyl)leucine-proline-alanine-leucine-pyroglutamate,
-(N-methyl)leucine-proline-(N-methyl-alanine)-leucine-pyroglutamate,
-(N-methyl)leucine-deoxo-proline,
-(N-methyl)leucine-deoxo-proline-lactate,
-(N-methyl)leucine-deoxo-proline-pyruvate,
-(N-methyl)leucine-deoxo-proline-lactate-(a first fluorophore),
-(N-methyl)leucine-deoxo-proline-lactate-glutamine-pyroglutamate,
-(N-methyl)leucine-deoxo-proline-lactate-glutamine-cyclopentanoate,
-(N-methyl)leucine-deoxo-proline-alanine-leucine-pyroglutamate, and
-(N-methyl)leucine-deoxo-proline-(N-methyl-alanine)-leucine-pyroglutamate,
-(N-methyl)leucine-dehydro-proline,
-(N-methyl)leucine-dehydro-proline-lactate,
-(N-methyl)leucine-dehydro-proline-pyruvate,
-(N-methyl)leucine-dehydro-proline-lactate-(a first fluorophore),
-(N-methyl)leucine-dehydro-proline-lactate-glutamine-pyroglutamate,
-(N-methyl)leucine-dehydro-proline-lactate-glutamine-cyclopentanoate,
-(N-methyl)leucine-dehydro-proline-alanine-leucine-pyroglutamate, and
-(N-methyl)leucine-dehydro-proline-(N-methyl-alanine)-leucine-pyroglutamate.

$R^2$ and $R^3$ in formula **I**, can be separate moieties or they can, together, be a single moiety. When $R^2$ and $R^3$ are separate moieties, $R^3$ is either a methyl group or a hydride radical and $R^2$ is selected from the group consisting of an isoleucine side chain, a valine side chain, an alanine side chain, a norleucine side chain, a norvaline side chain, leucine side chain, a histidine side chain, a tryptophan side chain, an arginine side chain, a lysine side chain, a second fluorophore, and a substituent having the structure of formula **III**

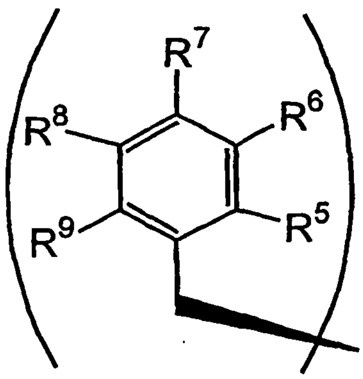

(III).

When $R^2$ and $R^3$ are, together, a single substituent, this substituent has the structure of formula **IV**

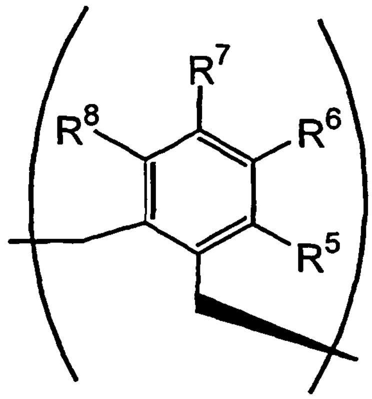

(IV).

In formulas **III** and **IV,** each of $R^5$, $R^6$, $R^7$, $R^8$, and $R^9$ is independently selected from the group consisting of -H, -OH, $-OCH_3$, $-CO(C_6H_5)$, Br, -I, -F, -Cl, $-CH_3$, and $-C_2H_5$;.

$R^4$ in formula **I** is an isoleucine side chain. Also, in formula **I,** X is either -O- or -(NH)-, Y is either a hydride radical or a hydroxyl protecting group, and $R^{10}$is either a leucine side chain or a lysine side chain. The didemnin analog is an analog other than tamandarin A (i.e., {(2S)Hiv$^2$ }didemnin B). In one embodiment, every proline or lactate moiety that is present in $R^1$ exhibits (S) stereochemistry. In another, every moiety capable of exhibiting stereochemistry in $R^1$ is present in its naturally occurring form (i.e., the (S) form for amino acid residues and lactate. It is believed that cyclopentanoate occurs naturally in an (S) stereochemistry.

In another embodiment, the invention relates to a composition comprising a didemnin analog having the structure of formula **XXI**

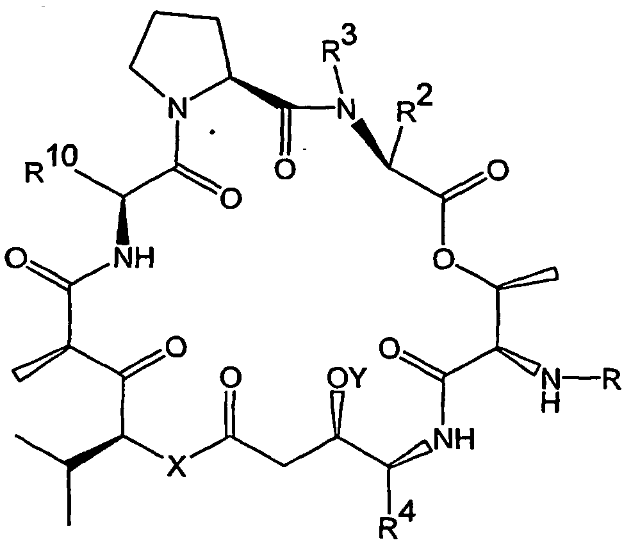

(XXI).

In formula **XXI,** each of $R^1$, $R^2$, $R^3$, $R^4$, $R^5$, $R^6$, $R^7$, $R^8$, $R^9$, and $R^{10}$ has the same meaning as in formula **I**.

In preferred classes of deoxo-proline tamandarin and didemnin analogs having the formula of **I** and **XXI**, respectively, $R^2$ has the structure of formula **III,** $R^3$ is methyl, $R^4$ is an isoleucine side chain, each of $R^5$, $R^6$, $R^8$, and $R^9$ is a hydride radical, $R^7$ is methoxy, $R^{10}$ is a leucine side chain, X is -O-, and Y is a hydride radical. Examples of tamandarin and didemnin analogs that are included in the invention are compounds **103, 104, 105, 106, 107, 108,109,110,115,116, 117,118,119,120,121, 122, 123, 124, 125, 126, 127, 128, 129, 130, 133,134,136, 137, 139, 141, 142, 143, 201, 202, 203**, and **204**, which are shown in the Figures.

In one embodiment, the tamandarin or didemnin analog has a photoreactive substituent, such as an $R^2$ moiety having the structure

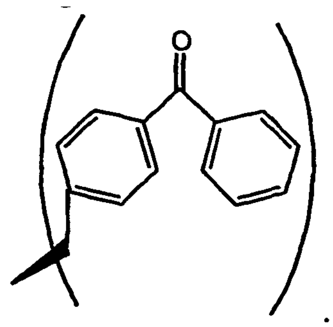

The photoreactive substituent can be directly attached to the analog, or it can be attached by way of a linker having a chain that comprises 1 to about 13 or more carbon atoms, and optionally having secondary amine or amide moieties in the chain.

In another embodiment, the tamandarin or didemnin analog has a fluorophore attached, such as an analog in which a fluorophore is attached at the omega amino moiety of a lysine side chain at $R^2$ or at $R^{10}$. An example of the structure of such a fluorescent didemnin analog is show in Figure 29. Alternatively, the didemnin analog can be attached (e.g., covalently) with a support. In most embodiments, Y in formulas **I** and **XXI** is preferably a hydride radical.

The invention includes an embodiment of a tamandarin or didemnin analog which can be activated (or the activity of which can be enhanced) by enzymatic cleavage of a moiety bound with the analog. For example, the invention includes compositions which comprise an analog having a structure selected from the group consisting of formulas (a)-(d), as follows.

(a)

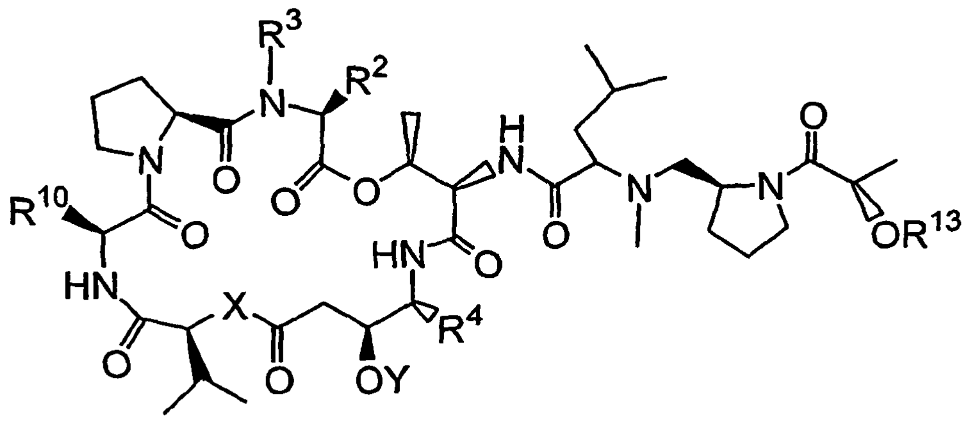

(b)

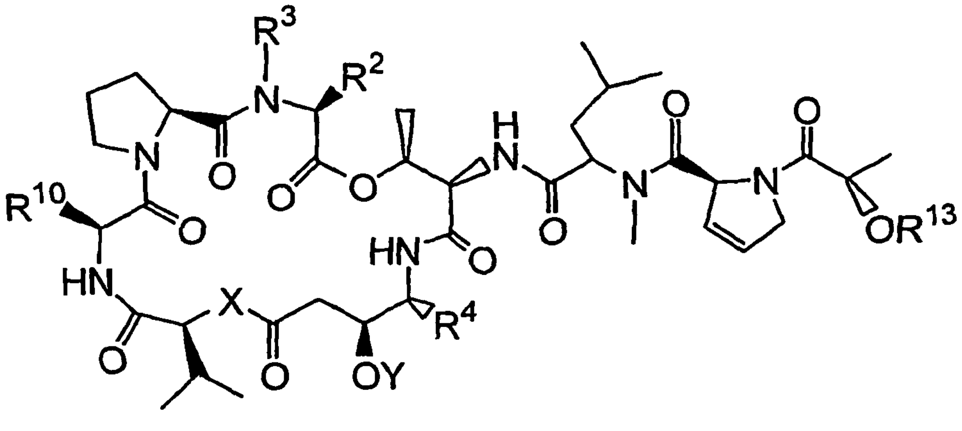

(c)

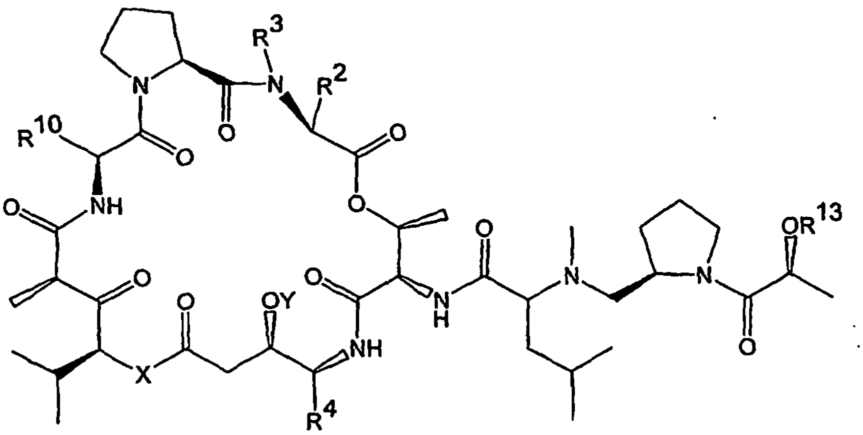

and

(d)

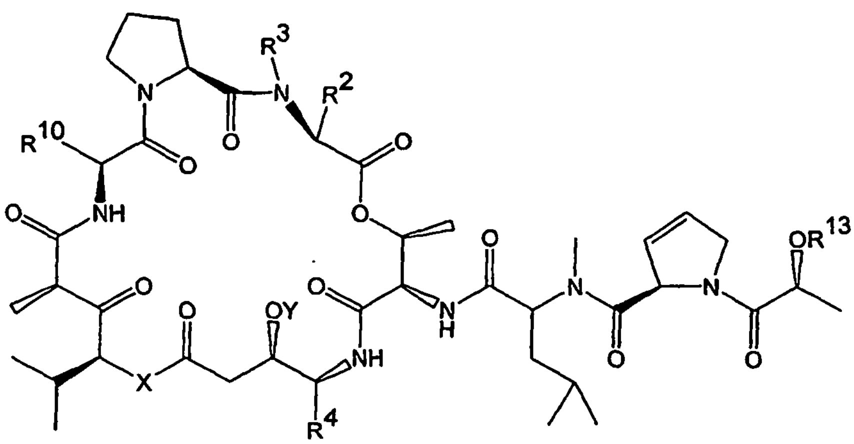

In formulas (a)-(d), $R^2$, $R^3$, $R^4$, $R^{10}$, X, and Y have the same identities described above for formula **I**. $R^{13}$ is an enzyme-cleavable moiety that is cleavable by an enzyme, such as one selected from the group consisting of a carboxypeptidase, a beta-lactamase, a beta-galactosidase, a penicillin V-amidase, a cytosine deaminase, a nitroreductase, a alkaline phosphatase, a beta-glucuronidase, and a catalytic antibody. By way of example, $R^{13}$ can have the structure of either of formulas **V** and **VI**

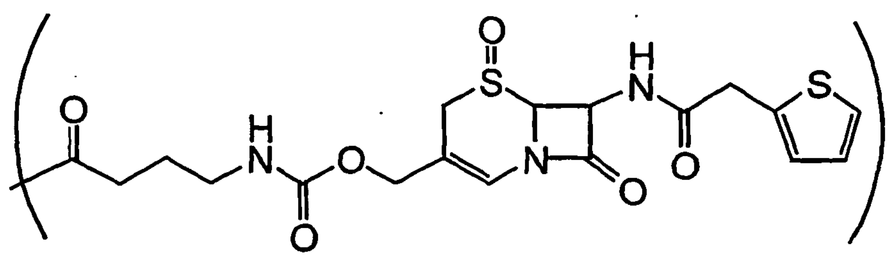

(V)

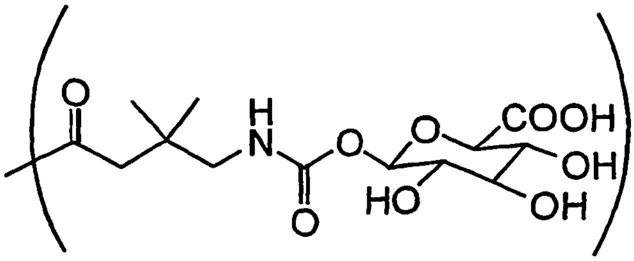

(VI).

Examples of such analogs include compound **131** and compound **132**.

The invention also relates to compositions which comprise a didemnin fragment having the structure of formula **VII**

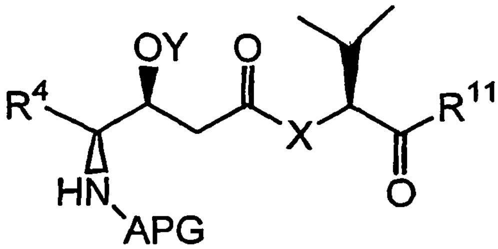

(VII).

In formula **VII,** Y is either a hydride radical or a hydroxyl protecting group, X is either -O- or -(NH)-, $R^4$ is either an isoleucine side chain or a valine side chain, and APG is a amine protecting group. $R^{11}$ can be any of -OH, $NH_2$, -O (allyl), -O(pentafluorophenyl), and a substituent having the structure of formula **VIII**

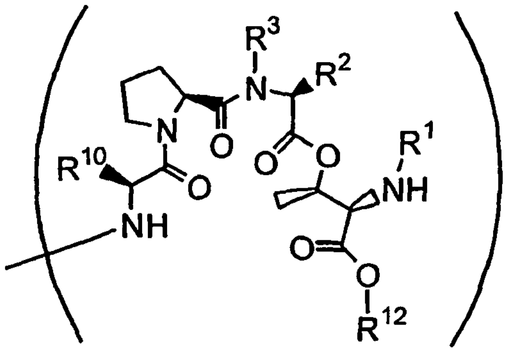

(VIII).

In formula **VIII**, $R^1$, $R^2$, $R^3$, and $R^{10}$ have the same identities described above for formula **I**, and $R^{12}$ can be either a hydride radical or a -2-(trimethylsilyl) ethoxycarbonyl moiety.

The tamandarin and didemnin analogs described herein can be formulated, together with one or more pharmaceutically acceptable carriers, to make pharmaceutical preparations. These preparations can be administered to a mammalian (e.g., human) cell (i.e., either in vitro or in vivo) in order to inhibit protein synthesis, inhibit growth, inhibit proliferation, inhibit tumorigenesis, or enhance apoptosis in the cell or in one or more tissues of the mammal.

The invention further includes a method of making a didemnin fragment. This method comprises coupling a first reactant having the structure

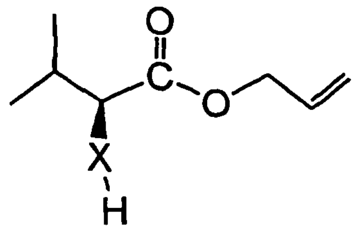

and a second reactant having the structure

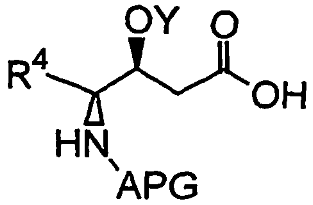

to yield a first didemnin fragment having the structure

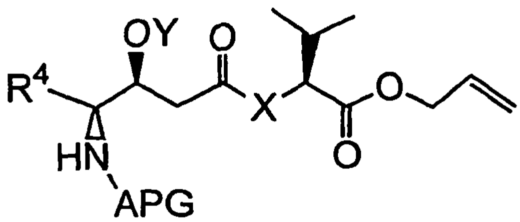

In this structure, X is either -O- or -(NH)-, APG is an amine protecting group; Y is a hydroxyl protecting group (e.g., a triisopropylsilyl group), and $R^4$ can be either an isoleucine side chain or a valine side chain. The first didemnin fragment can be hydrolyzed to yield a second didemnin fragment having the structure

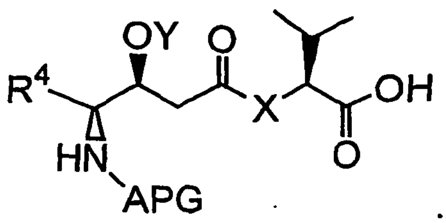

An activator (ACT) can be added to the carbonyl moiety of the second didemnin fragment to yield a third didemnin fragment having the structure

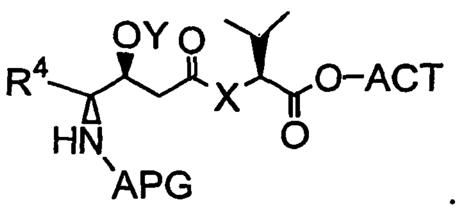

The third didemnin fragment can be coupled with a third reactant which has the structure

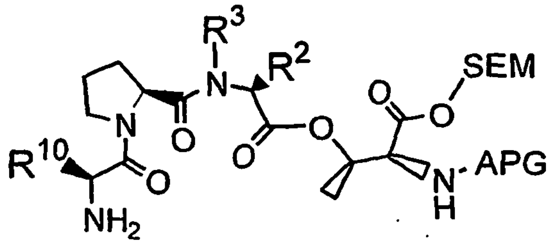

to yield a fourth didemnin fragment having the structure

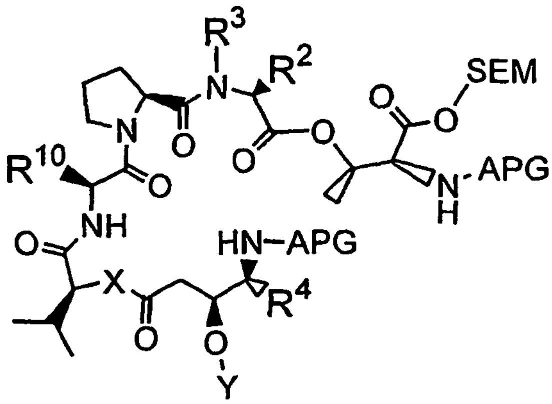

In this structure, $R^2$ and $R^3$ have the identities described above for formula **I**, APG is an amine protecting group, SEM is a 2-(trimethylsilyl)ethoxycarbonyl group, and $R^{10}$ is either a leucine side chain or a lysine side chain.

The invention also relates to a method of making a didemnin analog from the fourth didemnin fragment. This method comprises removing the SEM and CBZ moieties from the fourth didemnin fragment and cyclizing the fragment to yield a first didemnin analog having the following structure.

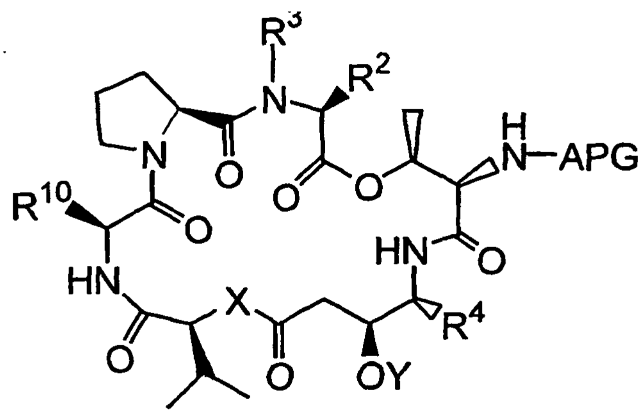

The APG group (which can, for example, be a carbobenzyloxy moiety or a tert-butyloxycarbonyl moiety) can be removed from the first didemnin analog to yield a second didemnin analog having the structure

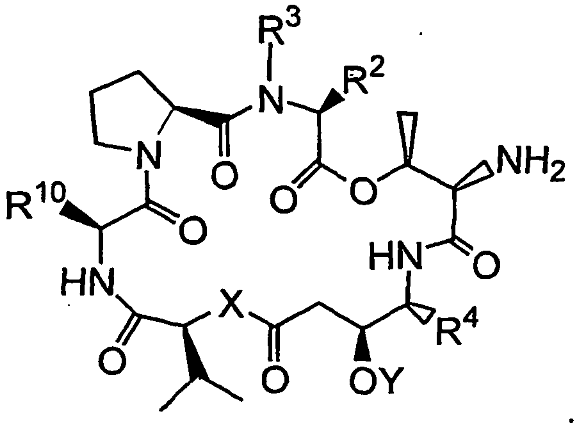

This second didemnin analog can be coupled with a fourth reagent having the structure

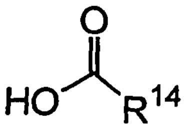

to yield a third didemnin analog having the structure

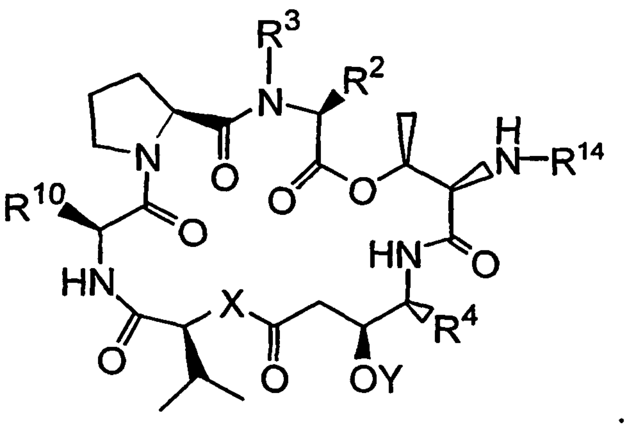

In these structures, $R^{14}$ is can be one of

-(tert-butyloxycarbonyl),
-leucine,
-(N-methyl)leucine,
-(N-methyl)leucine-(a first fluorophore),
-(N-methyl)leucine-proline,
-(N-methyl)leucine-proline lactate,
-(N-methyl)leucine-proline-pyruvate,
-(N-methyl)leucine-proline-lactate-(a first fluorophore),
-(N-methyl)leucine-proline-lactate-glutamine-pyroglutamate,
-(N-methyl)leucine-proline-lactate-glutamine-cyclopentanoate,
-(N-methyl)leucine-proline-alanine-leucine-pyroglutamate,
-(N-methyl)leucine-proline-4-(N-methyl-alanine)-leucine-pyroglutamate,
-(N-methyl)leucine-deoxo-proline,

-(N-methyl)leucine-deoxo-proline-lactate,
-(N-methyl)leucine-deoxo-proline-pyruvate,
-(N-methyl)leucine-deoxo-proline-lactate-(a first fluorophore),
-(N-methyl)leucine-deoxo-proline-lactate-glutamine-pyroglutamate,
-(N-methyl)leucine-deoxo-proline-lactate-glutamine-cyclopentanoate,
-(N-methyl)leucine-deoxo-proline-alanine-leucine-pyroglutamate, and
-(N-methyl)leucine-deoxo-proline-(N-methyl-alanine)-leucine-pyroglutamate,
-(N-methyl)leucine-dehydro-proline,
-(N-methyl)leucine-dehydro-proline-lactate,
-(N-methyl)leucine-dehydro-proline-pyruvate,
-(N-methyl)leucine-dehydro-proline-lactate-(a first fluorophore),
-(N-methyl)leucine-dehydro-proline-lactate-glutamine-pyroglutamate,
-(N-methyl)leucine-dehydro-proline-lactate-glutamine-cyclopentanoate,
-(N-methyl)leucine-dehydro-proline-alanine-leucine-pyroglutamate, and
-(N-methyl)leucine-dehydro-proline-(N-methyl-alanine)-leucine-pyroglutamate, or it can be one of these moieties coupled with an enzyme-cleavable moiety that is cleavable by an enzyme such as one of a carboxypeptidase, a beta-lactamase, a beta-galactosidase, a penicillin V-amidase, a cytosine deaminase, a nitroreductase, an alkaline phosphatase, a beta-glucuronidase, and a catalytic antibody. If Y is a hydroxyl protecting group, then that can be removed from the third didemnin analog (either before or after addition of $R^{14}$) to yield a fourth didemnin analog having the structure

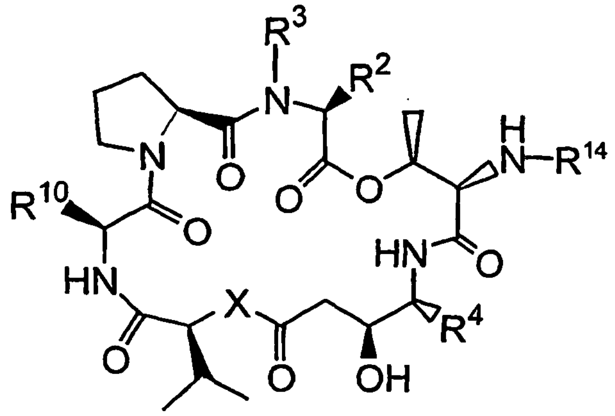

The invention further relates to a method of making deoxo-proline-containing tamandarin and didemnin analogs. These methods employ known methods for making tamandarin and didemnin analogs, and are modified to incorporate a deoxo-proline residue in place of a proline residue of the analog.
The invention still further relates to a method of making dehydro-proline-containing tamandarin and didemnin analogs. These methods employ known methods for making tamandarin and didemnin analogs, and are modified to incorporate a dehydro-proline residue in place of a proline residue of the analog.

BRIEF DESCRIPTION OF THE DRAWINGS

**Figure 1,** comprising Figures 1A and 1B, depicts the structure of tamandarin A (i.e., {(2S)HIV$^2$}didemnin B). Figure 1A is the structure of (-) tamandarin A (compound **101**). Figure 1B is the structure of a diastereomer (compound **102**) of (-) tamandarin A. The chiral center at which these two molecules differ is indicated with an arrow.
**Figure 2,** comprising Figures 2A and 2B, depicts the structure of tamandarin M (i.e., {(2S)HIV$^2$}didemnin M). Figure 2A is the structure of (-) tamandarin M (compound **103**). Figure 2B is the structure of a diastereomer (compound **104**) of (-) tamandarin M. The chiral center at which these two molecules differ is indicated with an arrow.
**Figure 3,** comprising Figures 3A and 3B, depicts the structure of tamandarin B (i.e., {(2S)HIV$^2$}didemnin B). Figure 3A is the structure of (-) tamandarin B (compound **105**). Figure 3B is the structure of a diastereomer (compound **106**) of (-) tamandarin B. The chiral center at which these two molecules differ is indicated with an arrow.
**Figure 4,** comprising Figures 4A, 4B, 4C, and 4D, depicts the structure of several fluorescent tamandarin-type

didemnin analogs. Figure 4A is the structure of compound **107**. Figure 4B is the structure of compound **108**. The chiral center at which compounds **107** and **108** differ is indicated with an arrow. Figure 4C is the structure of compound **109**. Figure 4D is the structure of compound **110**. The chiral center at which compounds **109** and **110** differ is indicated with an arrow.

**Figure 5**, comprising Figures 5A and 5B, depicts a class of immobilizable tamandarin-type didemnin analogs. Figure 5A is the structure of a didemnin analog of formula **I** herein, wherein $R^{10}$ is a lysine side chain. Figure 5B is the structure of the didemnin analog of Figure 5A bound with a solid support (SS).

**Figure 6,** comprising Figures 6A and 6B, depicts another class of immobilizable tamandarin-type didemnin analogs. Figure 6A is the structure of didemnin analog of formula **I** herein, wherein $R^1$ is -leucine. Figure 6B is the structure of the didemnin analog of Figure 6A bound with a solid support (SS).

**Figure 7** is the structure of compound **115.**

**Figure 8** is the structure of compound **116.**

**Figure 9** is the structure of compound **117.**

**Figure 10** is the structure of compound **118.**

**Figure 11** is the structure of compound **119.**

**Figure 12** is the structure of compound **120.**

**Figure 13** is the structure of compound **121.**

**Figure 14** is the structure of compound **122.**

**Figure 15** is the structure of compound **123.**

**Figure 16** is the structure of compound **124.**

**Figure 17** is the structure of compound **125**.

**Figure 18** is the structure of compound **126**.

**Figure 19** is the structure of compound **127**.

**Figure 20** is the structure of compound **128**.

**Figure 21** is the structure of compound **129**.

**Figure 22** is the structure of compound **130**.

**Figure 23** depicts enzymatic cleavage of the cephalosporin moiety of didemnin analog **131** by beta-lactamase to yield compound **101**.

**Figure 24** depicts enzymatic cleavage of the glucoside moiety of didemnin analog **132** by beta-glucuronidase to yield compound **101**.

**Figure 25,** comprising Figures 25A and 25B, is a pair of structures which illustrates the structural difference between tamandarin A (**101**; Figure 25A, and didemnin B (**201**; Figure 25B. The macrocyclic core of **101** differs from that of **201** in that **101** contains an alpha-hydroxyisovaleryl (Hiv) moiety, and **201** contains an alpha-(alpha-hydroxyisovaleryl)-propionyl (Hip) moiety at the analogous position, as indicated by the brackets and dotted lines in each of the figures.

**Figure 26,** comprising Figures 26A-26E, depicts a synthetic method for generating didemnin analogs described herein.

**Figure 27,** is the structure of (-) Tamandarin A (i.e., {(2S)$HIV^2$} didemnin B), illustrating the numbering convention used herein and in Sakai *et al.* (1996, J. Med. Chem. 39:2819-2834) for didemnin analogs.

**Figure 28,** comprising Figures 28A and 28B, depicts the structure of a dehydrotamandarin-type didemnin analog (i.e., {(2S)$HIV^2$}dehydrodidemnin B). Figure 28A is the structure of (-) dehydrotamandarin (compound **133**). Figure 28B is the structure of a diastereomer of (-) dehydrotamandarin (compound **134**). The chiral center at which these two molecules differ is indicated with an arrow. The position at which these dehydrotamandarin-type didemnin analogs differ from tamandarin-type didemnin analogs is indicated with an asterisk.

**Figure 29** is the structure of a fluorescent dehydrotamandarin-type didemnin analog. "FL" is a fluorophore.

**Figure 30** is the structure of compound **136**.

**Figure 31** is the structure of compound **137**.

**Figure 32** depicts a dehydrotamandarin-type didemnin analog bound with a solid support (SS).

**Figure 33** is the structure of compound **139**.

**Figure 34** is the structure of compound **140**

**Figure 35,** comprising Figures 35A and 35B, depicts the structure of dehydrotamandarin B, also designated {(2S) $Hiv^2$, $Norstal^1$}didemnin B. Figure 35A is the structure of (-) dehydrotamandarin B (compound **141**). Figure 35B is the structure of a diastereomer of (-) dehydrotamandarin B (compound **142**). The chiral center at which these two molecules differ is indicated with an arrow.

**Figure 36** is the structure of compound **143**

**Figure 37**, comprising Figures 37A and 37B, depicts a synthetic method for generating (-) dehydrotamandarin (i.e., {(2S)$HIV^2$}dehydrodidemnin B, compound **133**).

**Figure 38**, comprising Figures 38A, 38B, and 38C, depicts a synthetic method for generating fluorescent didemnin

analogs described herein.

**Figure 39** is the structure of a preferred deoxo-proline tamandarin analog designated compound **201a**.

**Figure 40** is the structure of a preferred deozo-proline didemnin analog designated compound **202**.

Each of **Figure 41, Figure 42,** and **Figure 43** depicts a method of making deoxo-proline-containing side chain moieties for tamandarin or didemnin analogs.

**Figure 44** depicts a method of making dehydro-proline-containing side chain moieties for tamandarin or didemnin analogs.

**Figure 45** is the structure of a preferred dehydro-proline tamandarin analog designated compound **203**.

**Figure 46** is the strudure of a preferred dehydro-proline didemnin analog designated compound **204.**

Each of **Figure 47, Figure 48, Figure 49**, and **Figure 50** depicts a method of making didemnin analogs having photoreactive side chain moieties.

DETAILED DESCRIPTION OF THE INVENTION

The invention relates to tamandarin and didemnin analogs, including analogs which have a deoxo-proline residue or a dehydro-proline residue in their structure. The invention includes compositions comprising such analogs, and methods for making and using these analogs. These analog are useful for, among other things, inhibiting protein synthesis, cell growth, cell proliferation, and tumorigenesis. The analogs of the invention can also exhibit anti-viral, anti-tumor, apoptosis-inducing, and immunosuppressive activities in animals, including in humans.

The invention includes compositions comprising a tamandarin analog having the structure

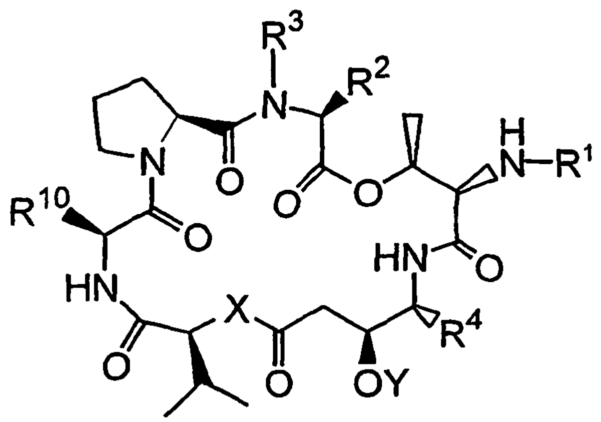

wherein $R^1$, $R^2$, $R^3$, $R^4$, $R^{10}$, X, and Y have the identities described herein. Examples of analogs according to this formula are shown in the figures.

The invention also includes compositions comprising a didemnin analog having the structure

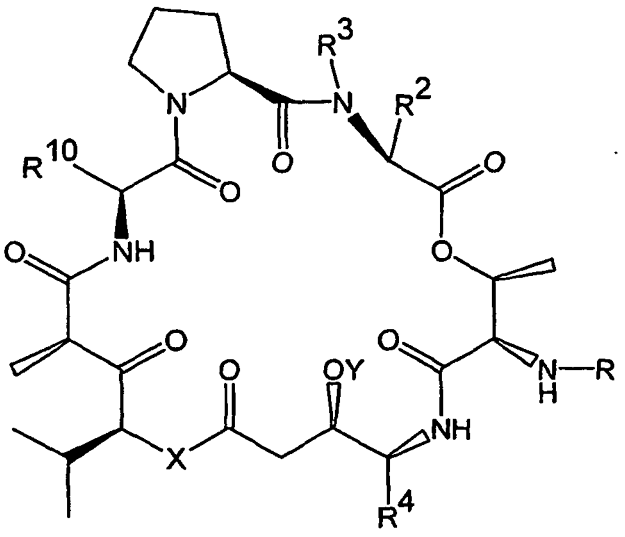

wherein $R^1$, $R^2$, $R^3$, $R^4$, $R^{10}$, X, and Y have the identities described herein.

Definitions

As used herein, each of the following terms has the meaning associated with it in this section.

The articles "a" and "an" are used herein to refer to one or to more than one (i.e., to at least one) of the grammatical object of the article. By way of example, "an element" means one element or more than one element.

As used herein, amino acid residues are represented by the full name thereof, by the three letter code corresponding thereto, or by the one-letter code corresponding thereto, as indicated by the following:

| Full Name | Three-Letter Code | One-Letter Code |
|---|---|---|
| Aspartic Acid | Asp | D |
| Glutamic Acid | Glu | E |
| Lysine | Lys | K |
| Arginine | Arg | R |
| Histidine | His | H |
| Tyrosine | Tyr | Y |
| Cysteine | Cys | C |
| Asparagine | Asn | N |
| Glutamine | Gln | Q |
| Serine | Ser | S |
| Threonine | Thr | T |
| Glycine | Gly | G |
| Alanine | Ala | A |
| Valine | Val | V |
| Leucine | Leu | L |
| Isoleucine | Ile | I |
| Methionine | Met | M |
| Proline | Pro | P |
| Phenylalanine | Phe | F |
| Tryptophan | Trp | W |

As used herein, the term "amino acid side chain" refers to a moiety comprising all of the atoms of an amino acid excluding the alpha-carbon atom, a hydrogen atom bound with the alpha-carbon, the atoms of the alpha-carboxyl moiety and the alpha-amine moiety. By way of example, an "alanine side chain" refers to a methyl group, and a "valine

side chain" refers to a 2-propyl group.

"Inhibition" of a process in a cell (e.g., inhibition of protein synthesis, inhibition of cell growth, inhibition of cell cycle progression, inhibition of cell proliferation, or inhibition of tumorigenesis) means reduction (e.g., by at least 10%, 25%, 50%, 75%, 90%, 95%, or even 100%) of the rate at which the process proceeds, reduction (e.g., by at least 10%, 25%, 50%, 75%, 90%, 95%, or even 100%) of the rate at which the process is initiated, or both.

"Enhancement" of a process in a cell (e.g., enhancement of apoptosis) means increasing (e.g., by at least 10%, 25%, 50%, 75%, 90%, 95%, or even 100%) the rate at which the process proceeds, increasing (e.g., by at least 10%, 25%, 50%, 75%, 90%, 95%, or even 100%) the rate at which the process is initiated, or both.

As used herein, the term "pharmaceutically acceptable carrier" means a chemical composition with which a didemnin analog or fragment, as described herein, can be combined and which, following the combination, can be administered to a subject (e.g., a human or other animal).

As used herein, the term "physiologically acceptable" ester or salt means an ester or salt form of a didemnin analog or fragment, as described herein, which is compatible with other ingredients of a pharmaceutical composition and which is not deleterious to a subject to which the composition is to be administered.

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration can include, but is not limited to, subcutaneous, intraperitoneal, intramuscular, intrasternal injection, and kidney dialytic infusion techniques.

As used herein, the term "anti-viral activity" means preventing replication of a virus in the cell, preventing infection of the cell by a virus, or reversing a physiological effect of infection of the cell by a virus. An anti-viral agent a composition of matter which, when delivered to a cell, exhibits anti-viral activities. Anti-viral agents are well known and described in the literature. By way of example, AZT (zidovudine, Retrovir® Glaxo Wellcome Inc., Research Triangle Park, NC) is an anti-viral agent which is thought to prevent replication of HIV in human cells.

As used herein, a "deoxo-proline" moiety or residue is a chemical moiety which has the following structure.

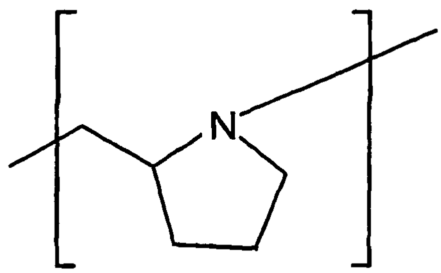

As used herein, a "dehydro-proline" moiety or residue is a chemical moiety which has the following structure.

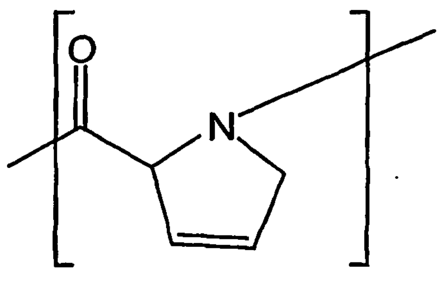

Description

The present invention relates to tamandarin and didemnin analogs, including those having a deoxo-proline moiety or a dehydro-proline in their structure (and, of course, those which do not have a deoxo-proline moiety or a dehydro-proline in their structure). These analogs exhibit potent pharmacological properties when administered to humans and other mammals. By way of example, these compounds can inhibit protein synthesis and cell growth and

proliferation. These compounds can also enhance apoptosis in cells. These properties render the compounds useful for treating a variety of disorders which are characterized by one or more of aberrant protein synthesis, aberrant cell growth, aberrant proliferation of cells, and aberrant apoptosis. Examples of such disorders include tumorigenesis, tumor growth, tumor metastasis, infection of a cell by a virus, and replication of a virus within a cell.

Among the compositions of the inventions are those which comprise a tamandarin analog having the structure of formula **I** or a didemnin analog having the structure of formula **XXI.**

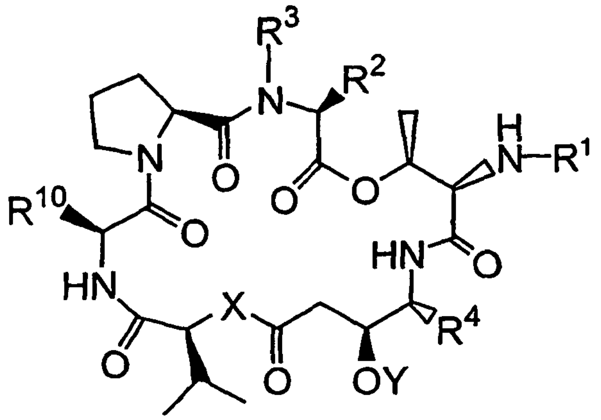

**(I).**

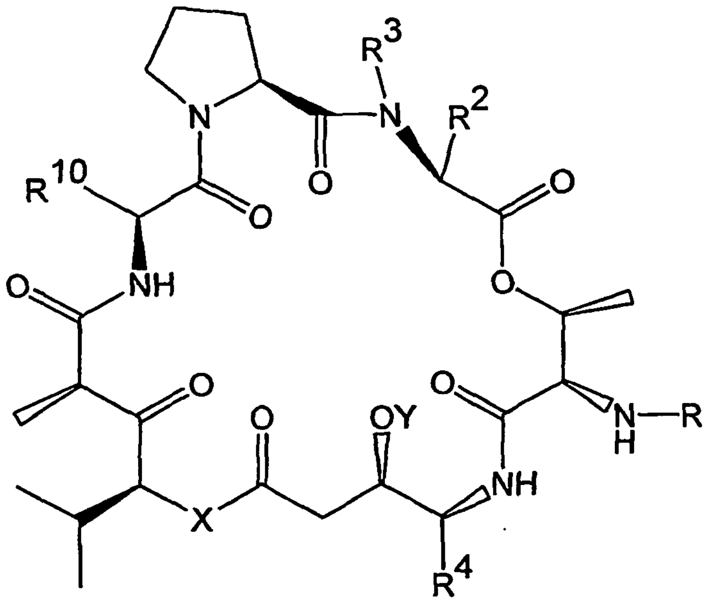

**(XXI).**

The $R^1$ substituent of formulas **I** and **XXI** can have a deoxo-proline moiety in its structure, and can, for example, be a hydrogen atom or an amine protecting group suitable for protection of amino acids. Such protecting groups are known in the art and referred to throughout this disclosure. Examples of suitable protecting groups can be found in references such as Green and Wutz (1999, Protecting Groups in Organic Synthesis, Wiley, New York) and Bodansky (1993, Principles of Peptide Synthesis, Springer, Berlin). Alternatively, the $R^1$ substituent can be an amino acid residue (e.g., a leucine residue) or a polypeptide comprising one or more amino acid residues. Examples of such residues and polypeptides include

- (N-methyl)leucine,
- (N-methyl)leucine-(a first fluorophore),
- (N-methyl)leucine-proline,

- (N-methyl)leucine-proline-lactate,
- (N-methyl)leucine-proline-pyruvate,
- (N-methyl)leucine-proline-lactate-glutamine-pyroglutamate,
- (N-methyl)leucine-proline-lactate-glutamine-cyclopentanoate,
- (N-methyl)leucine-proline-lactate-leucine-pyroglutamate,
- (N-methyl)leucine-proline-alanine-leucine-pyroglutamate,
- (N-methyl)leucine-proline-(N-methyl)alanine-leucine-pyroglutamate,
- (N-methyl)leucine-deoxo-proline,
- (N-methyl)leucine-deoxo-proline-lactate,
- (N-methyl)leucine-deoxo-proline-pyruvate,
- (N-methyl)leucine-deoxo-proline-lactate-glutamine-pyroglutamate,
- (N-methyl)leucine-deoxo-proline-lactate-glutamine-cyclopentanoate,
- (N-methyl)leucine-deoxo-proline-lactate-leucine-pyroglutamate,
- (N-methyl)leucine-deoxo-proline-alanine-leucine-pyroglutamate,
- (N-methyl)leucine-deoxo-proline-(N-methyl)alanine-leucine-pyroglutamate,
- (N-methyl)leucine-dehydro-proline,
- (N-methyl)leucine-dehydro-proline-lactate,
- (N-methyl)leucine-dehydro-proline-pyruvate,
- (N-methyl)leucine-dehydro-proline-lactate-(a first fluorophore),
- (N-methyl)leucine-dehydro-proline-lactate-glutamine-pyroglutamate,
- (N-methyl)leucine-dehydro-proline-lactate-glutamine-cyclopentanoate,
- (N-methyl)leucine-dehydro-proline-alanine-leucine-pyroglutamate, and
- (N-methyl)leucine-dehydro-proline-(N-methyl-alanine)-leucine-pyroglutamate.

Additional examples of alternative $R^1$ substituents include peptides which comprise a fluorophore (e.g., rhodamine or coumarin), an amino acid residue, a polypeptide, an enzymatically-cleavable group, or another chemical moiety bound (e.g., covalently attached) with a support (e.g., a glass or silica plate, an agarose or other polymeric bead, etc.). The fluorophore or enzymatically-cleavable group can be directly linked with the analog, or it can be bound thereto with a linker having 1 to about 13 or more carbon atoms (optionally including one or more secondary amine or amide moieties) therein. When $R^1$ comprises an N-methyl-leucine residue, the alpha-carbon atom of that residue can have either (R) or (S) stereochemistry. Other amino acid residues within $R^1$ can have either (R) or (S) stereochemistry, but they preferably have (S) stereochemistry at their alpha-carbon atom.
When $R^1$ comprises a lactate residue, the lactate residue is preferably an (S)lactate residue. In a preferable embodiment, every amino acid residue within $R^1$ other than the leucine (or N-methyl-leucine) residue (if present) attached directly to the nitrogen atom of the ring of formula **I** or **XXI** has (S) stereochemistry.
$R^3$ can be either of $-CH_3$ and -H. Alternatively, $R^3$ can, together with $R^2$, be a single substituent.
The $R^2$ substituent can be an amino acid side chain such as an isoleucine side chain (i.e., a 2-butyl moiety, preferably having (R) stereochemistry), a valine side chain (i.e., a 2-propyl moiety), an alanine side chain (i.e., a methyl moiety), a norleucine side chain (i.e., a 1-butyl moiety), a norvaline side chain (i.e., a 1-propyl moiety), a leucine side chain (i.e., an isobutyl moiety, preferably having (S) stereochemistry), a phenylalanine side chain (i.e., a phenylmethyl moiety), a histidine side chain (i.e., a 4-methyl-imidazole moiety), a tryptophan side chain (i.e., a 3-methyl-indole moiety), a tyrosine side chain (i.e., a 4-hydroxyphenylmethyl moiety), an arginine side chain (i.e., a 4-guanidinyl-butyl moiety), and a lysine side chain (i.e., a 4-aminobutyl moiety).
An $R^2$ substituent can comprise a fluorophore (e.g., a fluorophore linked with one of the amino acid side chains described above). In addition, $R^2$ substituent can have the structure of formula **III**

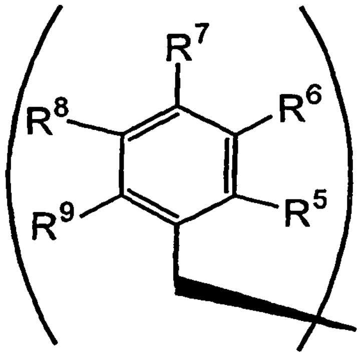

(III).

In an alternative embodiment, $R^2$ and $R^3$ together are a substituent having the structure of formula **IV**

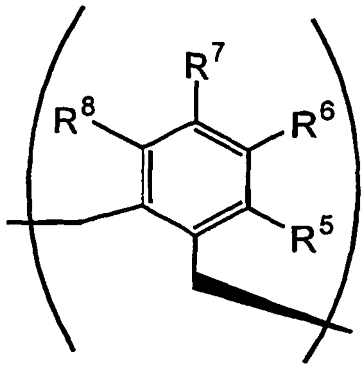

(IV).

In formulas **III** and **IV,** each of $R^5$, $R^6$, $R^7$, $R^8$, and $R^9$, independently, can be a substituent selected from the group consisting of H, -OH, $-OCH_3$,
$-CO(C_6H_5)$, -Br, -I, -F, -Cl, $-CH_3$, and $-CH_2CH_3$.
$R^4$ can be an isoleucine side chain or a valine side chain.
X can be -O- or -(NH)-.
Y can be -H or a hydroxyl protecting group. Examples of hydroxyl protecting groups which can be present at Y include an alkyl-substituted silyl moiety, an aryl- substituted silyl moiety, or a silane substituted with both alkyl- and aryl- moieties. An example of a useful hydroxyl protecting group is a triisopropylsilyl moiety. Other hydroxyl protecting groups which can be used at Y in formula I are described in references such as Green and Wutz (1999, Protecting Groups in Organic Synthesis, Wiley, New York).
$R^{10}$ can be an amino acid side chain such as a leucine side chain or a lysine side chain. Alternatively, $R^{10}$ can be an amino acid or other chemical moiety which is bound with (e.g., covalently attached to) a support (e.g., a solid support). An example of a support with an analog having the structure of formula I bound therewith is depicted in Figure 5B.
Another group of compositions included within the invention are those which comprise a tamandarin analog having a structure selected from the group consisting of formulas (a)-(d), set forth above.
Each of $R^2$, $R^3$, $R^4$, $R^{10}$, X, and Y has the same meaning in formulas (a)-(d) that it has in formulas **I** and **XXI.**
In formulas (a)-(d), $R^{13}$ can be hydrogen or a chemical moiety which can be enzymatically cleavable (i.e., an enzyme-cleavable moiety) or photoreactive. As used herein, an enzyme-cleavable moiety can include any chemical moiety which can be cleaved (i.e., chemically detached from) in the presence of a specific enzyme. Examples of enzymes capable of chemically detaching an enzyme-cleavable moiety include carboxypeptidases, beta-lactamase, beta-galactosidase, penicillin V-amidase, cytosine deaminase, nitroreductase, alkaline phosphatase, beta-glucuronidase, and catalytic antibodies. Examples of enzyme-cleavable moieties which can be incorporated in a compound described herein include cephalosporins, beta-glucosides, phosphate, pyrophosphate, beta-D-galactosides, nitrobenzamidine, cytosine, carbamates, peptides, and amino acids. Alternatively, $R^{13}$ can be an enzyme-cleavable moiety such as a di-peptide linked with glutamine-pyroglutamate, or a moiety having the structure of formula **V** or formula **VI**

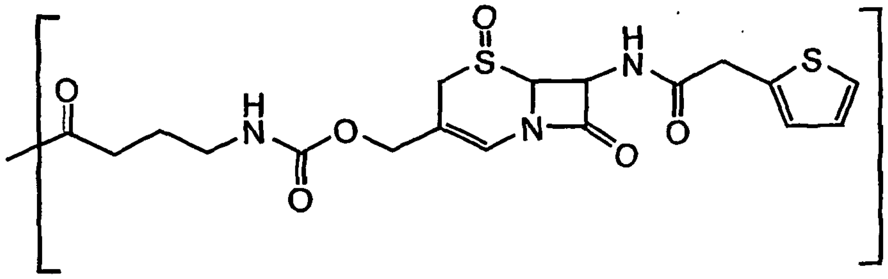

(V)

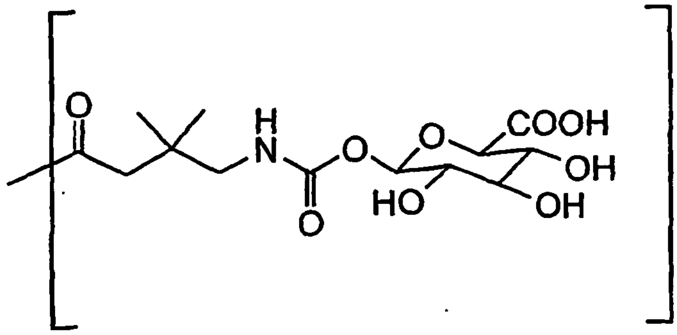

(VI).

By way of illustration, in compound **131**, depicted in Figure 23, $R^{13}$ is an enzyme-cleavable moiety having the structure of formula V (i.e., a cephalosporin moiety). The cephalosporin moiety of compound **131** can be cleaved by contact with the enzyme, beta-lactamase, to generate compound **101**. An $R^{13}$ substituent having the structure of formula **VI** can, for example, be in the form of a sodium or potassium salt.

After cleavage of an enzyme-cleavable moiety by an enzyme, the resulting didemnin analog can exhibit one or more of the physiological activities described herein. A tamandarin or didemnin analog having the structure of one of formulas (a)-(d), wherein $R^{13}$ is an enzyme-cleavable moiety, can, optionally, exhibit these activities before the cleavage of the enzyme-cleavable moiety. However, in a preferred embodiment, the analog exhibits therapeutic activity only following cleavage of the enzyme-cleavable moiety therefrom.

As described above, a tamandarin or didemnin analog having the structure of one of formulas **I**, **XXI**, and (a)-(d) can be bound with a support. The identity of the support is not critical. The support can be substantially any material with which such an analog can be bound (e.g., by covalent attachment through one of the $R^{10}$, $R^2$, $R^3$, or $R^1$ moieties). Examples of support materials include bonded silicates, cross-linked agarose, polyacrylamide, dextran, and allyl dextran. Such support materials can be chemically modified using reactive chemical moieties in order to facilitate covalent attachment of the analog with the support. Chemical modifications of this type are known in the art, and can, for example, include modification of a support with cyanogen bromide groups, epoxide groups, mesyl groups, and carboxyhexyl groups. Protocols for preparation of a support and subsequent attachment of a compound to the support are available in the art, and can be modified by one skilled in the art for use with a didemnin analog described herein.

Examples of didemnin analogs having the structure of formula **I** or formula **XXI,** include, compound **21,** and compounds **101-143,** some of which are depicted in one or more of Figures 1-39.

In compound **21**, $R^1$ is -(tert-butyloxycarbonyl), $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is an isoleucine side chain, $R^{10}$ is a leucine side chain, X is -O-, and Y is -(triisopropylsilyl).

Preferred tamandarin analogs are based on the structure of tamandarin A. In tamandarin A ({(2S)Hiv[2]}didemnin B; compound **101**), $R^1$ is-(N-methyl-R-leucine)-proline-lactate, $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is an isoleucine side chain, $R^{10}$ is a leucine side chain, X is -O-, and Y is -H.

In tamandarin M ({(2S)Hiv[2]}didemnin M; compound **103**), $R^1$ is -(N-methyl-R-leucine)-proline-lactate-glutamine-pyroglutamate, $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is an isoleucine side chain, $R^{10}$ is a leucine side chain, X is -O-, and Y is -H.

Preferred didemnin analogs are based on the structure of didemnin B. In tamandarin B ({(2S)Hiv[2], Norsta[1]} didemnin B; compound **105**), $R^1$ is -(N-methyl-R-leucine)-proline-lactate, $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is a valine side chain, $R^{10}$ is a leucine side chain, X is -O-, and Y is -H.

In compound **107,** $R^1$ is -(N-methyl-R-leucine)-glycine-(7-dimethylcoumarin-4-acetate), $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is an isoleucine side chain, $R^{10}$ is a leucine side chain, X is -O-, and Y is -H.

In compound **109,** $R^1$ is -(N-methyl-R-leucine)-proline-lactate-rhodamine, $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is an isoleucine side chain, $R^{10}$ is a leucine side chain, X is -O-, and Y is H.

In compound **111,** $R^1$ is -(N-methyl-R-leucine)-proline-lactate, $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is an isoleucine side chain, $R^{10}$ is a lysine side chain, X is -O-, and Y is -H.

In compound **113**, $R^1$ is -(N-methyl-R-leucine), $R^2$ is an O-methyl-tyrosine side chain (i.e., $R^5$, $R^6$, $R^8$, and $R^9$ are each -H, and $R^7$ is $-OCH_3$), $R^3$ is $-CH_3$, $R^4$ is an isoleucine side chain, $R^{10}$ is a leucine side chain, X is -O-, and Y is -H.

In compound **115**, $R^1$ is -(N-methyl-R-leucine)-proline-lactate, $R^2$ is a lysine side chain, $R^3$ is $-CH_3$, $R^4$ is an isoleucine side chain, $R^{10}$ is a leucine side chain, X is -O-, and Y is -H.

In compound **123**, $R^1$ is -(N-methyl-R-leucine)-proline-lactate, $R^2$ and $R^3$ together are a tetrahydroisoquinoline substituent having the structure of formula IV, $R^5$, $R^6$, and $R^8$ are each -H, $R^7$ is $-OCH_3$, $R^4$ is a valine side chain, $R^{10}$ is a leucine side chain, X is -O-, and Y is -H.

In compound **124**, $R^1$ is -(N-methyl-R-leucine)-proline-lactate, $R^2$ is an O-methyl-tyrosine side chain (i.e., $R^5$, $R^6$, $R^8$, and $R^9$ are each -H and $R^7$ is $-OCH_3$), $R^3$ is $-CH_3$, $R^4$ is an isoleucine side chain, $R^{10}$ is a leucine side chain, X is -(NH)-, and Y is -H.

In compound **128**, $R^1$ is -(N-methyl-R-leucine)-proline-lactate-glutamine-cyclopentanoate, $R^2$ is an O-methyl-tyrosine side chain (i.e., $R^5$, $R^6$, $R^8$, and $R^9$ are each -H and $R^7$ is $-OCH_3$), $R^3$ is $-CH_3$, $R^4$ is an isoleucine side chain, $R^{10}$ is a leucine side chain, X is -O-, and Y is -H.

In compound **129,** $R^1$ is -(N-methyl-R-leucine)-proline-(N-methyl-S-alanine)-leucine-pyroglutamate, $R^2$ is an O-methyl-tyrosine side chain (i.e., $R^5$, $R^6$, $R^8$, and $R^9$ are each -H and $R^7$ is $-OCH_3$), $R^3$ is $-CH_3$, $R^4$ is an isoleucine side chain, $R^{10}$ is a leucine side chain, X is -O-, and Y is -H.

In compound **131**, $R^1$ is-(N-methyl-R-leucine)-proline-lactate, $R^2$ is an O-methyl-tyrosine side chain (i.e., $R^5$, $R^6$, $R^8$ and $R^9$ are each -H and $R^7$ is $-OCH_3$), $R^3$ is $-CH_3$, $R^4$ is an isoleucine side chain, $R^{10}$ is a leucine side chain, $R^{13}$ is a cephalosporin moiety cleavable by the enzyme, beta-lactamase, X is -O- and Y is -H

In compuond **132**, $R^1$ is -(N-methyl-R-leucine-(S)proline-(S)lactate, $R^2$ is an O-methyl-tyrosine side chain (i.e., $R^5$, $R^6$, $R^8$, and $R^9$ are each -H and $R^7$ is $-OCH_3$), $R^3$ is $-CH_3$, $R^4$ is an isoleucine side chain, $R^{10}$ is a leucine side chain, $R^{13}$ is a beta-glucoside moiety cleavable by the enzyme, beta-glucuronidase, X is -O-, and Y is -H.

In compound **134**, $R^1$ is -(N-methyl-S-leucine)-(S)proline-pyravate, $R^2$ is an O-methyl-tyrosine side chain (i.e., $R^5$ $R^6$, $R^8$, and $R^9$ are each -H and $R^7$ is $-OCH_3$), $R^3$ is $-CH_3$, $R^4$ is an isoleucine side chain, $R^{10}$ is a leucine side chain, X is -O-, and Y is -H.

In compound **137**, $R^1$ is -(N-methyl-R-leucine)-(S)proline-pyruvate, $R^2$ and $R^3$ together are a tetrahydroisoquinoline substituent having the structure of formula IV, $R^5$, $R^6$, and $R^8$ are each -H, $R^4$ is an isoleucine side chain, $R^{10}$ is a leucine side chain, X is -O-, and Y is -H.

In compound **138**, $R^1$ is -(N-methyl-R-leucine)-(S)proline-pyroline-pyruvate, $R^2$ is an O-methyl-tyrosine side chain (i.e., $R^5$; $R^6$; $R^8$ and $R^9$ are each -H and $R^7$ is $-OCH_3$), $R^3$ is $-CH_3$, $R^4$ is an isoleucine side chain, $R^{10}$ is a lysine side chain, covalently attached to a support, X is -O-, and Y is -H.

In compound **142**, $R^1$ is -(N-methyl-S-leucine)-(S)proline-pyruvate, $R^2$ is an O-methyl-typrosine side chain (i.e., $R^5$, $R^6$, $R^8$, and $R^9$ are each -H and $R^7$ is $-OCH_3$), $R^3$ is$-CH_3$, $R^4$ is a valine side chain, $R^{10}$ is a leucine side chain, X is -O-, and Y is -H.

In compound **143**, $R^1$ is (N-methyl-R-leucine)-(S)proline-pyruvate, $R^2$ is an O-methyl-tyrosine side chain (i.e., $R^5$, $R^6$ $R^8$, and $R^9$ are each-H and $R^7$ is $-OCH_3$), $R^3$ is $-CH_3$, $R^4$ is an isoleucine side chain, $R^{10}$ is a leucine side chain, X is -NH-, and Y is -H.

The structural similarity of didemnin B and tamandarin A [i.e., {(2S)Hiv$^2$}didemnin B; compound **101**), is illustrated in Figure 25. The primary structural diaetence, indicated by brackets and dotted lines, is in the macrocycle portion of these. compounds. Tamandarin A, shown in Figure 25A, contains an alpha-hydroxyisovaleryl (Hiv) moiety, and didemnin B, shown in Figure 25B, contains an alpha-(alpha-hydroxyisovaleryl)-propionyl (Hip) moiety. The simpler macrocycle structure of tamandarin A, and of any compound having the structure of formula **I** or formula **XXI**, can be synthesized more readily than the macrocyclic structure of didemnin B. Compounds which have the structure of either of formula **I** and formula **XXI** can be more easily (and generally more inexpensively) prepared than compounds which are identical but for the presence of an Hip moiety in place of the Hiv moiety.

Another group of compounds that can exhibit the physiological activities described herein and which are included in the invention are compounds which correspond to fragments of didemnin analogs having the structure of formula **I** or formula **XXI.** Fragments which exhibit this activity include those which have the structure of formula **VII**

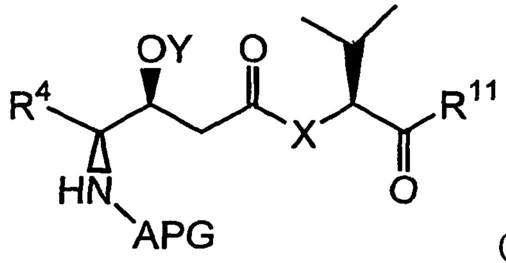

(VII)

In formula **VII**, X, Y, and $R^4$ have the identities described for formulas **I** and **XXI**, and APG is an amine protecting group. Examples of amine protecting groups which can be present in the active fragments include carbobenzyloxy (CBZ) and a tert-butyloxycarbonyl (BOC) moieties. Other useful amine protecting groups are described in references such as Green and Wutz (1999, Protecting Groups in Organic Synthesis, Wiley, New York) and Bodansky (1993, Principles of Peptide Synthesis, Springer, Berlin).

$R^{11}$ in formula **VII** can be any of-OH, $NH_2$, -(O-allyl), and -(O-pentafluorophenyl). Alternatively $R^{11}$ can be a substituent having the structure shown in formula **VIII**

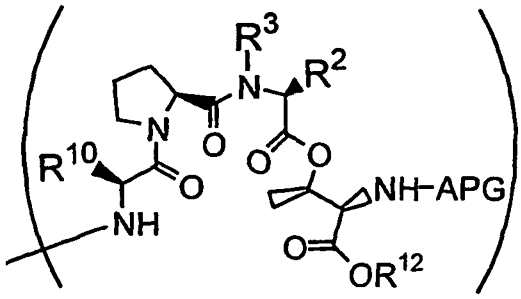

(VIII).

In formula **VIII,** $R^2$, $R^3$, and $R^{10}$ have the identities described for formulas **I** and **XXI,** and APG is an amine protecting group as described for formula **VII** (although it need not be the same APG as in formula **VII**). $R^{12}$ can be either -H or a hydroxyl protecting group, as described herein. Compounds having the structure of formula **VII** that can be made and used as described herein, and include the compounds designated 6, 17, 19, and 20 in Figure 26.

Methods of Using Compounds Described Herein

The didemnin and tamandarin analogs disclosed herein can be used to affect a variety of physiological processes. Each of these types of compounds can be used to inhibit protein synthesis. Furthermore, the compounds can be used to inhibit progression of a cell through the cell cycle. While not being bound by any particular theory of operation, it is believed that the cell cycle-inhibiting activity of the compounds can be attributed to inhibition of protein synthesis, to inhibition of other cellular activities associated with DNA replication or cell division, or to some combination of these activities. These tamandarin and didemnin analogs also induce apoptosis in cells. The physiological activities attributable to these tamandarin and didemnin analogs make these compounds useful for alleviating a variety of disorders in which one or more of cell growth, proliferation, and survival are aberrant. Examples of such disorders include cancers at various stages (e.g., tumorigenesis, tumor growth, and metastasis) and viral infections at various stages (e.g., infection of cells with virus particles, production of virus particles within a cell, and survival of virus-infected cells).

While still not being bound by any particular theory of operation, it is believed that the physiological activities attributable to the tamandarin and didemnin analogs described herein result from one or more interactions between such analogs and at least one cellular component. This interaction(s) leads, directly or indirectly, to the observed cellular response. Accordingly, the invention encompasses use of these compounds to identify one or more cellular components which contributes to a disorder phenotype in an individual. Identification of such a cellular component can indicate an effective course of treatment for alleviating the disorder. Examples of compounds useful for this purpose include analogs which comprise a fluorescent substituent (e.g., at $R^1$ or $R^2$), a photoreactive chemical moiety, such as a moiety having the structure

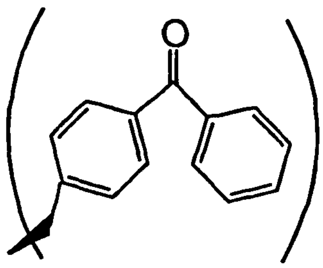

,

or a moiety bound with a support.

Fluorescent and other detectably labeled tamandarin and didemnin analogs described herein (as well as their physiologically active fragments) can be used to identify cells in which those analogs and fragments can exert their physiological effects. For example, cells which absorb or bind with a fluorescent analog can be identified or isolated. Identification or isolation of such cells can be used to diagnose a disorder associated with the presence of such cells. Identification or isolation of these cells can also indicate which of the tamandarin or didemnin analogs are efficacious for treating a disorder involving the cells.

The tamandarin and didemnin analogs described herein can be used for anti-proliferative, anti-tumor, anti-viral, and immunosuppressive purposes. For example, these compounds can be used in a pharmaceutical preparation or medicament to be administered to a patient afflicted with a disorder in which one or more of protein synthesis, cell growth, proliferation, and survival are aberrant. Such medicaments can be used to treat disorders such as cancers (e.g., breast cancer), viral, fungal, parasitic, and bacterial infections, auto-immune disorders, allergies, other hyper-immune disorders, and atherosclerosis.

Examples of anti-tumor activities that can be exhibited by the compounds described herein include inhibition of tumorigenesis, inhibition of metastasis, inhibition of tumor cell growth, inhibition of tumor cell proliferation, and enhancement of tumor cell apoptosis. Dehydrodidemnin exhibits activity against cell lines derived from several human solid tumor types, including non-small cell lung cancer and colon tumor cell lines, and exhibits selective anti-tumor activity against non-small cell lung cancer, melanomas, ovarian cancer, and colorectal cancer (Depenbrock et al., 1998, Brit. J. of Cancer 78(6): 739-744). The tamandarin and didemnin analogs described herein exhibit anti-tumor activities in cells of one or more of these lines, as well as in cells of the corresponding tumor type in vivo. Determination of the effectiveness of any particular analog against any particular tumor type can be made using standard methods involving, for example, one or more of the 60 standard tumor cell lines maintained in the U.S. National Cancer Institute drug screening program.

Examples of anti-viral activities that can be exhibited by the tamandarin and didemnin analogs described herein include inhibition of binding of a virus with a cellular target, inhibition of infection of a cell by a virus, inhibition of cellular synthesis of virus components, inhibition of intracellular assembly of virus particles, inhibition of release of virus particles from an infected cell, inhibition of growth of a cell infected by a virus, inhibition of proliferation of a cell infected by a virus, and induction of death (i.e., apoptosis) of a cell infected by a virus. The anti-viral activity of the compounds described herein can, for example, be used to treat or prevent viral infections of mammals and associated symptoms. By way of illustration, the didemnin and tamandarin analogs can be used to treat or prevent infections by viruses such as Rift Valley Fever virus, Dengue virus, or any of the equine encephalitis viruses.

Examples of immunosuppressive activities that can be exhibited by the tamandarin and didemnin analogs described herein include inhibition of a cellular immune response to an immunogen (e.g., an infectious agent, or a transplanted cell or tissue) and inhibition of a humoral immune response to an immunogen. Examples of disorders in which immunosupression can be desirable include autoimmune disorders, transplant rejection disorders (e.g., rejection of a solid tissue or bone marrow transplant), development of an immune response to an implanted device (e.g., a stent or a heart valve), immune hypersensitivity, and anaphylaxis.

The tamandarin and didemnin analogs described herein can be administered in vitro to a cell or tissue (e.g., a cultured cell or tissue, or a cell or tissue harvested from one animal prior to introduction into the same or a different animal). Alternatively, the analogs can be administered to the cell or tissue in vivo by administering the analog or a pharmaceutical composition comprising the analog to an animal (e.g., a mammal such as a human) that comprises the cell or tissue.

In one embodiment of the treatment methods described herein, a tamandarin or didemnin analog described herein and having an enzyme-cleavable group attached thereto (e.g., a compound having the structure of formula **XXI**) is administered to an animal. Upon cleavage of the enzyme-cleavable group, the compound is transformed from an inactive (or less active) form to an active (or more active) form, as shown in Figures 23 and 24. Thus, a tamandarin or

didemnin analog can be selectively activated at a body location at which the enzyme activity occurs.

The enzyme which is used to cleave a tamandarin or didemnin analog having an enzyme-cleavable moiety attached can be an enzyme which naturally occurs at a body location in an animal. Alternatively, the enzyme can be provided to the animal, for example as a composition comprising the enzyme or a nucleic acid which encodes the enzyme. As another example, the enzyme can be coupled (e.g., covalently, using a cross-linking agent or by expression as an enzyme-antibody fusion protein) with an antibody that specifically binds with a tissue (e.g., cancerous cells such as leukemic cells or cells of a solid tumor) at a body location in the animal, and the antibody-enzyme complex can be administered to an animal. Administration of a tamandarin or didemnin analog having an attached enzyme-cleavable group to the same animal results in preferential activation of the compound at the tissue or body location. The physiological effect of the compound can thereby be localized at the tissue or body location, and any side effect attributable to the activated compound can thereby be reduced or minimized.

A support-bound tamandarin or didemnin analog can be used to identify cells which comprise, on their surfaces or elsewhere, receptor proteins, glycoproteins, and the like, which are capable of interacting or binding with the analog. As an example, a tamandarin or didemnin analog having the structure of formula **I** or **XXI** and attached to a support can, by virtue of its interaction with a particular cellular receptor, be used to identify or physically isolate cells of a particular type (e.g., tumor cells) which are characterized by the presence of the particular receptor.

Methods of Making Compounds Described Herein

The present invention include methods of making didemnin analogs and fragments described herein. Methods of making tamandarin and didemnin analogs have been described (e.g., Harris et al., 1987, Tetrahedron Lett. 28: 2837-2840; Harris et al., 1988, Tetrahedron 44:3489-3500; Ewing et al., 1986, Tetrahedron 42:5863-5868; Ewing, W.R., 1988, Ph.D. Dissertation, University of Pennsylvania, Philadelphia PA; Ewing et al., 1989, Tetrahedron Lett. 30: 3757-3760; Li et al., 1990, J. Am. Chem. Soc. 112:7659-7672; Mayer et al., 1994, J. Org. Chem. 59:5192-5205; Mayer et al., 1994, Tetrahedron: Asymmetry 5:519-522; Xiao et al., 1997, Tetrahedron: Asymmetry 9:47-53; Pfizenmayer et al., 1998, 8:3653-3656; U.S. Patent Application serial number 09/545,848, filed April 7, 2000). The contents of each of these references and patent application are incorporated herein by reference. The precise method used to make a tamandarin or didemnin macrocycle or analog is not critical.

Preferably, the method that is used results in the stereoselective synthesis of a compound described herein. For example, synthesis of (-) tamandarin A ({(2S)Hiv$^2$}didemnin B; compound **101**) is exemplified herein at Example 1.

In reference to methods of making the analogs and fragments described herein, the substituents $R^2$, $R^3$, $R^4$, $R^5$, $R^6$, $R^7$, $R^8$, $R^9$, $R^{10}$, X, and Y, have the same meanings as used above.

As used in the present disclosure, a protection reaction can include any reaction whereby one or more chemical moieties are covalently (but reversibly) attached to one of a nitrogen atom, an oxygen atom, and a sulfur atom of a molecule. Such attachment prevents the atom or atoms from participating in non-desired chemical reactions, i.e., becoming covalently attached to other chemical moieties, and donating or accepting either of protons and electrons to other chemical moieties. A chemical moiety thus attached is referred to as "a protecting group." By way of example, the nitrogen atom of a compound having the structure of formula **IX**, such as D-*allo*-isoleucine, can be protected using a reagent such as carbobenzyloxy-succinimide(CBZ-succinimide). Use of this reagent in a standard protocol yields a protected D-*allo*-isoleucine, i.e., $N^{(alpha)}$-CBZ-D-*allo*-isoleucine, having the structure of compound **8** in Figure 26A. In compound 8, the CBZ moiety acts as an amine protecting group, and the nitrogen atom to which it is attached cannot readily undergo additional chemical reactions. Further by way of example, when X is -(NH)-, a protected amine group (e.g., -N(CBZ)-) can be used in the reactions described herein. As an alternative example, the hydroxyl moiety of compound **11** in Figure 26A, can be protected using a reagent such as triisopropylsilyltriflate (TIPSOTf) to yield compound **12** in Figure 26A. In this compound, Y is a triisopropylsilyl (TIPS) moiety and acts as a hydroxyl protecting group, preventing chemical reactions with the oxygen atom to which this moiety is attached.

Protocols for performing protection reactions and comprehensive information about chemical moieties can be used as protecting groups is found in references such as Green and Wutz (1999, Protecting Groups in Organic Synthesis, Wiley, New York) or Bodansky (1993, Principles of Peptide Synthesis, Springer, Berlin).

Didemnin analogs and fragments can be made by converting a compound having the structure of formula **IX**

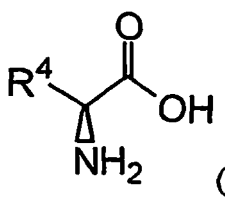

(IX)

to a compound having the structure of formula **X**

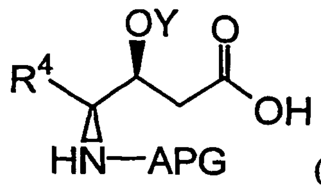

**(X).**

Such a series of reactions can include, but is not limited to, a protection reaction, an activation reaction, an esterification reaction, and an ester hydrolysis reaction. The amine group of formula **IX** is preferably protected prior to performing the esterification and hydrolysis reactions. A specific example of making a compound having the structure of formula X is given in Example 1. In formulas **X-XVIII,** "APG" refers to an amine protecting group such as carbobenzyloxy (CBZ) moiety or tert-butyloxycarbonyl (BOC) moiety. Alternative amine protecting groups can also be used, as described herein and in the art.

An example of an activation reaction included in the method of making a compound having the structure of formula **X** is depicted in Figure 26A, reaction B. Activation of a compound such as compound **8** can involve a reagent such as pentafluorophenol (PFPOH) to yield compound **9**. Compound **9** is an example of an activated intermediate that more readily undergoes subsequent reactions, such as esterification, at the carbonyl carbon of its PFP ester moiety. Esterification reactions which do not require an activated intermediate can also be employed to make a compound having the structure of formula **X**.

Any method of ester hydrolysis known in the art that does not comprise harsh conditions which favor racemization and can be used to make a compound having the structure of formula **X**. By way of example, a compound having the structure

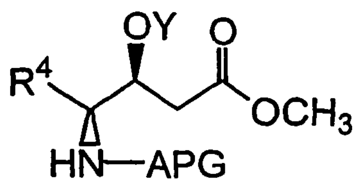

can be hydrolyzed using a strong base in a solvent mixture, as exemplified in Figure 26A, reaction F. Reagents and conditions suitable for ester hydrolysis under milder conditions (i.e., including conditions which do not favor racemization) can be readily selected by one skilled in the art.

A compound having the structure

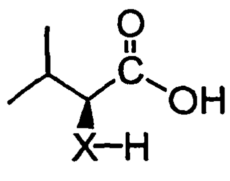

can be esterified with, for example, allyl bromide (e.g., as described in Example 1), to yield a compound having the structure of formula **XI**

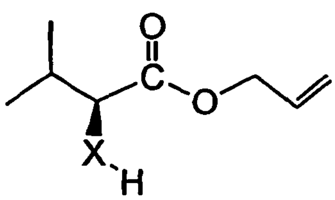

**(XI).**

A compound having the structure of formula **IX** and a compound having the structure of formula **XI** can be coupled (e.g., esterified) to yield a compound having the structure of formula **XII**

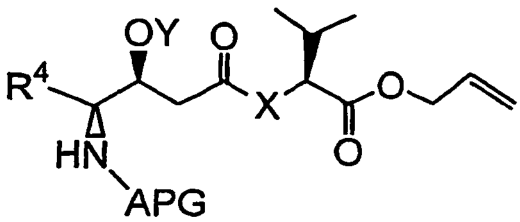

(XII).

(e.g., reaction I of Figure 26B). Optionally, such a reaction can be performed using a catalyst, a coupling reagent, or an esterification reagent. Reagents and conditions useful for this type of reaction are known in the art and exemplified in Example 1. Didemnin fragments having the structure of formula **XII** exhibit one or more of the pharmacological activities described herein.

A compound having the structure of formula **XII** can be hydrolyzed to yield a compound having the structure of formula **XIII**

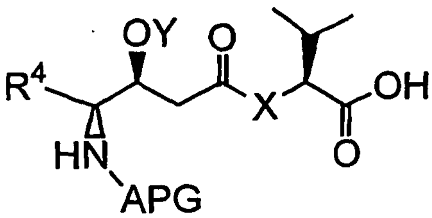

(XIII).

As described elsewhere herein, reaction conditions and reagents suitable for ester hydrolysis are known in the art, and can be readily applied by a skilled artisan. An example of this type of hydrolysis is depicted in Figure 26B, reaction J. Didemnin fragments having the structure of formula **XIII** exhibit one or more of the pharmacological activities described herein.

The carboxyl group of a compound having the structure of formula **XIII** can be activated to yield a compound having the structure of formula **XIV**

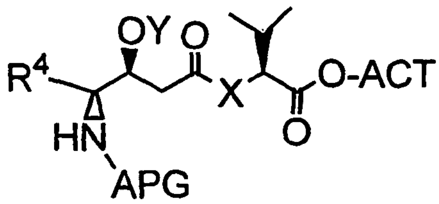

(XIV).

In formula **XIV**, "ACT" refers to an activating group, such as a pentafluorophenyl (PFP) moiety. Another example of an activating group is an N-hydroxysuccinimide moiety. Chemical activation can be performed using reagents such as an activating reagent, a catalyst, an activating group donor, or the like. By way of example, compound **6**, depicted in Figure 26C, is activated by covalent attachment of a PFP group to yield compound **19**. Protocols for activating a compound in the manner disclosed herein are known in the art. Didemnin fragments having the structure of formula **XIV** exhibit one or more of the pharmacological activities described herein.

The activated compound having the structure of formula **XIV** can be coupled with a third reactant having the structure of formula **XV**

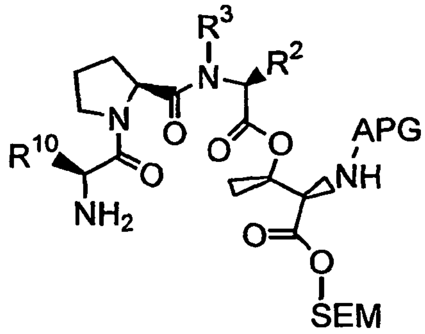

(XV)

to yield a compound having the structure of formula **XVI**

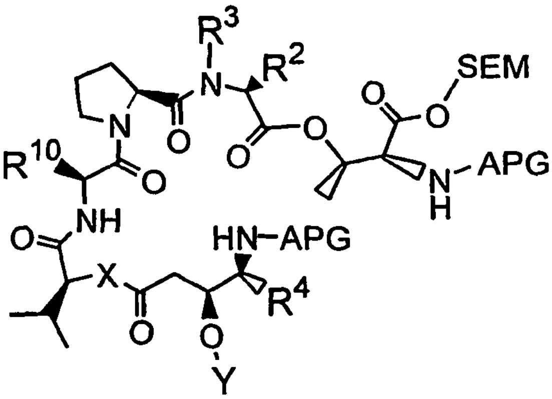

(XVI).

In formulas **XV** and **XVI**, SEM refers to 2-(trimethylsilyl)ethoxycarbonyl. An example of this reaction is depicted in reaction M of Figure 26C, in which compound **19** is coupled with compound **18** to yield compound **20**. The reagents and conditions necessary for preparation of a protected peptide such as compound **18** are described, for example, in Li *et al.* (1990, J. Am. Chem. Soc. 112: 7659-7672). Didemnin analogs having the structure of formula **XVI** exhibit one or more of the pharmacological activities described herein.

A didemnin analog having one or more of the pharmacological activities described herein can be made by removing one of the amino protecting groups and the carbonyl hydroxyl protecting group of a compound having the structure of formula **XVI** and cyclizing the compound to yield a PSI having the structure of formula **XVII**

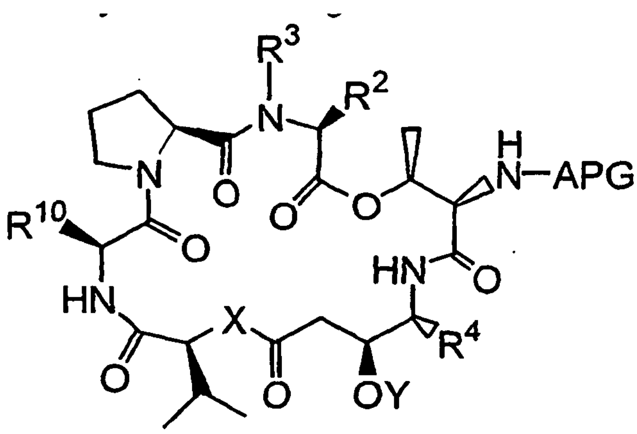

(XVII).

An example of reactions of this type is shown in step N of Figure 26D. Chemically de-protecting a compound such as one having the structure of formula **XVI** can be accomplished by reacting the compound with one or more reagents to remove a protecting group of the compound. Exemplary de-protection reactions are disclosed herein, for

example for compound **20**, as shown in Figure 26D. Other protocols for de-protecting a compound are known in the art, and can be readily applied by a skilled artisan to de-protection of a compound having the structure of formula **XVI.** Cyclization of a de-protected compound otherwise having the structure of the formula **XVI** can be accomplished using any method known in the art for macrocyclization of peptides. For example, the macrocyclization conditions can be similar or identical to those used in the cyclization that yields compound **21,** depicted in Figure 26D and described in Example 1. Didemnin analogs having the structure of formula **XVII** exhibit one or more of the therapeutic activities described herein.

One or more of the protecting groups of a compound having the structure of the formula **XVII** can be removed to yield a compound having the structure of formula **XVIII**

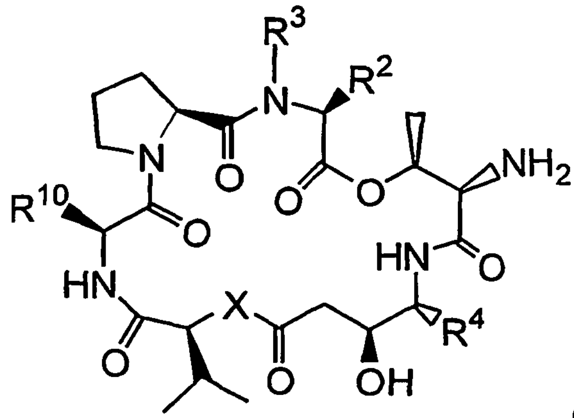

**(XVIII).**

This type of de-protection is exemplified in reaction O of Figure 26E. Didemnin analogs having the structure of formula **XVIII** exhibit one or more of the therapeutic activities described herein.

Yet another active compound can be made by coupling a compound having the structure of formula **XVIII** and a reagent having the structure of formula **XIX**

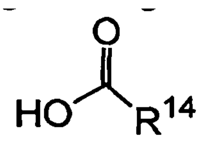

**(XIX)**

to yield a PSI having the structure of formula **XX**

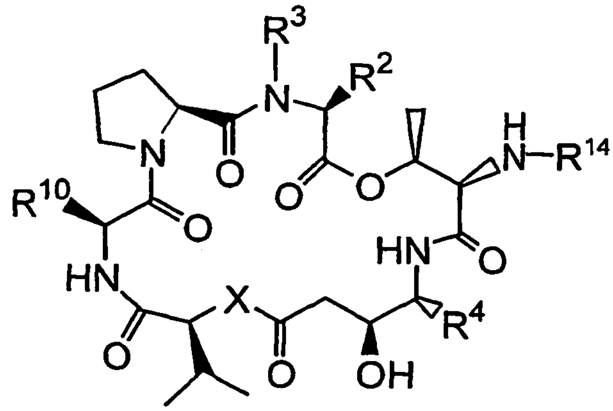

**(XX).**

This reaction is exemplified in reaction N of Figure 26E. $R^{14}$ can, for example, be any of the moieties described above as $R^1$. The $R^{14}$ substituent group can comprise an enzyme cleavable moiety, preferably at or near the distal end

thereof (relative to the macrocycle). Such a moiety can be cleavable by an enzyme, for example, a carboxypeptidase, a beta-lactamase, a beta-galactosidase, a penicillin V-amidase, a cytosine deaminase, a nitroreductase, an alkaline phosphatase, a beta-glucuronidase, and a catalytic antibody. An example of an $R^{14}$ moiety which comprises an enzyme-cleavable moiety is -(N-methyl)leucine-(S)proline-(S)lactate-(S)glutamine-(S)pyroglutamate. Other examples of enzyme-cleavable moieties are described herein. By way of illustration, compounds **131** and **132**, depicted in Figures 23 and 24, respectively, can be prepared using the methods described herein. Didemnin analogs and fragments which have an enzyme cleavable moiety attached thereto and which otherwise have the structure of one of formulas **XII-XVIII** upon cleavage of the enzyme-cleavable group therefrom exhibit one or more of the therapeutic activities described herein.

Variation of the substituents of the didemnin analogs and fragments can require slight modifications in the general methods described herein. It is understood that the invention includes such modifications, as they could be readily designed by one of ordinary skill in the art of synthetic chemistry.

Tamandarin and didemnin analogs can also be made by a method that includes incorporation of a deoxo-proline residue or a dehydro-proline residue in the side chain of a tamandarin or didemnin analog. The deoxo-proline residue or dehydro-proline residue can be used in place of a proline residue in any known tamandarin or didemnin analog. Particularly contemplated tamandarin and didemnin analogs are those in which the deoxo-proline residue or dehydro-proline residue is linked to the macrocycle by way of a leucine residue having a methylated amine moiety (i.e., an -(N-methyl)leucine-(deoxo or dehydro)-proline-containing tamandarin or didemnin analog). Of course, the (deoxo or dehydro)-proline residue can be further substituted, for example by lactate, by pyruvate, by lactate-fluorophore, by lactate-glutamine-pyroglutamate, by lactate-glutamine-cyclopentanoate, by -alanine-leucine-pyroglutamate, or by -(N-methyl-alanine)-leucine-pyroglutamate. One or more of the (deoxo or dehydro)-proline, lactate, glutamine, pyroglutamate, cyclopentanoate, and alanine residues is preferably the (S) enantiomer.

Incorporation of a deoxo-proline residue can be achieved by any method known in the art. Examples of methods of incorporating a deoxo-proline residue are included in this disclosure in Example 5 and in Figures 41-43.

In one embodiment, the method of incorporating a deoxo-proline residue comprises protecting the hydroxyl and amine moieties of leucine, methylating the leucine amine moiety, and de-protecting the leucine amine moiety. The amine group of proline is protected, and the ester function of the proline is reduced to an aldehyde (e.g., using a strong base such as $LiBH_4$ coupled with oxidation with an oxidizing agent such as pyridine-$SO_3$). Reductive amination (e.g., in the presence of a non-aqueous solvent, a strong base, and a carboxylic acid catalyst; e.g., in the presence of $Na(AcO)_3BH$, AcOH, and $CH_2Cl_2$) can be used to couple the hydroxyl-protected leucine with the amine protected proline (e.g., to form compound **43** in Figure 43, in one embodiment). The reductive amination can, for example, be performed as described by Abdel-Magid and co-workers (e.g., Abdel-Magid et al., 1990, Tetrahedron Lett. 31:5595-5598; Abdel-Magid et al., 1990, Synlett. 537-539).

The resulting leucine-deoxo-proline dipeptide can be further substituted with other moieties (e.g., with -lactate, -pyruvate, -lactate-fluorophore, -lactate-glutamine-pyroglutamate, -lactate-glutamine-cyclopentanoate, -alanine-leucine-pyroglutamate, or -(N-methyl-alanine)-leucine-pyroglutamate), with (preferably) or without first removing the protecting groups. The leucine-deoxo-proline dipeptide (optionally further substituted) can be attached to a tamandarin or didemnin macrocycle in the position identified as $R^1$ in formulas **I** and **XXI**.

Incorporation of a dehydro-proline residue can be achieved by any method known in the art. Examples of methods of incorporating a dehydro-proline residue are included in this disclosure in Example 6 and in Figure 44.

In one embodiment, the method of incorporating a dehydro-proline residue comprises protecting the carboxyl and amino groups of 4-hydroxyprolinate, mesylating the 4-hydroxyl moiety, displacing the mesylate moiety with an aryl-selenyl moiety, oxidatively eliminating the aryl-selenyl moiety, and de-protecting the carboxyl moiety. The resulting dehydro-proline moiety can be coupled with one or more additional amino acid residues or organic acids (e.g., those identified herein, removing the amino-protecting group if necessary or desired) and coupled with a tamandarin or didemnin macrocycle made or obtained by conventional means.

Pharmaceutical Compositions

The invention encompasses pharmaceutical compositions comprising at least one of the tamandarin and didemnin analogs and the physiologically active fragments described herein. Such compositions can comprise the analog/fragment and a pharmaceutically acceptable carrier. By way of example, a pharmaceutical composition can comprise a pharmaceutically acceptable carrier and a tamandarin or didemnin analog having the structure of any of formulas **I, XXI,** and (a)-(d) as an active agent. As a further example, a pharmaceutical composition can comprise a pharmaceutically-acceptable carrier and one or more of the compounds depicted in the figures in this disclosure.

Such pharmaceutical compositions can be used, for example, in the methods described herein for and for inhibiting one or more of protein synthesis, cell cycle progression, tumorigenesis, growth, and proliferation in a cell. In addition, such compositions can be used in the methods described herein for enhancing apoptosis in a cell.

Pharmaceutical compositions that are useful in the methods of the invention can be administered systemically

in oral solid formulations, ophthalmic, suppository, aerosol, topical or other similar formulations. In addition to the active agent, such pharmaceutical compositions can contain pharmaceutically-acceptable carriers and other ingredients known to enhance and facilitate drug administration. Other possible formulations, such as nanoparticles, liposomes, resealed erythrocytes, and immunologically based systems can also be used to administer the active agent according to the methods of the invention.

The invention encompasses pharmaceutical compositions which consist of the active agent, in a form suitable for administration to a subject, or the pharmaceutical composition can comprise the active agent and one or more pharmaceutically acceptable carriers, one or more additional ingredients, or some combination of these. The active agent can be present in the pharmaceutical composition in the form of a physiologically acceptable ester or salt, such as in combination with a physiologically acceptable cation or anion, as is well known in the art.

The formulations of the pharmaceutical compositions described herein can be prepared by any method known or hereafter developed in the art of pharmacology. In general, such preparatory methods include the step of bringing the active agent into association with a carrier or one or more other accessory ingredients, and then, if necessary or desirable, shaping or packaging the product into a desired single- or multi-dose unit.

Although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, and mammals including commercially relevant mammals such as cattle, pigs, horses, sheep, cats, and dogs.

Pharmaceutical compositions that are useful in the methods of the invention can be prepared, packaged, or sold in formulations suitable for oral, rectal, vaginal, parenteral, topical, pulmonary, intranasal, buccal, ophthalmic, or another route of administration. Other contemplated formulations include projected nanoparticles, liposomal preparations, resealed erythrocytes containing the active agent, and immunologically-based formulations.

A pharmaceutical composition of the invention can be prepared, packaged, or sold in bulk, as a single unit dose, or as a plurality of single unit doses. As used herein, a "unit dose" is discrete amount of the pharmaceutical composition comprising a predetermined amount of the active agent. The amount of the active agent is generally equal to the dosage of the active agent which would be administered to a subject or a convenient fraction of such a dosage such as, for example, one-half or one-third of such a dosage.

In addition to the active agent, a pharmaceutical composition of the invention can further comprise one or more additional pharmaceutically active agents such as, other tumor therapy agents, other anti-infective agents, and the like.

Controlled- or sustained-release formulations of a pharmaceutical composition of the invention can be made using conventional technology.

A formulation of a pharmaceutical composition of the invention suitable for oral administration can be prepared, packaged, or sold in the form of a discrete solid dose unit including, but not limited to, a tablet, a hard or soft capsule, a cachet, a troche, or a lozenge, each containing a predetermined amount of the active agent. Other formulations suitable for oral administration include, but are not limited to, a powdered or granular formulation, an aqueous or oily suspension, an aqueous or oily solution, or an emulsion.

As used herein, an "oily" liquid is one which comprises a carbon-containing liquid molecule and which exhibits a less polar character than water.

A tablet comprising the active agent may, for example, be made by compressing or molding the active agent, optionally with one or more additional ingredients. Compressed tablets can be prepared by compressing, in a suitable device, the active agent in a free-flowing form such as a powder or granular preparation, optionally mixed with one or more of a binder, a lubricant, an excipient, a surface active agent, and a dispersing agent. Molded tablets can be made by molding, in a suitable device, a mixture of the active agent, a pharmaceutically acceptable carrier, and at least sufficient liquid to moisten the mixture. Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycollate. Known surface active agents include, but are not limited to, sodium lauryl sulfate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginate. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearate, silica, and talc.

Tablets can be non-coated or they can be coated using known methods to achieve delayed disintegration in the gastrointestinal tract of a subject, thereby providing sustained release and absorption of the active agent. By way of

example, a material such as glyceryl monostearate or glyceryl distearate can be used to coat tablets. Further by way of example, tablets can be coated using methods described in U.S. Patents numbers 4,256,108; 4,160,452; and 4,265,874 to form osmotically-controlled release tablets. Tablets can further comprise a sweetening agent, a flavoring agent, a coloring agent, a preservative, or some combination of these in order to provide pharmaceutically elegant and palatable preparation.

Hard capsules comprising the active agent can be made using a physiologically degradable composition, such as gelatin. Such hard capsules comprise the active agent, and can further comprise additional ingredients including, for example, an inert solid diluent such as calcium carbonate, calcium phosphate, or kaolin.

Soft gelatin capsules comprising the active agent can be made using a physiologically degradable composition, such as gelatin. Such soft capsules comprise the active agent, which can be mixed with water or an oil medium such as peanut oil, liquid paraffin, or olive oil.

Liquid formulations of a pharmaceutical composition of the invention which are suitable for oral administration can be prepared, packaged, and sold either in liquid form or in the form of a dry product intended for reconstitution with water or another suitable vehicle prior to use.

Liquid suspensions can be prepared using conventional methods to achieve suspension of the active agent in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions can further comprise one or more additional ingredients including, but not limited to, suspending agents, dispersing or wetting agents, emulsifying agents, demulcents, preservatives, buffers, salts, flavorings, coloring agents, and sweetening agents. Oily suspensions can further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropylmethylcellulose. Known dispersing or wetting agents include, but are not limited to, naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g., polyoxyethylene stearate, heptadecaethyleneoxycetanol, polyoxyethylene sorbitol monooleate, and polyoxyethylene sorbitan mono-oleate, respectively). Known emulsifying agents include, but are not limited to, lecithin and acacia. Known preservatives include, but are not limited to, methyl, ethyl, or n-propyl-para- hydroxybenzoates, ascorbate, and sorbate. Known sweetening agents include, for example, glycerol, propylene glycol, sorbitol, sucrose, and saccharin. Known thickening agents for oily suspensions include, for example, beeswax, hard paraffin, and cetyl alcohol.

Liquid solutions of the active agent in aqueous or oily solvents can be prepared in substantially the same manner as liquid suspensions, the primary difference being that the active agent is dissolved, rather than suspended in the solvent. Liquid solutions of the pharmaceutical composition of the invention can comprise each of the components described with regard to liquid suspensions, it being understood that suspending agents will not necessarily aid dissolution of the active agent in the solvent. Aqueous solvents include, for example, water and isotonic saline. Oily solvents include, for example, almond oil, oily esters, ethyl alcohol, vegetable oils such as arachis, olive, sesame, or coconut oil, fractionated vegetable oils, and mineral oils such as liquid paraffin.

Powdered and granular formulations of a pharmaceutical preparation of the invention can be prepared using known methods. Such formulations can be administered directly to a subject, used, for example, to form tablets, to fill capsules, or to prepare an aqueous or oily suspension or solution by addition of an aqueous or oily vehicle thereto. Each of these formulations can further comprise one or more of dispersing or wetting agent, a suspending agent, and a preservative. Additional excipients, such as fillers and sweetening, flavoring, or coloring agents, can also be included in these formulations.

A pharmaceutical composition of the invention can also be prepared, packaged, or sold in the form of oil-in-water emulsion or a water-in-oil emulsion. The oily phase can be a vegetable oil such as olive or arachis oil, a mineral oil such as liquid paraffin, or a combination of these. Such compositions can further comprise one or more emulsifying agents such as naturally occurring gums such as gum acacia or gum tragacanth, naturally-occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions can also contain additional ingredients including, for example, sweetening or flavoring agents.

A pharmaceutical composition of the invention can be prepared, packaged, or sold in a formulation suitable for rectal administration. Such a composition can be in the form of, for example, a suppository, a retention enema preparation, and a solution for rectal or colonic irrigation.

Suppository formulations can be made by combining the active agent with a non-irritating pharmaceutically acceptable excipient which is solid at ordinary room temperature (i.e., about 20°C) and which is liquid at the rectal temperature of the subject (i.e., about 37°C in a healthy human). Suitable pharmaceutically acceptable excipients include,

but are not limited to, cocoa butter, polyethylene glycols, and various glycerides. Suppository formulations can further comprise various additional ingredients including, but not limited to, antioxidants and preservatives.

Retention enema preparations or solutions for rectal or colonic irrigation can be made by combining the active agent with a pharmaceutically acceptable liquid carrier. As is well known in the art, enema preparations can be administered using, and can be packaged within, a delivery device adapted to the rectal anatomy of the subject. Enema preparations can further comprise various additional ingredients including, but not limited to, antioxidants and preservatives.

A pharmaceutical composition of the invention can be prepared, packaged, or sold in a formulation suitable for vaginal administration. Such a composition can be in the form of, for example, a suppository, an impregnated or coated vaginally-insertable material such as a tampon, a douche preparation, or a solution for vaginal irrigation.

Methods for impregnating or coating a material with a chemical composition are known in the art, and include, but are not limited to methods of depositing or binding a chemical composition onto a surface, methods of incorporating a chemical composition into the structure of a material during the synthesis of the material (i.e., such as with a physiologically degradable material), and methods of absorbing an aqueous or oily solution or suspension into an absorbent material, with or without subsequent drying.

Douche preparations or solutions for vaginal irrigation can be made by combining the active agent with a pharmaceutically acceptable liquid carrier. As is well known in the art, douche preparations can be administered using, and can be packaged within, a delivery device adapted to the vaginal anatomy of the subject. Douche preparations can further comprise various additional ingredients including, but not limited to, antioxidants, antibiotics, anti-fungal agents, and preservatives.

Formulations of a pharmaceutical composition suitable for parenteral administration can comprise the active agent combined with a pharmaceutically acceptable carrier, such as sterile water or sterile isotonic saline. Such formulations can be prepared, packaged, or sold in a form suitable for bolus administration or for continuous administration. Injectable formulations can be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations can further comprise one or more additional ingredients including, but not limited to, suspending, stabilizing, or dispersing agents. In one embodiment of a formulation for parenteral administration, the active agent is provided in dry (i.e., powder or granular) form for reconstitution with a suitable vehicle (e.g., sterile pyrogen-free water) prior to parenteral administration of the reconstituted composition.

The pharmaceutical compositions can be prepared, packaged, or sold in the form of a sterile injectable aqueous or oily suspension or solution. This suspension or solution can be formulated according to the known art, and can comprise, in addition to the active agent, additional ingredients such as the dispersing agents, wetting agents, or suspending agents described herein. Such sterile injectable formulations can be prepared using a non-toxic parenterally-acceptable diluent or solvent, such as water or 1,3-butane diol, for example. Other acceptable diluents and solvents include, but are not limited to, Ringer's solution; isotonic sodium chloride solution, and fixed oils such as synthetic mono- or di-glycerides. Other parentally-administrable formulations which are useful include those which comprise the active agent in microcrystalline form, in a liposomal preparation, or as a component of a biodegradable polymer systems. Compositions for sustained release or implantation can comprise pharmaceutically acceptable polymeric or hydrophobic materials such as an emulsion, an ion exchange resin, a sparingly soluble polymer, or a sparingly soluble salt.

Formulations suitable for topical administration include, but are not limited to, liquid or semi-liquid preparations such as liniments, lotions, oil-in-water or water-in-oil emulsions such as creams, ointments or pastes, and solutions or suspensions. Topically-administrable formulations may, for example, comprise from about 1% to about 10% (w/w) active agent, although the concentration of the active agent can be as high as the solubility limit of the active agent in the solvent. Formulations for topical administration can further comprise one or more of the additional ingredients described herein.

A pharmaceutical composition of the invention can be prepared, packaged, or sold in a formulation suitable for pulmonary administration via the buccal cavity. Such a formulation can comprise dry particles which comprise the active agent and which have a diameter in the range from about 0.5 to about 7 nanometers, and preferably from about 1 to about 6 nanometers. Such compositions are conveniently in the form of dry powders for administration using a device comprising a dry powder reservoir to which a stream of propellant can be directed to disperse the powder or using a self-propelling solvent/powder-dispensing container such as a device comprising the active agent dissolved or suspended in a low-boiling propellant in a sealed container. Preferably, such powders comprise particles wherein at least 98% of the particles by weight have a diameter greater than 0.5 nanometers and at least 95% of the particles by number have a diameter less than 7 nanometers. More preferably, at least 95% of the particles by weight have a diameter greater than I nanometer and at least 90% of the particles by number have a diameter less than 6 nanometers. Dry powder compositions preferably include a solid fine powder diluent such as sugar and are conveniently provided in a unit dose form.

Low boiling propellants generally include liquid propellants having a boiling point of below 65°F at atmospheric pressure. Generally the propellant can constitute 50 to 99.9% (w/w) of the composition, and the active agent can constitute 0.1 to 20% (w/w) of the composition. The propellant can further comprise additional ingredients such as a liquid nonionic or solid anionic surfactant or a solid diluent (preferably having a particle size of the same order as particles comprising the active agent).

Pharmaceutical compositions of the invention formulated for pulmonary delivery can also provide the active agent in the form of droplets of a solution or suspension. Such formulations can be prepared, packaged, or sold as aqueous or dilute alcoholic solutions or suspensions, optionally sterile, comprising the active agent, and can conveniently be administered using any nebulization or atomization device. Such formulations can further comprise one or more additional ingredients including, but not limited to, a flavoring agent such as saccharin sodium, a volatile oil, a buffering agent, a surface active agent, or a preservative such as methylhydroxybenzoate. The droplets provided by this route of administration preferably have an average diameter in the range from about 0.1 to about 200 nanometers.

The formulations described herein as being useful for pulmonary delivery are also useful for intranasal delivery of a pharmaceutical composition of the invention.

Another formulation suitable for intranasal administration is a coarse powder comprising the active agent and having an average particle from about 0.2 to 500 micrometers. Such a formulation is administered in the manner in which snuff is taken i.e., by rapid inhalation through the nasal passage from a container of the powder held close to the nares.

Formulations suitable for nasal administration may, for example, comprise from about as little as 0.1% (w/w) and as much as 100% (w/w) of the active agent, and can further comprise one or more of the additional ingredients described herein.

A pharmaceutical composition of the invention can be prepared, packaged, or sold in a formulation suitable for buccal administration. Such formulations may, for example, be in the form of tablets or lozenges made using conventional methods, and may, for example, 0.1 to 20% (w/w) active agent, the balance comprising an orally dissolvable or degradable composition and, optionally, one or more of the additional ingredients described herein. Alternately, formulations suitable for buccal administration can comprise a powder or an aerosolized or atomized solution or suspension comprising the active agent. Such powdered, aerosolized, or aerosolized formulations, when dispersed, preferably have an average particle or droplet size in the range from about 0.1 to about 200 nanometers, and can further comprise one or more of the additional ingredients described herein.

A pharmaceutical composition of the invention can be prepared, packaged, or sold in a formulation suitable for ophthalmic administration. Such formulations may, for example, be in the form of eye drops including, for example, a 0.1-1.0% (w/w) solution or suspension of the active agent in an aqueous or oily liquid carrier. Such drops can further comprise buffering agents, salts, or one or more other of the additional ingredients described herein. Other ophthalmalmically-administrable formulations which are useful include those which comprise the active agent in microcrystalline form or in a liposomal preparation.

As used herein, "additional ingredients" include, but are not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; anti-fungal agents; stabilizing agents; and pharmaceutically acceptable polymeric or hydrophobic materials. Other "additional ingredients" which can be included in the pharmaceutical compositions of the invention are known in the art and described, for example in Genaro, ed., 1985, Remington's Pharmaceutical Sciences, Mack Publishing Co., Easton, PA, which is incorporated herein by reference.

The relative amounts of the active agent, the pharmaceutically acceptable carrier, and any additional ingredients in a pharmaceutical composition of the invention will vary, depending upon the identity, size, and the type and severity of condition of the subject treated and further depending upon the route by which the composition is to be administered. By way of example, the composition can comprise between 0.1 % and 100% (w/w) active agent.

Typically dosages of the active agent which can be administered to an animal, preferably a human, range in amount from 1 microgram to about 100 grams per kilogram of body weight of the animal. While the precise dosage administered will vary depending upon any number of factors, including but not limited to, the type of animal and type of disease state being treated, the age of the animal and the route of administration. Preferably, the dosage of the active agent will vary from about 1 milligram to about 10 g per kilogram of body weight of the animal. More preferably, the dosage will vary from about 10 milligram to about 1 gram per kilogram of body weight of the animal. Alternatively, the dosage can be determined in units of square meters of the body surface of an animal (i.e., milligrams or kilograms per square meter, $mg/m^2$ or $kg/m^2$). Preferably, this dosage will vary from about 0.1 milligram to about 5 grams per square meter of body surface of the animal. More preferably, the dosage will vary from about 1 milligram to about 1 gram per square meter of body surface of the animal.

The active agent can be administered to an animal as frequently as several times daily, or it can be administered less frequently, such as once a day, once a week, once every two weeks, once a month, or even lees frequently, such as once every several months or even once a year or less. The frequency of the dose is determinable by the skilled artisan and depends upon various factors including, but not limited to, the type and severity of the disease being treated, the type and age of the animal, etc.

The invention is now described with reference to the following Examples. These Examples are provided for the purpose of illustration only and the invention is not limited to these Examples, but rather encompasses all variations which are evident as a result of the teaching provided herein.

## Examples

The reagents and procedures which were used in the Examples are now presented.

Unless otherwise stated, all reactions were conducted in the presence of an inert atmosphere (e.g., argon or nitrogen). All solvents were reagent grade (e.g., distilled solvents, chromatography solvents, and reaction work-up solvents) or HPLC grade (i.e., reaction solvents). Anhydrous diethyl ether and tetrahydrofuran (THF) were distilled from sodium and benzophenone. The boiling point range of the hexane used was 38-55°C. Methylene chloride ($CH_2Cl_2$), benzene, toluene, and N,N-dimethyl formamide (DMF) were distilled from calcium hydride ($CaH_2$). Organic acids and bases were reagent grade. Triethylamine ($Et_3N$), diisopropylethylamine (DIPEA), morpholine, and N-methylmorpholine were distilled from calcium hydride ($CaH_2$). All other reagents, including dimethylaminophenol and diethyl 1,3-acetonedicarboxylate, were the highest purity commercial available. Analytical thin-layer chromatography (TLC) was performed using EM Separations Tech./Merck silica gel (60-F254) plates (0.25 millimeter) pre-coated with a fluorescent indicator. Visualization was effected using ultraviolet light (254 nanometers), phosphomolybdic acid (7% w/v) in 95% ethanol. Melting points (mp) were determined using a Thomas-Hoover capillary melting point apparatus and are reported without correction. Proton and carbon magnetic resonance spectra ($^1$H- and $^{13}$C-NMR, respectively) were recorded on a Bruker AM-500 (500MHz) Fourier transform spectrometer, and chemical shifts were expressed in parts per million (ppm) relative to $CHCl_3$ as an internal reference (7.24 ppm for $^1$H and 77.0 for $^{13}$C). Multiplicities are designated as singlet (s), doublet (d), doublet of doublets (dd), doublet of triplets (dt), triplet (t), quartet (q) multiplet (m), and broad singlet (s). Infrared spectra (IR) were obtained using a Perkin-Elmer Model 1600 FT-IR spectrophotometer. Absorptions are reported in wave number ($cm^{-1}$). Optical rotations (in degrees) were measured using a Perkin-Elmer Model 341 polarimeter. High resolution mass spectra (HRMS) were obtained using either a VG 70-70HS, or a Micromass AutoSpect. Elemental Analyses were performed using a Perkin-Elmer 2400 Series II CHNS/O Analyzer. Flash column chromatography was performed using Merck silica gel 60 (240-400 mesh) using the solvent systems indicated for individual experiments.

## Example 1

### Total Synthesis of (-) Tamandarin A

A method of synthesizing (-) Tamandarin A is described in this example. The method is illustrated in Figure 26. The method is initiated with the synthesis of compound 13, depicted in Figure 26A.

### Reaction A of Figure 26A: Synthesis of Compound **8**

A solution comprising 5.13 milliliters (36.9 millimoles) of $Et_3N$ was added drop-wise to a solution comprising 1.56 grams (11.9 millimoles) of compound 7, D-*allo*- isoleucine, and 50 milliliters of freshly distilled $CH_2Cl_2$ at 0°C. To the resulting mixture was added 3.114 grams (12.5 millimoles) of carbobenzyloxy succinimide (Cbz-succinimide). This reaction was stirred at 0°C for 1 hour, and maintained with stirring at room temperature overnight. The reaction mixture was concentrated, diluted with 20 milliliters of a saturated solution of $NaHCO_3$, and washed twice with 10 milliliters aliquots of ether. The combined ether layers were extracted with 10 milliliters of a saturated solution of sodium bicarbonate ($NaHCO_3$) The combined aqueous layers were cooled to 0°C, acidified to pH 2 by drop-wise addition of 1 normal $KHSO_4$, and extracted three times with 20 milliliters of ethyl acetate (EtOAc). The EtOAc layers were combined, washed with 20 milliliters of a saturated solution of NaCl, and dried in the presence of anhydrous sodium sulfate ($Na_2SO_4$). The resulting solution was filtered and concentrated under reduced pressure to yield 3.13 grams of compound **8** (99% yield). Compound **8** was obtained as a colorless oil and used directly in the next step without purification. Analytical data for compound **8** were as follows: $R_f$ 0.08 (20:80-ethyl acetate:hexane); $^1$H NMR (500 MHz, $CDCl_3$) δ 0.86-0.90 (m, 3H), 0.93-0.97 (m, 3H), 1.20-1.27 (m, 1H) and 1.42-1.47 (m, 1H), 1.98-2.09 (m, 1H), 4.47-4.50 (dd, $J_1$=9.1 Hz, $J_2$=3.4 Hz, 1H), 5.10 (s, 2H), 5.17-5.19 (d, J = 9.1 Hz, 1H), 7.29-7.35 (m, 5H); $^{13}$C NMR (250 MHz, $CDCl_3$) δ 11.69, 14.35, 26.20, 37.42, 56.9,6, 67.20, 128.14, 128.24, 128.55, 135.06, 156.42, 177.41; IR ($CHCl_3$) 2470-3540, 3440, 2980, 2950, 2890, 1720, 1510, 1455, 1405, 1385, 1325-1355, 1230-1280, 1165, 1095, 1040, 1005, 910 $cm^{-1}$.

Reactions B and C of Figure 26A: Synthesis of Compound **10**

A solution comprising 3.534 grams (13.3 millimoles) of compound **8** (i.e., crude Cbz-D-*allo*-isoleucine) 50 milliliters of anhydrous $CH_2Cl_2$ was cooled in an ice bath to 0°C. Each of the following was added in solid form to the cooled solution: 2.574 grams (14.0 millimoles) pentafluorophenol (PFPOH), 3.064 grams (16.0 millimoles) of 1-ethyl-3-(3-dimethylaminopropyl)carbodiimide hydrochloride (EDAC•HCl), and 0.325 gram (2.7 millimoles) 4-dimethylaminopyridine (DMAP). The resulting mixture was maintained at 0°C for half an hour with stirring, and at room temperature for an additional 4 hours. The mixture was diluted with 50 milliliters of $CH_2Cl_2$. The $CH_2Cl_2$ layer was washed once using 25 milliliters of a 10% solution of hydrochloric acid (HCl), once using 25 milliliters of a 5% solution of $NaHCO_3$, and once using 25 milliliters of a saturated solution of NaCl. The washed $CH_2Cl_2$ layer was dried in the presence of $Na_2SO_4$, filtered, and concentrated under reduced pressure. The resulting PFP ester, compound **9** (5.70 grams), was obtained as a colorless oil and used in the next step without purification. The following analytical data were obtained for compound **9**: $R_f$ 0.52 (20:80 EtOAc:hexane); $^1H$ NMR (500 MHz, $CDCl_3$) δ 0.90-1.12 (m, 6H), 1.29-1.39 (m, 1H) and 1.45-1.52 (m, 1H), 2.05-2.15 (m, 1H), 4.79-4.84 (m, 1H), 5.10 (s, 2H), 5.12-5.14 (d, J = 9.1 Hz, 1H), 7.29-7.37 (m, 5H); $^{13}C$ NMR (250 MHz, $CDCl_3$) δ 11.68, 14.25, 26.24 37.64, 57.09 67.45, 128.20, 128.35, 128.59, and 135.95, 136.9, 139.0, 140.0 and 142.0, 156.13, 168.87.

A solution comprising 5.70 grams of compound **9** and 25 milliliters of anhydrous THF was cooled to -78°C. An enolate solution comprising the lithium enolate of methyl acetate was prepared by cooling a solution comprising 49.2 millimoles of lithium dialdehyde and 25 milliliters anhydrous THF in a dry ice bath at -78°C, adding to the solution 3.92 milliliters (49.2 millimoles) of methyl acetate by syringe, and maintaining the resulting solution with stirring at -78°C for 1 hour. The resulting enolate solution was added drop-wise to the solution comprising compound **9**, and the resulting mixture was maintained with stirring for 0.75 hours at -78°C. The reaction was quenched at -78°C by adding 50 milliliters of a saturated solution of aqueous ammonium chloride ($NH_4Cl$). The quenched reaction was brought to room temperature, and THF was removed under reduced pressure. The resulting aqueous solution was extracted 3 times with 25 milliliters aliquots of $CH_2Cl_2$. The combined $CH_2Cl_2$ layers were washed once using 25 milliliters of a 10% solution of hydrochloric acid (HCl), once using 25 milliliters of a 5% solution of $NaHCO_3$, and once using 25 milliliters of a saturated solution of NaCl. The washed $CH_2Cl_2$ layer was dried in the presence of $Na_2SO_4$, filtered, and concentrated under reduced pressure. This reaction yielded a yellow oil which was purified by flash column chromatography. The yellow oil was applied to a column of silica gel and eluted with a solution comprising EtOAc and hexane in a ratio of 10 to 95, respectively. The product obtained from chromatography was 3.42 grams of a colorless oil corresponding to the beta-keto ester, compound **10**. The yield of compound **10** was 80%, as calculated for both Reactions B and C. The following analytical data were obtained for compound **10:** $R_f$ 0.42 (35:65, EtOAc:hexane); $^1H$ NMR (500 MHz, $CDCl_3$) δ 0.75-0.79 (m, 3H), 0.90-0.98 (m, 3H), 1.26-1.30 (m, 1H) and 1.42-1.46 (m, 1H), 1.97-1.99 (m, 1H), 3.53 (s, 2H), 3.72 (s, 3H), 4.56-4.58 (d, J = 7.6 Hz, 1H), 5.10 (s, 2H), 5.26-5.28 (d, J = 6.4 Hz, 1H), 7.30-7.36 (m, 5H); $^{13}C$ NMR (250 MHz, $CDCl_3$) δ 11.83, 13.79, 26.83, 36.12, 46.56, 52.46, 63.00, 67.19, 128.10, 128.24, 128.56, 136.16, 156.42, 166.96, 201.68; IR ($CHCl_3$) 3349.1, 2964.4, 1748.3, 1712.9, 1520.6, 1454.8, 1328.2, 1232.1 $cm^{-1}$; HRMS *m/z* calculated for $C_{17}H_{23}NO_5Na$ ($M^+Na^+$): 344.1498, found 344.1490; $[\alpha]_D^{20}$ -27.85 (c 0.53, $CHCl_3$); Anal. Calculated for $C_{17}H_{23}NO_5$: C, 63.52; H, 7.22, N, 4.36. Found: C, 63.32; H, 7.15, N, 4.24.

Reaction D of Figure 26A: Synthesis of Compound **11**

A solution comprising 2.797 grams (8.7 millimoles) of compound 10 and 30 milliliters of HPLC-grade methanol (MeOH) was cooled to -78°C, and 1.644 grams (30.5 millimoles) of potassium borohydride ($KBH_4$) was added in portions. The resulting mixture was initially maintained with stirring at -78°C for 10 minutes. The reaction vessel was next warmed to -20°C and maintained with stirring for 30 minutes, following which, the reaction vessel was warmed to 0°C and maintained with stirring for 10 minutes. The resulting mixture was quenched at 0 °C by adding a solution comprising glacial acetic acid in a drop-wise manner until the pH of the aqueous layer was not less pH 6 when tested with litmus paper. The resulting neutralized, bilayer solution was concentrated under reduced pressure, and 50 milliliters of a solution comprising EtOAc and $H_2O$ in a ratio of 1 to 1, was added. The organic layer was separated from the aqueous layer and washed with 10 milliliters of a saturated solution of NaCl. The washed organic layer was dried in the presence of $Na_2SO_4$, filtered, and concentrated under reduced pressure. The crude product thus obtained was 2.786 grams of a colorless oil comprising an 11:1 ratio of compound **11** and its stereoisomer, respectively. Crystallization of the colorless oil in a solution comprising ether and hexane afforded pure compound **11** as a white crystalline solid in 99% yield. The following analytical data were obtained for compound **11**: $R_f$ 0.29 (35:65, EtOAc:hexane); $^1H$ NMR (500 MHz, $CDCl_3$) δ 0.83-0.85 (m, 3H), 0.89-0.92 (m, 3H), 1.19-1.23 (m, 1H) and 1.32-1.34 (m, 1H), 1.91-1.93 (m, 1H), 2.45-2.51 (dd, $J_1$=16.7 Hz, $J_2$=9.1 Hz, 1H) and 2.58-2.62 (dd, $J_1$=16.7 Hz, $J_2$=2.7 Hz, 1H), 3.12-3.14 (d, J = 4.5 Hz, 1H), 3.68 (s, 3H), 3.90-3.91 (m, 1H), 4.62-4.65 (d, J =10.0 Hz, 1H), 5.07-5.08 (d, J = 5.1 Hz, 2H), 7.29-7.35 (m, 5H); $^{13}C$ NMR (250 MHz, $CDCl_3$) δ 12.10, 13.64, 27.52, 34.28, 38.74, 52.25, 57.57, 67.39, 69.43, 128.50, 128.61, 128.97 and 136.82, 157.08,

174.09; IR ($CHCl_3$) 3421, 3316, 2951, 1709, 1537, 1443, 1229 $cm^{-1}$; HRMS *m/z* calculated for $C_{17}H_{25}NO_5Na$ ($M^+Na^+$): 346.1630, found 346.1645; $[\alpha]_D^{20}$ -10.9 (c 0,595, $CHCl_3$); Anal. Calculated for $C_{17}H_{25}NO_5$: C, 63.12; H, 7.80, N, 4.33. Found: C, 63.23; H, 7.85, N, 4.06.

Reaction E of Figure 26A: Synthesis of Compound **12**

A solution comprising 0.8636 gram (2.67 millimoles) of compound **11** as a colorless oil and 10 milliliters of $CH_2Cl_2$ was placed under an argon atmosphere and cooled to 0°C. A solution comprising 0.778 milliliters (6.68 millimoles) of 2,6-lutidine was added, followed by addition of a solution comprising 1.08 milliliters (4.01 millimoles) of triisopropylsilyl triflate (*i*-$Pr_3SiOTf$). The reaction mixture was initially maintained with stirring at 0°C for 30 minutes, following which, the reaction mixture was maintained at room temperature for 2 hours. The resulting mixture was diluted with 20 milliliters of $CH_2Cl_2$. The $CH_2Cl_2$ layer was washed once using 15 milliliters of a 10% solution of hydrochloric acid (HCl), once using 15 milliliters of a 5% solution of $NaHCO_3$, and once using 15 milliliters of a saturated solution of NaCl. The resulting washed $CH_2Cl_2$ layer was dried in the presence of anhydrous $Na_2SO_4$, filtered, and concentrated under pressure. The concentrated residue was purified by flash column chromatography, eluting with solutions comprising ether and hexane in a ratio of from 2 to 98, respectively, to 15 to 85, respectively. Chromatography yielded 1.204 grams (94% yield) of compound 12 as the major isomer in the form of a colorless oil. The following analytical data were obtained for compound **12**: $R_f$ 0.65 (35:65, EtOAc:hexane); $^1H$ NMR (500 MHz, $CDCl_3$) δ 0.84-0.91 (m, 6H), 0.99-1.05 (m, 21H), 1.12-1.34 (m, 2H), 1.81-1.84 (m, 1H), 2.54-2.62 (m, 2H), 3.54 (s, 3H), 3.73-3.75 (m, 1H), 4.34-4.36 (m, 1H), 4.69-4.71 (d, J = 10.5 Hz, 1H), 5.01-5.04 (d, J = 12.3 Hz, 1H) and 5.09-5.11 (d, J = 12.3 Hz, 1H), 7.28-7.34 (m, 5H); $^{13}C$ NMR (250 MHz, $CDCl_3$) δ 12.50, 12.74, 13.97, 18.08, 27.44, 34.40, 40.45, 51.54, 58.62, 66.67, 70.49, 128.03, 128.08, 128.46 and 136.67, 156.51, 172.00; IR ($CHCl_3$) 3450, 3359, 2944, 2863, 1728, 1510, 1459, 1434, 1384, 1308, 1232, 1171, 1090 $cm^{-1}$ ; HRMS *m/z* calculated for $C_{26}H_{45}NSiO_5Na$ ($M^+Na^+$): 480.3145, found 480.3128; $[\alpha]^D_{20}$ +15.88 (c 0.57, $CHCl_3$); Anal. Calculated for $C_{17}H_{25}NO_5$: C, 65.09; H, 9.46, N, 2.92. Found: C, 64.80; H, 9.41, N, 2.69.

Reaction F of Figure 26A: Synthesis of Compound **13**

A solution comprising 1 normal NaOH (20 milliliters, 11 millimoles) was added to a solution comprising 0.84 gram (1.753 millimoles) of compound **12,** 10 milliliters of THF, and 10 milliliters of MeOH, which was cooled to 0°C. This reaction mixture was maintained with stirring at 0°C for 2 hours. The reaction mixture was then maintained with stirring at room temperature overnight. The reaction mixture was concentrated under reduced pressure and diluted with 10 milliliters of $H_2O$. The resulting mixture was cooled to 0°C in an ice bath, acidified to pH 2 by adding a solution comprising 1 normal $KHSO_4$, and extracted 3 times with 10 milliliters aliquots of EtOAc. The EtOAc layers were combined, washed with 10 milliliters of a saturated solution of NaCl, dried in the presence of anhydrous $Na_2SO_4$, filtered, and concentrated. This reaction yielded 0.7709 gram (95%yield) of compound **13** in the form of a white foam. Compound **13** was used in the subsequent reaction without purification. Analytical data for compound **13** were as follows: $R_f$ 0.08 (35:65-ethyl acetate:hexane); $^1H$ NMR (500 MHz, $CDCl_3$) δ 0.86-1.00 (m, 6H), , 1.06-1.08 (m, 21H), 1.19-1.24 (m, 1H) and 1.64-1.72 (m, 1H), 1.85-1.92 (m, 1H), 2.50-2.80 (m, 2H), 3.60-3.80 (m, 1H), 4.28-4.33 (m, 1H), 4.86-4.88 (d, J = 10.4 Hz, 1H), 5.06-5.22 (m, 2H), 7.29-7.45 (m, 5H).

Reaction G of Figure 26B: Synthesis of Compound **15**

Synthesis of a protected form of (2S)-α-hydroxyisovaleryl-isostatine (**6**) is depicted in Figure 26B. In reaction G of Figure 26B, L-valine (**14**), was dissolved in a solution comprising sodium nitrite ($NaNO_2$) and 1 normal sulfuric acid ($H_2SO_4$). The reaction was carried out using a standard method as described, for example, in Green and Wutz (1999, Protecting Groups in Organic Synthesis, Wiley, New York) or Bodansky (1993, Principles of Peptide Synthesis; Springer, Berlin). This reaction yielded alpha-hydroxy valine (**15**).

Reaction H of Figure 26B: Synthesis of Compound **16**

In reaction H, 2.46 grams (17.78 millimoles) of anhydrous potassium carbonate ($K_2CO_3$), and 1.25 grams (3.4 millimoles) of tetrabutyammonium iodide ($Bu_4NI$) were added to a solution comprising 2 grams (16.93 millimoles) of alpha-hydroxy valine (**15**) and 20 milliliters of re-distilled DMF, followed by drop-wise addition of 5.86 milliliters (67.72 millimoles) of allyl bromide. The resulting solution was stirred at room temperature for 1 hour. The reaction mixture was concentrated under reduced pressure, diluted with 20 milliliters of $H_2O$, and extracted three times with 20 milliliters aliquots of ether. The combined organic layers were washed once using 15 milliliters of a 10% solution of hydrochloric acid (HCl), once using 15 milliliters of a 5% solution of $NaHCO_3$, and once using 15 milliliters of a saturated solution of NaCl. The resulting washed organic layers were dried in the presence of $Na_2SO_4$, filtered, and concentrated under

reduced pressure. This reaction yielded 2.53 grams (96%yield) of compound **16** in the form of an orange oil. Compound **16** was used in the subsequent reaction without purification. The following analytical data were obtained for compound **16**: $R_f$ 0.50 (25:75, EtOAc:hexane); $^1$H NMR (500 MHz, $CDCl_3$) δ 0.71-0.86 (d, J = 6.8 Hz, 3H), 0.92-1.00 (d, J = 7.0 Hz, 3H), 2.04-2.10 (m, 1H), 2.65-2.66 (d, J = 6.1 Hz, 1H), 4.03-4.05 (dd, $J_1$=5.9 Hz, $J_2$=3.5 Hz, 1H), 4.62-4.70 (m, 2H), 5.24-5.27 (dd, $J_1$=10.4 Hz, $J_2$=1.1 Hz, 1H) and 5.31-5.35 (dd, $J_1$=17.2 Hz, $J_2$=3.5 Hz, 1H), 5.86-5.94 (m, 1H); $^{13}$C NMR (250 MHz, $CDCl_3$) δ 15.93, 18.76, 32.17, 66.04, 75.03, 119.12, 131.48, 174.62; IR ($CHCl_3$) 3521-3458, 2964, 2880, 1735, 1646, 1462, 1367, 1257, 1204, 1136, 1073, 1026, 983, 931 $cm^{-1}$.

Reaction I of Figure 26B: Synthesis of Compound **17**

A solution comprising 0.6827 gram (1.47 millimoles) of compound **13** and 2.5 milliliters of freshly distilled toluene, was placed under an inert atmosphere and cooled to 0°C. A solution comprising 0.232 gram (1.47 millimoles) of compound **16** and 2.5 milliliters of toluene was added drop-wise to the cooled solution of compound **13**, followed by the addition of 0.333 gram (1.61 millimoles) of dicyclohexylcarbodiimide (DCC) and 0.036 gram (0.29 millimole) of DMAP. The reaction mixture was stirred at 0°C for 2 hours and maintained at room temperature overnight. The reaction was quenched by adding 2 milliliters of a solution comprising MeOH and acetic acid (AcOH) in a ratio of 1:2, and EtOAc, and stirred for 20 minutes at room temperature. The resulting mixture was concentrated under reduced pressure. The residue which remained after this procedure was dissolved in 10 milliliters of ether, which resulted in the formation of a solid, which was removed by filtration. The filtrate was washed once using 10 milliliters of a 10% solution of citric acid, once using 10 milliliters of a 5% solution of $NaHCO_3$, and once using 10 milliliters of brine solution (i.e., a saturated NaCl solution). The organic layer was dried in the presence of anhydrous $Na_2SO_4$, filtered, and concentrated. The resulting residue was purified by flash column chromatography, eluting with a solvent mixture comprising ether and hexane in a ratio of from 2 to 98, respectively, to 12 to 88, respectively. Concentration of the eluant under reduced pressure yielded 0.5774 gram (65% yield) of compound **17** in the form of a colorless oil. The following analytical data were obtained for compound **17**: R*f* 0.55 (20:80-ethyl acetate:hexane); $^1$H NMR (500 MHz, $CDCl_3$) δ 0.85-1.08 (m, 33H), 1.16-1.19 (m, 1H), 1.34-1.36 (m, 1H), 1.80-1.82 (m, 1H), 2.18-2.21 (m, 1H), 2.68-2.73 (m, 2H), 3.78-3.82 (m, 1H), 4.37-4.42 (m, 1H), 4.55-4.64 (m, 2H), 4.77-4.78 (d, J = 4.4 Hz, 1H), 4.86-4.88 (d, J =10.7 Hz, 1H), 5.07 (s, 2H), 5.21-5.23 (dd, $J_1$=9.4 Hz, $J_2$=0.9 Hz, 1H), 5.28-5.32 (dd, $J_1$=17.0 Hz, $J_2$=1.2 Hz, 1H), 5.83-5.87 (m, 1H), 7.27-7.34 (m, 5H); $^{13}$C NMR (250 MHz, $CDCl_3$) δ 12.69, 12.93, 14.14, 17.25, 18.12, 18.78, 26.33, 29.98, 34.48, 40.28, 58.15, 66.69, 68.67, 70.66, 76.74, 118.82, 128.01, 128.40, 128.47, 136.75, 131.63, 156.48, 169.13, 170.78; IR ($CHCl_3$) 3380, 2964, 2867, 1743, 1508, 1463, 1374, 1201, 1129, 994, 882 $cm^{-1}$; HRMS *m*/*z* calculated for $C_{33}H_{55}NSiO_7Na$ ($M^+Na^+$): 628.364552, found 628.365878; Anal. Calculated for $C_{33}H_{55}NSiO_7$: C, 65.41; H, 9.16. Found: C, 65.09; H, 9.05.

Reaction J of Figure 26B: Synthesis of Compound **6**

In reaction J, tetrakis-(triphenylphosphine)palladium ($Pd(PPh_3)_4$, 0.044 grams, 0.038 millimole) was added to a solution comprising 0.2315 gram (0.38 millimole) of the isostatine-Hiv-allyl ester, compound **17**, and 3 milliliters of freshly distilled THF. Freshly distilled morpholine (0.33 milliliters, 3.8 millimoles) was added drop-wise to the resulting mixture. The addition of reagents was performed in a dark hood, and the reaction mixture was maintained with stirring at room temperature and in a dark hood for at least 8 hours. The reaction mixture was concentrated under reduced pressure and diluted with 5 milliliters of $CH_2Cl_2$. The solution obtained from this procedure was washed once with 5 milliliters of a solution comprising 1 normal HCl and once using 5 milliliters of $H_2O$. The washed organic layer was dried in the presence of anhydrous $Na_2SO_4$, filtered, and concentrated under reduced pressure. The residue was dissolved in 5 milliliters of ether, filtered, and concentrated again under reduced pressure. The residue which remained after these procedure was a white foam corresponding to compound 6 (0.218 grams, quantitative yield). Compound 6 was used in the subsequent reaction without purification.

The synthesis of a linear hexapeptide compound (**20**) is depicted in Figure 26C. Compound 5 was prepared as described (Li et al., 1990, J. Am. Chem. Soc. 112:7659-7672).

Reaction K of Figure 26C: Synthesis of Compound **18**

A solution comprising 0.6653 gram (0.74 millimole) of compound 5 and 10 milliliters of MeOH was added to a suspension comprising 0.1996 gram of 10% Palladium on carbon (Pd/C), 10 milliliters of MeOH, and 10 milliliters of EtOAc. The reaction mixture was agitated using a Parr apparatus for 5 hours at room temperature. The resulting slurry was filtered through Celite® and the Celite® was washed with an excess of a solvent mixture comprising MeOH and EtOAc in a ratio of 1 to 1. The filtrate was dried in the presence of anhydrous $Na_2SO_4$, filtered, and concentrated under reduced pressure to yield compound **18** (0.552 grams, 98% yield). Compound **18** (i.e., Leucylprolyl-*N*,*O*-dimethyltyrosine-*N*-Boc-*O*-SEM-threonine) was obtained in the form of a white solid using this procedure, and was used in the subsequent

reaction without purification. The following analytical data were obtained for compound 18: R*f* 0.03 (40:60, acetone: hexane); $^1$H NMR (500 MHz, $CDCl_3$) δ -0.001 (s, 9H), 0.64-0.86 (m, 2H), 0.88-0.96 (m, 6H), 1.19-1.21 (d, J = 6.3 Hz), 1.33-1.34 (d, J = 6.3 Hz, 3H, RI), 1.44 (s, 9H), 1.63-1.92 (m, 3H), 1.92-2.01, 2.08-2.24 (m, 4H), 2.73 (s, 3H), 2.86-2.96 (m), 3.10-3.19 (m, 2H), 3.45-3.72 (m, 4H), 3.75 (s, 3H), 4.34-4.69 (m, 3H), 4.78-4.81 (dd, $J_I$=8.0 Hz, $J_2$=3.3 Hz, 1H), 5.01-5.10 (m, 1H), 5.17-5.52 (m), 7.58-7.60 (d, J=9.7 Hz, 3H), 6.77-6.83 (m, 2H), 7.00-7.10 (m, 2H); $^{13}$C NMR (250 MHz, $CDCl_3$) δ -1.46, 16.87, 17.96, 22.00, 23.19, 23.76, 25.14, 28.16, 29.31, 33.65, 39.31, 47.34, 50.54, 55.37, 55.46, 58.66, 62.25, 68.16, 72.36, 81.15, 89.83, 114.39, 128.75, 128.75, 130.47, 157.04, 158.84, 168.15, 168.95, 169.57, 173.13; IR ($CHCl_3$) 3285, 2951, 1740, 1709, 1641, 1511, 1448, 1365, 1250, 1161 $cm^{-1}$; HRMS *m*/*z* calculated for $C_{37}H_{63}N_4SiO_{10}$ ($M^+H^+$): 751.4314, found 751.4343; [α]$D^{20}$ -44.68 (c 1.03, $CHCl_3$); Anal. Calculated for $C_{37}H_{62}N_4SiO_{10}$: C, 59.17; H, 8.33; N, 7.46. Found: C, 59.17; H, 8.35; N, 7.26.

Reaction L of Figure 26C: Synthesis of Compound **19**

In reaction L, the entire yield of compound **6** from reaction J was dissolved in 1 milliliter of freshly distilled $CH_2Cl_2$ and cooled to 0°C. To this solution was added 0.074 gram (0.40 millimole) of PFPOH, (0.088 grams, 0.46 millimole) of EDAC•HCl, and 0.0093 gram of DMAP (0.076 millimole). The resulting mixture was stirred at 0°C for 30 minutes. The reaction mixture was maintained at room temperature for an additional 4 hours and diluted with 10 milliliters of $CH_2Cl_2$. The organic layer was washed once using 5 milliliters of a 10% solution of HCl, once using 5 milliliters of a 5% solution of $NaHCO_3$, and once using 5 milliliters of a saturated solution of NaCl. The $CH_2Cl_2$ layer was dried in the presence of anhydrous $Na_2SO_4$, filtered, and concentrated under reduced pressure. The resulting residue was purified by flash column chromatography, eluting with a solvent mixture comprising ether and hexane in a ratio of from 3 to 97, respectively, to 7 to 93, respectively. Concentration of the eluate under reduced pressure yielded 0.2315 gram of a colorless oil corresponding to the PFP ester, compound **19** (83% yield for reaction J of Figure 26B and reaction L of Figure 26C). The following analytical data were obtained for compound **19:** R*f* 0.57 (20:80, EtOAc:hexane); $^1$H NMR (500 MHz, $CDCl_3$) δ 0.86-1.09 (m, 33H), 1.18-1.20 (m, 1H), 1.27-1.29 (m, 1H), 1.84-1.86 (m, 1H), 2.31-2.36 (m, 1H), 2.69-2.72 (m, 1H), 2.80-2.85 (m, 1H), 3.79-3. 82 (m, 1H), 4.38-4.42 (m, 1H), 4.78-4.80 (d, J = 10.7 Hz, 1H), 4.97-4.98 (d, J = 4.4 Hz, 1H), 5.00-5.06 (m, 2H), 7.25-7.29 (m, 5H); $^{13}$C NMR (250 MHz, $CDCl_3$) δ 12.72, 12.95, 14.07, 17.20, 18.09, 18.45, 27.52, 30.19, 34.40, 40.12, 58.17, 65.82, 66.74, 70.47, 127.92, 128.18, 128.33, 136.57, 138.83, 140.06, 140.65, 141.97, 156.56, 165.68, 170.76; IR ($CHCl_3$) 3480, 2962, 2868, 1793, 1730, 1516, 1464, 1381, 1214, 1094, 995, 880 $cm^{-1}$; HRMS *m*/*z* calculated for $C_{36}H_{50}NF_5SiO_7Na$ ($M^+Na^+$): 754.3174, found 754.3191.

Reaction M of Figure 26C: Synthesis of Compound **20**

A solution comprising 0.2855 gram (0.39 millimole) of compound **19** and 1.5 milliliters of $CH_2Cl_2$ was cooled to 0°C in an ice bath. To this solution 0.17 milliliters (0.98 millimole) of DIEA was added drop-wise, and the resulting mixture was maintained with stirring at 0°C for 20 minutes. A solution comprising 0.522 gram of compound **18,** 0.0095 gram (0.078 millimole) of DMAP, and 1.5 milliliters of $CH_2Cl_2$ was added to the solution comprising compound **19** using a syringe. The resulting mixture was stirred at 0°C for 1 hour and maintained at room temperature for an additional 1 hour. The reaction was quenched at 0°C by adding 3 milliliters of a saturated solution of $NH_4Cl$ and diluting the reaction with 10 milliliters of $CH_2Cl_2$. The resulting mixture was separated at room temperature. The aqueous layer was extracted 3 times using 10 milliliters aliquots of $CH_2Cl_2$, and the combined organic layers were washed once using 10 milliliters of a 10% solution of HCl, once using 10 milliliters of a 5% solution of $NaHCO_3$, and once using 10 milliliters of a saturated NaCl solution. The washed organic layer was dried in the presence of anhydrous $Na_2SO_4$, filtered, and concentrated under reduced pressure. This reaction yielded 0.4861 gram (96% yield) of the fully protected hexapeptide precursor, compound **20.** Compound **20** were obtained in the form of a white foam using this procedure, and was used in the subsequent reaction without further purification. Analytical data for hexapeptide **20** was as follows: R*f* 0.47 (05:95-acetone:$CH_2Cl_2$); $^1$H NMR (500 MHz, $CDCl_3$) δ -0.0008 (s, 9H), 0.73-0.83 (m, 2H), 0.85-0.92 (m, 9H), 0.92-1.08 (m, 21H), 1.45 (s, 9H), 1.13-2.19 (m, 11H), 2.43-2.46 (m, 1H) and 2.54-2.58 (m, 1H), 2.64 and 2.88 (s, 3H, RI), 3.09-3.17 (m, 2H), 3.44-3.73 (m, 4H), 3.75 (s, 3H), 3.79-3.89 (m, 1H), 4.38-4.45 (m, 114),4.21-4.35 (m, 3H), 4.70-4.81 (m, 1H), 4.96-5.06 (m, 3H), 5.18-5.43 (m, 3H) and 8.32-8.34 (d, J = 9.0 Hz, 3H), 5.46-5.48 (d, J = 6.1 Hz, 1H), 6.74-6.83 (m, 2H), 6.95-7.11 (m, 2H), 7.25-7.38 (m, 5H), 7.75-7.77 (d, J = 8.5 Hz) and 8.85-8.87 (d, J = 10.1 Hz, 2H); $^{13}$C NMR (250 MHz, $CDCl_3$) δ -1.45, 11.73, 12.73, 14.47, 16.54, 17.73, 17.99, 18.14, 18.92, 21.40, 23.54, 24.42, 25.13, 28.21, 28.27, 29.20, 29.67, 30.13, 33.59, 34.86, 39.58, 39.73, 46.87, 49.04, 55.13, 55.39, 56.90, 58.94, 62.24, 66.42, 68.12, 70.96, 72.25, 78.71, 80.39, 89.85, 113.92, 114.37, 127.70, 127.76, 127.86, 128.35, 128.45, 128.81, 128.91, 137.01, 130.44, 130.86, 156.39, 158.40, 158.82, 169.20, 169.75, 169.97, 170.58, 171.77, 173.85; IR ($CHCl_3$) 3275, 2952, 2868, 1735, 1704, 1636, 1511, 1454, 1380, 1365, 1250, 1167, 1110, 1047 $cm^{-1}$; HRMS *m*/*z* calculated for $C_{67}H_{111}N_5Si_2O_{16}Na$ ($M^+Na^+$): 1320.7462, found 1320.7520; [α]$D^{20}$ -44.56 (c 1.13, $CHCl_3$); Anal. Calculated for $C_{67}H_{111}N_5Si_2O_{16}$: C, 61.95; H, 8.62; N, 5.40. Found: C, 61.75; H, 8.59; N, 5.15.

Cyclization of hexapeptide **20** to yield compound **21** is depicted in Figure 26D.

Reaction N of figure 26D: Synthesis of Compound **21**

A solution comprising 0.3505 gram (0.27 millimole) of compound **20** and 5 milliliters of $CH_2Cl_2$ was cooled to 0°C, and 0.21 gram (0.81 millimole) of magnesium bromide etherate ($MgBr_2$•$Et_2O$) was added. The resulting mixture was stirred at 0°C for 2 hours and maintained at room temperature for an additional 4 hours. The reaction mixture was diluted by adding 10 milliliters of $CH_2Cl_2$, and washed once using 10 milliliters of a 10% solution of HCl, and once using 10 milliliters of a saturated NaCl solution. The $CH_2Cl_2$ layer was dried in the presence of anhydrous $Na_2SO_4$, filtered, and concentrated under reduced pressure. The white foam obtained as a product of this de-protection reaction was used in the subsequent hydrogenolysis reaction without purification. The entire yield of white foam (0.3195 grams) was dissolved in 10 milliliters of MeOH and subjected to hydrogenolysis as described for preparation of compound 18. This hydrogenolysis reaction yielded 0.296 gram of a white foam which was used in the subsequent coupling reaction without purification. The hydrogenolysis product was dissolved in 27 milliliters of freshly-distilled DMF and cooled to 0°C. To the cooled solution was added 0.123 gram (0.32 millimole) of 2-(1H-9-azobenzotriazole-1-yl)-1,1,3,3-tetramethyluronium hexafluorophosphate (HATU), followed by drop-wise addition of 0.141 milliliters (0.81 millimole) of DIEA. The resulting mixture was stirred at 0°C for 1 hour and maintained with stirring at room temperature for at least 8 hours. The reaction mixture was concentrated under reduced pressure, diluted with 10 milliliters of EtOAc, and washed once using 10 milliliters of a 10% solution of HCl, once using 10 milliliters of a 5% solution of $NaHCO_3$, and once using 10 milliliters of a saturated NaCl solution. The $CH_2Cl_2$ layer was dried in the presence of anhydrous $Na_2SO_4$, filtered, and concentrated under reduced pressure. The crude residue obtained from this procedure was purified by flash column chromatography using a solvent mixture comprising acetone and hexane in a ratio of from 5 to 95, respectively, to 15 to 85, respectively. The resulting eluate was concentrated under reduced pressure to yield 0.173 gram of the protected macrocycle, compound **21,** in the form of a white foam. The yield of compound **21** represents a 63% yield for the three reactions starting with compound **20** (i.e., the de-protection, hydrogenolysis, and coupling reactions). The following analytical data were obtained for compound **21:** R*f* 0.55 (30:70, acetone:hexane); $^1H$ NMR (500 MHz, $CDCl_3$) $\delta$ 0.78-1.07 (m, 39H), 1.21-1.48 (m, 17H), 1.56-1.92 (m, 4H), 1.95-2.11 (m, 2H), 2.43-2.44 (m, 1H), 3.11-3.17 (m, 1H), 2.53 (s, 3H), 2.89-3.02 (m, 1H), 3.30-3.34 (m, 1H), 3.49-3.52 (m, 1H), 3.60-3.62 (m, 1H), 3.66-3.70 (m, 1H), 3.77 (s, 3H), 4.14-4.19 (m, 1H), 4.37-4.43 (m, 1H), 4.46-4.48 (d, J = 7.6 Hz, 1H), 4.55-4.86 (m, 1H), 4.88-4.91 (m), 7.60-7.66 (m, 4H), 6.82-6.83 (d, J = 8.5 Hz, 2H), 7.06-7.07 (d, J = 8.5 Hz, 2H), 7.41-7.48 (m, 2H); $^{13}C$ NMR (250 MHz, $CDCl_3$) $\delta$ 12.24, 12.66, 14.21, 15.11,15.61,17.98,18.60,18.74,19.31,21.10,23.92,25.24,27.21, 28.42,30.48,34.87,37.92, 39.00, 41.65, 47.10, 48.52, 55.67, 56.16, 57.25, 63.26, 66.33, 68.65, 71.78, 80.46, 81.66, 114.50, 130.31, 130.82, 156.40, 159.01, 169.13, 170.90, 171.34, 172.78, 176.75; IR ($CHCl_3$) 3330, 2952, 2876, 1735, 1629, 1508, 1447, 1243, 1168, 1024, 843 $cm^{-1}$; HRMS *m/z* calculated for$C_{53}H_{89}N_5SiO_{12}Na$ ($N4^+Na^+$): 1038.6175, found 1038.6166.

The final reactions in the total synthesis of (-) Tamandarin A, **101**, are depicted in Figure 26E. Compound **4** was prepared in the manner previously reported in Li et al. (1990, J. Am. Chem. Soc. 112:7659-7672).

Reaction O of Figure 26E: Synthesis of Compound **22**

A solution comprising 167 milligrams (0.165 millimole) of protected macrocycle (compound **21**) and 20 milliliters of EtOAc was cooled to -30°C. Gaseous HCl was introduced into the solution, and the temperature of the reaction mixture was maintained between -10°C to -20°C during introduction of HCl. The reaction mixture was stirred and maintained at a temperature of between -10°C to -20°C for an additional 30 minutes. The reaction mixture was warmed to 0°C and maintained at 0°C for 1 hour. The reaction vessel was purged with $N_2$ gas for at least 30 minutes while the temperature of the vessel was maintained at 0°C. The purged solution was warmed to room temperature and concentrated under reduced pressure. The residue remaining after concentration of the reaction was triturated and washed by decanting using three 5 milliliter aliquots of a solvent mixture comprising *tert*-butylmethyl ether and hexane in a ratio of 1 to 4, respectively. The solid which was produced by this procedure was collected by filtration and dried under reduced pressure to provide 127.5 milligrams (quantitative yield) of the hydrochloride salt of compound **22**. Compound **22** was obtained as a white solid, and was used in the final coupling reaction without purification.

Reaction P of Figure 26E: Synthesis of (-) Tamandarin A (Compound **101**)

A solution comprising 63.3 milligrams (0.082 millimole) of compound **22**, 37.7 milligrams (0.12 millimole) of compound 4, and 0.50 milliliters of $CH_2Cl_2$ was cooled to 0°C in an ice bath. To this cooled solution was added 53.1 milligrams (0.12 millimole) of benzotriazole-1-yl-oxy-tris(dimethylamino)-phosphonium hexafluorophosphate (BOP) and 0.035 milliliters (0.32 millimole) of NMM. The reaction mixture was stirred for 30 minutes at 0°C and allowed to warm to room temperature. The reaction mixture was maintained with stirring at room temperature for at least an additional 8

hours. The resulting solution was diluted with 2 milliliters of a saturated solution of NaCl and extracted with 10 milliliters of EtOAc. The extracted organic layer was washed once using 10 milliliters of a 10% solution of HCl, once using 10 milliliters of a 5% solution of $NaHCO_3$, and once using 10 milliliters of a saturated solution of NaCl. The washed organic layer was dried in the presence of anhydrous $Na_2SO_4$, filtered, and concentrated under reduced pressure. The crude residue was purified by flash column chromatography using a solvent mixture comprising MeOH and $CH_2Cl_2$ in a ratio of from 2 to 98, respectively, to 5 to 95, respectively. This procedure yielded 0.0471 gram of tamandarin A (compound 101) in the form of a yellow-greenish solid. The yield of compound **101** was 56%, as calculated for both reactions O and P, and 12% as calculated for reactions A-F, I, J, and L-P (i.e., starting with D-*allo*-isoleucine). The following analytical data were obtained for tamandarin A (**101**)**:** R*f* 0.57 (10:90-MeOH:$CH_2Cl_2$); $^1$H NMR (500 MHz, $CDCl_3$) δ 0.86-1.08 (m, 24H), 1.19-2.30 (dd, $J_1$=17.1 Hz, $J_2$=7.9 Hz, 1H), 3.29-3.33 (d, J = 17.0 Hz, 1H), 2.62 (s, 3H), 3.14 (s, 3H), 3.16-3.21 (dd, $J_1$=14.4 Hz, $J_2$=11.1 Hz, 1H), 3.41-3.45 (m, 1H), 3.59-3.80 (m, 5H), 3.82 (s, 3H), 3.90-3.95 (m, 1H), 4.03-4.08 (m, 1H), 4.29-4.30 (m, 1H), 4.37-4.42 (m, 1H), 4.67-4.69 (m, 1H), 4.74-4.77 (m, 1H), 4.89-4.93 (m, 1H), 5.06 (d, J = 4.3 Hz, 1H), 5.31-5.35 (q, $J_1$=11.6 Hz, $J_2$=3.3 Hz, 1H), 5.44-5.46 (m, 1H), 6.86-6.88 (d, J = 8.3 Hz, 2H), 7.09-7.11 (d, J = 8.3 Hz, 2H), 7.37-7.39 (d, J = 9.1 Hz, 1H), 7.48-7.50 (d, J = 5.1 Hz, 1H), 7.78-7.80 (d, J = 9.7 Hz, 1H); $^{13}$C NMR (250 MHz, $CDCl_3$) δ 11.78, 14.07, 16.52, 17.55, 18.93, 20.28, 20.90, 21.32, 23.47, 23.79, 24.83, 24.90, 25.98, 27.33, 27.96, 28.40, 30.11, 31.26, 33.55, 33.93, 35.73, 38.70, 39.41, 39.65, 46.66, 47.02, 48.28, 54.91, 55.27, 56.71, 56.97, 57.90, 66.07, 66.22, 68.98, 70.70, 78.87, 114.08, 130.07, 130.33, 156.62, 168.52, 169.57, 170.11, 170.35, 170.59, 171.09, 172.67, 173.84, 174.53; IR ($CHCl_3$) 3330, 2952, 2876, 1735, 1629, 1508, 1447, 1243, 1168, 1024, 843 $cm^{-1}$; HRMS *m/z* calculated for $C_{54}H_{85}N_7O_{14}Na$ ($M^+Na^+$): 1078.6052, found 1078.6044; [α]$D^{20}$ -43.93 (c 1.05, $CHCl_3$).

The inventors believe that the series of reactions disclosed above represent the first stereoselective synthesis of (-) Tamandarin A.

Example 2

Biological Activity of Tamandarin A

The experiments described in this example demonstrate that tamandarin A is an effective protein synthesis inhibitor and anti-tumor agent. Initial results are shown in Table 1 for inhibition of protein biosynthesis (column 1), cytotoxicity (column 2), and anti-tumor activity (column 3) of tamandarin A, as compared to the related anti-tumor compound didemnin B. The results in Table 1 are given in units of concentration of the selected compound.

**Table 1**

| Compound | Protein Synthesis | Cytotoxicity (NCI-60 Mean) | | Anti-tumor activity (Assay Mean) |
|---|---|---|---|---|
| Tamandarin A | 1.3 μM | $GI_{50}$ = 10.4 μM | $LC_{50}$ = 4.8 μM | 1.31 nM |
| Didemnin B | 4 μM | $GI_{50}$ = 1.8 pM | $LC_{50}$ = 7.4 nM | 1.58 nM |

As used herein, " $GI_{50}$" refers to the dose of a compound which is capable of producing 50% inhibition of cell growth. $GI_{50}$ is assessed by comparing growth of cells to which a compound has been administered with growth of the same cells to which the compound has not been administered.

As used herein, "$LC_{50}$" refers to the dose of a compound which is capable of producing 50% lethality in cells. $LC_{50}$ is assessed by comparing death of cells in a population of cells to which a compound has been administered with the death of cells in a population of the same cells to which the compound has not been administered.

"NCI-60" refers to a 60 tumor cell line panel which from the National Cancer Institute (NCI, Frederick, MD). "NCI-60 Mean" is the average $GI_{50}$ or $LC_{50}$ for the panel treated with the selected compound.

These *in vitro* findings indicate that tamandarin A exhibits comparable potency to didemnin B, a known anticancer agent. Tamandarin A is significantly less cytotoxic in these assays than is didemnin B. The comparison indicate the usefulness of tamandarin A and other didemnin analogs as pharmacological agents having anti-cancer and other activities characteristic of didemnin B.

Example 3

Synthesis of (-)Dehydrotamandarin

A method of synthesizing (-) dehydrotamandarin is described in this example. The method involves the macrocyclic compound **22**, which can be generated as described in Example 1. The method described in this Example is illustrated in Figure 37, and begins with synthesis of compound **27**, depicted in Figure 37A.

Reaction O of Figure 37A: Synthesis of Compound **26**

To a solution comprising 0.1084 gram (0.33 millimole) of compound **25** and 1 milliliter of freshly-distilled $CH_2Cl_2$ was added 0.182 g (0.43 millimole) of Dess-martin periodinane. The resulting mixture was stirred at room temperature for 4 hours. The reaction mixture was diluted with 10 milliliters of ether and poured into 6 milliliters of a saturated $NaHCO_3$ solution comprising 5% $Na_2S_2O_3$. Another 10 milliliter aliquot of ether was added to the bi-phasic mixture, and the layers were separated. The organic layer was washed once using 10 milliliters of a saturated $NaHCO_3$ solution, and once using 10 milliliters of water. The washed organic layer was dried in the presence of anhydrous $Na_2SO_4$, filtered, and concentrated under reduced pressure. The residue obtained by this procedure was purified by flash column chromatography, eluting with a solvent mixture comprising MeOH and $CH_2Cl_2$ in a ratio of 5 to 95, respectively, to yield 0.083 gram of purified compound **26** (77% yield) in the form of a white foam. The following analytical data were obtained for compound **26**: $R_f$ 0.61 (10:90-MeOH:$CH_2Cl_2$); $^1H$ NMR (500 MHz, $CDCl_3$) $\delta$ 0.86-1.05 (m, 6H), 1.41-1.54 (m, 1H), 1.70-1.74 (m, 2H), 1.87-1.89 (m, 3H) and 2.05-2.19 (m, 1H), 2.33 (s, 3H), 2.97 (s, 2H), 3.59-3.63 (m, 2H), 3.70 (s, 3H), 4.95-4.98 (t, J=7.97Hz, 1H), 5.11-5.13 (m, 1H); HRMS *m/z* calculated for $C_{16}H_{26}N_2O_5$ ($M^+H^+$): 327.1920, found 327.1912; $[\alpha]^{20}_D$ +1.14 (c 0.49, $CHCl_3$).

Reaction R of Figure 37A: Synthesis of Compound **27**

A solution comprising 0.0678 gram (0.21 millimole) of compound **26**, 4 milliliters of distilled THF, and 4 milliliters of MeOH was cooled to 0°C. To this solution, 8 milliliters of a solution comprising 0.2 molar LiOH was added. The reaction mixture was initially maintained with stirring at 0°C for 1 hour, after which the reaction mixture was warmed to room temperature and maintained with stirring for at least an additional 8 hours. The resulting mixture was concentrated under reduced pressure, cooled to 0°C, and acidified to pH 3 by adding 1 normal $KHSO_4$. The acidified mixture was extracted three times with 5 milliliters aliquots of EtOAc. The combined EtOAc layers were dried in the presence of anhydrous $Na_2SO_4$, filtered, and concentrated under reduced pressure to yield 0.042 gram (64% yield) of compound **27** in the form of a white solid. The following analytical data were obtained for compound **27**: $^1H$ NMR (500 MHz, $CDCl_3$) $\delta$ 0.86-1.03 (m, 6H), 1.35-1.50 (m, 1H), 1.70-1.77 (m, 2H), 1.85-1.93 (m, 2H) and 2.07-2.18 (m, 2H), 2.33 (s, 3H), 3.02 (s, 3H), 3.58-3.86 (m, 2H), 4.78-4.81 (m, 1H), 5.08-5.12 (m, 1H); $^{13}C$ NMR (250 MHz, $CDCl_3$) $\delta$ 21.26 and 22.55, 23.21, 24.64, 26.36 and 28.17, 31.16, 37.09, 45.70, 56.79, 58.66, 163.32, 173.12, 174.59, 199.07.

Reaction S of Figure 37B: Synthesis of (-) Dehydrotamandarin (Compound **133**)

A solution comprising 19.7 milligrams (0.063 millimole) of compound **27**, 33.4 milligrams (0.042 millimole) of compound 22, and 0.50 milliliters of $CH_2Cl_2$ was cooled to 0°C in an ice bath. To this cooled solution was added 28 milligrams (0.063 millimole) of BOP and 0.0185 milliliters (0.17 millimole) of NMM. The reaction mixture was stirred for 30 minutes at 0°C and allowed to warm to room temperature. The reaction mixture was maintained with stirring at room temperature for at least an additional 8 hours. The resulting solution was diluted with 2 milliliters of a saturated solution of NaCl and extracted with 10 milliliters of EtOAc. The extracted organic layer was washed once using 5 milliliters of a 10% solution of HCl, once using 5 milliliters of a 5% solution of $NaHCO_3$, and once using 5 milliliters of a saturated solution of NaCl. The washed organic layer was dried in the presence of anhydrous $Na_2SO_4$, filtered, and concentrated under reduced pressure. The crude residue was purified by flash column chromatography, eluting with a combination of solvent mixtures comprising MeOH and $CH_2Cl_2$ in ratios from 2 to 98, respectively, to 10 to 90, respectively, to yield 18.1 milligrams of dehydrotamandarin (compound **13**3) in the form of a yellow-white solid. The yield of compound **133** was 41%, as calculated for both reaction O (i.e., the de-protection of compound 21 to yield compound **22** in Example 1) and reaction S. The following analytical data were obtained for dehydrotamandarin A: $R_f$ 0.48 (10:90-MeOH: $CH_2Cl_2$); $^1H$ NMR (500 MHz, $CDCl_3$) $\delta$ 0.80-1.00 (m, 24H), 1.16-1.45 (m 11H), 1.51-2.25 (m, 10H), 2.38-2.48 and 3.19-3.30 (m, 2H), 2.52-2.53 (d, J=6.2Hz, 3H), 2.57 (s, 3H), 3.04 and 3.08 (s, 3H, rotomers), 3.12-3.16 (m, 1H) and 3.31-3.35 (m, 1H), 3.53-3.72 (m, 5H), 3.77 (s, 3H), 3.81-4.03 (m, 1H), 4.05-4.10 (m, 1H), 4.25-4.26 (m, 1H), 4.61-4.65 (m, 1H), 4.68-4.71 (m, 1H), 4.85-4.88 (m, 1H), 5.00-5.01 (d, J=4.6 Hz, 1H), 5.15-5.20 (m, 1H), 5.28-5.31 (m, 1H), 6.81-6.83 (d, J=7.5 Hz, 2H), 7.05-7.07 (d, J=8.2 Hz, 2H), 7.28-7.30 (d, J=9.8 Hz, 1H) and 7.33-7.35 (d, J=10.2 Hz, 1H), 7.39-7.40 (d, J=5.5 Hz, 1H), 7.72-7.74 (d, J=9.7 Hz, 1H) and 7.78-7.80 (d, J=9.7 Hz, 1H); $^{13}C$ NMR (500 MHz, $CDCl_3$) $\delta$ 11.80, 14.11, 16.52, 17.63, 18.91, 20.87, 21.33, 22.32, 23.53, 24.89 (overlap), 24.83, 27.08, 27.35, 27.99, 29.62, 30.13, 31.00, 33.56, 34.04, 35.10, 35.84, 38.90,39.65, 46.72, 48.29, 48.81, 54.78, 55.27 (overlap), 56.91, 57.46, 58.91, 66.10, 68.92, 70.90, 78.94, 114.12, 130.00, 130.36, 158.68, 161.50, 168.43, 169.59, 170.61, 171.00, 172.26, 173.00, 174.52, 197.36; IR (KBr) 3339,2960,2927,2872,1736,1715, 1655, 1633, 1508, 1460, 1438, 1248, 1177, 1085, 1031, 830 $cm^{-1}$; HRMS *m/z* calculated for $C_{54}H_{83}N_7O_{14}Na$ ($M+Na^+$): 1076.5896, found 1076.5946; $[\alpha]^{20}_D$ - 35.29 (c 0.35, $CHCl_3$).

Example 4

Synthesis of Fluorescent Tamandarin analogs

A method of synthesizing a fluorescent tamandarin, compound **108**, is described in this example. As in Example 3, the method illustrated in this Example involves the macrocyclic compound **22**, which can be generated as described in Example 1. The method described in this Example is illustrated in Figure 38, and is initiated with synthesis of compound 206a, depicted in Figure 38A.

Reaction T of Figure 38A: Synthesis of Compound **205a**.

A solution comprising 10.00 grams (49.5 millimoles) of diethyl 1,3-acetonedicarboxylate, 7.12 grams (51.9 millimoles) of *m*-dimethylaminophenol, 8.56 grams (62.8 millimoles) of zinc chloride, and 25 milliliters of absolute ethanol was maintained at reflux for 13 hours. The reaction mixture was diluted with 50 milliliters of EtOAc and washed using 25 milliliters of $H_2O$. The aqueous layer obtained from this procedure was extracted three times with 50 milliliters aliquots of EtOAc. The combined EtOAc layers were dried in the presence of anhydrous magnesium sulfate ($MgSO_4$) filtered, and concentrated under reduced pressure. The resulting crude solid was re-crystallized from absolute ethanol to yield 2.64 grams (20% yield) of compound **205a** in the form of orange crystals. The following analytical data were obtained for compound **205a**: mp 131-132°C; $R_f$ 0.20 (acetone/hexane 30:70); $^1$H NMR (500 MHz, $CDCl_3$) δ 1.22 (3H, t, *J*=7.1 Hz), 3.01 (6H, s), 3.64 (2H, s), 4.15 (2H, q, *J*=7.2 Hz), 6.02 (1H, s), 6.48 (1H, d, *J*=2.6 Hz), 6.58 (1H, dd, $J^1$ = 2.6 Hz, $J^2$ = 8.9 Hz), 7.37 (1H, d, J = 8.9); $^{13}$C NMR (125 MHz, $CDCl_3$) δ 14.02, 38.16, 40.01, 61.49, 98.28, 108.45, 108.93, 110.63, 125.22, 148.39, 152.91, 155.89, 161.65, 169.01; IR (KBr) 2907 (w), 1720 (s), 1600 (2), 1534 (m), 1484 (m), 1427 (m), 1405 (s), 1371 (m), 1330 (m), 1232 (m) $cm^{-1}$; HRMS Calculated for $C_{15}H_{18}NO_4$ ($M^+H$): 2.76.1236, found 276.1244. Anal. Calculated for $C_{15}H_{17}NO_4$: C, 65.43; H, 6.23; N, 5.09. Found: C, 65.26; H. 6.36; N, 5.03.

Reaction U of Figure 38A: Synthesis of Compound **205b**.

A solution comprising 0.46 gram (11.0 millimoles) of lithium hydroxide hydrate ($LiOH-H_2O$) and 25 milliliters of $H_2O$ was added to a solution comprising 1.50 grams (5.5 millimoles) of compound **205a** and 10 milliliters of THF. The reaction mixture was maintained with stirring at room temperature for 2.5 hours. The resulting solution was washed with 10 milliliters of ether and acidified to pH 2. A precipitate formed upon acidification and was collected by filtration. The precipitate comprised 0.364 gram of compound **205b** in the form of yellow crystals. The following analytical data were obtained for compound **205b**: mp 166-167°C, $^1$H NMR (500 MHz, $CDCl_3$/DMSO) δ 3.01 (6H, s), 3.62 (2H, s), 6.00 (1H, s), 6.44 (1H, d, J = 2.3 Hz), 6.58 (1H, dd, $J^1$ = 2.4 Hz, $J^2$ = 8.9 Hz), 7.40 (1H, d, J = 9.0 Hz); $^{13}$C NMR (125 MHz, $CDCl_3$/DMSO) δ 38.42, 40.25, 98.34, 108.89, 109.35, 110.67, 125.84, 149.59, 153.22, 156.15, 162.00, 171.33; IR (KBr) 2923 (m), 1690 (s), 1619 (s), 1534 (m), 1404 (m), 1248 (m), 1145 (m), 1055 (m) $cm^{-1}$; HRMS Calculated for $C_{13}H_{14}NO_4$ ($M^+H$): 248.0922, found 248.0929.

Reaction V of Figure 38A: Synthesis of Compound **206a**.

A solution comprising 0.364 gram (1.47 millimoles) of compound **205b** and 5 milliliters of freshly-distilled $CH_2Cl_2$ was placed under an argon atmosphere and cooled to °C. To this solution was added 0.282 gram (1.47 millimole) of EDAC•HCl and 0.035 gram (0.29 millimole) of DMAP. The resulting mixture was stirred for 10 minutes, and 0.246 gram (1.47 millimoles) of glycine *tert*-butyl ester was added. The reaction mixture was maintained with stirring at 0°C for 2 hours, warmed to room temperature, and maintained at room temperature for at least 8 additional hours. The reaction mixture was diluted with $CH_2Cl_2$ and washed once using 10 milliliters of a 10% solution of HCl and once using 10 milliliters of a saturated NaCl solution. The combined, washed $CH_2Cl_2$ layers were dried in the presence of anhydrous $MgSO_4$, filtered, and concentrated under reduced pressure. The resulting residue was purified by flash column chromatography, eluting with a solvent mixture comprising equal amounts of EtOAc and $CH_2Cl_2$ to yield 0.250 gram (42% yield) of compound **206a** in the form of a yellow solid. The following analytical data were obtained for compound **206a**: $^1$H NMR (500 MHz, $CDCl_3$) δ 1.42 (s, 9H), 3.03 (s, 6H), 3.64 (s, 2H), 3.88 (d, J = 5.12 Hz, 2H), 6.05 (s, 1H), 6.48 (brs, 1H), 6.58 (d, J = 2.5 Hz, 1H), 6.60 (dd, $J^1$ = 2.6 Hz, $J^2$ = 8.9 Hz, 1H), 7.45 (d, J = 8.9 Hz, 1H); $^{13}$C NMR (125 MHz, $CDCl_3$) δ 168.5, 167.8, 161.6, 156.0, 153.1, 149.2, 125.6, 110.5, 109.2, 108.3, 98.3, 82.5, 42.4, 40.3, 40.1, 27.9; IR ($CHCl_3$) 3450, 1679, 1618, 1530, 1405, 1370, 1230, 1157 $cm^{-1}$; HRMS (CI) *m*/*z* calculated for $C_{19}H_{24}N_2O_5$ ($M^+$): 360.1685, Found 360.1686. Anal. Calculated for $C_{19}H_{24}N_2O_5$: C, 63.30; H, 6.72; N, 7.78. Found: C, 62.99; H, 6.60; N, 7.52.

Reaction W of Figure 38B: Synthesis of Compound **207a.**

A solution comprising 0.250 gram (0.69 millimole) of Gly-DACA-*tert*-butyl ester, compound **206a**, and $CH_2Cl_2$ was cooled to 0°C, and maintained for 10 minutes. To the cooled solution a solution comprising 10% trifluoroacetic acid (TFA) was added drop-wise. This addition was completed in 10 minutes. The resulting mixture was maintained with stirring at room temperature for at least 8 hours. The reaction mixture was evaporated to dryness using a rotary evaporator, and the resulting residue, comprising 0.033 gram (0.108 millimole) of compound **206b**, was used in the subsequent reaction without further purification.

A solution comprising 0.033 gram (0.108 millimole) of compound **206b** and $CH_2Cl_2$ was cooled to 0°C. To the cooled solution was added 0.027 gram (0.108 millimole) of BOP and 0.012 gram (0.108 millimole) ofNMM. The resulting mixture was maintained for 30 minutes with stirring. An excess of the methyl ester of *N*-methyl-D-leucine was added to the stirred mixture, after which the reaction was maintained with stirring at 0°C for at least 8 hours. The reaction mixture was diluted with $CH_2Cl_2$, and washed once using 10 milliliters of a 10% solution of HCl and once using 10 milliliters of a saturated NaCl solution. The combined, washed $CH_2Cl_2$ layers were dried in the presence of anhydrous $MgSO_4$, filtered, and concentrated under reduced pressure. The resulting residue was purified by flash column chromatography, eluting with a solvent mixture comprising equal amounts of EtOAc and $CH_2Cl_2$. Evaporation of the eluate yielded 0.030 gram (63% yield) of compound **207a** in the form of a yellow solid. The following analytical data were obtained for compound **207a**: $[\alpha]_D^{20}$ +19.08 (c 0.865, $CHCl_3$); $^1$ H NMR (500 MHz, $CDCl_3$) δ 0.87 (, J = 6.52 Hz, 3H), 0.91 (d, J = 6.79 Hz, 3H), 1.40 (m, 1H), 1.69 (m, 2H), 2.85 (s, 3H), 3.01 (s, 6H), 3.63 (s, 2H), 3.67 (s, 3H), 4.06 (m, 2H), 5.22 (dd, $J^1$ = 10.6 Hz, $J^2$ = 5.2 Hz, 1H), 6.04 (s, 1H), 6.47 (d, J = 2.5 Hz, 1H), 6.57 (dd, $J^1$ = 2. 5 Hz, $J^2$ = 8.9 Hz, 1H), 6.76 (brs, 1H), 7.42 (d, J = 8.9 Hz, 1H); $^{13}$C NMR (125 MHz, $CDCl_3$) δ 171.7, 168.6 167.8, 161.6, 155.9, 152.9, 149.1, 125.4, 110.5, 109.1, 108.4, 98.3, 54.7, 52.3, 41.8, 40.1, 39.9, 37.1, 30.0, 24.8, 23.1, 21.3 $cm^{-1}$. HRMS *m/z* calculated for $C_{23}H_{31}N_3O_6$ $(M^+Na^+)$ : 468.2111, Found 468.2133.

Reaction X of Figure 38B: Synthesis of Compound **207b**

A solution comprising 0.135 gram (0.304 millimole) of compound **207a** and THF was cooled to 0°C, and a solution comprising 0.025 gram (0.606 millimole) of $LiOH\cdot H_2O$ in 2 milliliters of water was added. This addition was completed over a period of 5 minutes. The resulting mixture was maintained with stirring at 0°C for 30 minutes and then warmed to room temperature. The reaction mixture was maintained at room temperature for 1.5 hours, and then washed twice using 4 milliliter aliquots of ether. The washed aqueous layer was evaporated to dryness under reduced pressure. The resulting residue, comprising compound **207b,** was dissolved in a solution comprising 2 milliliters of water and 4 milliliters of EtOAc, cooled to 0°C, and acidified to pH 2 by adding a solution comprising 1 normal $KHSO_4$. The separated aqueous layer formed by this procedure was washed twice with 4 milliliters aliquots of EtOAc. The combined organic layers were dried in the presence of anhydrous $MgSO_4$, filtered, and concentrated to dryness under reduced pressure. The residue resulting from this procedure comprised 0.010 gram (0.023 millimole) of compound **207b,** and was used in the subsequent reaction without purification.

Reaction Y of Figure 38C: Synthesis of Compound **107**

A solution comprising 0.010 gram (0.023 millimole) of compound **207b** and $CH_2Cl_2$ was cooled to 0°C. To this cooled solution were added 0.010 gram (0.023 millimole) of BOP and 0.010 milliliter (0.092 millimole) of NMM. The resulting mixture was maintained with stirring at 0°C for 10 minutes, and the hydrochloride salt of compound **22** (0.018 gram, 0.023 millimole) was added. The reaction was maintained at 0°C for 1 hour and at room temperature for at least an additional 8 hours. The resulting solution was diluted with 1 milliliter of brine and extracted twice using 2 milliliter aliquots of EtOAc. The organic layer formed by extraction was washed once using 1 milliliter of a 10% solution of HCl and twice using 1 milliliter aliquots of water. The washed organic layer was dried in the presence of anhydrous $MgSO_4$, filtered, and concentrated under reduced pressure. The resulting residue was purified by flash chromatography, eluting with a solvent mixture comprising acetone and hexane in a ratio of 30 to 70, respectively. Evaporation of the eluate yielded 0.008 gram (30% yield) of fluorescent compound **107**. The following analytical data were obtained for compound **107:** $[\alpha]_D^{20}$ -160.1 (c 0.3, $CHCl_3$); $^1$H NMR (500 MHz, $CDCl_3$) δ 0.73-0.93 (m, 24H), 1.07-1.74 (m, 15H), 2.04 (m, 2H), 2.14 (s, 3H), 2.15 (m, 2H), 2.31 (m, 2H), 2.53 (s, 4H), 2.84 (s, 2H), 3.03 (s, 6H), 3.17 (brd, J =10.4 Hz, 2H), 3.35 (m, 1H), 3.57 (m, 2H), 3.68 (s, 2H), 3.78 (s, 3H), 3.99 (brd, 2H), 4.08-4.12 (m, 3H), 4.55-4.56 (m, 1H), 4.78-4.80 (m, 1H), 4.82-5.01 (m, 2H), 5.13-5.14 (brd, 1H), 6.08 (s, 1H), 6.51 (s, 1H), 6.61 (d, J = 9.1 Hz, 1H), 6.83 (d, J = 8.54 Hz, 2H), 7.07 (d, J = 8.35 Hz, 2H), 7.24-7.41 (m, 3H), 7.51 (d, J = 8.90 Hz, 1H), 7.81 (d, J = 8.65 Hz, 1H); IR (KBr) 3462, 2953, 2358, 1732, 1658, 1632, 1555, 1538, 1456, $cm^{-1}$; HRMS *m/z* calculated for $C_{64}H_{92}N_8O_{16}$ $(M^+Na^+)$: 1251, 6529, Found 1251.6528.

Example 5

Synthesis of a Deoxo-Proline Side Chain Moiety and Coupling to a Didemnin Macrocycle

An amino methylene single bond was used to replace the amide bond between D-leucine and L-proline in the side chain of didemnin B. Synthetically, the amino methylene bond was prepared by reductive amination, as described (Abdel-Magid et al., 1990, Tetrahedron Lett. 31:5595-5598; Abdel-Magid et al., 1990, Synlett. 537-539).

In a first synthetic method, exhaustive methylation of Cbz-D-leucine was performed using dimethylsulfate, as illustrated in Figure 41. The Cbz group was removed using hydrogenolysis to yield dimethylated D-leucine free amine (Compound **40**). The amine was used in reductive amination without purification. Commercially available L-proline was first esterified using $SOCl_2$ in MeOH, and its amino group was subsequently protected using $Boc_2O$. After purification, the ester function was reduced to an aldehyde in two steps. $NaBH_4$ was combined with LiCl to generate $LiBH_4$ in situ, and this compound was used to reduce the ester to the corresponding alcohol. Oxidation with $SO_3$•pyridine reagent gave the aldehyde, designated compound **42**, in good yield. Reduction of the ester to the aldehyde in one step using DIBAL could be performed, but depended on the freshness of the reducing reagent and was not very reproducible. Reductive amination between the aldehyde designated compound **40** and the free amine (compound **42**) yielded compound **43**. The Boc group of compound **43** was removed using $TFA/CH_2Cl_2$. The resulting free amine was coupled with pyruvic acid using BOP to yield the protected $\Psi(CH_2NH)$ side chain designated compound **44**.

The next step was hydrolysis of the methyl ester of compound **44**. But this step was proved to be non-trivial. Perhaps owing to steric hindrance, the methyl ester was difficult to cleave using 2 equivalents of $LiOH•H_2O$ in $THF/H_2O$. When the amount of $LiOH•H_2O$ was increased to 10 equivalents, the methyl ester appeared to hydrolyze, since the mass spectrum of the reaction mixture did show the acid peak. But the acid was so hydrophilic and buried inside the excess inorganic salts, that it could not be extracted by any organic solvent. We also tried to precipitate the acid using ether but only precipitated the inorganic salts. On account of this purification problem, we decided not to use the methyl ester to protect the acid. We needed a protective group which could survive all the synthetic steps but did not require aqueous conditions for its removal. The benzyl group was found to serve this purpose well.

The synthetic procedure was modified as indicated in Figure 42. In order to be compatible with the benzyl ester, the leucine amino group was protected by Boc instead of Cbz. Selective methylation of the amino group gave the acid designated compound **46**. The free acid was benzylated to yield the desired benzyl ester, compound **47**. The Boc protecting group was removed using TFA. The resulting amine (compound **48**) was condensed with protected prolinal by reductive amination. Removal of the Boc protecting group and subsequent coupling with pyruvic acid yielded compound **50**. Hydrogenolysis was used to remove the benzyl group and yielded the desired $\Psi(CH_2NH)$ side chain, in the form of the free acid (compound 51). Coupling of the free acid side chain with a didemnin macrocycle gave the desired deoxo-proline didemnin analog designated compound 12.

A second deoxo-proline didemnin analog (designated compound **72a**) was synthesized using a similar synthetic strategy, as illustrated in Figure 43 and described in detail as follows.

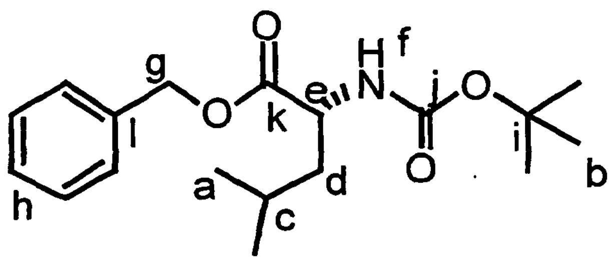

Benzyl N-Boc D-leucinate (Compound **46**).

A solution of N-Boc-D-leucine (1.0 gram, 4.1 millimoles) in 20 milliliters of DMF was cooled to 0° C. Finely powdered $Li_2CO_3$ (1.5 grams, 20.5 millimoles) was added, followed by the addition of benzyl bromide (2.43 milliliters, 20.5 millimoles). The reaction mixture was stirred for 6 hours and monitored by TLC. When the reaction was complete, the reaction mixture was diluted with $H_2O$ and extracted with EtOAc three times. The EtOAc extracts were combined and washed with brine. DMF was removed in vacuo. The crude mixture was purified with column chromatography eluting with 20% acetone/hexane to afford compound **46** in 78% yield. The following analytical data were obtained for compound **46**: $R_f$ 0.60 (40% acetone/hexane); $[\alpha]^{25}_{D}$ +16 (c=1.0, $CHCl_3$); $^1H$ NMR (500 MHz, $CDCl_3$) $\delta$ $H_a$) 0.92 (m, 6H), $H_b$)

1.48 (s, 9H), $H_c$) 1.50-1.59 (m, 1H), $H_d$) 1.60-1.69 (dd, 2H), He) 4.36 (m, 1H), $H_f$) 4.90 (d, 1H), $H_g$) 5.11-5.20 (m, 2H), $H_h$) 7.33 (m, 5H); $^{13}$C NMR (125 MHz, $CDCl_3$) $\delta$ $C_a$) 21.8, $C_{a'}$) 22.7, $C_c$) 24.7, $C_b$) 28.2, $C_d$) 41.6, $C_e$) 52.1, $C_g$) 66.7, $C_i$) 79.6, $C_h$) 128.0, 128.2, 128.4, $C_l$) 135.5, $C_j$) 155.3, $C_k$) 173.2; IR (neat) 3367 (br), 2958 (s), 1732 (s), 1715 (s), 1500 (s), 1455 (m), 1366 (w), 1120 (m) $cm^{-1}$; HRMS m/z calculated for $C_{11}H_{23}N_4O_2$ (M+H) 322.2017, found 322.2018.

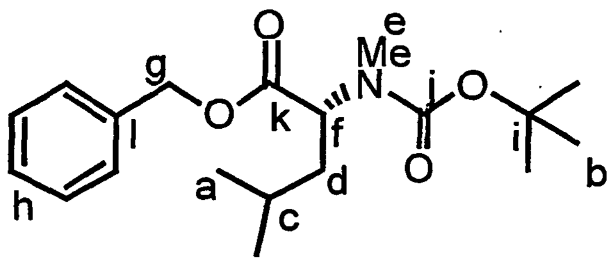

N-Boc N-Methyl benzyl D-leucinate (compound **47**).

A solution of compound **46** (1.2 grams, 3.74 millimoles) in 200 milliliters of THF was cooled to 0° C. NaHMDS (1 molar in THF; 5.6milliliters, 5.6 millimoles) was added, followed by the addition of methyl iodide (1.0 milliliter, 18.7 millimoles). The reaction mixture was stirred overnight and monitored by TLC. When the reaction was complete, the reaction mixture was diluted with ether. The organic layer was washed with 5% HCl, 5% $NaHCO_3$, and brine. The resulting solution was dried over $Na_2SO_4$ and concentrated. The crude mixture was purified by column chromatography, eluting with 20% acetone/hexane to afford compound 47 in 71% yield. The following analytical data were obtained for compound **47**: $R_f$ 0.55 (30% Acetone/Hexane); $[\alpha]^{25}_{D}$ +20.4 (c=1.2, $CHCl_3$); $^1$H NMR (500 MHz, $CDCl_3$) $\delta$ $H_a$) 0.85-0.90 (m, 6H), $H_c$) & $H_d$) 1.35-1.65 (m, 3H), $H_b$) 1.45 (s, 9H), $H_e$) 2.75 (d, 3H), $H_f$) 4.53-4.58 & 4.81-4.88 (rm, 1H), $H_g$) 5.10 (s, 2H), $H_h$) 7.15-7.28 (m, 5H); $^{13}$C NMR (125 MHz, $CDCl_3$) $\delta$ $C_a$) 22.0 and 23.5, $C_c$) 25.8, $C_b$) 29.0, $C_d$) 31.0, $C_e$) 38.0, $C_f$) 57.0, $C_g$) 66.1, $C_i$) 80.2, $C_h$) 128.0, 128.2, 128.4, $C_l$) 136.5, $C_j$) 156.3, $C_k$) 173.2; IR (neat) 2958.3 (m), 1742.7 (s), 1696.8 (s), 1455.6 (s), 1390.7 (s), 1366.6 (s), 1323.5 (s), 1151.3 (s) $cm^{-1}$; HRMS m/z calculated for $C_{11}H_{23}N_4O_2$ (M+H) 336.2174, found 336.2178.

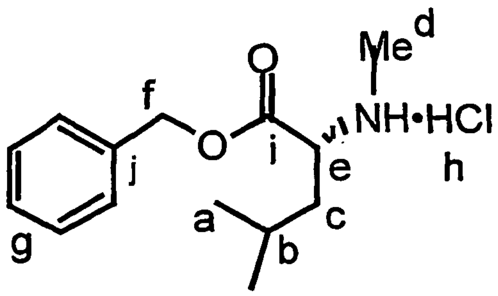

Benzyl N-Methyl D-leucinate hydrochloride salt (compound **48**).

Compound **47** (0.1 gram) was dissolved in HCl•dioxane (5 milliliters) and stirred at room temperature. When the reaction was completed, the solvent was removed in vacuo. Toluene was added twice and concentrated. The residue was dried under reduced pressure overnight to afford the desired HCl salt (compound **48**) in 98%. The following analytical data were obtained for compound **48**: $R_f$: baseline (10% MeOH/$CH_2Cl_2$); $[\alpha]^{25}_{D}$ +48.5 (c=0.2, $CHCl_3$); $^1$H NMR (500 MHz, $CDCl_3$) $\delta$ $H_a$) 0.91 (d, 6H), $H_b$) 1.75 (m, 1H) $H_c$) 1.90-1.95 (dd, 2H), $H_d$) 2.71 (s, 3H), $H_e$) 3.83 (t, 1H), $H_f$) 5.20-5.30 (rm, 2H), $H_g$) 7.35-7.40 (m, 5H), $H_h$) 9.85 & 10.15 (br, 2H); $^{13}$C NMR (125 MHz, $CDCl_3$) $\delta$ $C_a$) 21.8, and 23.4, $C_c$) 25.8, $C_b$) 31.9, $C_d$) 38.2, $C_e$) 60.1, $C_f$) 68.8, $C_g$) 128.4, 128.6, 128.8, $C_j$) 134.6, $C_i$) 168.2; IR (neat) 2958.3 (m), 1742 (s), 1696 (s), 1455 (s), 1390 (s), 1366 (s), 1323 (s), 1151 (s) $cm^{-1}$; HRMS m/z calculated for $C_{11}H_{23}N_4O_2$ (M+H) 336.2174, found

336.2178.

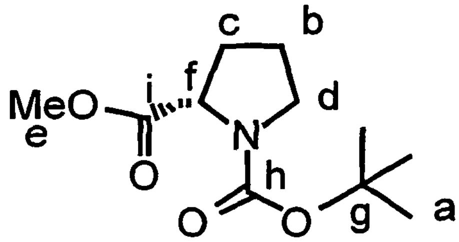

Methyl N-Boc L-Prolinate (compound **41**).

Commercially available N-Boc-L-proline (5.0 grams, 23.2 millimoles) was dissolved in acetone (200 milliliters). $K_2CO_3$ (3.8 grams, 27.84 millimoles) was added at 0° C, followed by the addition of MeI (2.9 milliliters, 46.4 millimoles). The reaction mixture was stirred overnight. The reaction was quenched with 5% $NaHCO_3$ solution and extracted with ether. The combined ether layers were washed with 5% HCl and brine, and concentrated to afford the crude product (compound **41**) in 91% yield. This compound was purified by column chromatography eluting with 20% acetone/hexane. The following analytical data were obtained for compound **41:** $R_f$ 0.4 (30% acetone/hexane); $[\alpha]_D^{25}$ -61.2 (c=0.8, $CHCl_3$); $^1H$ NMR (500 MHz, $CDCl_3$) δ $H_a$) 1.45 (s, 9H), $H_b$) 1.98 (dd, 2H) $H_c$) 1.88 & 2.22 (m, rm, 2H), $H_d$) 3.42-3.58 (m, 2H), $H_e$) 3.74 (s, 3H), $H_f$) 4.24 & 4.35 (rm, 1H); $^{13}C$ NMR (125 MHz, $CDCl_3$) δ $C_b$) 24.2, and 24.4, $C_a$) 28.3, $C_c$) 31.6 and 31.8, $C_d$) 47.3, $C_f$) 52.1, $C_e$) 59.7, $C_g$) 80.1, $C_h$) 153.8, $C_i$) 174.0; IR (neat) 2975 (m), 1749 (s), 1700 (s), 1396 (s), 1365 (s), 1200 (s), 1161 (s), 1151 (s); HRMS m/z calculated for $C_{11}H_{23}N_4O_2$ (M+H) 230.1391, found 230.1389.

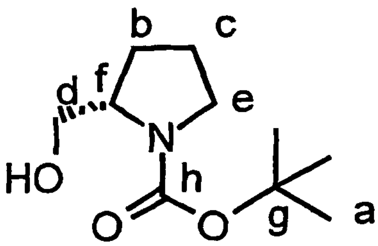

N-Boc L-Prolinol (Compound **41a**).

Compound **41** was dissolved in 200 milliliters THF/EtOH (1:1). LiCl (1.8 grams, 32.7 millimoles) and $NaBH_4$ (1.2 grams, 32.7 millimoles) was added in portions at 0°C. The reaction mixture was stirred overnight and monitored by TLC; more LiCl and $NaBH_4$ were added during the operation. When the reaction was complete, the white solid was collected and washed with ether. The solvent was removed using a rotary evaporator. The residue was neutralized to pH 4 and then extracted twice with EtOAc. The EtOAc extracts were combined, washed with brine, dried, and concentrated. The crude product was purified by column chromatography eluting with 10% acetone/hexane to afford the desired alcohol (compound **41a**) in 83% yield. The following analytical data were obtained for compound **41a:** $R_f$ 0.30 (30% Acetone/Hexane); ; $[\alpha]_D^{25}$ -60.0 (c=0.8, $CHCl_3$); $^1H$ NMR (500 MHz, $CDCl_3$) δ $H_a$) 1.48 (s, 9H), $H_b$) 1.60 & 2.05 (rm, 2H) $H_c$) 1.83-1.98 (dd, 2H), $H_d$) 3.35 & 3.45 (rm, 2H), $H_e$) 3.61-3.70 (m, 2H), $H_f$) 4.03 (m, 1H); $^{13}C$ NMR (125 MHz, $CDCl_3$) δ $C_c$) 23.8, $C_a$) 28.4, $C_b$) 28.9, $C_f$) 47.3, $C_e$) 59.9, $C_d$) 67.3, $C_g$) 80.0, $C_h$) 156.8; IR (neat) 3424 (br), 2973 (s), 2877 (s), 1695 (s), 1670 (s), 1477 (s), 1406 (s), 1366.2 (s); HRMS m/z calculated for $C_{11}H_{23}N_4O_2$ (M+H) 202.1443, found 202.1449.

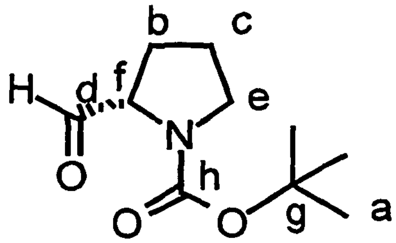

N-Boc L-prolinal (compound **42**).

A solution of compound **41a** (2.0 grams, 9.9 millimoles) and $Et_3N$ in 200 milliliters of $CH_2Cl_2$ was cooled to -78° C. $SO_3$•pyridine complex (4.7 grams, 29.7 millimoles) in DMSO (30 milliliters) was added to the previous solution. The reaction mixture was warmed to room temperature, stirred overnight, and monitored by TLC. When the reaction was complete, the reaction mixture was diluted with ether. The organic layer was washed with 5% HCl, 5% $NaHCO_3$, and brine. The resulting solution was dried over $Na_2SO_4$ and concentrated. The crude mixture was purified with a short column to afford the desired aldehyde (compound **42**) in 81 % yield. The following analytical data were obtained for compound **42**: $R_f$ 0.55 (30% Acetone/Hexane); $[\alpha]^{25}_D$ +20.4° (c=1.2, $CHCl_3$); $^1$H NMR (500 MHz, $CDCl_3$) δ $H_a$) 1.45 (s, 9H), $H_b$) 1.75-1.90 (m, 2H), $H_c$) 1.90-2.15 (m, 2H), $H_d$) 3.20-3.50 (t, 2H), $H_e$) 3.90-4.20 (d, 1H), $H_f$) 9.72 (d, 1H); $^{13}$C NMR (125 MHz, $CDCl_3$) δ $C_b$) 24.0, $C_c$) 27.3, $C_a$) 28.9, $C_d$) 47.5, $C_e$) 65.5, $C_g$) 80.1, $C_h$) 154.2, $C_f$) 200.1.

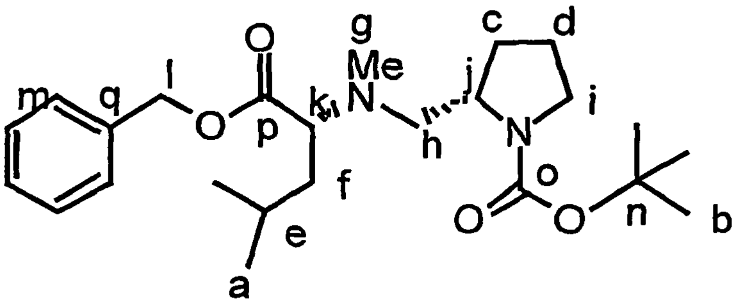

N-Boc-pro y($NHCH_2$) N-Methyl-Benzyl-D-Leucinate (compound **43**).

Compound **42** (0.1 gram, 0.5 millimoles) was dissolved in $CH_2Cl_2$ (8 milliliters), and free amine compound **48** (0.15 gram, 0.55 millimoles) was added with efficient stirring. At 0°C, AcOH (0.02 milliliters, 0.3 millimoles) was added as a catalyst and stirred for 10 minutes before $NaBH(OAc)_3$ (0.13 gram, 0.6 millimoles) was added to the reaction mixture. The reaction mixture was stirred at room temperature and monitored by TLC. When the reaction was complete, the mixture was diluted with $CH_2Cl_2$. The excess reagent was quenched by dropwise addition of saturated $NH_4Cl$ solution. The organic layer was washed with 5% HCl, 5% $NaHCO_3$, and brine, dried, and concentrated. The crude product was purified by flash chromatography, eluting with 20% EtOAc/petroleum ether to afford the desired amine (compound **43**). The following analytical data were obtained for compound **43**: $R_f$0.50 (30% Acetone/Hexane); $[\alpha]^{25}_D$ -44.0 (c=1.1, $CHCl_3$); $^1$H NMR (500 MHz, $CDCl_3$) δ $H_a$) 0.92 (m, 6H), $H_b$) 1.45 (s, 9H), $H_c$), $H_d$) & $H_e$) 1.50-1.90 (m, 5H), $H_f$) & $H_g$) 2.3-2.45 (m, 5H), $H_{h1}$) 2.22-2.90 (m, 1H), $H_{h2}$) & Hi) 3.25-3.4 (m, 3H), $H_j$) & $H_k$) 3.70-3.95 (m, 2H), $H_l$) 5.35 (m, 5H), $H_m$) 7.30 (m, 5H); $^{13}$C NMR (125 MHz, $CDCl_3$) δ $C_a$) 22.2, $C_d$) 23.4, $C_c$) 25.6, $C_b$) 28.4, $C_e$) 28.8, $C_g$) 36.6, $C_f$) 38.4, $C_h$) 46.2, $C_i$) 56.1, $C_j$) 58.5, $C_l$) 56.2, $C_k$) 66.0, $C_n$) 79.6, $C_m$) 128.2, $C_q$) 136.2, $C_o$) 154.1, $C_p$) 174.2; IR (neat) 2955 (s), 2868 (s), 1730 (s), 1693 (s), 1455 (s), 1392 (s), 1364 (s), 1170 (s) $cm^{-1}$; HRMS m/z calculated for $C_{24}H_{38}N_2O_4$ (M+H) 419.2910 , found 419.2897.

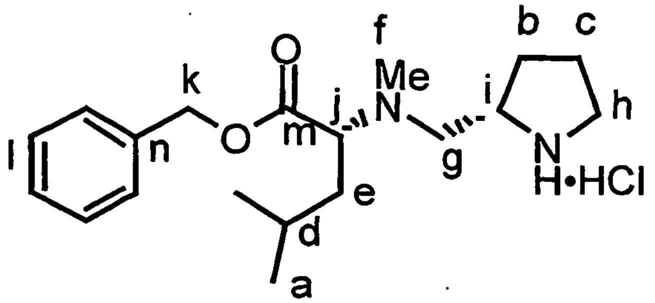

L-Pro Ψ($NHCH_2$) N-Methyl-Benzyl-D-Leucinate Hydrochloride salt (compound **44**).

Compound **43** (0.1 gram) was dissolved in HCl•dioxane (5 milliliters), and the solution was stirred at room temperature. When the reaction was completed, all solvent was removed in vacuo. Toluene was added twice and concentrated. The residue was dried under reduced pressure overnight to afford the desired HCl salt (compound **44**) in 98% yield. The following analytical data were obtained for compound **44**: $R_f$ baseline (60% EA/PE); $[\alpha]_D^{25}$ +15.3 (c=0.6, $CHCl_3$); $^1H$ NMR (500 MHz, $CDCl_3$) δ $H_a$) 0.91-1.10 (d, 6H), $H_b$), $H_c$) & $H_d$) 1.68-2.30 (m, 5H), $H_e$) 2.83 (dd, 2H), $H_f$) 3.12 (s, 3H), $H_g$) & $H_h$) 3.31-3.65 (m, 4H), $H_i$) & Hj) 4.11-4.30 (m, 2H), $H_k$) 5.28 (s, 2H), $H_k$) 7.40 (m, 5H); $^{13}C$ NMR (125 MHz, $CDCl_3$) δ $C_c$) 20.8, $C_b$) 23.5, $C_a$) 25.9, $C_d$) 26.1, $C_e$) 36.2, $C_f$) 46.2, $C_g$) 55.8, $C_h$) 61.6, $C_i$) 64.2, $C_j$) 67.0, $C_k$) 68.2, $C_l$) 125.0, 128.0, 128.8, 128.9 & 134.1, $C_n$) 137.9, $C_m$) 167.0; IR (neat) 2954 (s), 2360 (s), 2338 (s), 1740 (s), 1455 (s), 1389 (s), 1197 (s), 1141 (s); $cm^{-1}$; HRMS m/z calculated for $C_{19}H_{30}N_2O_2$ (M+H) 319.2386 , found 319.2392.

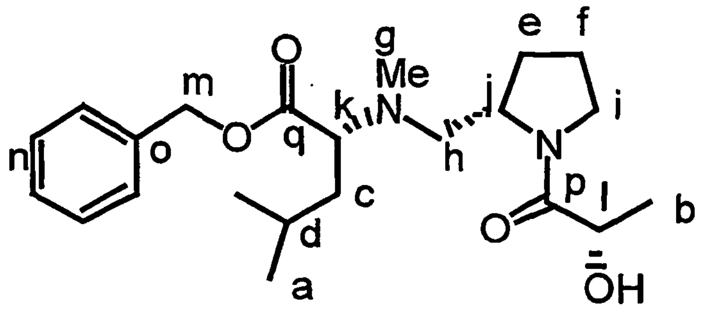

L-Lactyl-Pro Ψ($NHCH_2$) N-Methyl-Benzyl-D-Leucinate (compound **50a**).

Compound **44** (20 milligrams, 0.063 millimoles) was dissolved in $CH_2Cl_2$ (0.5 milliliters), and lactic acid (5.55 milligrams, 0.063 millimoles) was added at 0°C, followed by the addition of BOP (28 milligrams, 0.063 millimoles and NMM (0.035 milliliters, 0.31 millimoles). The reaction mixture was stirred at 0°C and monitored by TLC. When the reaction was completed, the reaction mixture was diluted with ether. The organic layer was washed with 5% HCl, 5% $NaHCO_3$, and brine. The resulting solution was dried over $Na_2SO_4$ and concentrated. The crude mixture was purified by column chromatography, eluting with 30% acetone/hexane to afford compound **50a** in 61% yield. The following analytical data were obtained for compound **50a**: $R_f$ 0.50 (50% Acetone/Hexane); $[\alpha]_D^{25}$ +21.0 (c=0.2, $CHCl_3$); $^1H$ NMR (500 MHz, $CDCl_3$) δ $H_a$) 0.82-0.92 (d, 6H), $H_b$) 1.20 (d, 3H), $H_c$) 1.45-1.66 (m, 2H), $H_d$) 1.66-1.72 (m, 1H), $H_e$) & $H_f$) 1.76-1.90 (m, 4H), $H_g$) & $H_h$) 2.25-2.45 (m, 5H), $H_i$), Hj) & $H_k$) 3.19-3.85 (m, 4H), $H_l$) 4.10-4.21 (rm, 1H), $H_m$) 5.05-5.18 (s, 2H), $H_n$) 7.12-7.38 (m, 5H); $^{13}C$ NMR (125 MHz, $CDCl_3$) δ $C_a$) 22.0, $C_c$) 22.8, $C_d$) 24.2, $C_e$) 24.8, $C_b$) 25.4, $C_f$) 28.1, $C_g$) 37.8, $C_h$) 38.6, $C_j$) 45.8, $C_i$) 46.2, $C_k$) 56.0, $C_l$) 56.8, $C_m$) 66.0, $C_n$) 128.8, $C_o$) 136.1, $C_p$) 173.8, $C_q$) 174.1; IR (neat), 3415 (br), 2955 (s), 2869 (m), 1729 (s), 1638 (s), 1455 (m), 1379 (m), 1366 (m), 1147 (m), 1126 (m); HRMS m/z calculated for $C_{22}H_{34}N_2O_4$ (M+Na) 413.2416 , found 413.2423.

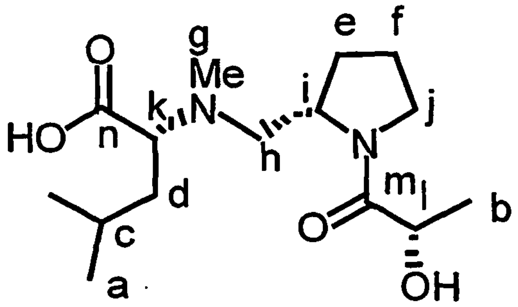

N-Methyl-leu Ψ($NHCH_2$) lac-pro acid (compound **51a**).

Compound **50a** (20 milligrams, 0.063 millimoles) was dissolved in 0.5 milliliters MeOH/EtOAc (1:1). The mixture was added to a solution of MeOH and EtOH (1:1) containing Pd/C catalyst (10 milligrams). The reaction mixture was shaken in a Parr hydrogenator under $H_2$ (40 pounds per square inch, gauge) and monitored by TLC. When the reaction was completed, the catalyst was removed by filtration. The remaining solution was concentrated in vacuo. The crude product (compound **51a**) was used directly in the next step. The following analytical data were obtained for compound **51a**: $R_f$ 0.20 (10% MeOH/$CH_2Cl_2$); $[\alpha]^{25}_D$ +65.0 (c=0.2, $CHCl_3$); $^1H$ NMR (500 MHz, $CDCl_3$) δ $H_a$) 0.82-0.92 (d, 6H), $H_b$) 1.34 (d, 3H), $H_c$) 1.45-1.55 (m, 1H), $H_d$) 1.78-1.91 (m, 2H), $H_e$) 1.91-2.0 (m, 2H), $H_f$) 2.0-2.18 (m, 2H), $H_g$) 2.81 (s, 3H), $H_h$) 3.03-3.18 (d, 2H), $H_i$) 3.45-3.54 (m, 1H), $H_j$) 3.56-3.68 (t, 2H), $H_k$) 4.25-4.35 (m, 1H), $H_l$) 4.40-4.49 (m, 1H); $^{13}C$ NMR (125 MHz, $CDCl_3$) δ $C_a$) 21.0, $C_b$) 22.8, $C_c$) 23.4, $C_d$) 24.8, $C_e$) 25.8, $C_f$) 29.4, $C_g$) 36.5, $C_h$) 38.6, $C_i$) 47.2, $C_j$) 55.2, $C_k$) 66.4, $C_l$) 68.2, $C_m$) 172.4, $C_n$) 176.2; IR (neat), 3336 (br), 2957 (s), 2870 (m), 1718 (m), 1627 (s), 1466 (s), 1368 (s), 1250 (m), 1197 (w), 1128 (w); HRMS m/z calculated for $C_{15}H_{27}N_2O_4$ (M+Na) 323.1947, found 323.1939.

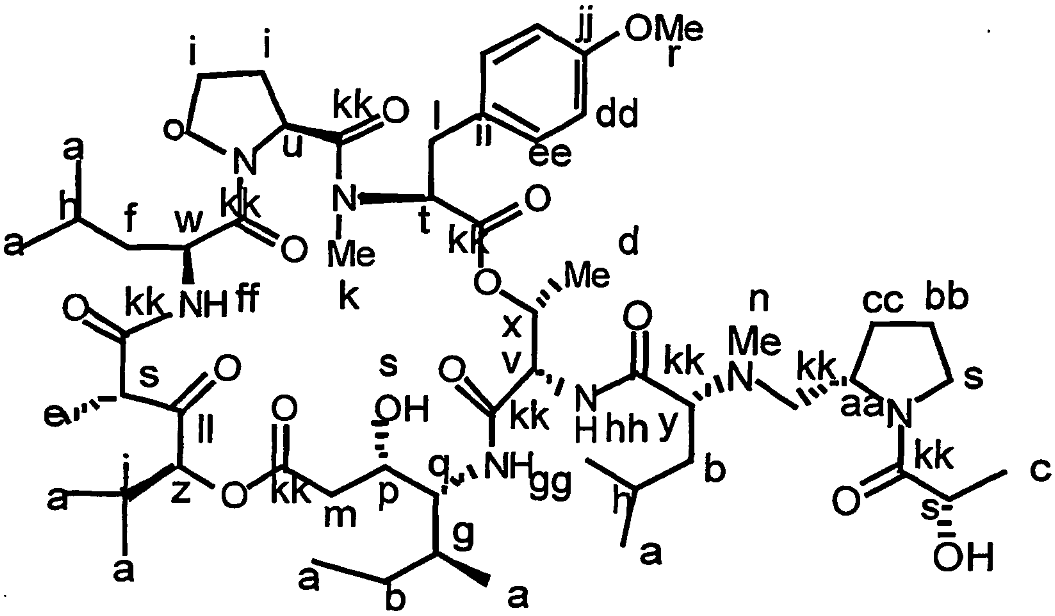

Cyclo[N-(N-3,4-didehydro-L-prolyl-N-methyl-D-leucyl)-O[[N-[(2S,3 S,4S)-4-[(3S, 4R,5S)-4-amino-3-hydroxy-5-methyl-heptanoyl]oxy-3-oxo-2,5-dimethylhexanoyl] -L-leucyl]-L-prolyl-N,O-dimethyl-L-tyrosyl]-L-threonyl] (compound **72a**).

The crude acid (12.5 milligrams, 0.038 millimoles) was combined with the macrocycle HCl salt (15.0 milligrams, 0.019 millimole) in $CH_2Cl_2$ (0.25 milliliter) at 0° C. HATU (8.2 milligrams, 0.020 millimole) and DIEA (0.026 milliliter, 4 equivalents) was added. The reaction was stirred at 0°C overnight and monitored by TLC. When the reaction was complete, the mixture was diluted with $Et_2O$ and the organic layer was washed with 5% HCl, 5% $NaHCO_3$, and brine.

The resulting solution was dried over $Na_2SO_4$ and concentrated. The crude residue was purified by column chromatography eluting with 5% $MeOH/CH_2Cl_2$ to yield the desired product (compound **72a**) in 72% yield. The following analytical data were obtained for compound **72a:** $R_f$ 0.40 (10% $MeOH/CH_2Cl_2$); $[\alpha]^{25}_{D}$ +81.2 (c=0.15, $CHCl_3$); $^1H$ NMR (500 MHz, $CDCl_3$) δ $H_a$) 0.85-0.97 (m, 24H), $H_b$) 1.17-1.27 (m, 2H), $H_c$) 1.28-1.39 (s, 3H), $H_d$) 1.40 (d, 3H), $H_e$) 1.43 (d, 3H), $H_f$) 1.46-1.50 (m, 1H) and 1.61 (t, 1H), $H_g$) 1.68-1.73 (m, 1H), $H_h$) 1.74-1.79 (m, 1H), $H_i$) 1.82-1.88 (m, 2H), 2.02-2.08 (m, 1H) and 2.11-2.17 (m, 2H), $H_j$) 2.34-2.37 (m, 1H), $H_k$) 2.57 (s, 3H), $H_l$) 2.38 (d, 2H), $H_m$) 3.18 and 3.39 (dd, 1H), $H_n$ 2.83 (s, 3H), $H_o$) 3.37-3.57 (m, 2H), $H_p$) 3.60 (d, 1H), $H_q$) 3.70-3.73 (m, 1H), $H_r$) 3.80 (s, 3H), $H_s$) 4.05-4.14 (m, 3H) and 4.36-4.48 (m, 2H), $H_t$) 4.27 (q, 1H), $H_u$) 4.56 (dd, 1H), $H_v$) 4.65 (dd, 1H), $H_w$) 4.81 (t, 1H), $H_x$) 5.19 (d, 1H), $H_y$) 5.35 (dd, 1H), $H_z$) 5.42 (dd, 1H), $H_{aa}$) 5.61 (dd, 1H), $H_{bb}$) 5.73-5.75 (m, 1H), $H_{cc}$) 6.11 (dd, 1H), $H_{dd}$) 6.85 (d, 2H), $H_{ee}$) 7.08 (d, 2H), $H_{ff}$) 7.20 (d, 1H), $H_{gg}$) 7.54 (d, 1H), $H_{hh}$) 7.78 (d, 1H); $^{13}C$ NMR (125 MHz, $CDCl_3$) δ $C_a$), $C_c$)11.7, 14.7, 15.2, 16.3, 16.9, 18.6, 20.1 and 20.9, $C_d$) 23.3, $C_e$) 23.7, $C_b$) 24.8 and 27.2, $C_i$) 24.9, 25.0 and 33.9, $C_f$) 27.9, $C_g$) 31.2, $C_h$) 31.3, $C_j$) 33.9, $C_l$) 36.2, $C_m$) 38.7, $C_k$) 38.8, $C_n$) 41.3, $C_o$) 46.9, $C_q$) 49.46, $C_s$) 49.52, 53.0, and 67.9, $C_r$) 55.3, $C_v$) 55.4, $C_w$) 55.5, $C_u$) 57.2, $C_y$) 57.6, $C_t$) 63.9, $C_{aa}$) 65.9, $C_s$) 66.4, $C_p$) 66.5, $C_x$) 70.4, $C_z$) 81.5, $C_{dd}$) 114.1, $C_{cc}$) 124.0, $C_{bb}$) 129.1, $C_{ii}$) 130.0, $C_{ee}$) 130.3, $C_{jj}$) 158.6, $C_{kk}$) 168.6, 169.3, 170.5, 170.6, 171.3, 171.5, 172.4, 173.9, $C_{ll}$) 204.9; IR (neat) 3331 (br), 2956 (s), 2871 (m), 1732 (s), 1638.3 (br, overlap), 1543 (m), 1513 (s), 1448 (m), 1379 (m), 1247 (w), 1167 (w) $cm^{-1}$; HRMS m/z calculated for $C_{57}H_{91}N_7O_{14}$ (M+H) 1098.6702, found 1098.6726.

Example 6

Synthesis of a 3,4-Dehydro-Proline Side Chain Moiety and Coupling to a Didemnin Macrocycle

Synthesis of the 3,4-dehydroproline unit began with trans-4-hydroxy-L-proline (compound **52**), which was produced as described (Rueger et al., 1982, Can. J. Chem. 60:2918; see Figure 44). The acid was first protected as its ethyl ester. The amino group was further protected using $Boc_2O$ to yield compound **53**. The hydroxyl group was mesylated using MsCl and pyridine. The mesylate (i.e., methyl sulfonate moiety of compound **53**) was displaced by sodium benzene selenide with inversion of stereochemistry to yield compound **54**. Oxidative elimination of the phenyl selenium group afforded the corresponding alkene (compound **55**) in 73% yield.

If compound **54** was directly exposed to the basic elimination condition, two regioisomers would be generated. But oxidative elimination of compound **54** through phenyl selenium as the intermediate gave only the desired regioisomer. This regioselectivity may be due to the fact that the transition state leading to the undesired isomer has larger dipole moment with higher energy.

Hydrolysis of the ethyl ester (compound 55) yielded the free dehydroproline acid (compound **56**), which was coupled with D-leucine ester to yield compound **57**. Hydrolysis of the methyl ester afforded the free acid, which was coupled to the didemnin macrocycle salt using HATU to afford compound **58**. The Boc protecting group was removed using HCl gas, and the HCl salt was neutralized using saturated $NaHCO_3$ solution to afford the final analog, compound **59**.

The steps in this synthesis are now described in greater detail.

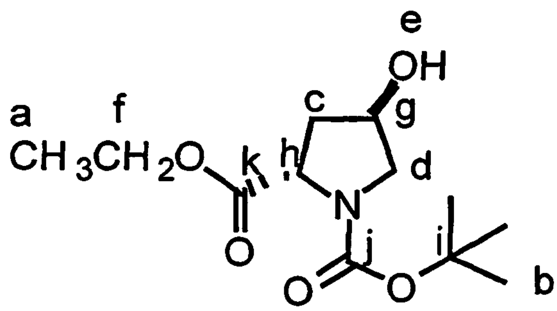

Ethyl N-Boc-trans-4-Hydroxyprolinate (compound **53**). Ethyl trans-4-hydroxy prolinate hydrochloride salt (1.0 gram, 0.005 mole) was dissolved in saturated $CH_2Cl_2$ (10 milliliter). $Et_3N$ (2.09 milliliter, 0.015 mole) was added at 0°C, followed by addition of $Boc_2O$ (2.23 g, 0.01 mole). The reaction mixture was stirred overnight. When the reaction was complete, the pH was measured, and was then 8. The reaction mixture was washed with ether, and the ether layer was discarded. The aqueous layer was acidified with 1 normal $KHSO_4$ to pH 4, followed by extraction three times with ethyl

acetate. The organic extracts were combined, washed with brine, dried, and concentrated. The crude mixture was purified by column chromatography, eluting with 20% acetone/hexane to afford the desired product (compound **53**) in 71% yield.

The following analytical data were obtained for compound **53**: $R_f$ 0.50 (40% Acetone/Hexane); $[\alpha]_D^{25}$ +71.3 (c=0.2, $CHCl_3$); $^1H$ NMR (500 MHz, $CDCl_3$) δ $H_a$) 1.07-1.13 (t, 6H), $H_b$) 1.32-1.36 (m, 9H), $H_c$) 1.84-2.15 (m, dr, 2H), $H_d$) 3.23-3.49 (m, dr, 2H), $H_e$) 3.78-3.95 (br, 1H), $H_f$) 3.96-4.09 (m, 2H), $H_g$) 4.17-4.26 (m, 1H), $H_h$) 4.27-4.32 (t, 1H); $^{13}C$ NMR (125 MHz, $CDCl_3$) δ $C_a$) 14.0, $C_b$) 28.1, $C_c$) 38.2, $C_d$) 54.2, $C_f$) 57.7, $C_g$) 61.0, $C_h$) 69.0, $C_i$) 80.0, $C_j$) 153.9, $C_k$) 172.6; IR (neat) 3448 (br), 2978 (s), 2935 (m), 1746 (m), 1702 (s), 1676 (s), 1477 (m), 1402(s), 1367 (m), 1339 (m) $cm^{-1}$; HRMS m/z calculated for $C_{11}H_{23}N_4O_2$ (M+H) 260.1497, found 260.1503.

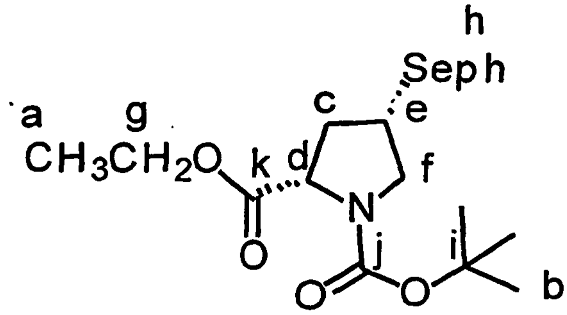

Ethyl N-Boc-cis-4-phenylselenyl-L-prolinate (compound **54**). Sodium borohydride (0.15 gram, 0.004 mole) was added in small portions, at room temperature, to a solution of diphenyl diselenide (0.556 gram, 0.0018 mole) in EtOH. The mixture was stirred for about 5 minutes, until the bright yellow color disappeared. The previously prepared mesylate (1.0 gram, 0.003 mole) was added, the solution was refluxed for 2 hours, and the solvent was removed in vacuo. The residue was diluted with $Et_2O$ (5 milliliters), and the organic layer was washed with $H_2O$ (10 milliliters) and brine. The resulting organic layer was dried and concentrated. The crude oil was purified by column chromatography, eluting with 10% acetone/hexane to afford the product (compound **54**) in 85% yield. The following analytical data were obtained for compound **54:** $R_f$ 0.53 (30% Acetone/Hexane); $[\alpha]_D^{25}$ -16.4 (c=0.3, $CHCl_3$); $^1H$ NMR (500 MHz, $CDCl_3$) δ $H_a$) 1.21-1.24 (t, 3H), $H_b$) 1.45 (s, 9H), $H_c$) 2.05 & 2.68 (dr, 2H), $H_d$) 3.40 (m, 1H), $H_e$) 3.58 (m, 1H), $H_{f1}$) 3.95 (m, 1H), $H_{f2}$) & $H_g$) 4.10-4.28 (m, 2H), $H_h$) 7.25, 7.55 (m, 5H); $^{13}C$ NMR (125 MHz, $CDCl_3$) δ $C_a$) 14.0, $C_b$) 28.2, $C_c$) 36.1, $C_d$) 39.8, $C_e$) 53.6, $C_f$) 58.8, $C_g$) 60.8, $C_i$) 80.2, $C_h$) 127.0, 128.2, 134.3, $C_j$) 152.8, $C_k$) 171.8; IR (neat) 2977.0, 1747.1, 1701.9, 1477.4, 1394.4, 1190.1, 1114.1 (m) $cm^{-1}$; HRMS m/z calculated for $C_{18}H_{25}NO_4Se$ (M+H) 399.0948, found 399.0957.

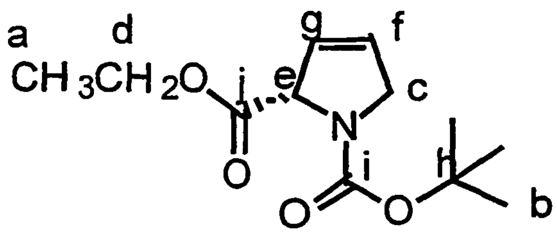

Ethyl N-Boc-3,4-Dehydro-L-prolinate (compound **55**). A mixture of compound **54** (0.9 gram, 2.26 millimoles) and $CH_2Cl_2$ was initially cooled to 0°C in an ice bath. Pyridine (0.27 milliliter, 3.4 millimoles) was added dropwise to this solution. A solution of 30% aqueous $H_2O_2$ (0.58 milliliter) was then gradually added over a 5 minute period. The reaction was stirred at room temperature for 1 hour, and then diluted with EtOAc. The organic layer was washed with 5% HCl, saturated $Na_2CO_3$ solution, and brine. The resulting solution was dried and concentrated. The residue was purified by column chromatography to afford the desired product (compound **55**) in 73% yield. The following analytical data were obtained for compound **55**: $R_f$ 0.63 (30% Acetone/Hexane); $[\alpha]_D^{25}$ -32.3 (c=0.3, $CHCl_3$); $^1H$ NMR (500 MHz, $CDCl_3$)

δ $H_a$) 1.15-1.30 (t, 3H), $H_b$) 1.45 (s, 9H), $H_c$) & $H_d$) 4.15-4.30 (m, 4H), $H_e$) 4.98 (d, 1H), $H_f$) 5.75 (dt, 1H), $H_g$) 5.95 (dd, 1H); $^{13}C$ NMR (125 MHz, $CDCl_3$) δ $C_a$) 14.1, $C_b$) 29.2, $C_c$) 53.8, $C_d$) 61.8, $C_e$) 67.0, $C_b$) 80.1, $C_f$) 125.0, $C_g$) 129.2, $C_i$) 154.0, $C_j$) 170.2; IR (neat) 3458 (br), 2979 (s), 1786 (s), 1750 (s), 1710 (s), 1448 (m), 1395 (m), 1369 (s), 1318 (s), 1258 (m), 1159 (s), 1896 (m) $cm^{-1}$; HRMS m/z calculated for $C_{12}H_{19}NO_4$ (M+H) 242.1392, found 242.1386.

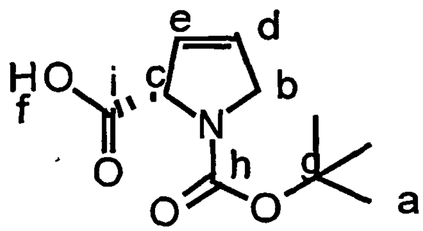

N-Boc-3,4-Dehydro-L-proline (compound **56**). Ethyl ester 55 (0.18 gram, 0.8 millimole) was dissolved in THF/$H_2O$ (1:1, 10 milliliters). LiOH•$H_2O$ (0.33 gram, 8 millimoles) was added to the solution at 0°C. The mixture was stirred for about 6 hours and monitored by TLC. When the reaction was completed, THF was removed in vacuo. Saturated $NaHCO_3$ solution was added and washed with ether twice. All aqueous layers were combined and acidified to pH 4 with 1 normal $KHSO_4$. Ethyl acetate was used to extract the acidified aqueous solution three times. The extracts were dried and concentrated to afford the desired acid (compound **56**) in 92% yield. The following analytical data were obtained for compound **56**: $R_f$ 0.20 (30% acetone/hexane); $[\alpha]_D^{25}$ +8.4 (c=0.2, $CHCl_3$); $^1H$ NMR (500 MHz, $CDCl_3$) δ $H_a$) 1.49 (s, rm, 9H), $H_b$) 4.25 (d, 2H), $H_c$) 5.05 (d, 1H), $H_d$) 5.80 (dt, 1H), $H_e$) 6.01 (dd, 1H), $H_f$) 8.40 (br, 1H); $^{13}C$ NMR (125 MHz, $CDCl_3$) δ $C_a$) 28.9, $C_b$) 53.9, $C_c$) 66.2, $C_g$) 81.8, $C_d$) 124.2, $C_e$) 129.9, $C_h$) 154.5, $C_i$) 165.3; IR (neat) 3000-3400 (br), 2957 (s), 1736 (s), 1704 (s), 1666 (s), 1400.2 (s), 1367 (m), 1177 (s), 1136 (s) $cm^{-1}$; HRMS m/z calculated for $C_{10}H_{15}NO_4$ (M+H) 213.1079, found 242.1086.

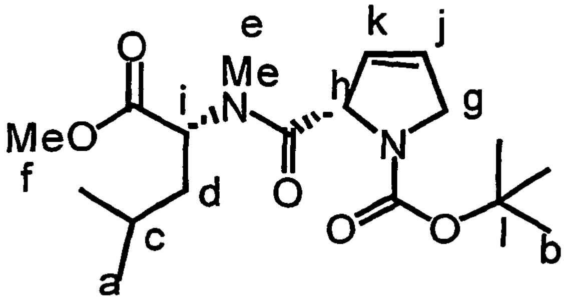

N-Boc-3,4-Dehydro-L-Prolyl-Methyl-N-Methyl-Leucinate (compound **57**). Compound **56** (0.1 gram, 0.469 millimole) was dissolved in $CH_2Cl_2$ (5 milliliters). At 0°C, HATU (0.26 gram, 0.58 millimole) was added, followed by the addition of DIEA (0.26 milliliter, 1.88 millimoles). Finally, dimethyl D-leucine hydrochloride salt (0.09 gram, 0.469 millimole) was added to the mixture. The reaction mixture was stirred for 6 hours and monitored by TLC. When the reaction was completed, the mixture was diluted with $Et_2O$ and the organic layer was washed with 5% HCl, 5% NaHCO3, and brine. The resulting solution was dried and concentrated. The crude residue was purified by column to afford the desired product (compound **57**) in 76% yield. The following analytical data were obtained for compound **57**: $R_f$ 0.32 (30% Acetone/Hexane); $[\alpha]_D^{25}$ -22.7 (c=0.4, $CHCl_3$); $^1H$ NMR (500 MHz, $CDCl_3$) δ $H_a$) 0.90-1.05 (d, rm, 6H), $H_b$) 1.48 (s, rm, 9H), $H_c$) & $H_d$) 1.65-1.80 (m, 3H), He) 3.05 (s, 3H), $H_f$) 3.75 (s, 3H), $H_g$) 4.20-4.35 (m, 2H), $H_h$) 5.28 (t, 1H), $H_i$) 5.40 (d, 1H), Hj) 5.70 (dt, 1H), $H_k$) 6.01 (dd, 1H); $^{13}C$ NMR (125 MHz, $CDCl_3$) δ $C_a$) 22.0,23.8, $C_c$) 25.2, $C_b$) 29.0, $C_d$) 37.8, $C_e$) 52.2, $C_h$) 53.8, $C_i$) 65.8, $C_l$) 80.0, $C_j$) 124.0, $C_k$) 129.2, $C_m$) 159.5, $C_n$) 171.0, $C_o$) 172.2; IR (neat) 2956 (s), 1743 (s), 1705 (s), 1667 (s), 1400 (s), 1366 (m), 1316 (w), 1258 (w), 1177 (m), 1128 (m) $cm^{-1}$; HRMS m/z calculated for $C_{18}H_{30}N_2O_5$ (M+H) 355.2233, found 355.2225.

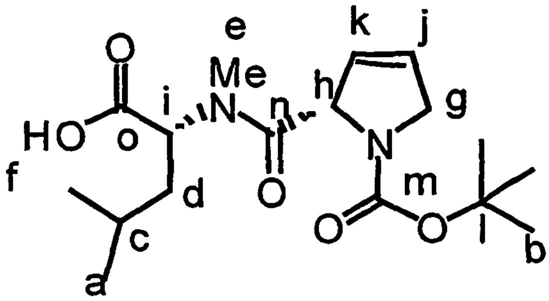

N-Boc-3,4-Dehydro-L-Prolyl-N-Methyl-Leucine (compound **57a**). Compound **57** (0.13 gram, 0.37 millimole) was dissolved in THF/$H_2O$ (1:1, 5 milliliters). LiOH•$H_2O$ (0.15 gram, 3.7 millimoles) was added to the solution at 0°C. The mixture was stirred for about 6 hours and monitored by TLC. When the reaction was completed, THF was removed in vacuo. Saturated $NaHCO_3$ solution was added and washed with ether twice. All aqueous layers were combined and acidified to pH 4 with 1 normal $KHSO_4$. Ethyl acetate was used to extract the acidified aqueous solution three times. The extracts were dried and concentrated to afford the desired acid (compound **57a**) in 92% yield. The following analytical data were obtained for compound **57a:** $R_f$ 0.20 (40% Acetone/Hexane); $[\alpha]_D^{25}$ +52.3 (c=0.2, $CHCl_3$); $^1H$ NMR (500 MHz, $CDCl_3$) δ $H_a$) 0.85-1.05 (d, rm, 6H), $H_b$) 1.48 (s, rm, 9H), $H_c$) & $H_d$) 1.65-1.92 (m, 3H), $H_e$) 3.12 (s, rm, 3H), $H_f$) br, 1H, $H_g$) 4.10-4.28 (m, 2H), $H_h$) 5.15 (t, 1H), $H_i$) 5.35 (d, 1H), $H_j$) 5.70 (dt, 1H), $H_k$) 6.01 (dd, 1H); $^{13}C$ NMR (125 MHz, $CDCl_3$) δ $C_a$) 23.3, 24.5, $C_c$) 25.0, $C_b$) 28.3, $C_d$) 31.5, $C_g$) 38.5, $C_e$) 53.6, $C_i$) 55.8, $C_h$) 65.2, $C_l$) 80.0, $C_j$) 124.1, $C_k$) 129.5, $C_m$) 153.8, $C_n$) 171.3, $C_o$) 175.3; IR (neat) 3300 (br), 2957 (s), 1731 (m), 1783 (s), 1667 (s), 1612 (m), 1481 (m), 1400 (s), 1367 (m), 1177 (s), 1136.7 (s) $cm^{-1}$; HRMS m/z calculated for $C_{17}H_{28}N_2O_5$ (M+H) 341.2076, found 341.2063.

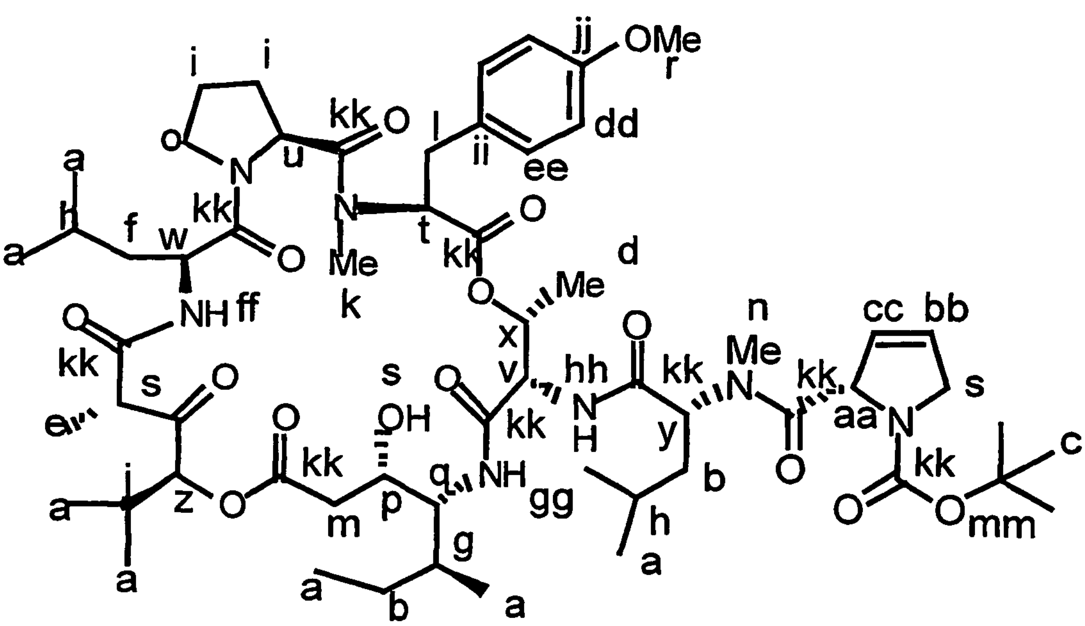

Cyclo[N-(N-Boc-3,4-dehydro-L-prolyl-N-methyl-D-leucyl)-O[[N-[(2S,3S,4S)-4[(3S,4R,5S)-4-amino-3-hydroxy-5-methyl-heptanoyl]oxy-3-oxo-2,5-dimethylhexanoyl]-L-leucyl]-L-prolyl-N,O-dimethyl-L-tyrosyl]-L-threonyl] (compound **58**). The crude acid **57a** (5.0 milligrams, 0.012 millimole) was combined with the macrocycle HCl salt (14.9 milligrams, 0.012 millimole) in $CH_2Cl_2$ (0.1 milliliter) at 0°C. HATU (48 milligrams, 0.012 millimole) and DIEA (0.048 milliliter, 0.048 millimole) was added. The reaction was stirred at 0°C overnight and monitored by TLC. When the reaction was complete, the mixture was diluted with $Et_2O$ and the organic layer was washed with 5% HCl, 5% $NaHCO_3$, and brine. The resulting solution was dried and concentrated. The crude residue was purified by column chromatography, eluting with 5% MeOH/$CH_2Cl_2$ to afford the desired product (compound **58**) in 72% yield. The following analytical data were obtained for

compound **58**: $R_f$ 0.40 (10% MeOH/$CH_2Cl_2$); $[\alpha]_D^{25}$ -83.2 (c=0.3, $CHCl_3$); $^1H$ NMR (500 MHz, $CDCl_3$) δ $H_a$) 0.85-0.97 (m, 24H), $H_b$) 1.17-1.27 (m, 3H), $H_c$) 1.45 (s, 9H), $H_d$) 1.40 (d, 3H), $H_e$) 1.43 (d, 3H), $H_f$) 1.46-1.50 (m, 1H) and 1.61 (t, 1H), $H_g$) 1.68-1.73 (m, 1H), $H_h$) 1.74-1.79 (m, 1H), $H_i$) 1.82-1.88 (m, 2H), 2.02-2.08 (m, 1H) and 2.11-2.17 (m, 2H), $H_j$) 2.34-2.37 (m, 1H), $H_k$) 2.57 (s, 3H), $H_l$) 2.63 (dd, 1H) and 2.95 (d, 1H), $H_m$) 3.18 and 3.39 (dd, 1H), $H_n$) 3.23 (s, 3H), $H_o$) 3.57-3.61 (m, 2H), $H_p$) 3.33 (d, 1H), $H_q$) 3.69-3.71 (m, 1H), $H_r$) 3.80 (s, 3H), $H_s$) 4.05-4.14 (m, 2H) and 4.36-4.48 (m, 2H), $H_t$) 4.27 (q, 1H), $H_u$) 4.56 (dd, 1H), $H_v$) 4.65 (dd, 1H), $H_w$) 4.81 (t, 1H), $H_x$) 5.19 (d, 1H), $H_y$) 5.35 (dd, 1H), $H_z$) 5.42 (dd, 1H), $H_{aa}$) 5.61 (dd, 1H), $H_{bb}$) 5.73-5.75 (m, 1H), $H_{cc}$) 6.11 (dd, 1H), $H_{dd}$) 6.85 (d, 2H), $H_{ee}$) 7.08 (d, 2H), $H_{ff}$) 7.20 (d, 1H), $H_{gg}$) 7.54 (d, 1H), $H_{hh}$) 7.78 (d, 1H); $^{13}C$ NMR (125 MHz, $CDCl_3$) δ $C_a$) 11.7, 14.7, 15.2, 16.3, 16.9, 18.6, 20.1 and 20.9, $C_d$) 23.3, $C_e$) 23.7, $C_a$) 24.8 and 27.2, $C_i$) 24.9, 25.0 and 33.9, $C_f$) 27.9, $C_c$) 28.6, $C_g$) 31.2, $C_h$) 31.3, $C_j$) 33.9, $C_l$) 36.2, $C_m$) 38.7, $C_k$) 38.8, $C_n$) 41.3, $C_o$) 46.9, $C_q$) 49.46, $C_s$) 49.52, 53.0, and 67.9, $C_r$) 55.3, $C_v$) 55.4, $C_w$) 55.5, $C_u$) 57.2, $C_y$) 57.6, $C_t$) 63.9, $C_{aa}$) 65.9, $C_p$) 66.5, $C_x$) 70.4, $C_z$) 81.5, $C_z$) 81.7, $C_{dd}$) 114.1, $C_{cc}$) 124.0, $C_{bb}$) 129.1, $C_{ii}$) 130.0, $C_{ee}$) 130.3, $C_{jj}$) 158.6, $C_{kk}$) 168.6, 169.3, 170.5, 170.6, 171.3, 171.5, 172.4, 173.9, 204.9; IR (neat) 3337 (s), 2959 (s), 2870 (m), 1733 (s), 1645 (s), 1640 (s), 1543 (m), 1514 (m), 1454 (m), 1407 (m), 1368 (m), 1302 (w), 1248 (m), 1171 (m) $cm^{-1}$; HRMS m/z calculated for $C_{59}H_{91}N_7O_{15}$ (M+Na) 1160.6471, found 1160.6415.

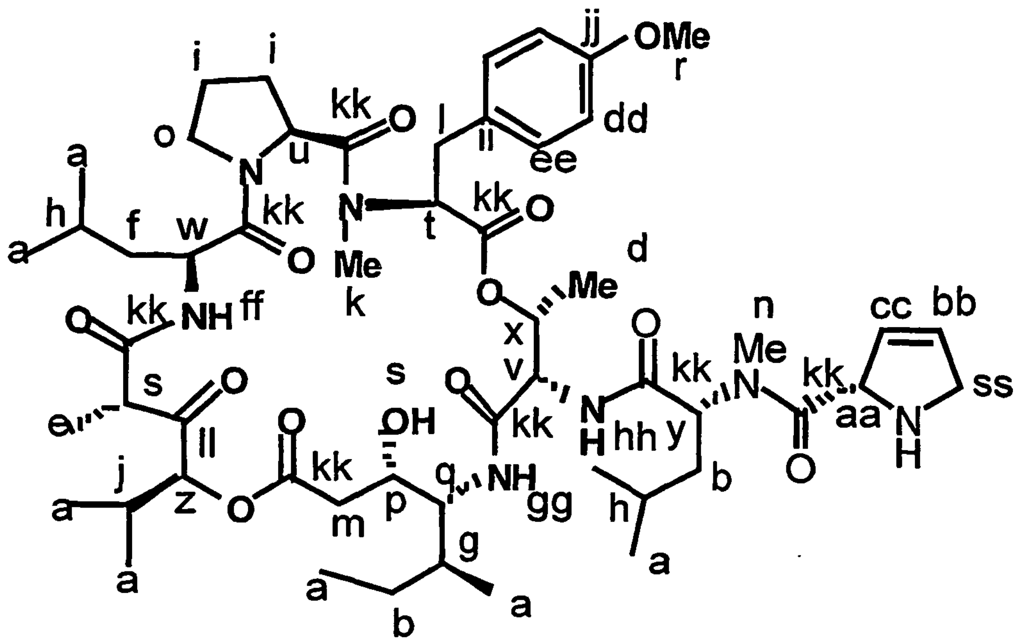

Cyclo[N-(N-3,4-dehydro-L-prolyl-N-methyl-D-leucyl)-O[[N-[(2S,3S,4S)-4[(3S,4R,5S)-4-amino-3-hydroxy-5-methyl-heptanoyl]oxy-3-oxo-2,5-dimethylhexanoyl]-L-leucyl]-L-prolyl-N,O-dimethyl-L-tyrosyl]-L-threonyl] (compound **59**). Compound **58** (4 milligrams) was dissolved in HCl•dioxane (0.5 milliliter) and stirred at room temperature. When the reaction was completed, the solvent was removed in vacuo. Toluene was added twice, and the solution was concentrated. The residue was dried in vacuo overnight to afford the desired HCl salt in 98%. The HCl salt was dissolved in EtOAc, washed with saturated $NaHCO_3$, and the organic layer washed again with brine, dried over $Na_2SO_4$ and concentrated to afford the desired product (compound **59**) in 75% yield. The following analytical data were obtained for compound **59**: $R_f$ 0.20 (10% MeOH/$CH_2Cl_2$); $[\alpha]_D^{25}$ -242.3 (c=0.2, $CHCl_3$); $^1H$ NMR (500 MHz, $CDCl_3$) δ $H_a$) 0.79-0.97 (m, 24H), $H_b$) 1.12-1.23 (m, 3H), $H_d$) 1.32-1.40 (d, 3H), $H_e$) 1.43 (d, 3H), $H_f$) 1.46-1.50 (m, 1H) and 1.61 (t, 1H), $H_g$) 1.68-1.73 (m, 1H), $H_h$) 1.74-1.79 (m, 1H), $H_i$) & $H_j$) 1.82-1.88 (m, 2H), 2.02-2.08 (m, 1H) and 2.11-2.17 (m, 3H), $H_k$) 2.25 (s, 3H), $H_l$) 2.51 (d, 2H), $H_m$) 3.18 and 3.39 (dd, 1H), $H_n$) 2.95 (s, 3H), $H_o$) 3.57-3.61 (m, 2H), $H_p$) 3.33 (d, 1H), $H_q$) 3.69-3.71 (m, 1H), $H_r$) 3.73 (s, 3H), $H_s$) 3.90-4.18 (m, 2H), $H_t$) 4.49 (q, 1H), $H_u$) 4.56 (dd, 1H), $H_v$) 4.65 (dd, 1H), $H_w$) 4.81 (t, 1H), $H_x$) 5.19 (m, 3H), $H_{bb}$) & $H_{aa}$) 5.73-5.75 (m, 3H), $H_{cc}$) & $H_z$) 6.01 (dd, 3H), $H_{dd}$) 6.85 (d, 2H), $H_{ee}$) 7.08 (d, 2H), $H_{ff}$) 7.20 (d, 1H), $H_{gg}$) 7.54 (d, 1H), $H_{hh}$) 7.78 (d, 1H); $^{13}C$ NMR (125 MHz, $CDCl_3$) δ $C_a$) 11.7, 14.7, 15.2, 16.3, 16.9, 18.6, 20.1 and 20.9, $C_d$) 23.3, $C_e$) 23.7, $C_a$) 24.8 and 27.2, $C_i$) 24.9, 25.0 and 33.9, $C_f$) 27.9, $C_c$) 28.6, $C_g$) 31.2,

$C_h$) 31.3, $C_j$) 33.9, $C_l$) 36.2, $C_m$) 38.7, $C_k$) 38.8, $C_n$) 41.3, $C_o$) 46.9, $C_q$) 49.46, $C_s$) 49.52, 53.0, and 67.9, $C_r$) 55.3, $C_v$) 55.4, $C_w$) 55.5, $C_u$) 57.2, $C_y$) 57.6, $C_t$) 63.9, $C_{aa}$) 65.9, $C_p$) 66.5, $C_x$) 70.4, $C_z$) 81.5, $C_{dd}$) 114.1, $C_{cc}$) 124.0, $C_{bb}$) 129.1, $C_{ii}$) 130.0, $C_{ee}$) 130.3, $C_{jj}$) 158.6, $C_{kk}$) 168.6, 169.3, 170.5, 171.3, 171.5, 172.4, 173.9, $C_{ll}$) 204.9; IR (neat) 3337 (s), 2958 (s), 2861 (m), 1734 (s), 1642 (s), 1638 (s), 1547 (m), 1514 (m), 1451 (m), 1385 (w), 1243 (w), 1166 (w) $cm^{-1}$; HRMS m/z calculated for $C_{54}H_{83}N_7O_{13}$ (M+H) 1038.6127, found 1038.6103.

Example 7

Synthesis and Biological Evaluation of Didemnin Photoaffinity Analogs

Two proteins which bind with didemnins have been identified, namely, eukaryotic elongation factor 1 alpha (EF-1a; Crews et al., 1994 J. Biol. Chem. 269:15411-15414) and palmitoyl protein thioesterase 1 (PPT1; Crews et al., 1996, Proc. Natl. Acad. Sci. USA 93:4316-4319). However, the precise structural interaction between didemnin B and these two proteins are not fully understood. The experiments described in this Example were performed in order to generate photo-reactive didemnin derivatives that can be used to investigate binding of the corresponding didemnins with these and other cellular targets, and that analogous photo-reactive tamandarin derivatives can be synthesized.

Synthesis of didemnin analogs designated compounds **403, 404, 405**, and **406** is described in this example. These compounds have the structure of formula **XXI,** wherein $R^2$ is a substituent having the structure of formula **III,** $R^3$ is methyl, $R^4$ is an isoleucine side chain, each of $R^5$, $R^6$, $R^8$ and $R^9$ is a hydride radical, $R^7$ is a methoxy radical, $R^{10}$ is a leucine side chain, X is -O-, Y is a hydride radical, and $R^1$ has the identity shown in Figures 47A, 47B, 47C, and 47D, respectively.

Each of compounds **403, 404, 405**, and **406** is a didemnin A derivative, and each of these molecules comprises a 4-benzoylbenzoic acid moiety. This type of benzophenone derivative was selected as the photo-reactive group because of its multiple advantages in photoaffinity labeling, as described (Dormán et al., 1994, Biochemistry 33:5661-5673; Fleming, 1995, Tetrahedron 51:12479-12520). Benzophenones are easily manipulated in ambient light, but are photoactivated by light having a wavelength of about 350 nanometers. Light having this wavelength does not generally damage proteins, and was used in order to avoid light-induced changes in the target proteins used in these experiments. The activated diradical of benzophenone tends to avoid solvent molecules and nucleophiles, and cross-links preferentially at unreactive carbon centers. Because photo-excitation of benzophenones is reversible, a given molecule of the analog which is not proximal to the ligand binding site can undergo several excitation-relaxation cycles without suffering non-specific side reactions. As a result, benzophenones tend to cross-link with greater site-specificity than photophores such as aryl azides, which undergo an irreversible photolysis reaction.

The side chain portions of compounds **403, 404, 405**, and **406** were synthesized and then coupled to an advanced macrolide intermediate as a final step. Figure 48 shows the synthesis of the D-leucine moiety which is common to all four analogs. D-Leucine was converted to its benzyl carbamate (compound **407**) using benzyl chloroformate in $NaHCO_3$ and water. Compound **407** was dialkylated using dimethyl sulfate in the presence of powdered KOH, THF, and $Bu_4N^+HSO_4^-$. Catalytic hydrogenolysis of the carbamate over a Pd/C catalyst in the presence of hydrogen and (1:1) ethyl acetate:methanol, followed by trapping with HCl in the presence of methanol yielded the amine hydrochloride salt, compound **409**. As shown in Figure 49, compound **409** was coupled with 4-benzoylbenzoic acid by maintaining the acid with BOP-Cl, a reagent which is generally useful in coupling to *N*-methyl amines in the presence of NMM and DMF at 0°C for 30 minutes, and then contacting the reaction product with compound **409.** Removal of the methyl group by ester saponification yielded compound **412**.

The benzophenone derivative designated compound **410** (see Figure 50) was elongated by BOP-mediated coupling with a glycine methyl ester linker in the presence of BOP, NMM, DMF, and the glycine methyl ester hydrochloride salt. Saponification of the ester compound **413** in the presence of LiOH, water, THF, and methanol, followed by coupling with amine salt **409** in the presence of BOP-Cl, NMM, and DMF, and subsequent saponification in the presence of LiOH, water, THF, and methanol yielded compound **415**. Compound **417** was prepared in an analogous fashion, using a 6-aminohexanoic acid methyl ester. Compound **419** was prepared by twice coupling compound **410** with 6-aminohexanoic acid methyl ester prior to coupling with compound **409** and subsequent saponification.

Compounds **412, 415, 417**, and **419** were coupled with the previously reported macrocyclic salt (prepared as described, Mayer et al., 1994, J. Org. Chem. 59:5192-5205; Mayer et al., 1995, Acta Crystallogr. C51:1609-1614) using the BOP coupling reagent to obtain compounds **403, 404, 405**, and **406**, respectively, in 68-81 % yield.

In order to evaluate their suitability for photoaffinity labeling of the EF-1a binding protein, compounds **403, 404, 405**, and **406** were assayed for protein biosynthesis inhibition potency. The results of this evaluation are listed in Table 2. The cell-free translation assay system was as described (SirDeshpande et al., 1995, Biochemistry 34:9177-9184; Ahuja et al., J. Med. Chem. 2000 43:4212-4218). In this assay, didemnin B exhibited a half-maximal inhibitory concentration ($IC_{50}$) of 3 micromolar. The results in Table 2 demonstrate that protein biosynthesis inhibition potency remains

intact even when a large benzophenone moiety is incorporated into the structure of didemnin B. The length of the linker seems to exert a marginal effect, with the two shortest side chains (i.e., compounds **403** and **404**) being nearly equipotent with didemnin B. Increasing side chain length correlates with modest reductions in inhibitor potency. These results are consistent with those obtained by others (Sakai et al., 1996, J. Med. Chem. 39:2819-2834; Ahuja et al., J. Med. Chem. 2000 43:4212-4218), and demonstrate that protein biosynthesis inhibition is broadly tolerant of side chain modification.

Table 2

| Compound | Coupling Yield | IC50 (micromolar) |
|---|---|---|
| Didemnin B | | 3.0 |
| **403** | 68 % | 4.0 |
| **404** | 78 % | 4.5 |
| **405** | 74 % | 24 |
| **406** | 81 % | 19 |

Table 3 lists preliminary results obtained using the NCI-60 tumor cell screen (described in Boyd et al., 1995, Drug Dev. Res. 34:91-109). The data in Table 3 indicate that the benzophenone photoaffinity analogs can be used to study anti-tumor activity of didemnins. Examination of the 50% growth inhibition parameter ($GI_{50}$) shows that all four analogs retain potency comparable to didemnin B. In contrast to the results obtained with regard to in vitro protein biosynthesis, there does not appear to be any clear-cut relationship between linker length and activity. However, total growth inhibition (TGI) by the four analogs requires significantly higher concentrations than are required by didemnin B. Interestingly, all four of the analogs exhibit three to ten-fold lower toxicity as measured by the lethal concentration parameter, $LC_{50}$.

Table 3

| Compound | $GI_{50}$ (nanomolar) | TGI (micromolar) | $LC_{50}$ (micromolar) |
|---|---|---|---|
| Didemnin B | 13 | 0.066 | 3.8 |
| **403** | 3.0 | 0.35 | 15 |
| **404** | 13 | 2.0 | 23 |
| **405** | 4.3 | 20.5 | 19 |
| **406** | 17 | 5.0 | 48 |

The results of the experiments presented in this Example demonstrate that benzophenone-containing analogs of didemnins can be made by total synthesis. Biological evaluation of the analogs indicates that incorporation of benzophenone in the side chain peptide is a feasible strategy for photoaffinity labeling of molecular targets of didemnins.

The disclosure of every patent, patent application, and publication cited herein is hereby incorporated herein by reference in its entirety.

While this invention has been disclosed with reference to specific embodiments, other embodiments and variations of this invention can be devised by others skilled in the art without departing from the true spirit and scope of the invention. The appended claims include all such embodiments and equivalent variations.

## Claims

**1.** A composition composing a tamandarin analog having the structure

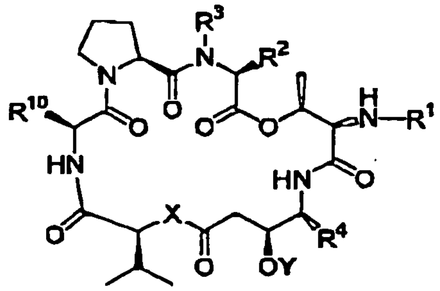

wherein:

i) $R^1$ is selected from the group consisting of

-H,
-(tert-butyloxycarbonyl).
-leucine,
-(N-methyl)leucine,
-(N-methyl)leucina-(a first fluorophore),
-(N-methyl)leucine-proline,
-(N-methyl)leucine-proline-lactale,
-(N-methyl)leucine-proline-pyruvate,
-(N-methyl)leucine-proline-lactate-(a first fluorophore),
-(N-methyl)leucine-proline-lactate-glutamine-pyroglutamate,
-(N-methyl)leucine-proline-lactate-glutamine-cyclopentanoate,
-(N-methyl)leucine-proline-alanine-leucine-pyroglutamate,
-(N-methyl)leucine-proline-(N-methyl-alanine)-leucine-pyroglutamate,
-(N-methyl)leucine-deoxo-proline,
-(N-methyl)leucine-deoxo-proline-actate,
-(N-methyl)leucine-deoxo-proline-pyruvate,
-(N-methyl)leucine-deoxo-proline-lactate-(a first-fluorophore),
-(N-methyl)leucine-deoxo-proline-betate-glutamine-pyroglutamate,
-(N-methyl)leucine-deoxo-proline-lactate-glutamine-cyclopentanoate,
-(N-methyl)leucine-deoxo-proline-alanine-leucine-pyruglutamate,
-(N-methyl)leucine-deoxo-proline-(N-methyl-alanine)-leucine-pyroglutamate,
-(N-methyl)leucine-dehydro-proline,
-(N-methyl)leucine-dehydro-proline-lactate,
-(N-methyl)leucine-dehydro-proline-pyruvate,
-(N-methyl)leucine-dehydro-proline-lactate-(a first fluorophore),
-(N-methyl)leucine-dehydro-proline-lactate-glutamine-pyroglutamate,
-(N-methyl)leucine-dehydro-proline-lactate-glutamine-cyclopentanoate,
-(N-methyl)leucine-dehydro-proline-alanine-leucine-pyroglutomate, and
-(N-methyl)leucine-dehydro-proline-(N-methyl-alanine)-leucine-pyroglutamate;

ii) either

(a) $R^3$ is selected from the group consisting of $-CH_3$ and -H; and $R^2$ is selected from the group consisting of an isoleucine side chain, a valine side chain, an alanine side chain, a norleucine side chain, a norvaline side chain, leucine side chain, a histidine side chain, a tryptophan side chain, an arginine side chain, a lysine side chain, a second fluorophore, and a substituent having the structure

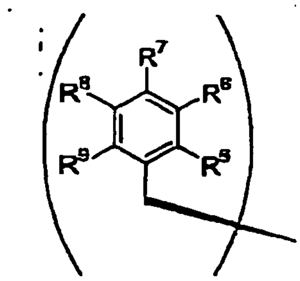

(b) $R^2$ and $R^3$ together are a substituent having the structure

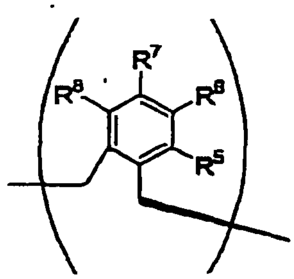

iii) each of $R^5$, $R^6$, $R^7$, $R^8$, and $R^9$, when present, is independently selected from the group consisting of -H, -OH, $-OCH_3$, $-CO(C_6H_5)$, -Br, -I, -F, -Cl, $-CH_3$, and $C_2H_5$.
iv) $R^4$ is an isoleucine side chain:
v) X is selected from the group consisting of -O- and -(NH)-:
vi) Y is selected from the group consisting of-H and a hydroxyl protecting group;
vii) $R^{10}$ is selected from the group consisting of a leucine side chain and a lysine side chain; and
viii) the molecule is not tamandarin A.

**2.** The composition of claim 1, wherein $R^1$ is selected from the group consisting of

-H.
-(tert-butyloxycarbonyl),
-leucine,
-(N-methyl)leucine,
-(N-methyl)leucine-(a first fluorophore),
-(N-methyl)leucine-(S)-proline,
-(N-methyl)leucine-(S)-proline-(S)-lactate,
-(N-methyl)leucine-(S)-proline-pyruvate,
-(N-methyl)leucine-(S)-proline-(S)-lactate-(a first fluorophore),
-(N-methyl)leucine-(S)-proline-(S)-lactate-glutamine-pyroglutamate,
-(N-methyl)leucine-(S)-proline-(S)-lactate-glutamine-cyclopentanoate,
-(N-methyl)leucine-(S)-proline-alanine-leucine-pyroglutamate,
-(N-methyl)leucine-(S)-proline-(N-methyl-alanine)-leucine-pyroglutamate,
-(N-methyl)leucine-deoxo-(S)-proline,
-(N-methyl)leucine-deoxo-(S)-proline-(S)-lactate,
-(N-methyl)leucine-deoxo-(S)-proline-pyruvate,
-(N-methyl)leucine-deoxo-(S)-proline-(S)-lactate-(a first fluorophore).
-(N-methyl)leucine-deoxo-(S)-proline-(S)-lactate-glutamine-pyroglutamate,
-(N-methyl)leucine-deoxo-(S)-proline-(S)-lactate-glutamine-cyclopentanoate,
-(N-methyl)leucine deoxo-(S)-proline-alanine-leucine-pyroglutamate.
-(N-methyl)leucine-deoxo-(S)-proline-(N-methyl-alanine)-leucine-pyroglutamate,
-(N-methyl)leucine-dehydro-(S)-proline,
-(N-methyl)leucine-dehydro-(S)-proline-(S)-lactate,

-(N-methyl)leucine-dehydro-(S)-proline-pyruvate,
-(N-methyl)leucine-dehydro-(S)-proline-(S)-lactate-(a first fluorophore),
-(N-methyl)leucine-dehydro-(S)-proline-(S)-lactate-glutamine-pyroglutamate,
-(N-methyl)leucine-dehydro-(S)-proline-(S)-lactate-glutamine-cyclopentanoate,
-(N-methyl)leucine-dehydro-(S)-proline-alanine-leucine-pyroglutamate, and
-(N-methyl)leucine-dehydro-(S)-proline-(N-methyl-alanine)-leucine-pyroglutamate.

**3.** The composition of claim 1, wherein $R^1$ is selected from the group consisting of

-H,
-(tert-butyloxycarbonyl),
-leucine,
-(N-methyl)leucine,
-(N-methyl)leucine-(a first fluorophore),
-(N-methyl)leucine-(S)-proline,
-(N-methyl)leucine-(S)-proline-(S)-lactate,
-(N-methyl)leucine-(S)-proline-pyruvate,
-(N-methyl)leucine-(S)-proline-(S)-lactate-(a first fluorophore),
-(N-methyl)leucine-(S)-proline-(S)-lactate-(S)-glutamine-(S)-pyroglutamate,
-(N-methyl)leucine-(S)-proline-(S)-lactate-(S)-glutamine-(S)-cyclopentanoate,
-(N-methyl)leucine-(S)-proline-(S)-alanine-(S)-leucine-(S)pyroglutamate,
-(N-methyl)leucine-(S)-proline-(N-methyl-S-alanine)-(S)-leucine-(S)-pyroglutamate,
-(N-methyl)leucine-deoxo-(S)-proline,
-(N-methyl)leucine-deoxo-(S)-proline-(S)-lactate,
-(N-methyl)leucine-deoxo-(S)-proline-pyruvate,
-(N-methyl)leucine-deoxo-(S)-proline-(S)-lactate (a first fluorophore),
-(N-methyl)leucine-deoxo-(S)-proline-(S)-lactate-(S)-glutamine-(S)-Pyroglutamate,
-(N-methyl)leucine-deoxo-(S)-proline-(S)-lactate-(S)-glutamine-(S)-cyclopentanoate,
-(N-methyl)leucine-deoxo-(S)-proline-(S)-alanine-(S)leucine-(S)-pyroglutamate, and
-(N-methyl)leucine-deoxo-(S)-proline-(N-methyl-S-alanine)-(S)-leucine-(S)-pyroglutamate,
-(N-methyl)leucine-dehydro-(S)-proline,
-(N-methyl)leucine-dehydro-(S)-proline-(S)-lactate,
-(N-mathyl)leucine-dehydro-(S)-proline-pyruvate,
-(N-methyl)leucine-ehydro-(S)-Proline-(S)-lactate-(a first fluorophore).
-(N-methyl)leucine-dehydro-(S)-proline-(S)-lactate-(S)-glutamine-(S)-pyroglutamate,
-(N-methyl)leucine-dehydro-(S)-proline-(S)-lactate-(S)-glutamine-(S)-cyclopentanoate,
-(N-methyl)leucine-dehydro-(S)-proline-(S)-alanine-(S)leucine-(S)-pyroglutamate, and
-(N-methyl)leucine-dehydro-(S)-proline-(N-methyl-S-alanine)(S)-leucine-(S)-pyroglutamate.

**4.** The composition of claim 1, wherein $R^2$ is

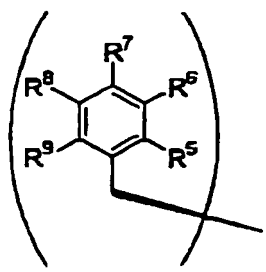

$R^3$ is methyl, each of $R^5$, $R^6$, $R^8$, and $R^9$ is a hydride radical, $R^7$ is methoxy, $R^{10}$ is a leucine side chain, X is -O-, and Y is a hydride radical.

**5.** The composition of claim 1, wherein the tamandarin analog is

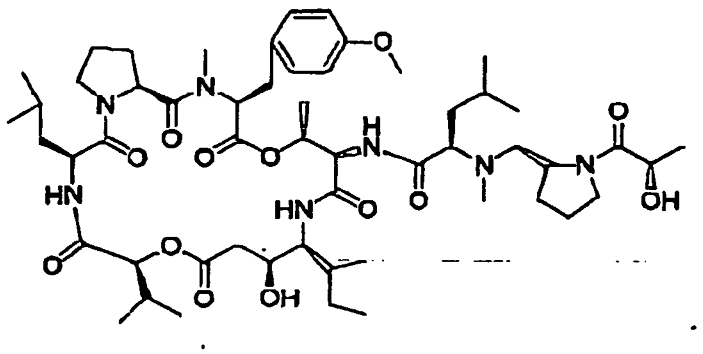

6. The composition of claim 1, wherein the tamandarin analog is

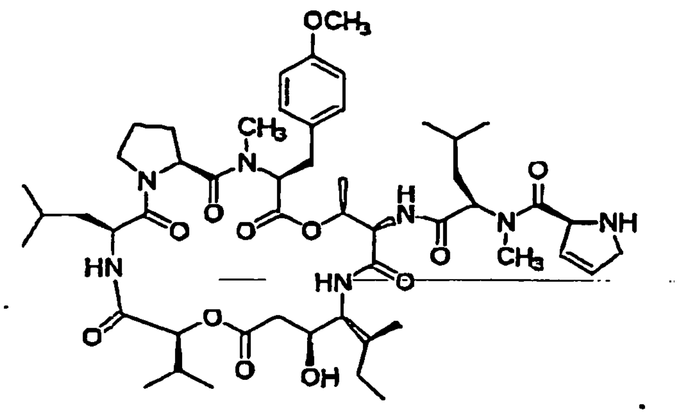

7. The composition of claim 1, wherein $R^1$ is -(N-methyl)leucine-deoxo-(S)-proline-lactate.

8. The composition of daim 1, wherein $R^1$ is -(N-methyl-R-leucine).

9. The composition of claim 1, wherein $R^1$ is -(N-methyl-R-leucine)-(S)-proline-(S)-lactate-(S)-glutamine-(S)-pyroglutamate, Y is -H, and X is -O-.

10. The composition of claim 9, wherein the tamandarin analog is

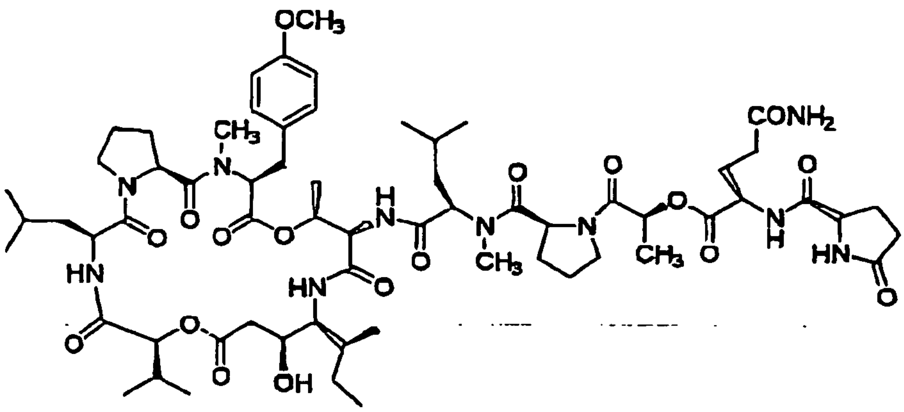

or

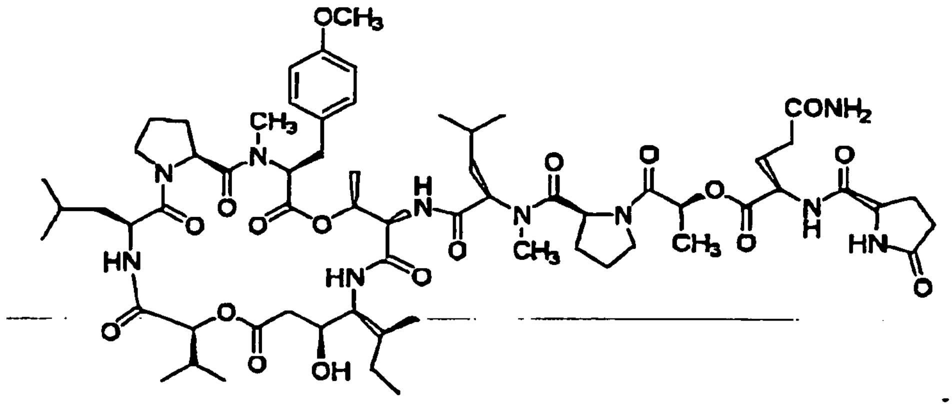

11. The composition of claim 1, wherein $R^1$ is -(N-methyl)leucine-(S)-proline-(S)-lactate-(a first fluorphore).

12. The composition of claim 11, wherein the tamandarin analog is selected from the group consisting of

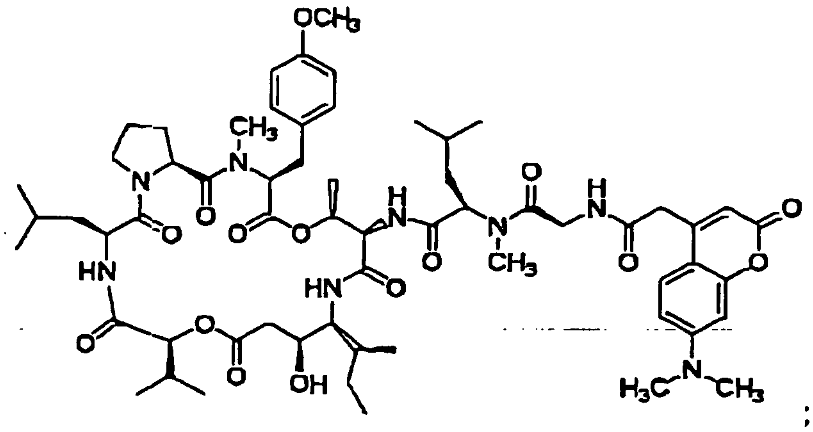

;

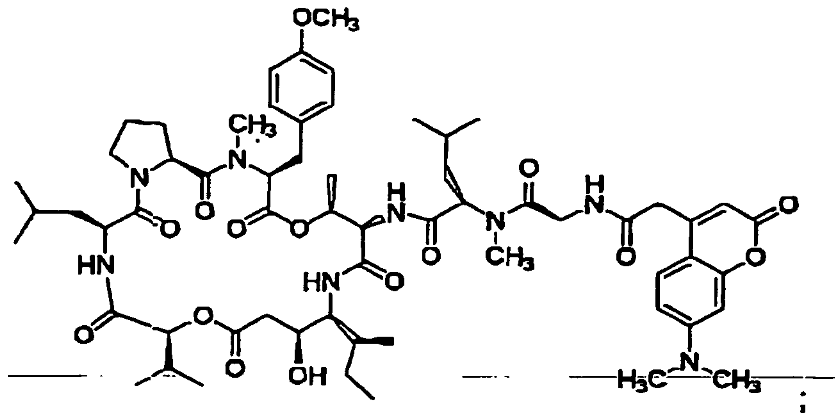

;

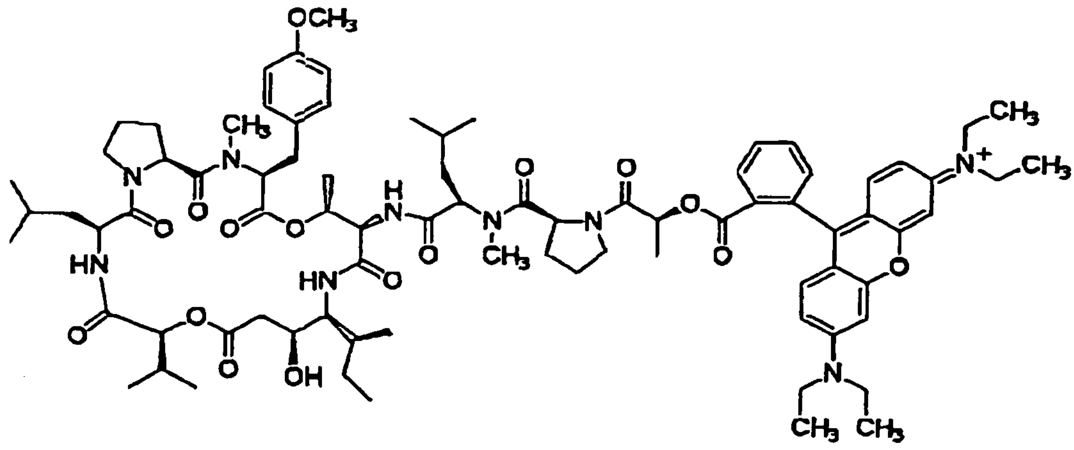

and

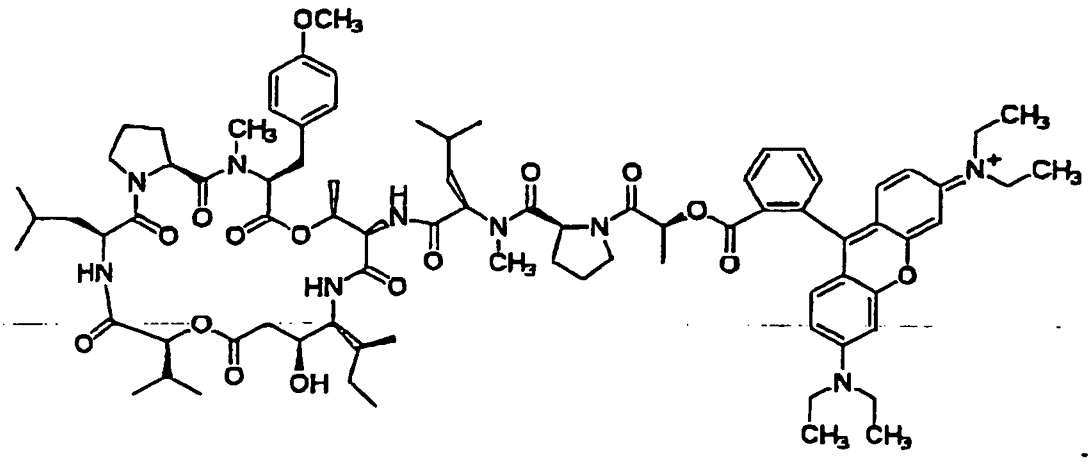

**13.** The composition of claim 1, wherein $R^1$ is -(N-methyl)leucine-(S)-prolinepyruvate.

**14.** The composition of claim 13, wherein the tamandarin analog is

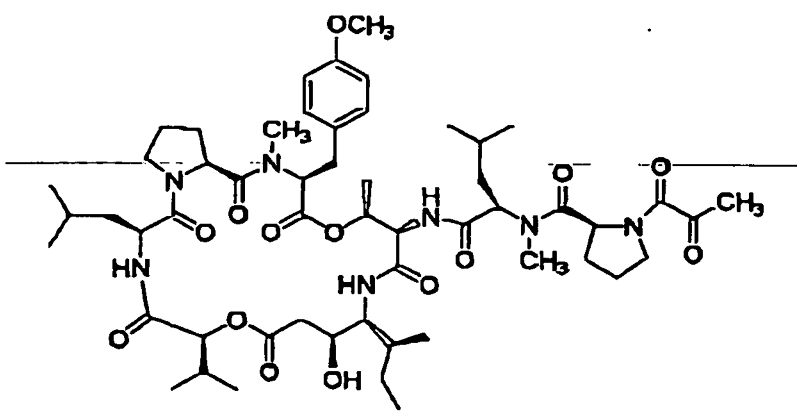

or

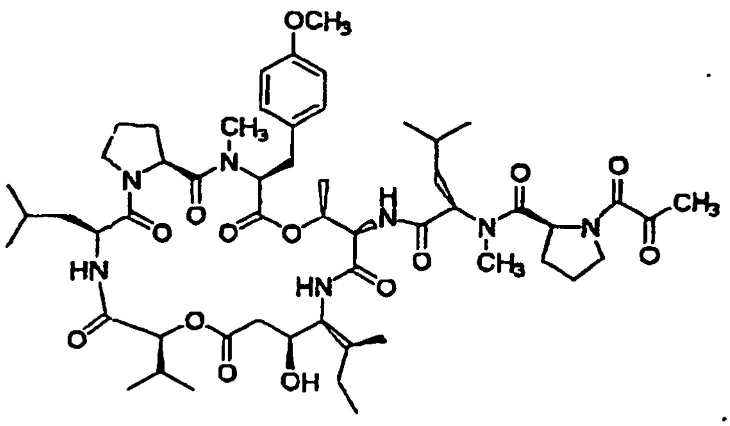

15. The composition of claim 1, wherein Y is -H, and wherein $R^2$ has the structure

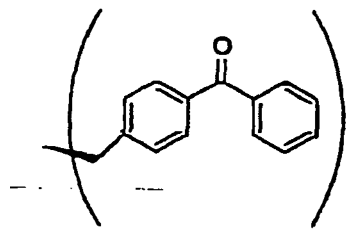

16. The composition of claim 1, wherein $R^2$ is a lysine side chain and Y is -H.

17. The composition of claim 1, wherein the tamandarin analog has the following structure

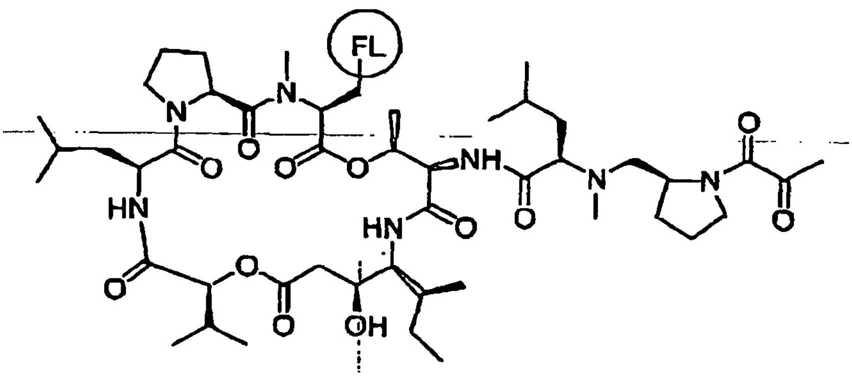

wherein FL is a fluorophore.

18. The composition of claim 1, wherein X is -(NH)-.

19. The composition of claim 1, wherein the tamandarin analog is selected from the group consisting of

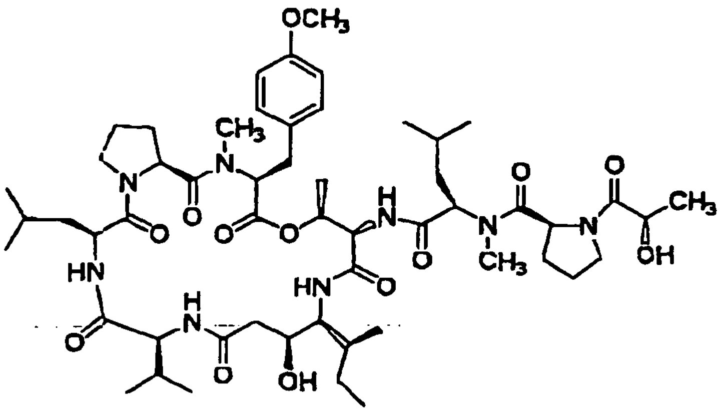

;

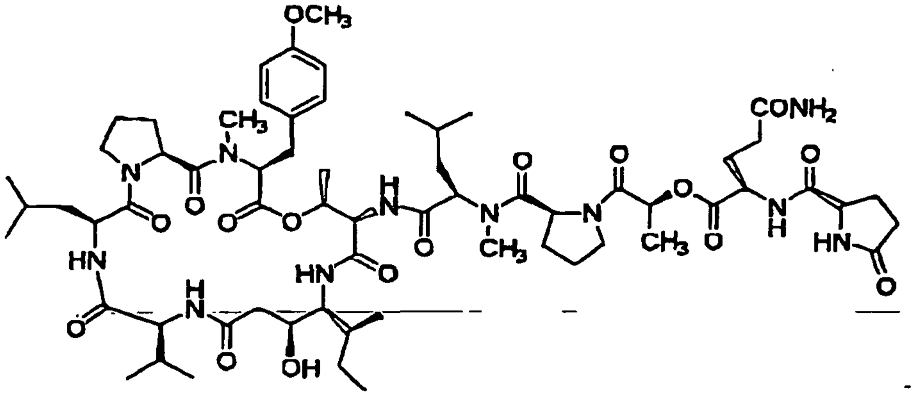

;

and

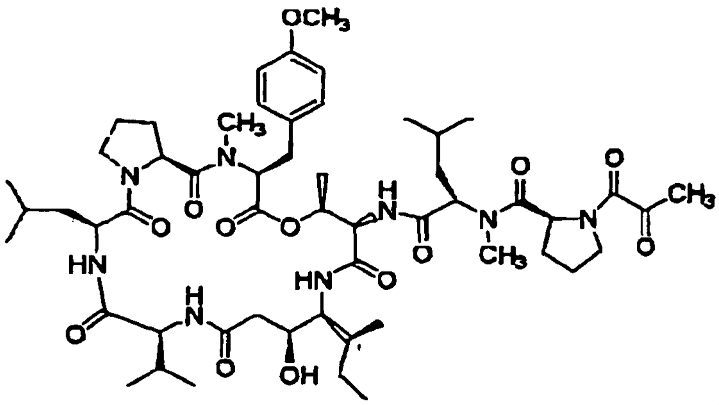

.

**20.** The composition of claim 1, wherein the tamandarin analog is selected from the group consisting of

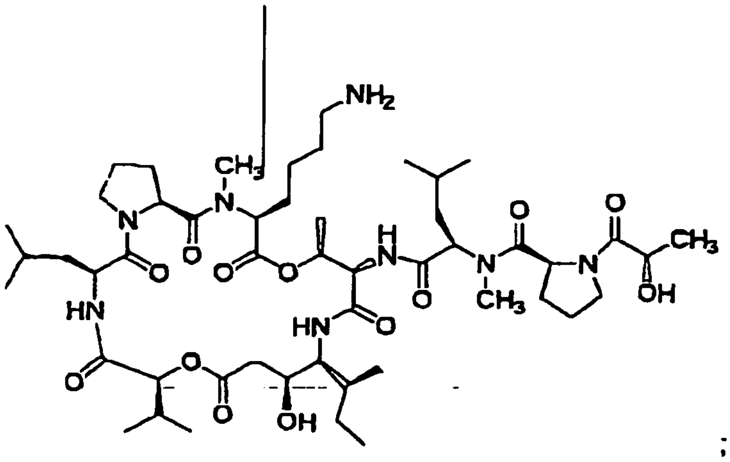

;

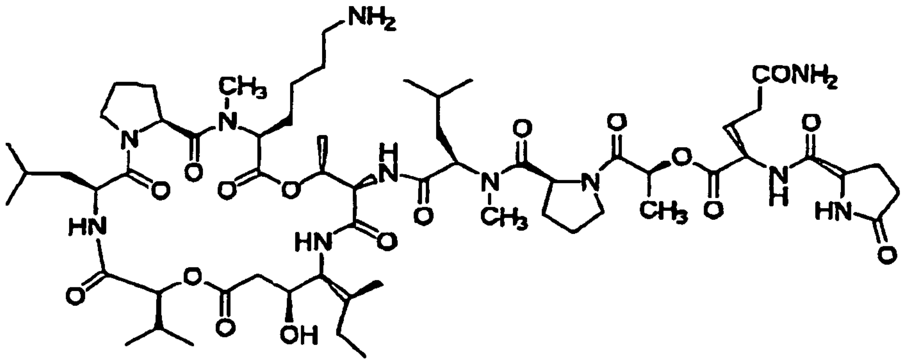

;

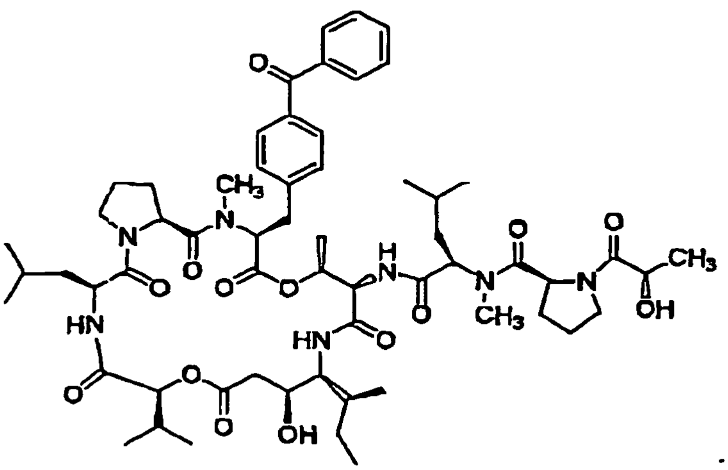

;

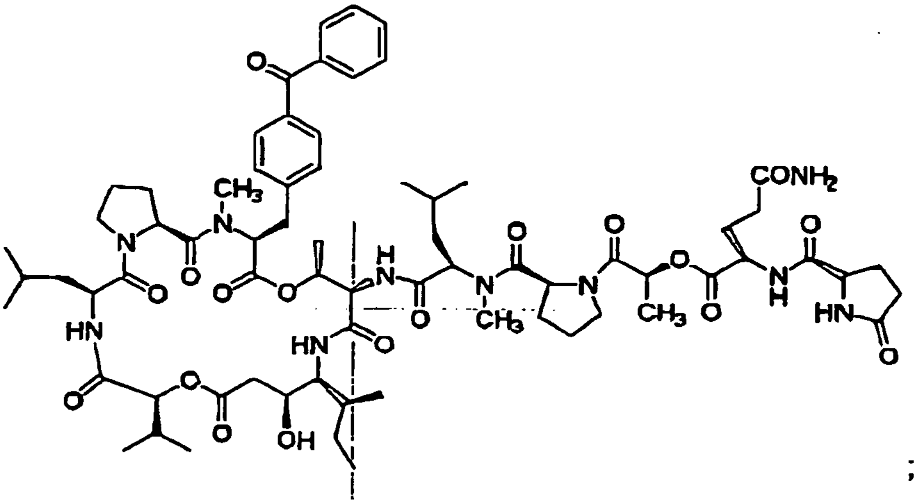

;

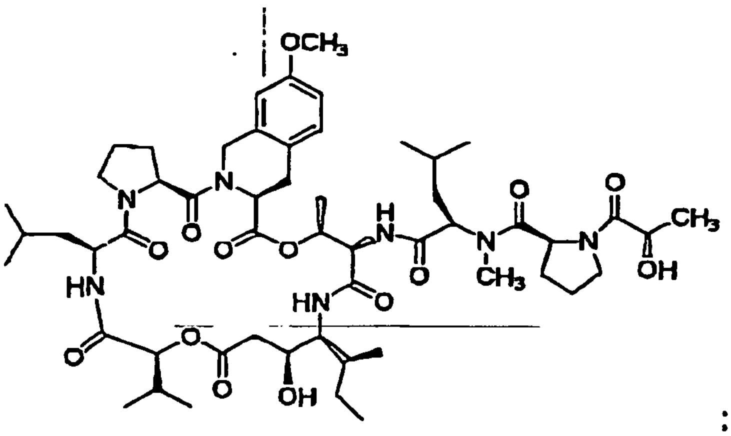

;

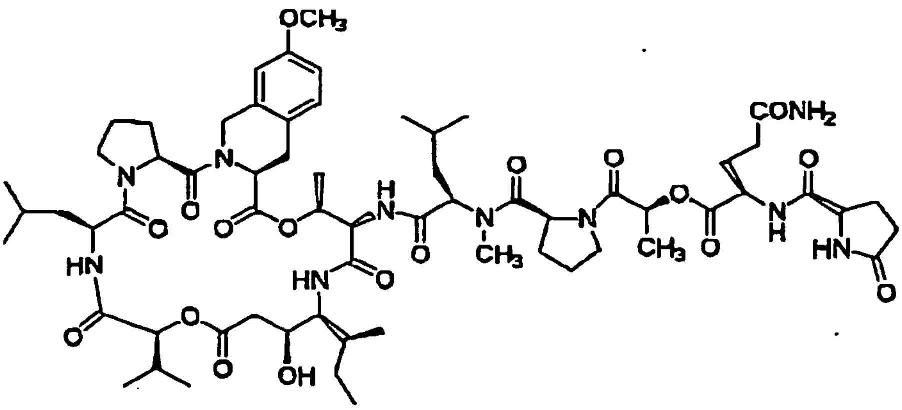

;

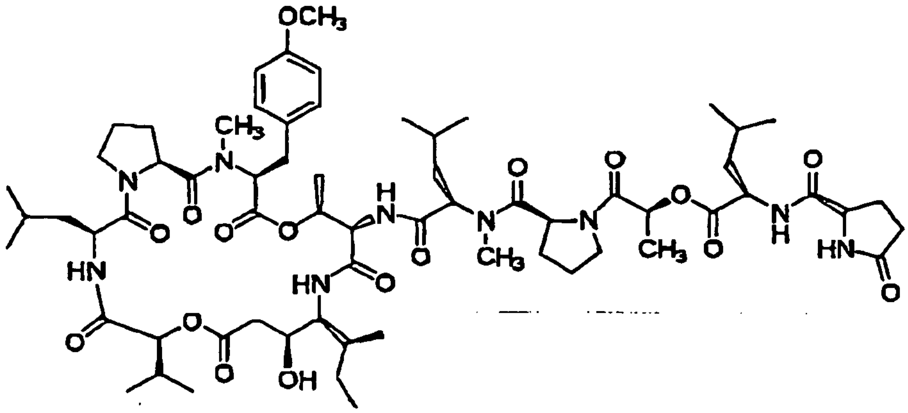

;

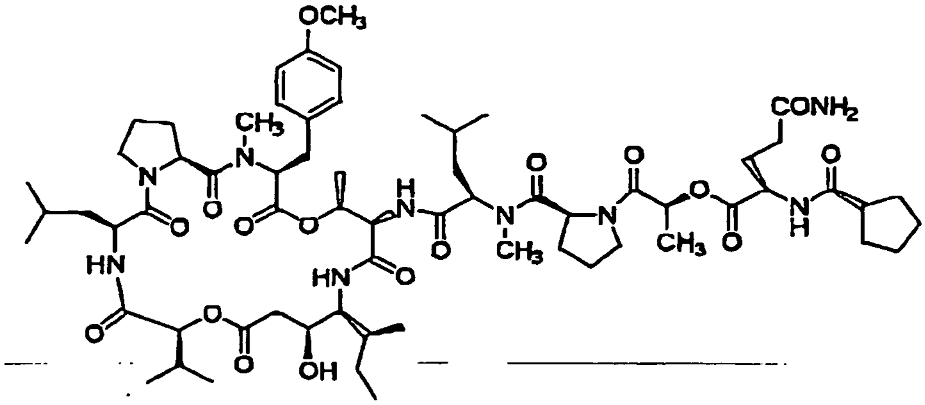

;

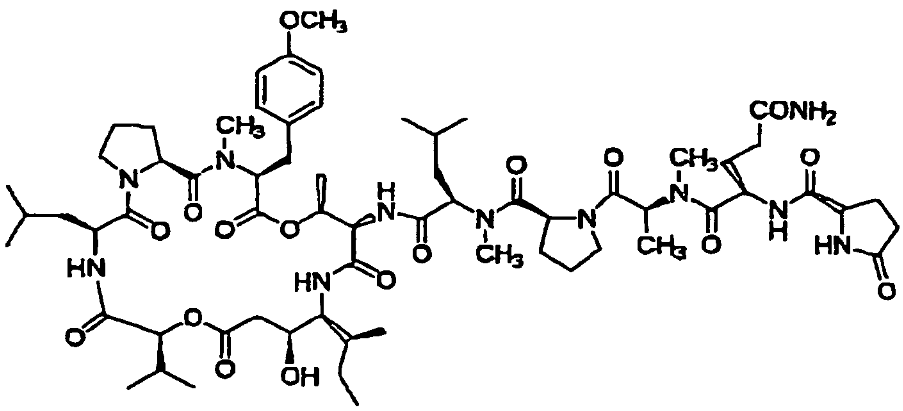

;

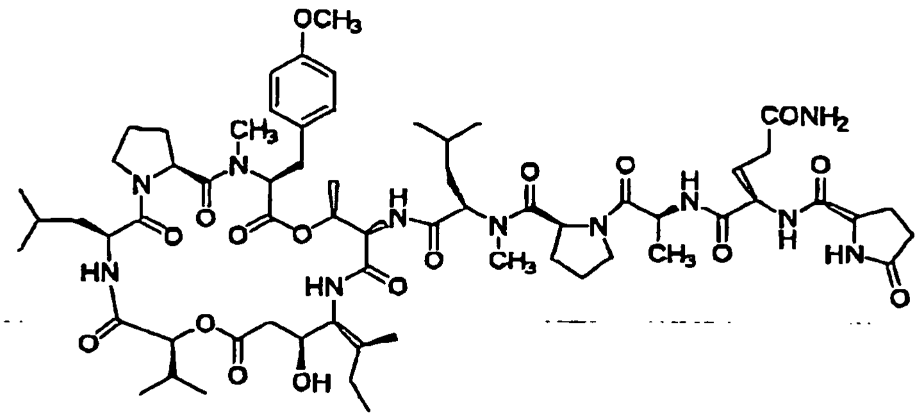

;

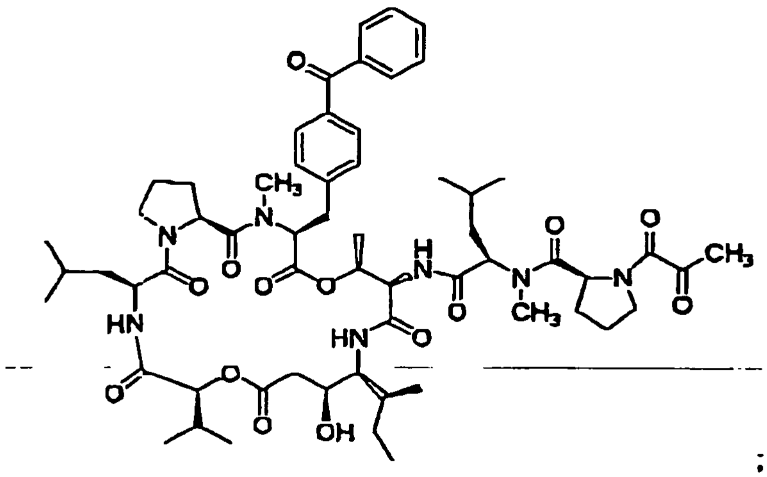

;

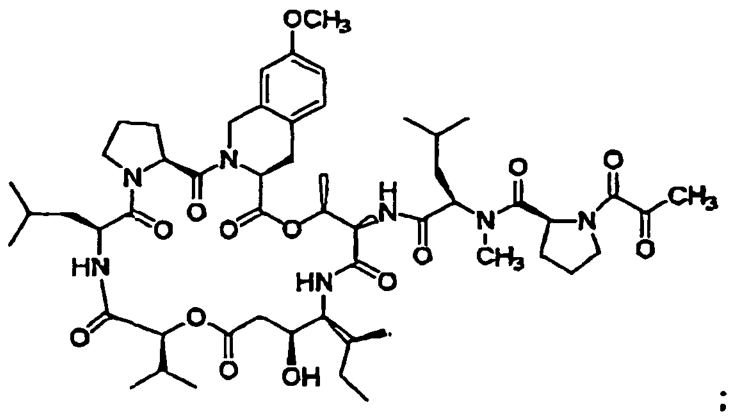

;

and

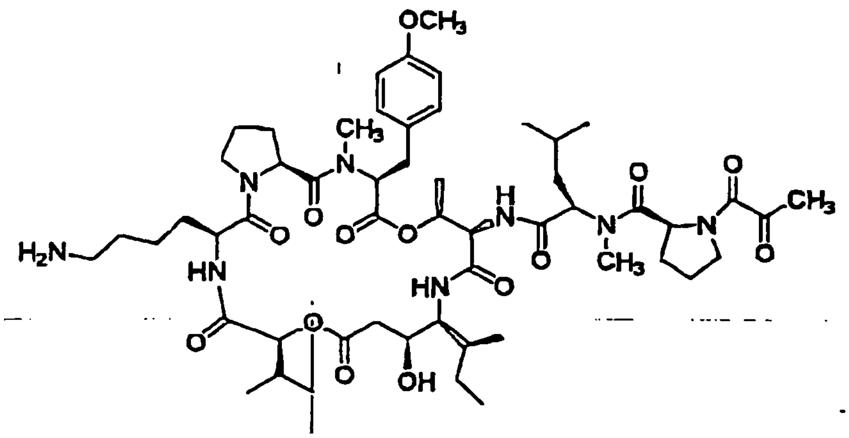

**21.** The composition of claim 1, further composing a pharmaceutically acceptable carrier

**22.** A support having the tamandarin analog of claim 1 covalently attached thereto.

**23.** A composition comprising a compound having a structure selected from the group consisting of

(a)

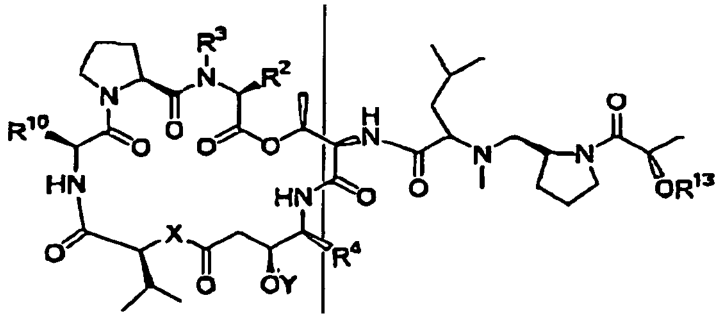

(b)

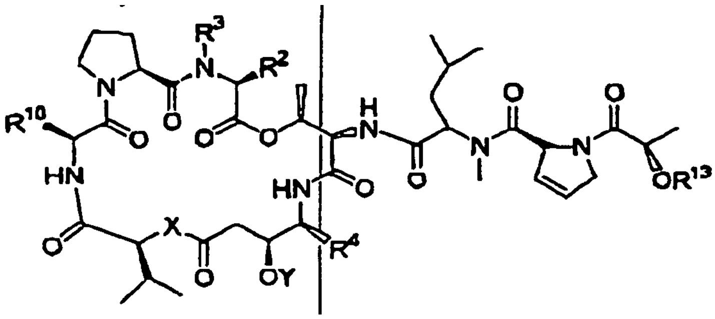

(c)

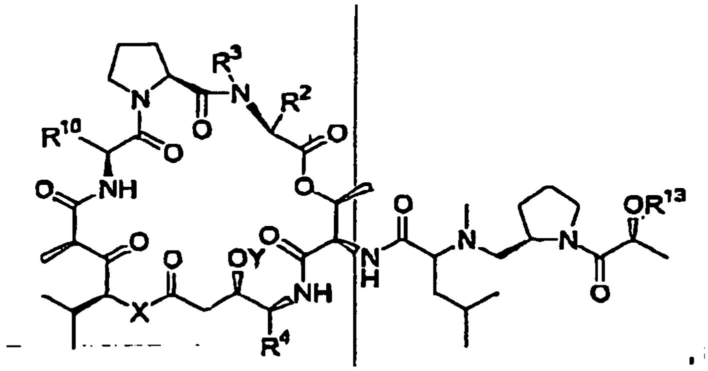

(d)

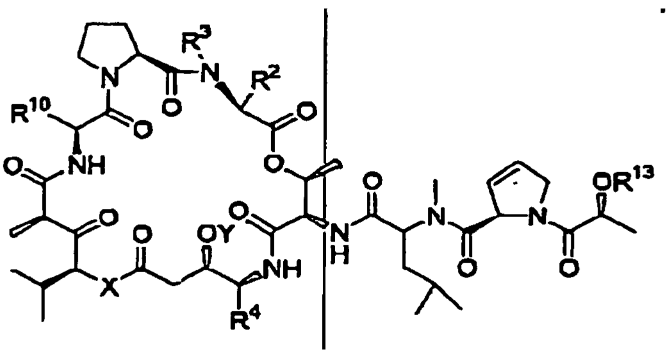

wherein:

i) either

(a) $R^3$ is selected from the group consisting of $-CH_3$ and -H; and $R^2$ is selected from the group consisting of an isoleucine side chain, a valine side chain, an alanine side chain, a norleucine side chain, a norvaline side chain, a proline side chain, leucine side chain, a histidine side chain a tryptophan side chain, an arginine side chain, a lysine side chain, a second fluorophore, and a substituent having the structure

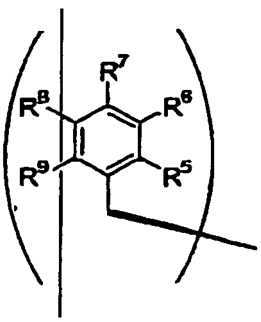

(b) $R^2$ and $R^3$ together are a substituent having the structure

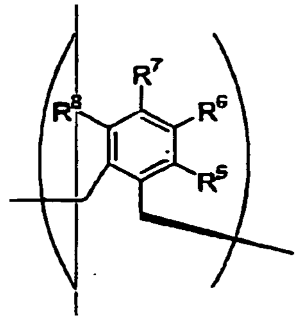

ii) each of $R^5$, $R^6$, $R^7$, $R^8$, and $R^9$, when present is independently selected from the group consisting of -H, -OH, $-OCH_3$, $-CO(C_6H_5)$, -Br, -I, -F, -Cl, $-CH_3$, and $-C_2H_5$;
iii) $R^4$ is selected from the group consisting of an isoleucine side chain and a valine side chain;
iv) X is selected from the group consisting of -O- and -(NH)-:
v) Y is selected from the group consisting of -H and a hydroxyl protecting group;
vi) $R^{10}$ is selected from the group consisting of a leucine side chain and a lysine side chain; and
vii) $R^{13}$ is an enzyme-cleavable moiety that is cleavable by an enzyme selected from the group consisting of a carboxypeptidase, a beta-lactamase, a beta-galactosidase, a penicillin V-amidase, a cytosine deaminase, a nitroreductase, a alkaline phosphatase, a betaglucuronidase, and a catalytic antibody.

**24.** The composition of claim 25, wherein $R^{13}$ has the structure

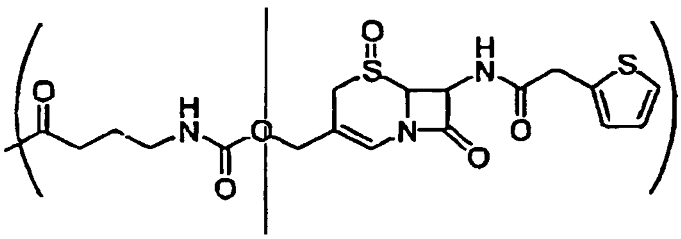

**25.** The composition of claim 25, wherein $R^{13}$ has the structure

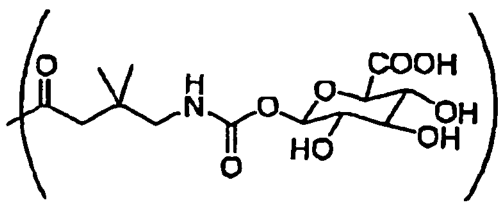

**26.** A composition comprising a didemnin analog having the structure

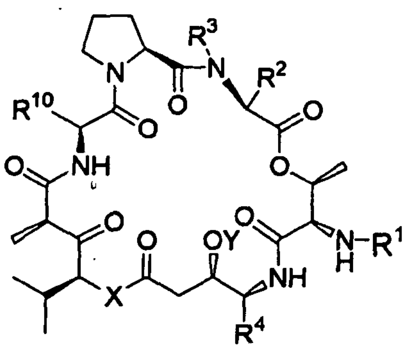

wherein:

i) $R^1$ is selected from the group consisting of

-(tert-butyloxycarbonyl),
-leucine,
-(N-methyl)leucine-(a first fluorophore),
-(N-methyl)leucine-proline,
-(N-methyl)leucine-proline-lactate-(a first fluorophore),
-(N-methyl)leucine-proline-lactate-glutamine-pyroglutamate,
-(N-methyl)leucine-proline-lactate-glutamine-cyclopentanoate,
-(N-methyl)leucine-proline-alanine-leucine-pyroglutamate,
-(N-methyl)leucine-proline-(N-methyl-alanine)-leucine-pyroglutamate,
-(N-methyl)leucine-deoxo-proline,
-(N-methyl)leucine-deoxo-proline-lactate,
-(N-methyl)leucine-deoxo-proline-pyruvate,
-(N-methyl)leucine-deoxo-proline-lactate-(a first fluorophore),
-(N-methyl)leucine-deoxo-proline-lactate-glutamine-pyroglutamate,
-(N-methyl)leucine-deoxo-proline-lactate-glutamine-cyclopentanoate,
-(N-methyl)leucine-deoxo-proline-alanine-leucine-pyroglutamate,
-(N-methyl)leucine-deoxo-proline-(N-methyl-alanine)-leucine-pyroglutamate,
-(N-methyl)leucine-dehydro-proline,
-(N-methyl)leucine-dehydro-proline-lactate,
-(N-methyl)leucine-dehydro-proline-pyruvate,
-(N-methyl)leucine-dehydro-proline-lactate-(a first fluorophore),
-(N-methyl)leucine-dehydro-proline-lactate-glutamine-pyroglutamate,
-(N-methyl)leucine-dehydro-proline-lactate-glutamine-cyclopentanoate,
-(N-methyl)leucine-dehydro-proline-alanine-leucine-pyroglutamate, and
-(N-methyl)leucine-dehydro-proline-(N-methyl-alanine)-leucine-pyroglutamate;

ii) either

(a) $R^3$ is selected from the group consisting of $-CH_3$ and -H; and $R^2$ is selected from the group consisting of an isoleucine side chain, a valine side chain, an alanine side chain, a norleucine side chain, a norvaline side chain, leucine side chain, a histidine side chain, a tryptophan side chain, an arginine side chain, a lysine side chain, a second fluorophore, and a substituent having the structure

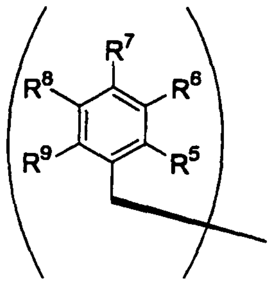

(b) $R^2$ and $R^3$ together are a substituent having the structure

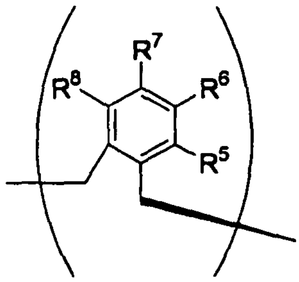

iii) each of $R^5$, $R^6$, $R^7$, $R^8$, and $R^9$, when present, is independently selected from the group consisting of -H, -OH, $-OCH_3$, -CO ($C_6H_5$), -Br, -I, -F, -Cl, $-CH_3$, and $-C_2H_5$;
iv) $R^4$ is selected from the group consisting of an isoleucine side chain and a valine side chain;
v) X is selected from the group consisting of -O- and -(NH)-;
vi) Y is selected from the group consisting of -H and a hydroxyl protecting group; and
vii) $R^{10}$ is selected from the group consisting of a leucine side chain and a lysine side chain.

**27.** The composition of claim 26, wherein $R^1$ is selected from the group consisting of

-(tert-butyloxycarbonyl),
-leucine,
-(N-methyl)leucine-(a first fluorophore),
-(N-methyl)leucine-(S)-proline,
-(N-methyl)leucine-(S)-proline-(S)-lactate-(a first fluorophore),
-(N-methyl)leucine-(S)-proline-(S)-lactate-glutamine-pyroglutamate,
-(N-methyl)leucine-(S)-proline-(S)-lactate-glutamine-cyclopentanoate,
-(N-methyl)leucine-(S)-proline-alanine-leucine-pyroglutamate,
-(N-methyl)leucine-(S)-proline-(N-methyl-alanine)-leucine-pyroglutamate,
-(N-methyl)leucine-deoxo-(S)-proline,
-(N-methyl)leucine-deoxo-(S)-proline-(S)-lactate,
-(N-methyl)leucine-deoxo-(S)-proline-pyruvate,
-(N-methyl)leucine-deoxo-(S)-proline-(S)-lactate-(a first fluorophore),
-(N-methyl)leucine-deoxo-(S)-proline-(S)-lactate-glutamine-pyroglutamate,
-(N-methyl)leucine-deoxo-(S)-proline-(S)-lactate-glutamine-cyclopentanoate,
-(N-methyl)leucine-deoxo-(S)-proline-alanine-leucine-pyroglutamate,
-(N-methyl)leucine-deoxo-(S)-proline-(N-methyl-alanine)-leucine-pyroglutamate,
-(N-methyl)leucine-dehydro-(S)-proline,
-(N-methyl)leucine-dehydro-(S)-proline-(S)-lactate,
-(N-methyl)leucine-dehydro-(S)-proline-pyruvate,
-(N-methyl)leucine-dehydro-(S)-proline-(S)-lactate-(a first fluorophore),
-(N-methyl)leucine-dehydro-(S)-proline-(S)-lactate-glutamine-pyroglutamate,
-(N-methyl)leucine-dehydro-(S)-proline-(S)-lactate-glutamine-cyclopentanoate,

-(N-methyl)leucine-dehydro-(S)-proline-alanine-leucine-pyroglutamate, and
-(N-methyl)leucine-dehydro-(S)-proline-(N-methyl-alanine)-leucine-pyroglutamate.

**28.** The composition of claim 26, wherein $R^1$ is selected from the group consisting of

-(tert-butyloxycarbonyl),
-leucine,
-(N-methyl)leucine-(a first fluorophore),
-(N-methyl)leucine-(S)-proline,
-(N-methyl)leucine-(S)-proline-(S)-lactate-(a first fluorophore),
-(N-methyl)leucine-(S)-proline-(S)-lactate-(S)-glutamine-(S)-pyroglutamate,
-(N-methyl)leucine-(S)-proline-(S)-lactate-(S)-glutamine-(S)-cyclopentanoate,
-(N-methyl)leucine-(S)-proline-(S)-alanine-(S)-leucine-(S)-pyroglutamate,
-(N-methyl)leucine-(S)-proline-(N-methyl-S-alanine)-(S)-leucine-(S)-pyroglutamate,
-(N-methyl)leucine-deoxo-(S)-proline,
-(N-methyl)leucine-deoxo-(S)-proline-(S)-lactate,
-(N-methyl)leucine-deoxo-(S)-proline-pyruvate,
-(N-methyl)leucine-deoxo-(S)-proline-(S)-lactate-(a first fluorophore),
-(N-methyl)leucine-deoxo-(S)-proline-(S)-lactate-(S)-glutamine-(S)-pyroglutamate,
-(N-methyl)leucine-deoxo-(S)-proline-(S)-lactate-(S)-glutamine-(S)-cyclopentanoate,
-(N-methyl)leucine-deoxo-(S)-proline-(S)-alanine-(S)-leucine-(S)-pyroglutamate, and
-(N-methyl)leucine-deoxo-(S)-proline-(N-methyl-S-alanine)-(S)-leucine-(S)-pyroglutamate,
-(N-methyl)leucine-dehydro-(S)-proline,
-(N-methyl)leucine-dehydro- (S)-proline-(S)-lactate,
-(N-methyl)leucine-deoxo-(S)-proline-pyruvate,
-(N-methyl)leucine-deoxo-(S)-proline-(S)-lactate- (a first fluorophore),
-(N-methyl)leucine-deoxo-(S)-proline-(S)-lactate-(S)-glutamine-(S)-pyroglutamate,
-(N-methyl)leucine-deoxo-(S)-proline-(S)-lactate-(S)-glutamine-(S)-cyclopentanoate,
-(N-methyl)leucine-deoxo-(S)-proline-(S)-alanine-(S)-leucine-(S)-pyroglutamate, and
-(N-methyl)leucine-deoxo-(S)-proline-(N-methyl-S-alanine)-(S)-leucine-(S)-pyroglutamate.

**29.** The composition of claim 26, wherein $R^2$ is

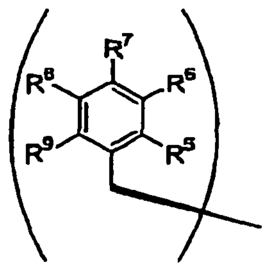

$R^3$ is methyl, $R^4$ is an isoleucine side chain, each of $R^5$, $R^6$, $R^8$, and $R^9$ is a hydride radical, $R^7$ is methoxy, $R^{10}$ is a leucine side chain, X is -O-, and Y is a hydride radical.

**30.** The composition of claim 26, wherein the didemnin analog is

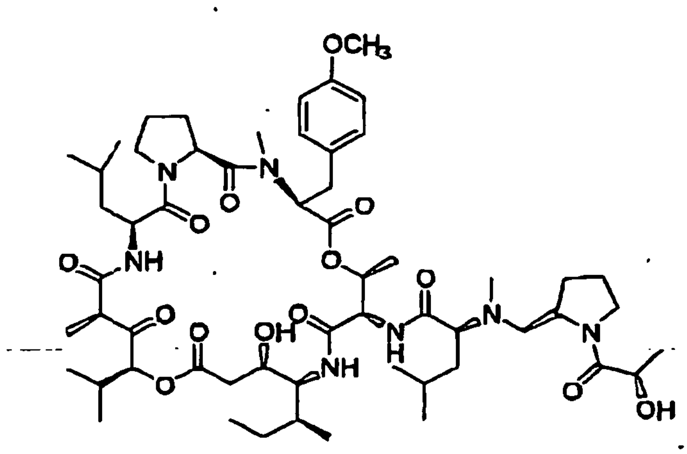

**31.** The composition of claim 26, wherein the didemnin analog is

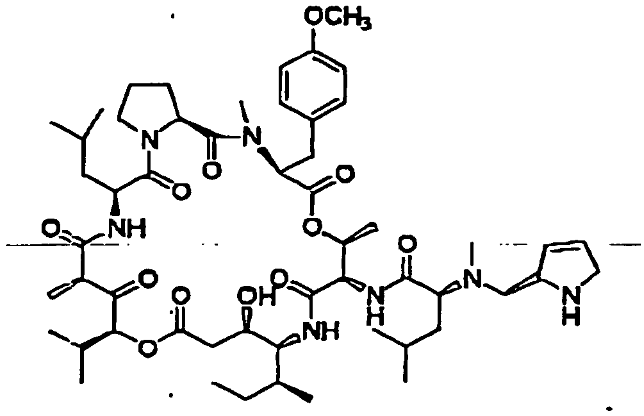

**32.** The composition of claim 26, wherein R is -(N-methyl)leucine-deoxo-(S)-proline-lactate.

**33.** The composition of claim 26, wherein Y is -H, and wherein $R^2$ has the structure

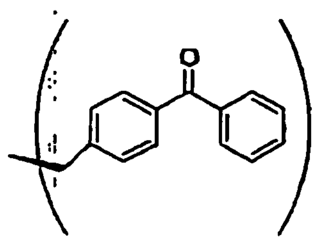

**34.** The composition of claim 26, wherein $R^2$ is a lysine side chain and Y is -H.

**35.** The composition of claim 26, wherein X is -(NH)-.

**36.** The composition of claim 26, further comprising a pharmaceutically acceptable carrier.

**37.** A support covalently attached with the didemnin analog of claim 26.

**38.** A method of making a tamandarin analog of claim 1 or a didemnin analog of claim 26, the improvement comprising

incorporating a deoxo-proline residue in place of a proline residue of the analog, wherein $R^1$ comprises a deoxo-proline residue.

**39.** The method of claim 38, wherein the analog comprises an (N-methyl)leucine-proline moiety and wherein the (N-methyl)leucine-proline moiety is replaced by an (N-methyl)leucine-deoxo-proline moiety.

**40.** The method of claim 39, wherein the (N-methyl)leucine-deoxoproline moiety is made by reducing the ester function of proline to an aldehyde function; and coupling the proline with the (N-methyl) leucine moiety by reductive amination to yield the (N-methy)leucine-deoxo-proline moiety.

**41.** The method of claim 40, wherein the amine moiety of the proline is protected with an amine-protecting group prior to the reductive amination.

**42.** The method of claim 40, wherein the ester function of the proline is reduced to an aldehyde function by contacting the proline with a strong base and then contacting the proline with an oxidizing agent.

**43.** The method of claim 40, wherein the reductive amination is performed in a non-aqueous solvent in the presence of a strong base and a carboxylic acid catalyst

**44.** A method of making a tamandarin analog of claim 1 or a didemnin analog of claim 26. the improvement comprising incorporating a dehydro-proline residue in place of a proline residue of the analog, wherein $R^1$ comprises a dehydro-proline residue.

**45.** The method of claim 44, wherein the analog comprises an (N-methyl)leucine-proline moiety and wherein the (N-methyl) leucine-proline moiety is replaced by an (N-mothyl)leucine-dehydro-proline moiety.

**46.** The method of claim 44, wherein the dehydro-proline residue is made by protecting the carboxyl and amino moieties of 4-hydroxyprolinate, alkyl-sulfonylating the 4-hydroxyl moiety, displacing the alkyl-sulfonate moiety with an aryl-selenyl moiety, oxidatively eliminating the aryl-selenyl moiety to yield a dehyro-proline moiety having protected carboxyl and amine moieties, and coupling the dehydro-proline moiety with an amine moiety of the analog.

**47.** The method of claim 46, wherein the alkyl-sulfonate moiety is a methyl-sulfonate moiety.

**48.** The method of claim 46, wherein the aryl selenyl moiety is a phenylselenyl moiety.

**49.** The method of claim 44, wherein the 4-hydroxyprolinate is trans-4-hydroxyprolinate.

**50.** A composition comprising a didemnin fragment having the structure

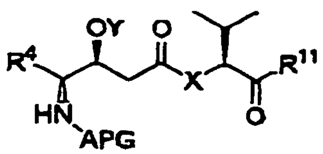

wherein:

i) Y is selected from the group consisting of-H and a hydroxyl protecting group;
ii) X is selected from the group consisting of -O- and -(NH)-;
iii) $R^4$ is selected from the group consisting of an isoleucine side chain and a valine side chain;
iv) APG is a amine protecting group; and
v) $R^{11}$ is selected from the group consisting of -OH, $-NH_2$, -O(allyl), -O(pentafluorophenyl), and a substituent having the structure

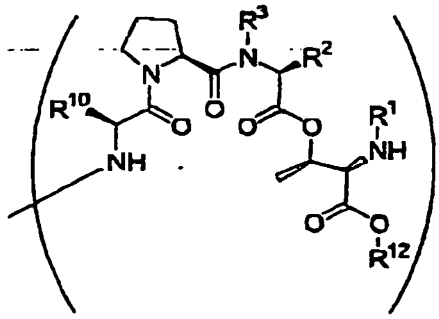

wherein:

a) $R^1$ is selected from the group consisting of-H, and an amine protecting group;
b) either

(I) $R^3$ is selected from the group consisting of $-CH_3$ and -H; and $R^2$ is selected from the group consisting of an isoleucine side chain, a valine side chain, an alanine side chain, a norleucine side chain, a norvaline side chain, a proline side chain, leucine side chain, a histidine side chain, a tryptophan side chain, an arginine side chain, a lysine side chain, a second fluorophore, and a substituent having the structure

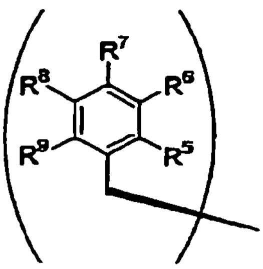

or
(II) $R^2$ and $R^3$ together are a substituent having the structure

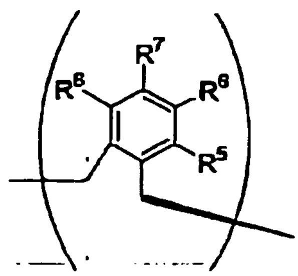

c) each of $R^6$, $R^6$, $R^7$, $R^8$, and $R^9$, when present, is independently selected from the group consisting of-H, -OH, $-OCH_3$, $-CO(C_6H_5)$, -Br, -I, -F, -Cl, $-CH_3$, and $-C_7H_5$;
d) $R^{10}$ is selected from the group consisting of a leucine side chain, a lysine side chain, and a protected lysine side chain; and
e) $R^{12}$ is selected from the group consisting of -H, and 2-(trimethylsilyl)ethoxycarbonyl.

**51.** A method of making a didemnin fragment, the method comprising coupling a first reactant having the structure

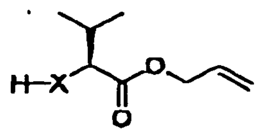

and a second reactant having the structure

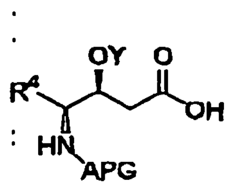

to yield a first didemnin fragment having the structure

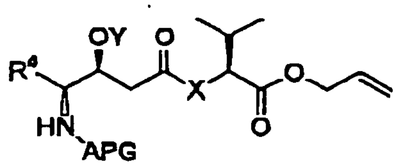

wherein X is selected from the group consisting of -O-, and -(NH)-: wherein APG is an amine protecting group; wherein Y is a hydroxyl protecting group; and wherein $R^4$ is selected from the group consisting of an isoleucine side chain and a valine side chain.

**52.** The method of claim 51, wherein Y is -triisopropylsilyl.

**53.** The method of claim 52, further comprising hydrolyzing the first didemnin fragment to yield a second didemnin fragment having the structure

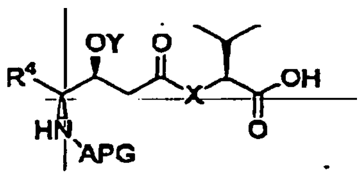

**54.** The method of claim 53, further comprising adding an activator to the carbonyl moiety of the second didemnin fragment to yield a third didemnin fragment having the structure

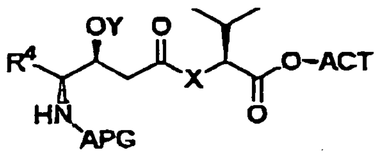

wherein -ACT is an activator.

**55.** The method of claim 54, further comprising coupling the third didemnin fragment and a third reactant having the structure

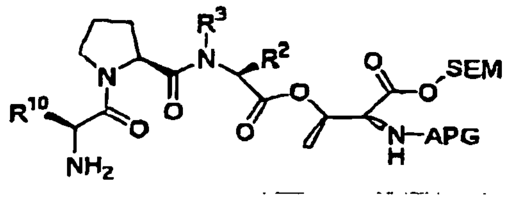

to yield a fourth didemnin fragment having the structure

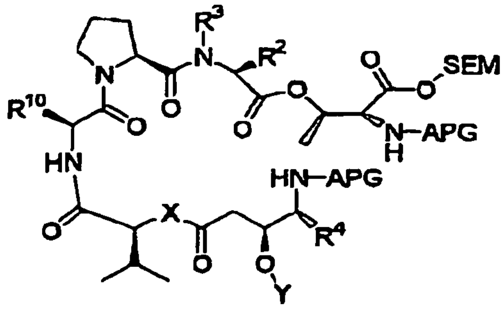

wherein:

i) either

(a) $R^3$ is selected from the group consisting of $-CH_3$ and -H; and $R^2$ is selected from the group consisting of an isoleucine side chain, a valine side chain, an alanine side chain, a norleucine side chain, a norvaline side chain, a proline side chain, leucine side chain, a histidine side chain, a tryptophan side chain, an arginine side chain, a lysine side chain, a second fluorophore, and a substituent having the structure

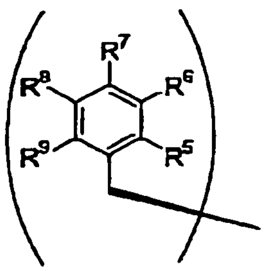

or

(b) $R^2$ and $R^3$ together are a substitutent having the structure

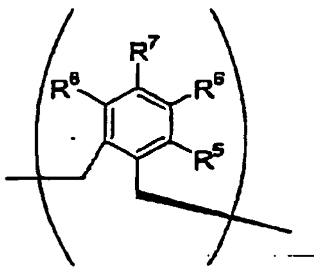

ii) each of $R^5$, $R^8$, $R^7$, $R^8$, and $R^9$, when present is independently selected from the group consisting of -H, -OH. -$OCH_3$ -$CO(C_6H_5)$, -Br. -I, -F, -Cl. -$CH_3$, and -$C_2H_5$;
iii) APG is an amine protecting group;
iv) SEM is 2-(trimethylsilyl)ethoxycarbonyl and
iii) $R^{10}$ is selected from the group consisting of a leucine side chain and a lysine side chain.

**56.** A method of making a didemnin analog, the method comprising removing the SEM moiety and the APG moiety protecting the amino group attached to the carbon atom to which R4 is bound, from the fourth didemnin fragment of claim 55 and cyclizing the fourth didemnin fragment to yield a first didemnin analog having the structure

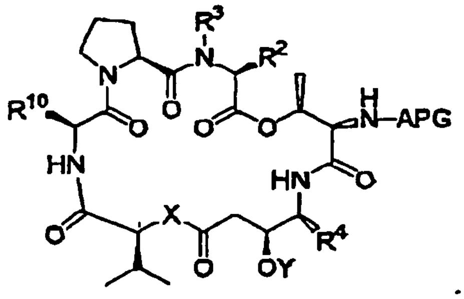

**57.** The method of claim 56, further comprising removing the APG group from the first didemnin analog to yield a second didemnin analog having the structure

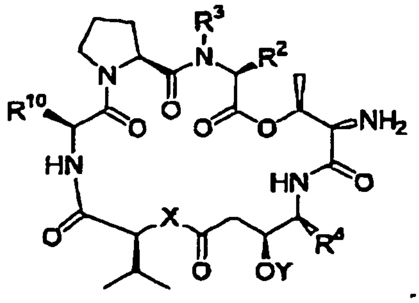

**58.** The method of claim 56, wherein the APG group is selected from the group consisting of a carbobenzyloxy moiety and a tert-butyloxycarbonyl moiety.

**59.** The method of claim 57, further comprising coupling the second didemnin analog and a fourth reagent having the structure

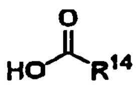

to yield a third didemnin analog having the structure

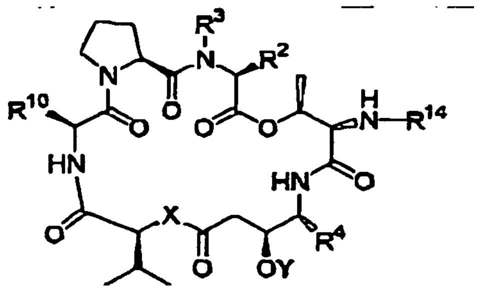

wherein $R^{14}$ is selected from the group consisting of

-leucine,
-(N-methyl)leucine,
-(N-methyl)leucine-(a first fluorophore),
-(N-methyl)leucine-proline,
-(N-methyl)leucine-proline-lactate,
-(N-methyl)leucine-proline-pyruvate,
-(N-methyl)leucine-proline-lactate-(a first fluorophore),
-(N-methyl)leucine-proline-lactate-glutamine-pyroglutamate,
-(N-methyl)leucine-proline-lactate-glutamine-cyclopentanoate,
-(N-methyl)leucine-proline-alanine-leucine-pyroglutamate,
-(N-methyl)leucine-proline-(N-methyl-alanine)-leucine-pyroglutamate,
-(N-methyl)leucine-deoxo-proline,
-(N-methyl)leucine-deoxo-proline-lactate,
-(N-methyl)leucine-deoxo-proline-pyruvate,
-(N-methyl)leucine-deoxo-proline-lactate-(a first fluorophore),
-(N-methyl)leucine-deoxo-proline-lactate-glutamine-pyroglutamate,
-(N-melhyl)leucine-deoxo-proline-lactate-glutamine-cyclopentanoate,
-(N-methyl)leucine-deoxo-proline-alanine-leucine-pyroglutamate,
-(N-methyl)leucine-deoxo-proline-(N-methyl-alanine)-leucine-pyroglutamate,
-(N-methyl)leucine-dehydro-proline,
-(N-methyl)leucine-dehydro-proline-lactate,
-(N-methyl)leucine-dehydro-proline-pyruvate,
-(N-methyl)leucine-dehydro-proline-lactate-(a first fluorophore),
-(N-methyl)leucine-dehydro-proline-lactate-glutamine-pyroglutamate,
-(N-methyl)leucine-dehydro-proline-lactate-glutamine-cyclopentanoate,
-(N-methyl)leucine-dehydro-proline-alanine-leucine-pyroglutamate,
-(N-methyl)leucine-dehydro-proline-(N-methyl-alanine}-leucine-pyroglutamate;

and
any one of the above moieties coupled with an enzyme-cleavable moiety that is cleavable by an enzyme selected from the group consisting of a carboxypeptidase, abeta-lactamase, a betagalactosidase, a penicillin V-amidase, a cytosine deaminase, a nitroreductase, an alkaline phosphatase, a beta-glucuronidase, and a catalytic antibody.

**60.** The method of claim 59, wherein Y is a hydroxyl protecting group, the method further comprising removing the hydroxyl protecting group from the second didemnin analog to yield a fourth didemnin analog having the structure

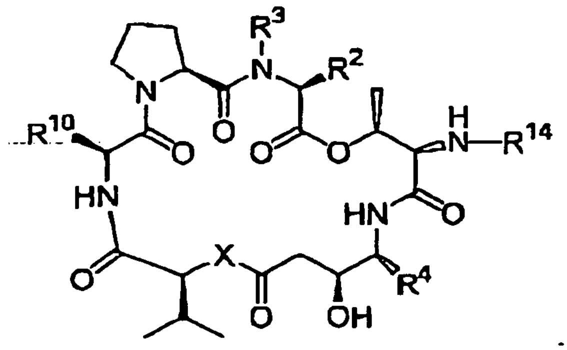

**61.** Use of any one of the compositions of claim 1, 23, 26, or 50 for preparing a medicament for inhibiting protein synthesis in a cell.

**62.** Use of any one of the compositions of claim 1, 23, 26, or 50 for preparing a medicament for inhibiting growth of a cell.

**63.** Use of any one of the compositions of claim 1, 23, 26, or 50 for preparing a medicament for inhibiting proliferation of a cell.

**64.** Use of any one of the compositions of claim 1, 23, 26, or 50 for preparing a medicament for inhibiting tumorigenesis in a cell.

**65.** Use of any one of the compositions of claim 1, 23, 26, or 50 for preparing a medicament for inhibiting enhancing apoptosis of a cell.

**66.** A composition comprising a tamandarin analog having the structure

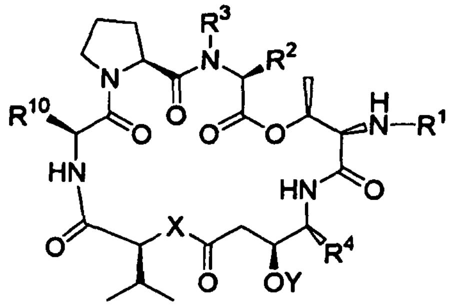

wherein:

i) $R^1$ is selected from the group consisting of

-H,
-(tert-butyloxycarbonyl),
-leucine,
-(N-methyl)leucine,
-(N-methyl)leucine-(a first fluorophore),
-(N-methyl)leucine-proline,
-(N-methyl)leucine-proline-pyruvate,
-(N-methyl)leucine-proline-lactate-(a first fluorophore),
-(N-methyl)leucine-proline-lactate-glutamine-pyroglutamate,
-(N-methyl)leucine-proline-lactate-glutamine-cyclopentanoate,
-(N-methyl)leucine-proline-alanine-leucine-pyroglutamate,
-(N-methyl)leucine-proline-(N-methyl-alanine)-leucine-pyroglutamate,

-(N-methyl)leucine-deoxo-proline,
-(N-methyl)leucine-deoxo-proline-lactate,
-(N-methyl)leucine-deoxo-proline-pyruvate,
-(N-methyl)leucine-deoxo-proline-lactate-(a first fluorophore),
-(N-methyl)leucine-deoxo-proline-lactate-glutamine-pyroglutamate,
-(N-methyl)leucine-deoxo-proline-lactate-glutamine-cyclopentanoate,
-(N-methyl)leucine-deoxo-proline-alanine-leucine-pyroglutamate,
-(N-methyl)leucine-deoxo-proline-(N-methyl-alanine)-leucine-pyroglutamate,
-(N-methyl)leucine-dehydro-proline,
-(N-methyl)leucine-dehydro-proline-lactate,
-(N-methyl)leucine-dehydro-proline-pyruvate,
-(N-methyl)leucine-dehydro-proline-lactate-(a first fluorophore),
-(N-methyl)leucine-dehydro-proline-lactate-glutamine-pyroglutamate,
-(N-methyl)leucine-dehydro-proline-lactate-glutamine-cyclopentanoate,
-(N-methyl)leucine-dehydro-proline-alanine-leucine-pyroglutamate, and
-(N-methyl)leucine-dehydro-proline-(N-methyl-alanine)-leucine-pyroglutamate;

ii) either

(a) $R^3$ is selected from the group consisting of $-CH_3$ and -H; and $R^2$ is selected from the group consisting of an isoleucine side chain, a valine side chain, an alanine side chain, a norleucine side chain, a norvaline side chain, a leucine side chain, a histidine side chain, a tryptophan side chain, an arginine side chain, a lysine side chain, a second fluorophore, and a substituent having the structure

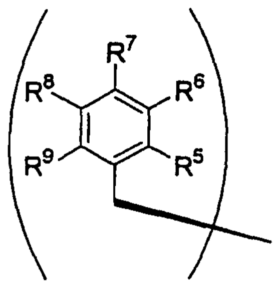

,

or

(b) $R^2$ and $R^3$ together are a substituent having the structure

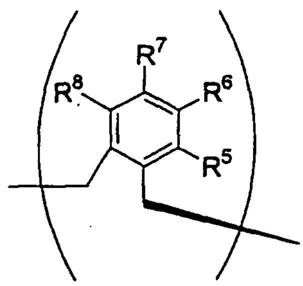

iii) each of $R^5$, $R^6$, $R^7$, $R^8$, and $R^9$, when present, is independently selected from the group consisting of -H, -OH, $-OCH_3$, $-CO(C_6H_5)$, -Br, -I, -F, -Cl, $-CH_3$, and $C_2H_5$;
iv) $R^4$ is a valine side chain;
v) X is selected from the group consisting of -O- and -(NH)-;
vi) Y is selected from the group consisting of -H and a hydroxyl protecting group;
vii) $R^{10}$ is selected from the group consisting of a leucine side chain and a lysine side chain; and
viii) the molecule is not tamandarin B.

**67.** The composition of claim 66, wherein $R^4$ is a valine side chain, Y is -H, and X is -O-.

**68.** A tamandarin analog selected from the group consisting of

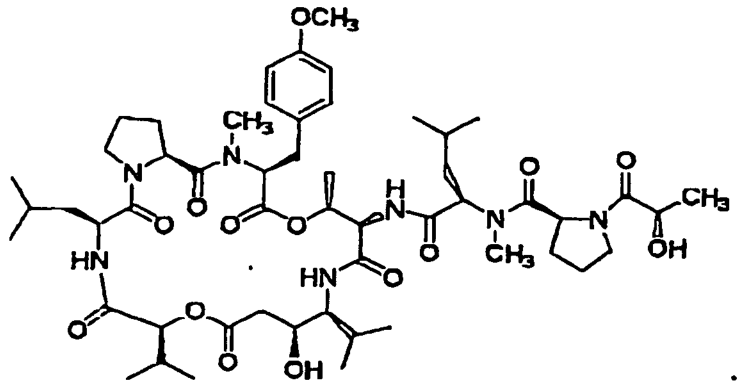

;

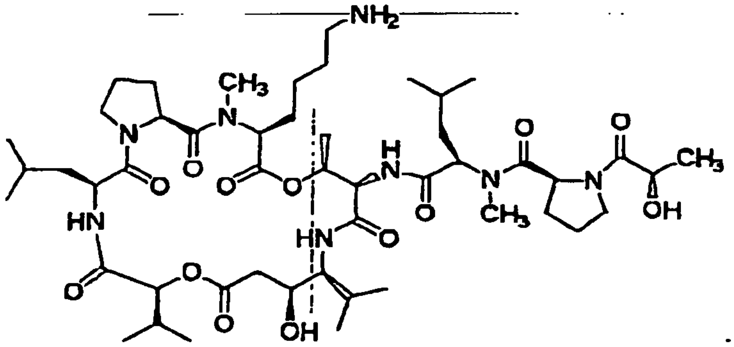

;

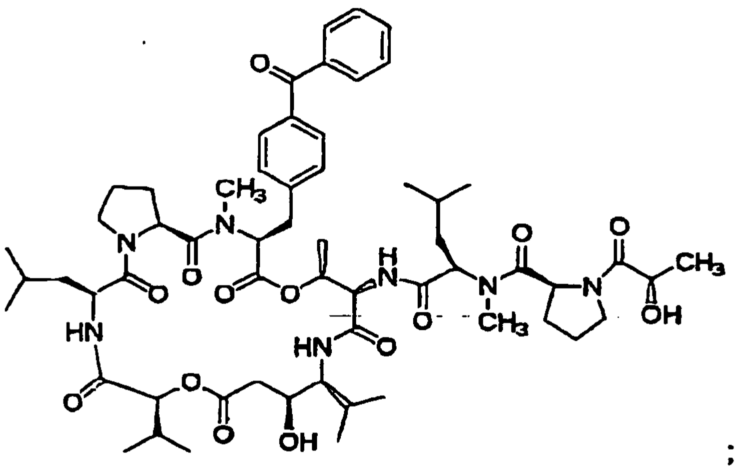

;

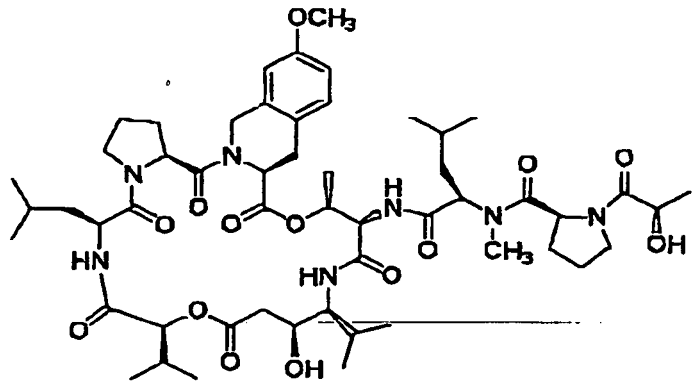

;

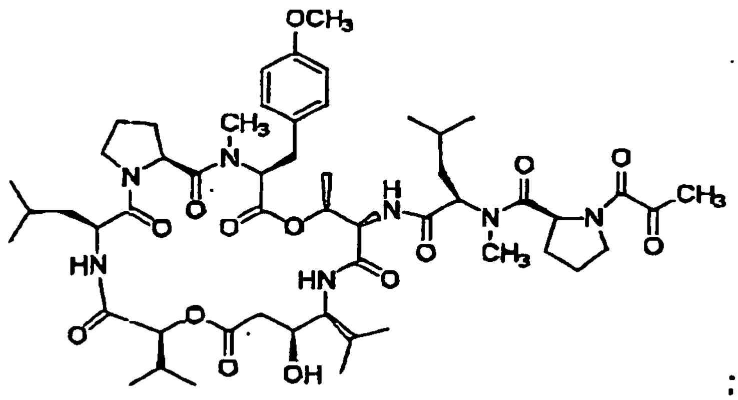

;

and

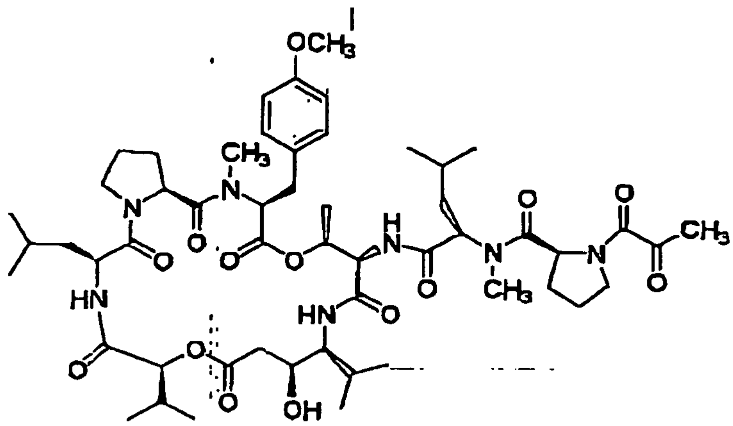

.

**69.** The composition of claim 67, wherein the tamandarin analog is

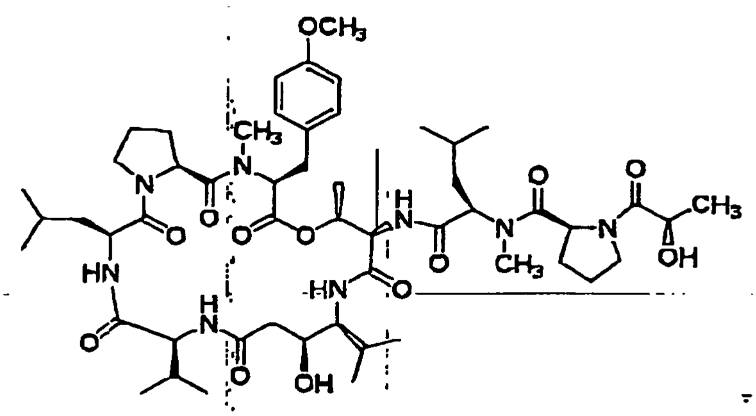

**70.** A composition comprising a didemnin analog having the structure

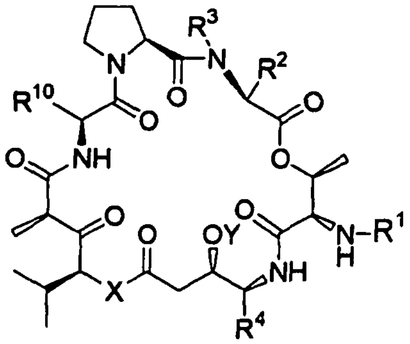

wherein:

i) $R^1$ is selected from the group consisting of

-H,
-(tert-butyloxycarbonyl),
-leucine,
-(N-methyl)leucine,
-(N-methyl)leucine-(a first fluorophore),
-(N-methyl)leucine-proline,
-(N-methyl)leucine-proline-lactate,
-(N-methyl)leucine-proline-pyruvate,
-(N-methyl)leucine-proline-lactate-(a first fluorophore),
-(N-methyl)leucine-proline-lactate-glutamine-pyroglutamate,
-(N-methyl)leucine-proline-lactate-glutamine-cyclopentanoate,
-(N-methyl)leucine-proline-alanine-leucine-pyroglutamate,
-(N-methyl)leucine-proline-(N-methyl-alanine)-leucine-pyroglutamate,
-(N-methyl)leucine-deoxo-proline,
-(N-methyl)leucine-deoxo-proline-lactate,
-(N-methyl)leucine-deoxo-proline-pyruvate,
-(N-methyl)leucine-deoxo-proline-lactate-(a first fluorophore),
-(N-methyl)leucine-deoxo-proline-lactate-glutamine-pyroglutamate,
-(N-methyl)leucine-deoxo-proline-lactate-glutamine-cyclopentanoate,
-(N-methyl)leucine-deoxo-proline-alanine-leucine-pyroglutamate,
-(N-methyl)leucine-deoxo-proline-(N-methyl-alanine)-leucine-pyroglutamate,
-(N-methyl)leucine-dehydro-proline,
-(N-methyl)leucine-dehydro-proline-lactate,

-(N-methyl)leucine-dehydro-proline-pyruvate,
-(N-methyl)leucine-dehydro-proline-lactate-(a first fluorophore),
-(N-methyl)leucine-dehydro-proline-lactate-glutamine-pyroglutamate,
-(N-methyl)leucine-dehydro-proline-lactate-glutamine-cyclopentanoate,
-(N-methyl)leucine-dehydro-proline-alanine-leucine-pyroglutamate, and
-(N-methyl)leucine-dehydro-proline-(N-methyl-alanine)-leucine-pyroglutamate;

ii) either

(a) $R^3$ is selected from the group consisting of $-CH_3$ and -H; and $R^2$ is selected from the group consisting of an isoleucine side chain, a valine side chain, an alanine side chain, a norleucine side chain, a norvaline side chain, a leucine side chain, a histidine side chain, a tryptophan side chain, an arginine side chain, a lysine side chain, a second fluorophore, and a substituent having the structure

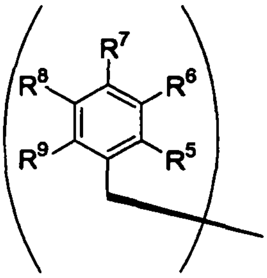

(b) $R^2$ and $R^3$ together are a substituent having the structure

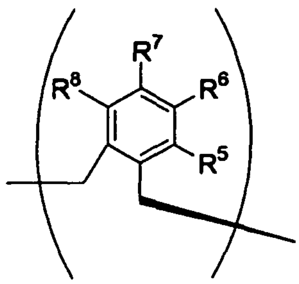

iii) each of $R^5$, $R^6$, $R^7$, $R^8$, and $R^9$, when present, is independently selected from the group consisting of -H, -OH, $-OCH_3$, -CO $(C_6H_5)$, -Br, -I, -F, -Cl, $-CH_3$, and $-C_2H_5$;
iv) $R^4$ is a valine side chain;
v) X is selected from the group consisting of -O- and -(NH)-;
vi) Y is selected from the group consisting of-H and a hydroxyl protecting group;
vii) $R^{10}$ is selected from the group consisting of a leucine side chain and a lysine side.

**71.** The composition of claim 70. wherein $R^1$ is selected from the group consisting of

-H,
-(tert-butyloxycarbonyl),
-leucine,
-(N-methyl)leucine,
-(N-methyl)leucine-(a first fluorophore),
-(N-methyl)leucine-(S)-proline,
-(N-methyl)leucine-(S)-proline-(S)-lactate,
-(N-methyl)leucine-(S)-proline-pyruvate,
-(N-methyl)leucine-(S)-proline-(S)-lactate-(a first fluorophore),

-(N-methyl)leucine-(S)-proline-(S)-lactate-glutamine-pyroglutamate,
-(N-methyl)leucine-(S)-proline-(S)-lactate-glutamine-cyclopentanoate,
-(N-methyl)leucine-(S)-proline-alanine-leucine-pyroglutamate,
-(N-methyl)leucine-(S)-proline-(N-methyl-alanine)-leucine-pyroglutamate,
-(N-methyl)leucine-deoxo-(S)-proline,
-(N-methyl)leucine-deoxo-(S)-proline-(S)-lactate,
-(N-methyl)leucine-deoxo-(S)-proline-pyruvate,
-(N-methyl)leucine-deoxo-(S)-proline-(S)-lactate-(a first fluorophore).
-(N-methyl)leucine-deoxo-(S)-proline-(S)-lactate-glutamine-pyroglutamate,
-(N-methyl)leucine-deoxo-(S)-proline-(S)-lactate-glutamine-cyclopentanoate,
-(N-methyl)leucine-deoxo-(S)-proline-alanine-leucine-pyroglutamate,
-(N-methyl)leucine-deoxo-(S)-proline-(N-methyl-alanine-)-leucine-pyroglutamate,
-(N-methyl)leucine-dehydro-(S)-proline,
-(N-methyl)leucine-dehydro-(S)-prolin-(S)-lactate,
-(N-methyl)leucine-dehydro-(S)-proline-pyruvate,
-(N-methyl)leucine-dehydro-(S)-proline-(S)-lactate-(a first fluorophore),
-(N-methyl)leucine-dehydro(S)-proline-(S)-lactate-glutamine-pyroglutamate,
-(N-methyl)leucine-dehydro-(S)-proline-(S)-lactate-glutamine-cyclopentanoate,
-(N-methyl)leucine-dehydro-(S)-proline-alanine-leucine-pyroglutamate,
-(N-methyl)leucine-dehydro-(S)-proline-(N-methyl-alanine)-leucine-pyroglutamate,
-(N-methyl)leucine-(S)-proline-(S)-lactate-(S)-glutamine-(S)-pyroglutamate,
-(N-methyl)leucine-(S)-proline-(S)-lactate-(S)-glutamine-(S)-cyclopentanoate,
-(N-methyl)leucine-(S)-proline-(S)-alanine-(S)-leucine-(S)-pyroglutamate,
-(N-methyl)leucine-(S)-proline-(N-methyl-S-alanine)-(S)-leucine-(S)-pyroglutamate,
-(N-methyl)leucine-deoxo-(S)-proline-(S)-lactate-(S)-glutamine-(S)-pyroglutamate,
-(N-methyl)leucine-deoxo-(S)-proline-(S)-lactate-(S)-glutamine-(S)-cyclopentanoate,
-(N-methyl)leucine-deoxo-(S)-proline-(S)-alanine-(S)-leucine-(S)-pyruglutamate, and
-(N-methyl)leucine-deoxo-(S)-proline-(N-methyl-S-alanine)-(S)-leucine-(S)-pyroglutamate.
-(N-methyl)leucine-deoxo-(S)-proline-pyruvate,
-(N-methyl)leucine-deoxo-(S)-proline-(S)-lactate-(a first fluorophore),
-(N-methyl)leucine-deoxo-(S)-proline-(S)-lactate-(S)-glutamine-(S)-pyroglutamate,
-(N-methyl)leucine-deoxo-(S)-proline-(S)-lactate-(S)-glutamine-(S)-cyclopentanoate.
-(N-methyl)leucine-deoxo-(S)-proline-(S)-alanine-(S)-leucine-(S)-pyroglutamate, and
-(N-methyl)leucine-deoxo-(S)-proline-(N-methyl-S-alanine)-(S)-leucine-(S)-pyroglutamate.

**72.** The composition of claim 70, wherein $R^2$ is

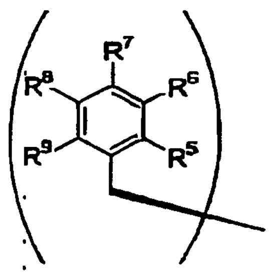

$R^3$ is Methyl, each of $R^5$, $R^6$, $R^8$, and $R^9$ is a hydride radical, $R^7$ is methoxy, $R^{10}$ is a leucine side chain, X is -O-, and Y is a hydride radical.

**73.** The composition of claim 70, wherein R is -(N-methyl)leucine-deoxo-(S)-proline-lactate.

**74.** The composition of claim 70, wherein Y is -H. and wherein $R^2$ has the structure

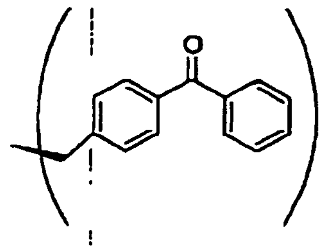

**75.** The composition of claim 70, wherein $R^2$ is a lysine side chain and Y is -H.

**76.** The composition of claim 70, wherein X is -(NH)-.

**77.** The composition of claim 70, further comprising a pharmaceutically acceptable carrier.

**78.** A support covalently attached with the didemnin analog of claim 70.

**Patentansprüche**

**1.** Eine Zusammensetzung umfassend ein Tamandarin-Analog mit der Struktur

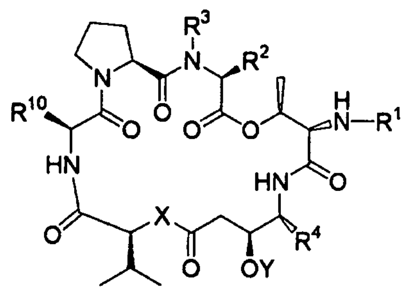

wobei:

i) $R^1$ ausgewählt ist aus der Gruppe von:

-H,
-(tert-Butyloxycarbonyl),
-Leucin,
-(N-Methyl)leucin,
-(N-Methyl)leucin-(ein erster Fluorophor),
-(N-Methyl)leucin-prolin,
-(N-Methyl)leucin-prolin-lactat,
-(N-Methyl)leucin-prolin-pyruvat,
-(N-Methyl)leucin-prolin-lactat-(ein erster Fluorophor),
-(N-Methyl)leucin-prolin-lactat-glutamin-pyroglutamat,
-(N-Methyl)leucin-prolin-lactat-glutamin-cyclopentanoat,
-(N-Methyl)leucin-prolin-alanin-leucin-pyroglutamat,
-(N-Methyl)leucin-prolin-(N-methyl-alanin)-leucin-pyroglutamat,
-(N-Methyl)leucin-deoxo-prolin,
-(N-Methyl)leucin-deoxo-prolin-lactat,
-(N-Methyl)leucin-deoxo-prolin-pyruvat,
-(N-Methyl)leucin-deoxo-prolin-lactat-(ein erster Fluorophor),
-(N-Methyl)leucin-deoxo-prolin-lactat-glutamin-pyroglutamat,
-(N-Methyl)leucin-deoxo-prolin-lactat-glutamin-cyclopentanoat,

-(N-Methyl)leucin-deoxo-prolin-alanin-leucin-pyroglutamat,
-(N-Methyl)leucin-deoxo-prolin-(N-methyl-alanin)-leucin-pyroglutamat,
-(N-Methyl)leucin-dehydro-prolin,
-(N-Methyl)leucin-dehydro-prolin-lactat,
-(N-Methyl)leucin-dehydro-prolin-pyruvat,
-(N-Methyl)leucin-dehydro-prolin-lactat-(ein erster Fluorophor),
-(N-Methyl)leucin-dehydro-prolin-lactat-glutamin-pyroglutamat,
-(N-Methyl)leucin-dehydro-prolin-lactat-glutamin-cyclopentanoat,
-(N-Methyl)leucin-dehydro-prolin-alanin-leucin-pyroglutamat, und
-(N-Methyl)leucin-dehydro-prolin-(N-methyl-alanin)-leucin-pyroglutamat;

ii) entweder

(a) $R^3$ ausgewählt ist aus der Gruppe von: $-CH_3$ und -H; und $R^2$ ausgewählt ist aus der Gruppe von: einer Isoleucin- Seitenkette, a Valin Seitenkette, einer Alanin-Seitenkette, einer Norleucin-Seitenkette, einer Norvalin-Seitenkette, einer Leucin-Seitenkette, einer Histidin-Seitenkette, einer Tryptophan-Seitenkette, einer Arginin-Seitenkette, einer Lysine-Seitenkette, einem zweiten Fluorophor, und einem Substituenten mit der Struktur

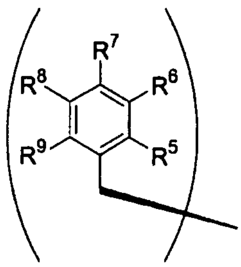

oder
(b) $R^2$ und $R^3$ together are einem Substituenten mit der Struktur

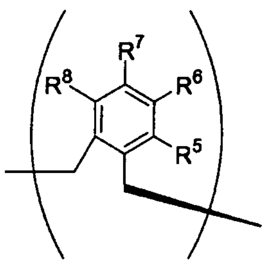

iii) jeder Rest von $R^5$, $R^6$, $R^7$, $R^8$, und $R^9$, wenn vorliegend, unabhängig ausgewählt ist aus der Gruppe bestehend aus -H, -OH, $-OCH_3$, $-CO(C_6H_5)$, -Br, -I, -F, -Cl, $-CH_3$, und $C_2H_5$;
iv) $R^4$ eine Isoleucin-Seitenkette ist;
v) X ausgewählt ist aus der Gruppe von: -O- und -(NH)-;
vi) Y ausgewählt ist aus der Gruppe von: -H und einer Hydroxyl-Schutzgruppe;
vii) $R^{10}$ ausgewählt ist aus der Gruppe von: einer Leucin-Seitenkette und einer Lysine- Seitenkette; und
viii) das Molekül nicht Tamandarin A ist.

**2.** Die Zusammensetzung von Anspruch 1, wobei $R^1$ ausgewählt ist aus der Gruppe von:

-H,
-(tert-Butyloxycarbonyl),
-Leucin,

-(N-Methyl)leucin,
-(N-Methyl)leucin-(ein erster Fluorophor),
-(N-Methyl)leucin-(S)-prolin,
-(N-Methyl)leucin-(S)-prolin-(S)-lactat,
-(N-Methyl)leucin-(S)-prolin-pyruvat,
-(N-Methyl)leucin-(S)-prolin-(S)-lactat-(ein erster Fluorophor),
-(N-Methyl)leucin-(S)-prolin-(S)-lactat-glutamin-pyroglutamat,
-(N-Methyl)leucin-(S)-prolin-(S)-lactat-glutamin-cyclopentanoat,
-(N-Methyl)leucin-(S)-prolin-alanin-leucin-pyroglutamat,
-(N-Methyl)leucin-(S)-prolin-(N-methyl-alanin)-leucin-pyroglutamat,
-(N-Methyl)leucin-deoxo-(S)-prolin,
-(N-Methyl)leucin-deoxo-(S)-prolin-(S)-lactat,
-(N-Methyl)leucin-deoxo-(S)-prolin-pyruvat,
-(N-Methyl)leucin-deoxo-(S)-prolin-(S)-lactat-(ein erster Fluorophor),
-(N-Methyl)leucin-deoxo-(S)-prolin-(S)-lactat-glutamin-pyroglutamat,
-(N-Methyl)leucin-deoxo-(S)-prolin-(S)-lactat-glutamin-cyclopentanoat,
-(N-Methyl)leucin-deoxo-(S)-prolin-alanin-leucin-pyroglutamat,
-(N-Methyl)leucin-deoxo-(S)-prolin-(N-methyl-alanin)-leucin-pyroglutamat,
-(N-Methyl)leucin-dehydro-(S)-prolin,
-(N-Methyl)leucin-dehydro-(S)-prolin-(S)-lactat,
-(N-Methyl)leucin-dehydro-(S)-prolin-pyruvat,
-(N-Methyl)leucin-dehydro-(S)-prolin-(S)-lactat-(ein erster Fluorophor),
-(N-Methyl)leucin-dehydro-(S)-prolin-(S)-lactat-glutamin-pyroglutamat,
-(N-Methyl)leucin-dehydro-(S)-prolin-(S)-lactat-glutamin-cyclopentanoat,
-(N-Methyl)leucin-dehydro-(S)-prolin-alanin-leucin-pyroglutamat, und
-(N-Methyl)leucin-dehydro-(S)-prolin-(N-methyl-alanin)-leucin-pyroglutamat.

**3.** Die Zusammensetzung von Anspruch 1, wobei $R^1$ ausgewählt ist aus der Gruppe von:

-H,
-(tert-Butyloxycarbonyl),
-Leucin,
-(N-Methyl)leucin,
-(N-Methyl)leucin-(ein erster Fluorophor),
-(N-Methyl)leucin-(S)-prolin,
-(N-Methyl)leucin-(S)-prolin-(S)-lactat,
-(N-Methyl)leucin-(S)-prolin-pyruvat,
-(N-Methyl)leucin-(S)-prolin(S)-lactat-(ein erster Fluorophor),
-(N-Methyl)leucin-(S)-profin-(S)-lactat-(S)-glutamin-(S)-pyroglutamat,
-(N-Methyl)leucin-(S)-prolin-(S)-lactat-(S)-glutamin-(S)-cyclopentanoat,
-(N-Methyl)leucin-(S)-prolin-(S)-alanin-(S)-leucin-(S)-pyroglutamat,
-(N-Methyl)leucin-(S)-prolin-(N-methyl-S-alanin)-(S)-leucin-(S)-pyroglutamat,
-(N-Methyl)leucin-deoxo-(S)-prolin,
-(N-Methyl)leucin-deoxo-(S)-prolin-(S)-lactat,
-(N-Methyl)leucin-deoxo-(S)-prolin-pyruvat,
-(N-Methyl)leucin-deoxo-(S)-prolin-(S)-lactat-(ein erster Fluorophor),
-(N-Methyl)leucin-deoxo-(S)-prolin-(S)-lactat-(S)-glutamin-(S)-pyroglutamat,
-(N-Methyl)leucin-deoxo-(S)-prolin-(S)-lactat-(S)-glutamin-(S)-cyclopentanoat,
-(N-Methyl)leucin-deoxo-(S)-prolin-(S)-alanin-(S)-leucin-(S)-pyroglutamat, und
-(N-Methyl)leucin-deoxo-(S)-prolin-(N-methyl-S-alanin)-(S)-leucin-(S)-pyroglutamat,
-(N-Methyl)leucin-dehydro-(S)-prolin,
-(N-Methyl)leucin-dehydro-(S)-prolin-(S)-lactat,
-(N-Methyl)leucin- dehydro-(S)-prolin-pyruvat,
-(N-Methyl)leucin- dehydro-(S)-prolin-(S)-lactat- (ein erster Fluorophor),
-(N-Methyl)leucin-dehydro-(S)-prolin-(S)-lactat-(S)-glutamin-(S)-pyroglutamat,
-(N-Methyl)leucin-dehydro-(8)-prolin-(S)-lactat -(8)-glutamin-(S)-cyclopentanoat,
-(N-Methyl)leucin-dehydro-(S)-prolin-(S)-alanin-(S)-leucin-(S)-pyroglutamat, und
-(N-Methyl)leucin-dehydro-(S)-prolin-(N-methyl-S-alanin)-(S)-leucin-(S)-pyroglutamat.

4. Die Zusammensetzung von Anspruch 1, wobei $R^2$

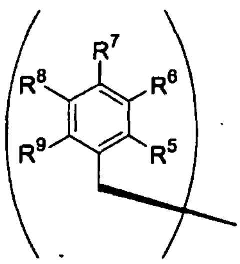

ist,
$R^3$ Methyl ist, jeder Rest von $R^5$, $R^6$, $R^8$, und $R^9$ ein Hydrid Radikal ist, $R^7$ Methoxy ist, $R^{10}$ eine Leucin-Seitenkette ist, X -O- ist, und Y ein Hydrid Radikal ist.

5. Die Zusammensetzung von Anspruch 1, wobei das Tamandarin-Analog

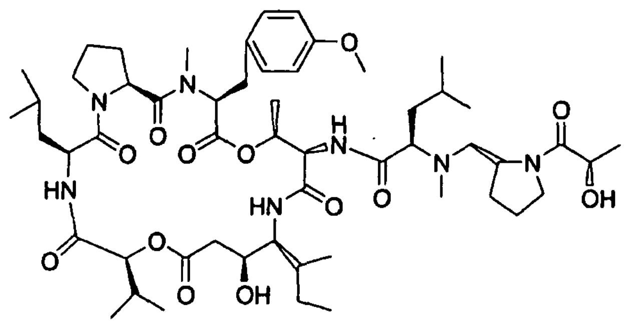

ist.

6. Die Zusammensetzung von Anspruch 1, wobei das Tamandarin-Analog

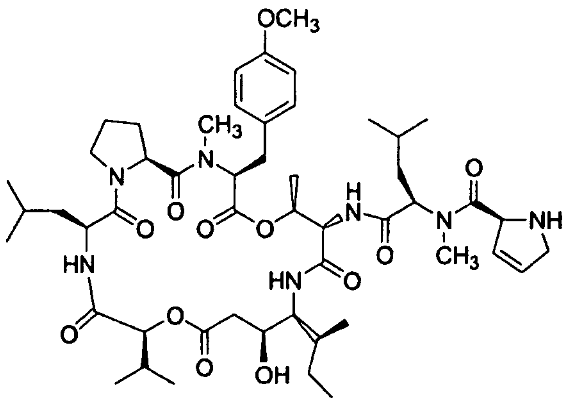

ist.

7. Die Zusammensetzung von Anspruch 1, wobei $R^1$ -(N-Methyl)leucin-deoxo-(S)-prolin-lactat ist.

8. Die Zusammensetzung von Anspruch 1, wobei $R^1$ -(N-Methyl-R-leucin)ist.

9. Die Zusammensetzung von Anspruch 1, wobei $R^1$ -(N-Methyl-R-leucin)-(S)-prolin-(S)-lactat-(S)-glutamin-(S)-pyroglutamat ist, Y -H ist, und X -O- ist.

10. Die Zusammensetzung von Anspruch 9, wobei das Tamandarin-Analog

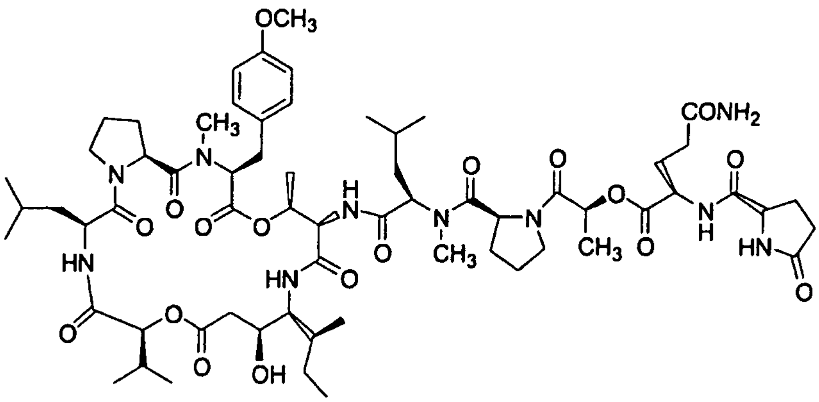

oder

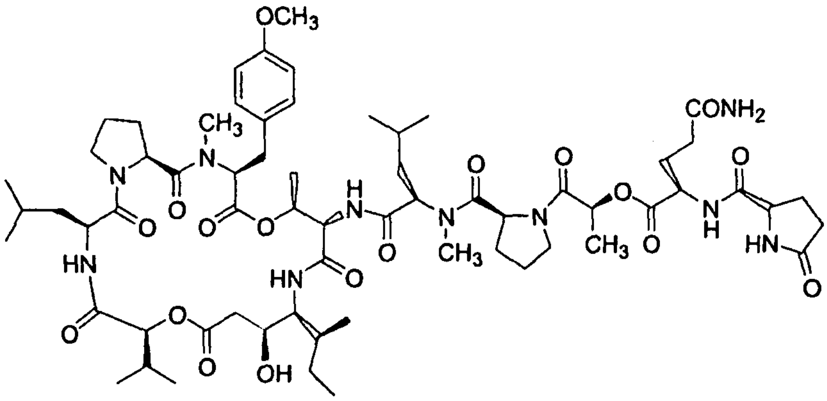

ist.

11. Die Zusammensetzung von Anspruch 1, wobei $R^1$ -(N-Methyl)leucin-(S)-prolin-(S)-lactat- (ein erster Fluorophor)ist.

12. Die Zusammensetzung von Anspruch 11, wobei das Tamandarin-Analog ausgewählt ist aus der Gruppe von:

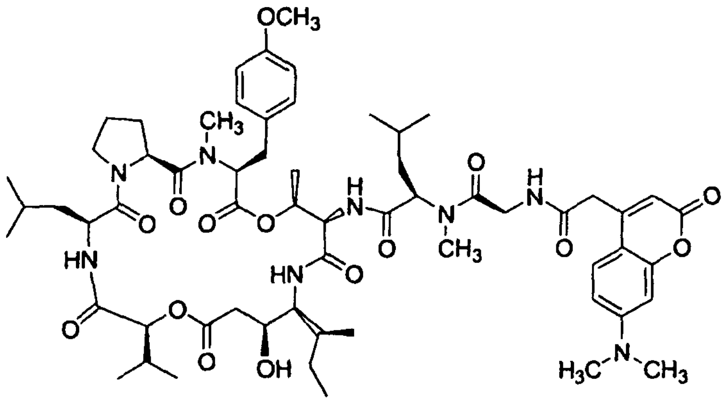

;

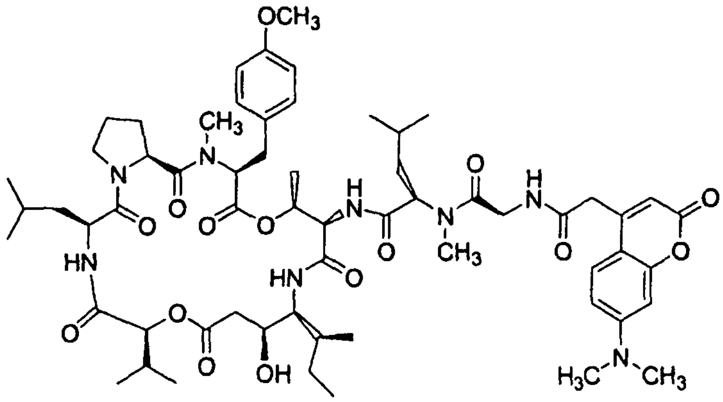

;

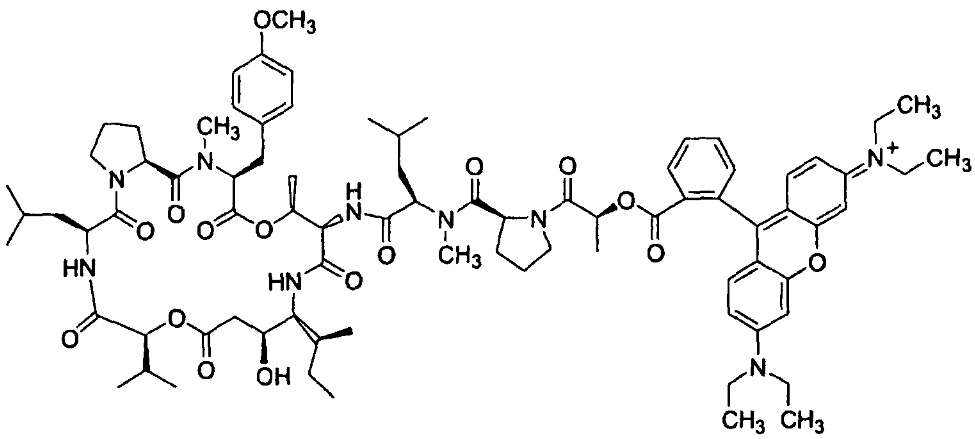

;

und

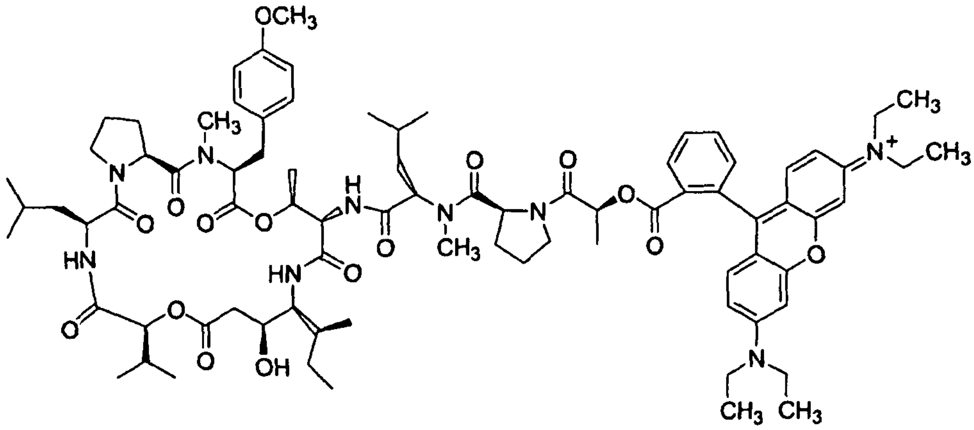

.

13. Die Zusammensetzung von Anspruch 1, wobei $R^1$ -(N-Methyl)leucin-(S)-prolinpyruvat ist.

14. Die Zusammensetzung von Anspruch 13, wobei das Tamandarin-Analog

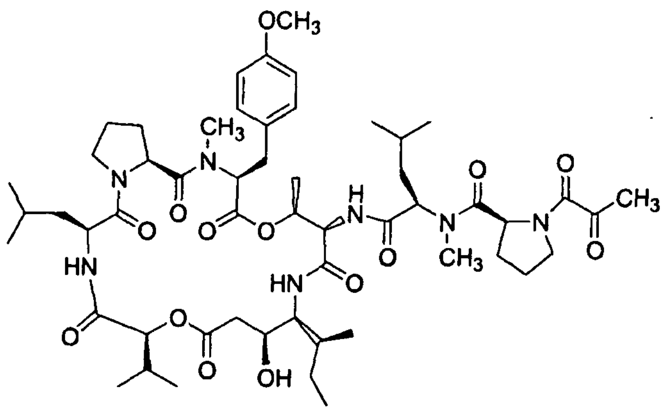

oder

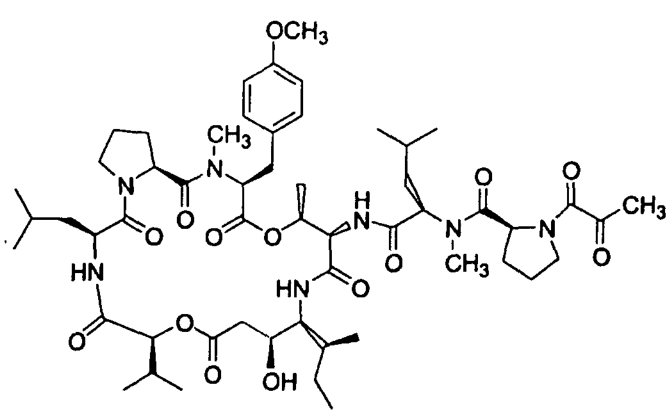

ist.

15. Die Zusammensetzung von Anspruch 1, wobei Y -H ist, und wobei $R^2$ die Struktur

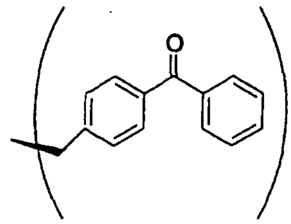

aufweist.

**16.** Die Zusammensetzung von Anspruch 1, wobei $R^2$ eine Lysin-Seitenkette ist und Y -H ist.

**17.** Die Zusammensetzung von Anspruch 1, wobei das Tamandarin-Analog die folgende Struktur

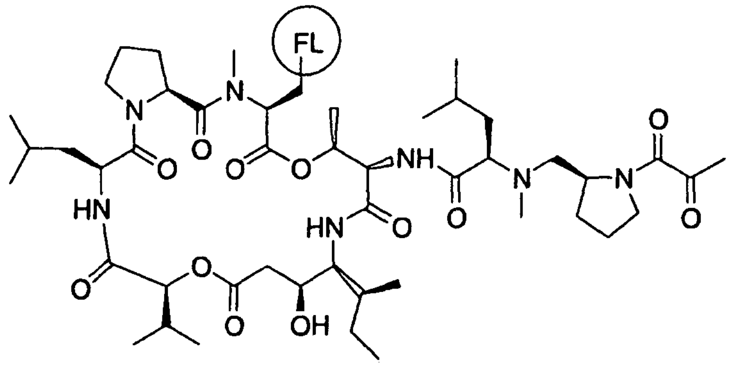

aufweist,
wobei FL ein Fluorophor ist.

**18.** Die Zusammensetzung von Anspruch 1, wobei X -(NH)- Ist.

**19.** Die Zusammensetzung von Anspruch 1, wobei das Tamandarin-Analog ausgewählt ist aus der Gruppe von:

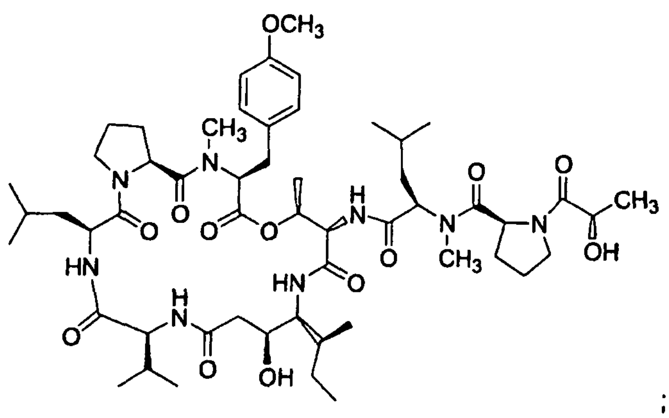

;

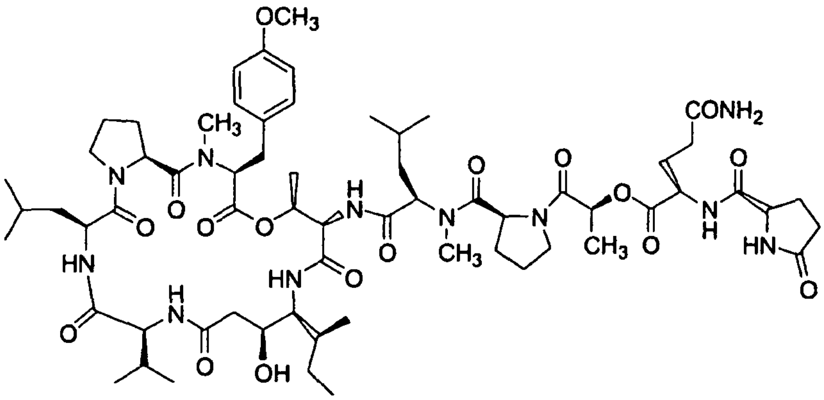

;

und

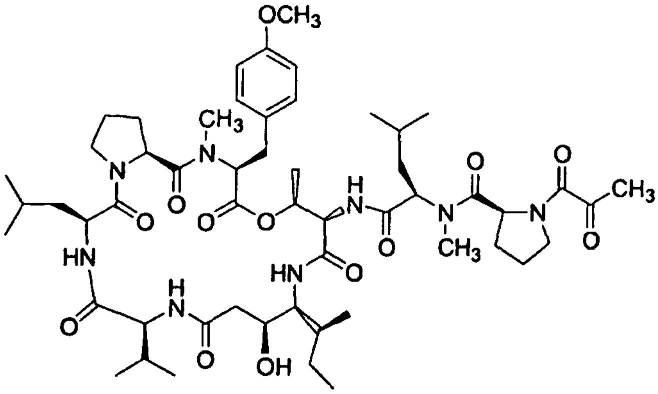

.

**20.** Die Zusammensetzung von Anspruch 1, wobei das Tamandarin-Analog ausgewählt ist aus der Gruppe von:

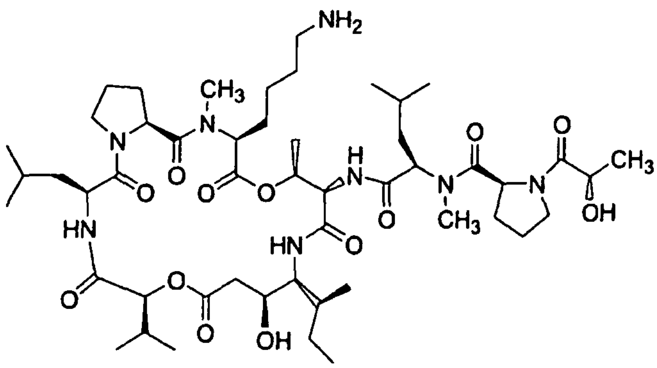

;

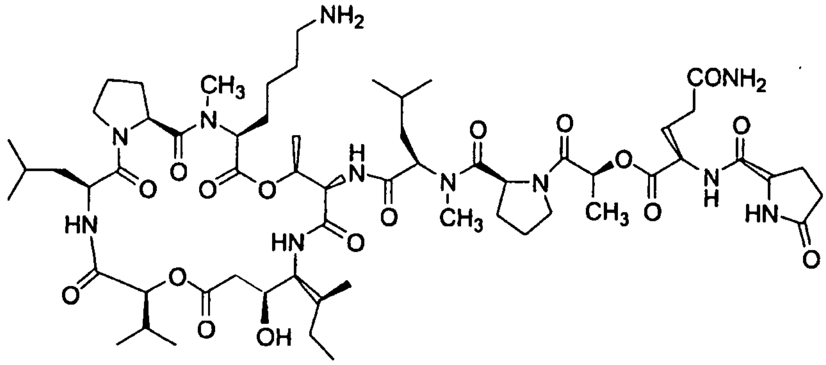

;

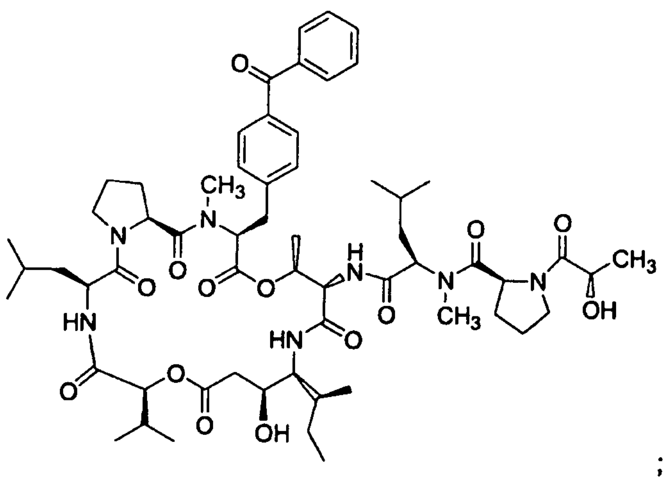

;

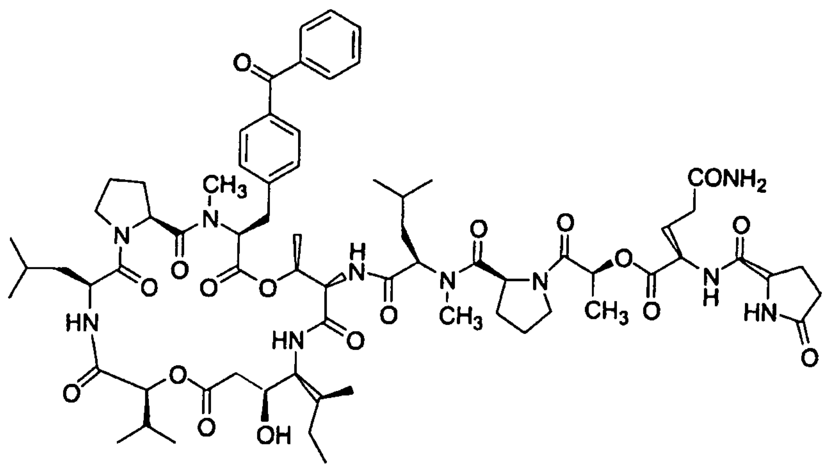

;

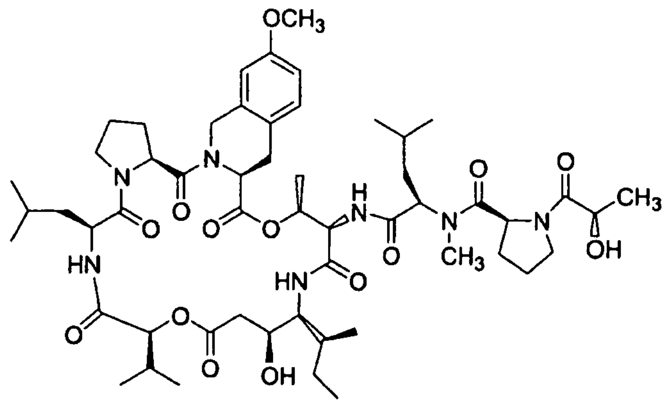

;

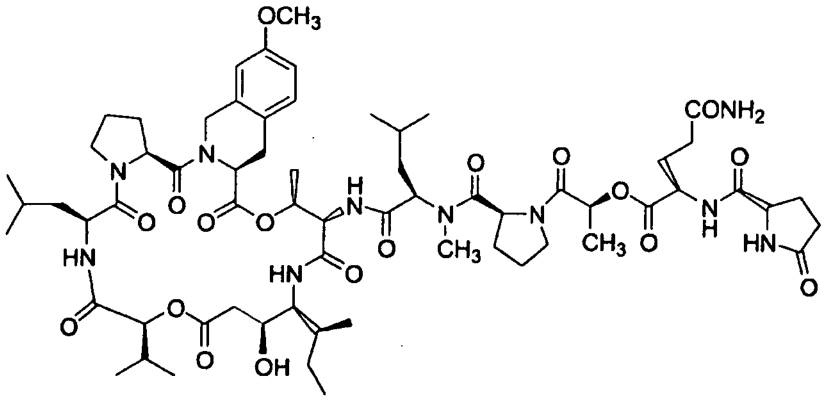

;

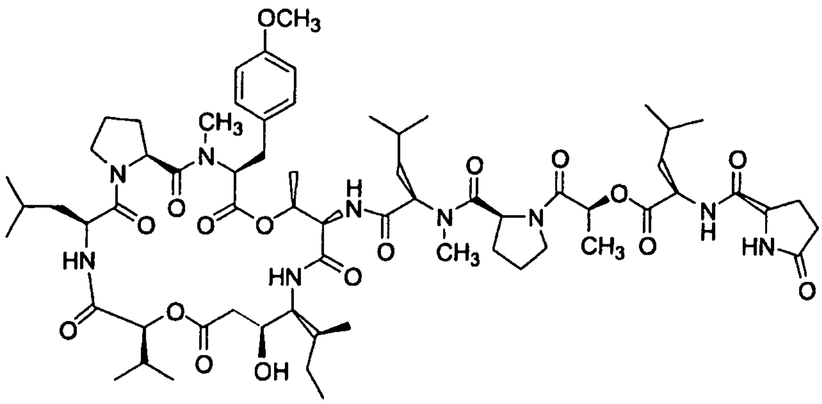

;

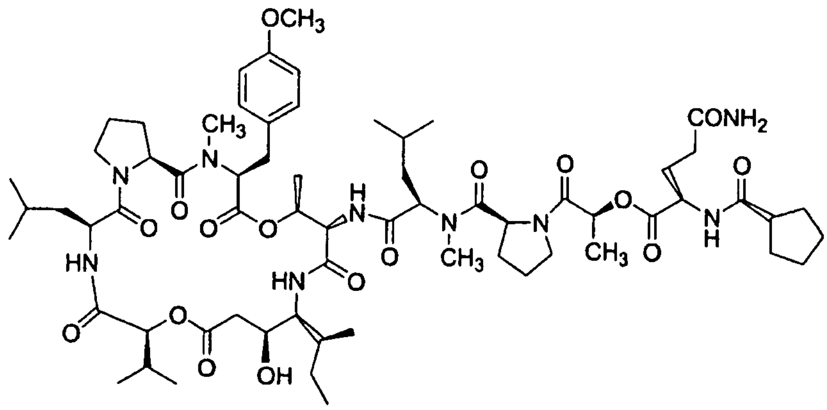

;

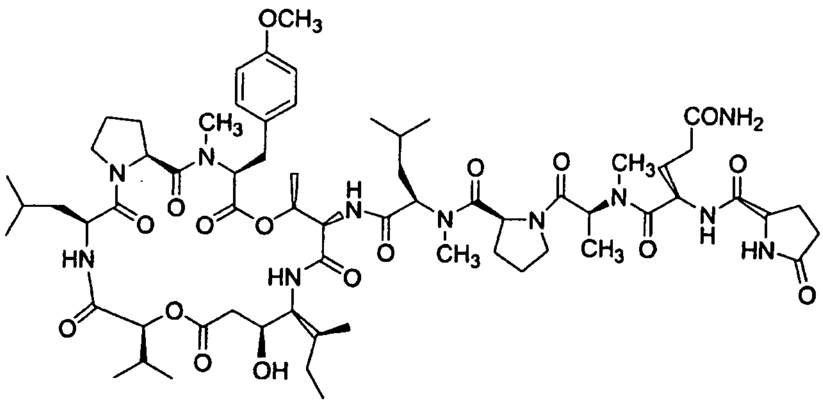

;

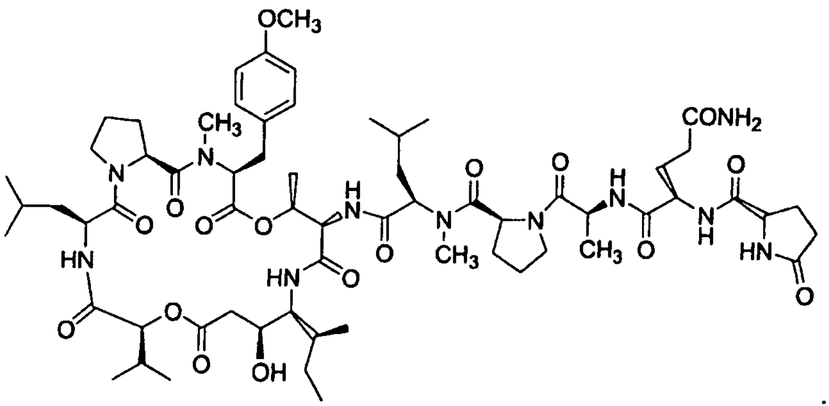

;

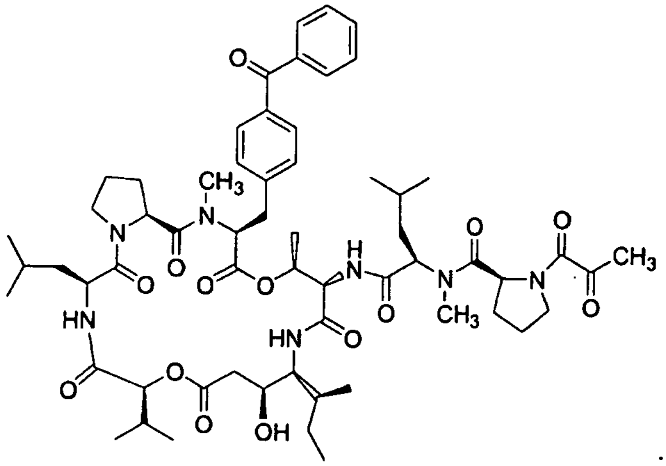

;

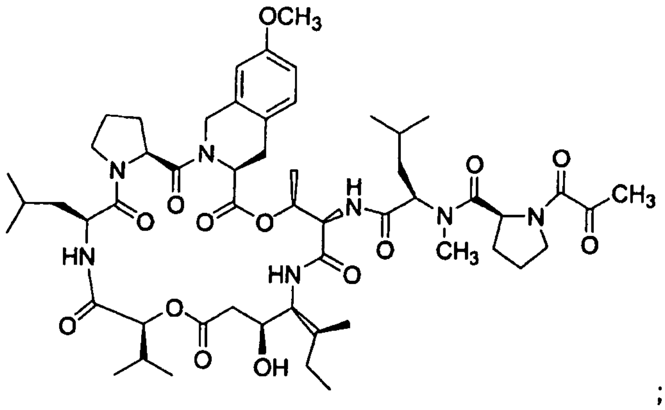

;

und

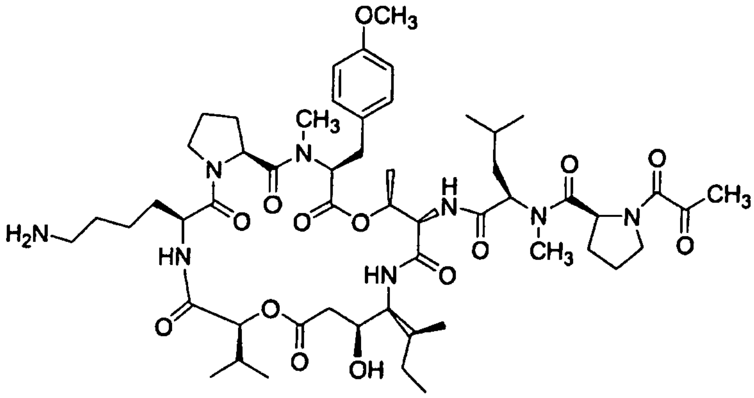

**21.** Die Zusammensetzung von Anspruch 1, desweiteren umfassend einen pharmazeutisch verträglichen Träger.

**22.** Eine Unterlage aufweisend das Tamandarin-Analog von Anspruch 1 kovalent daran angebunden.

**23.** Eine Zusammensetzung umfassend eine Verbindung mit einer Struktur ausgewählt aus der Gruppe bestehend aus

(a)

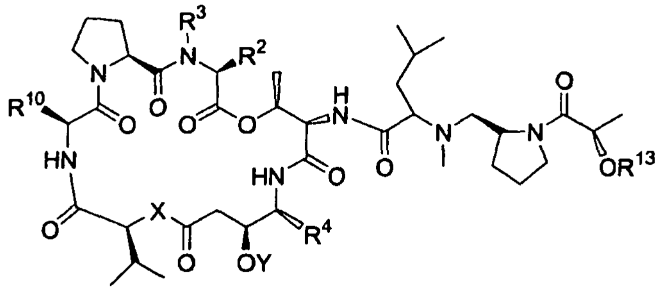

(b)

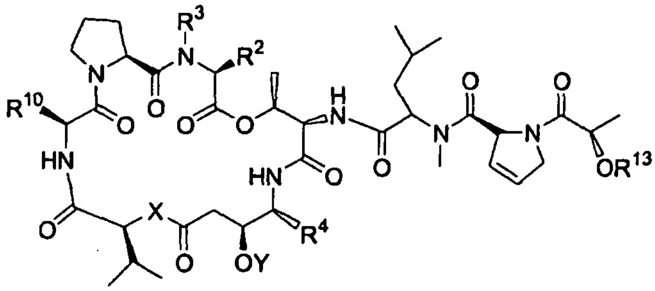

(c)

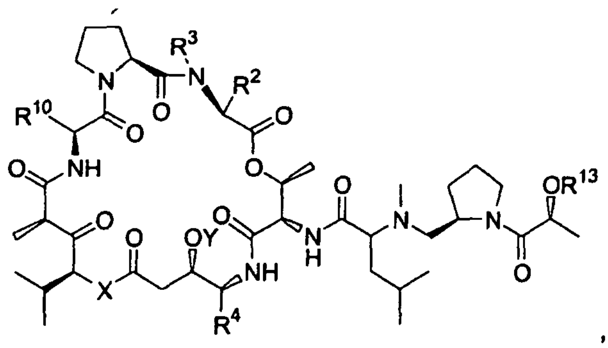

,

und

(d)

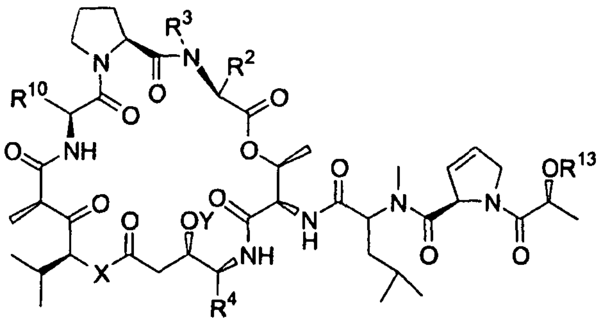

wobei:

i) entweder

(a) $R^3$ ausgewählt ist aus der Gruppe von: $-CH_3$ und -H; und $R^2$ ausgewählt ist aus der Gruppe von: einer Isoleucin-Seitenkette, einer Valin-Seitenkette, einer Alanin-Seitenkette, einer Norleucin-Seitenkette, einer Norvalin-Seitenkette, einer Prolin-Seitenkette, einer Leucin-Seitenkette, einer Histidin-Seitenkette, einer Tryptophan-Seitenkette, einer Arginin-Seitenkette, einer Lysine-Seitenkette, einem zweiten Fluorophor, und einem Substituenten mit der Struktur

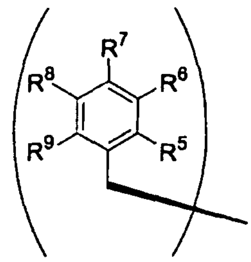

oder
(b) $R^2$ und $R^3$ zusammen einen Substituenten mit der Struktur

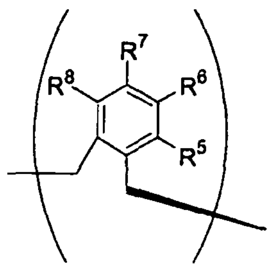

ausbilden

ii) jeder Rest von $R^5$, $R^6$, $R^7$, $R^8$, und $R^9$, wenn vorliegend, unabhängig ausgewählt ist aus der Gruppe bestehend aus -H, -OH, $-OCH_3$, $-CO(C_6H_5)$, -Br, -I, -F, -Cl, $-CH_3$, und $-C_2H_5$;
iii) $R^4$ ausgewählt ist aus der Gruppe von: einer Isoleucin-Seitenkette und einer Valin-Seitenkette;
iv) X ausgewählt ist aus der Gruppe von: -O- und -(NH)-;
v) Y ausgewählt ist aus der Gruppe von: -H und einer Hydroxyl-Schutzgruppe;
vi) $R^{10}$ ausgewählt ist aus der Gruppe von: einer Leucin-Seitenkette und einer Lysine-Seitenkette; und
vii) $R^{13}$ eine Enzyme-abspaltbare Gruppe ist, welche spaltbar ist durch ein Enzym ausgewählt aus der Gruppe bestehend aus einer Carboxypeptidase, einer beta-Lactamase, einer beta-Galactosidase, einer Penicillin V-Amidase, einer Cytosin-Deaminase, einer Nitroreductase, einer alkalischen Phosphatase, einer beta-Glucuronidase, und einem katalytischen Antikörper.

**24.** Die Zusammensetzung von Anspruch 25, wobei $R^{13}$ die Struktur

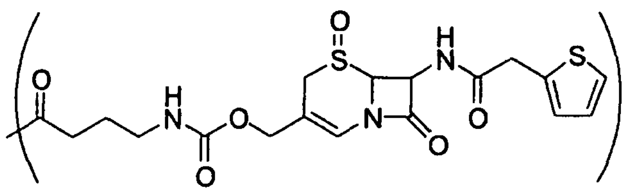

aufweist.

**25.** Die Zusammensetzung von Anspruch 25, wobei $R^{13}$ die Struktur

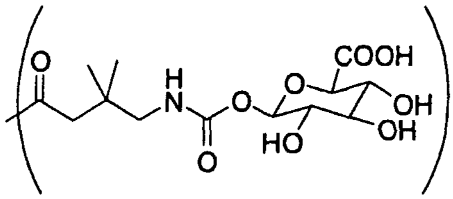

aufweist.

**26.** Eine Zusammensetzung umfassend ein Didemnin-Analog mit der Struktur

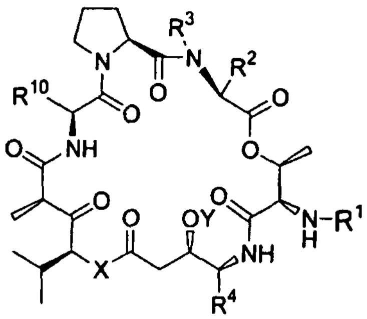

wobei:

i) $R^1$ ausgewählt ist aus der Gruppe von:

-(tert-Butyloxycarbonyl),
-Leucin,
-(N-Methyl)leucin-(ein erster Fluorophor),
-(N-Methyl)leucin-prolin,
-(N-Methyl)leucin-prolin-lactat-(ein erster Fluorophor),
-(N-Methyl)leucin-prolin-lactat-glutamin-pyroglutamat,
-(N-Methyl)leucin-prolin-lactat-glutamin-cyclopentanoat,
-(N-Methyl)leucin-prolin-alanin-leucin-pyroglutamat,
-(N-Methyl)leucin-prolin-(N-methyl-alanin)-leucin-pyroglutamat,
-(N-Methyl)leucin-deoxo-prolin,
-(N-Methyl)leucin-deoxo-prolin-lactat,
-(N-Methyl)leucin-deoxo-prolin-pyruvat,
-(N-Methyl)leucin-deoxo-prolin-lactat-(ein erster Fluorophor),
-(N-Methyl)leucin-deoxo-prolin-lactat-glutamin-pyroglutamat,
-(N-Methyl)leucin-deoxo-prolin-lactat-glutamin-cyclopentanoat,
-(N-Methyl)leucin-deoxo-prolin-alanin-leucin-pyroglutamat,
-(N-Methyl)leucin-deoxo-prolin-(N-methyl-alanin)-leucin-pyroglutamat,
-(N-Methyl)leucin-dehydro-prolin,
-(N-Methyl)leucin-dehydro-prolin-lactat,
-(N-Methyl)leucin-dehydro-prolin-pyruvat,
-(N-Methyl)leucin-dehydro-prolin-lactat-(ein erster Fluorophor),
-(N-Methyl)leucin-dehydro-prolin-lactat-glutamin-pyroglutamat,
-(N-Methyl)leucin-dehydro-prolin-lactat-glutamin-cyclopentanoat,
-(N-Methyl)leucin-dehydro-prolin-alanin-leucin-pyroglutamat, und
-(N-Methyl)leucin-dehydro-prolin-(N-methyl-alanin)-leucin-pyroglutamat;

ii) entweder

(a) $R^3$ ausgewählt ist aus der Gruppe von: $-CH_3$ und -H; und $R^2$ ausgewählt ist aus der Gruppe von: einer Isoleucin-Seitenkette, einer Valin-Seitenkette, einer Alanin-Seitenkette, einer Norleucin-Seitenkette, einer Norvalin-Seitenkette, einer Leucin-Seitenkette, einer Histidin-Seitenkette, einer Tryptophan-Seitenkette, einer Arginin-Seitenkette, einer Lysine-Seitenkette, einem zweiten Fluorophor, und einem Substituenten mit der Struktur

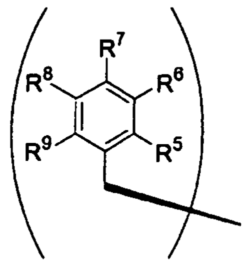

,

oder
(b) $R^2$ und $R^3$ zusammen einen Substituenten mit der Struktur

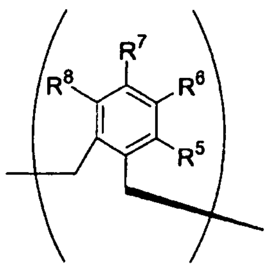

darstellen,

iii) jeder Rest von $R^5$, $R^6$, $R^7$, $R^8$, und $R^9$, wenn vorliegend, unabhängig ausgewählt ist aus der Gruppe bestehend aus -H, -OH, $-OCH_3$, -CO $(C_6H_5)$, -Br, -I, -F, -Cl, $-CH_3$, und $-C_2H_5$;
iv) $R^4$ ausgewählt ist aus der Gruppe von: einer Isoleucin-Seitenkette und einer Valin-Seitenkette;
v) X ausgewählt ist aus der Gruppe von: -O- und -(NH)-;
vi) Y ausgewählt ist aus der Gruppe von: -H und einer Hydroxyl-Schutzgruppe; und
vii) $R^{10}$ ausgewählt ist aus der Gruppe von: einer Leucin-Seitenkette und einer Lysine-Seitenkette.

**27.** Die Zusammensetzung von Anspruch 26, wobei $R^1$ ausgewählt ist aus der Gruppe von:

-(tert-butyloxycarbonyl),
-leucin,
-(N-Methyl)leucin-(ein erster Fluorophor),
-(N-Methyl)leucin-(S)-prolin,
-(N-Methyl)leucin-(S)-prolin-(S)-lactat-(ein erster Fluorophor),
-(N-Methyl)leucin-(S)-prolin-(S)-lactat-glutamin-pyroglutamat,
-(N-Methyl)leucin-(S)-prolin-(S)-lactat-glutamin-cyclopentanoat,
-(N-Methyl)leucin-(S)-prolin-alanin-leucin-pyroglutamat,
-(N-Methyl)leucin-(S)-prolin-(N-methyl-alanin)-leucin-pyroglutamat,
-(N-Methyl)leucin-deoxo-(S)-prolin,
-(N-Methyl)leucin-deoxo-(S)-prolin-(S)-lactat,
-(N-Methyl)leucin-deoxo-(S)-prolin-pyruvat,

-(N-Methyl)leucin-deoxo-(S)-prolin-(S)-lactat-(ein erster Fluorophor),
-(N-Methyl)leucin-deoxo-(S)-prolin-(S)-lactat-glutamin-pyroglutamat,
-(N-Methyl)leucin-deoxo-(S)-prolin-(S)-lactat-glutamin-cyclopentanoat,
-(N-Methyl)leucin-deoxo-(S)-prolin-alanin-leucin-pyroglutamat,
-(N-Methyl)leucin-deoxo-(S)-prolin-(N-methyl-alanin)-leucin-pyroglutamat,
-(N-Methyl)leucin-dehydro-(S)-prolin,
-(N-Methyl)leucin-dehydro-(S)-prolin-(S)-lactat,
-(N-Methyl)leucin-dehydro-(S)-prolin-pyruvat,
-(N-Methyl)leucin-dehydro-(S)-prolin-(S)-lactat-(ein erster Fluorophor),
-(N-Methyl)leucin-dehydro-(S)-prolin-(S)-lactat-glutamin-pyroglutamat,
-(N-Methyl)leucin-dehydro-(S)-prolin-(S)-lactat-glutamin-cyclopentanoat,
-(N-Methyl)leucin-dehydro-(S)-prolin-alanin-leucin-pyroglutamat, und
-(N-methyl)leucin-dehydro-(S)-prolin-(N-methyl-alanin)-leucin-pyroglutamat.

**28.** Die Zusammensetzung von Anspruch 26, wobei $R^1$ ausgewählt ist aus der Gruppe von:

-(tert-butyloxycarbonyl),
-Leucin,
-(N-Methyl)leucin-(ein erster Fluorophor),
-(N-Methyl)leucin-(S)-prolin,
-(N-Methyl)leucin-(S)-prolin-(S)-lactat-(ein erster Fluorophor),
-(N-Methyl)leucin-(S)-prolin-(S)-lactat-(S)-glutamin-(S)-pyroglutamat,
-(N-Methyl)leucin-(S)-prolin-(S)-lactat-(S)-glutamin-(S)-cyclopentanoat,
-(N-Methyl)leucin-(S)-prolin-(S)-alanin-(S)-leucin-(S)-pyroglutamat,
-(N-Methyl)leucin-(S)-prolin-(N-methyl-S-alanin)-(S)-leucin-(S)-pyroglutamat,
-(N-Methyl)leucin-deoxo-(S)-prolin,
-(N-Methyl)leucin-deoxo-(S)-prolin-(S)-lactat,
-(N-Methyl)leucin-deoxo-(S)-prolin-pyruvat,
-(N-Methyl)leucin-deoxo-(S)-prolin-(S)-lactat-(ein erster Fluorophor),
-(N-Methy)leucin-deoxo-(S)-prolin-(S)-lactat-(S)-glutamin-(S)-pyroglutamat,
-(N-Methyl)leucin-deoxo-(S)-prolin-(S)-lactat-(S)-glutamin-(S)-cyclopentanoat,
-(N-Methyl)leucin-deoxo-(S)-prolin-(S)-alanin-(S)-leucin-(S)-pyroglutamat, und
-(N-Methyl)leucin-deoxo-(S)-prolin-(N-methyl-S-alanin)-(S)-leucin-(S)-pyroglutamat,
-(N-Methy)leucin-dehydro-(S)-prolin,
-(N-Methyl)leucin-dehydro- (S)-prolin-(S)-lactat,
-(N-Methyl)leucin-deoxo-(S)-prolin-pyruvat,
-(N-Methyl)leucin-deoxo-(S)-prolin-(S)-lactat- (ein erster Fluorophor),
-(N-Methyl)leucin-deoxo-(S)-prolin-(S)-lactat-(S)-glutamin-(S)-pyroglutamat,
-(N-Methyl)leucin-deaxo-(5)-prolin-(S)-lactat -(5)-glutamin-(S)-cyclopentanoat.
-(N-Methyl)leucin-deoxo-(S)-prolin-(S)-alanin-(5)-leucin-(S)-pyroglutamat, und
-(N-Methyl)leucin-deoxo-(S)-prolin-(N-methyl-S-alanin)-(S)-leucin-(S)-pyroglutamat.

**29.** Die Zusammensetzung von Anspruch 26, wobei $R^2$

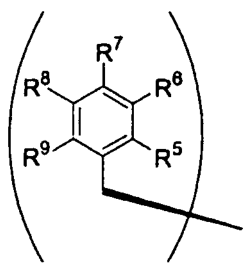

ist,
$R^3$ Methyl ist, $R^4$ eine Isoleucin-Seitenkette ist, jeder Rest von $R^5$, $R^6$, $R^8$, und $R^9$ ein Hydrid Radikal ist, $R^7$ Methoxy ist, $R^{10}$ eine Leucin-Seitenkette ist, X -O- ist, und Y ein Hydrid Radikal ist.

**30.** Die Zusammensetzung von Anspruch 26, wobei das Didemnin-Analog

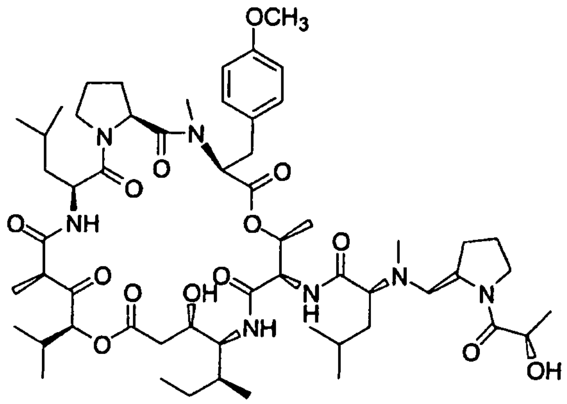

ist.

**31.** Die Zusammensetzung von Anspruch 26, wobei das Didemnin-Analog

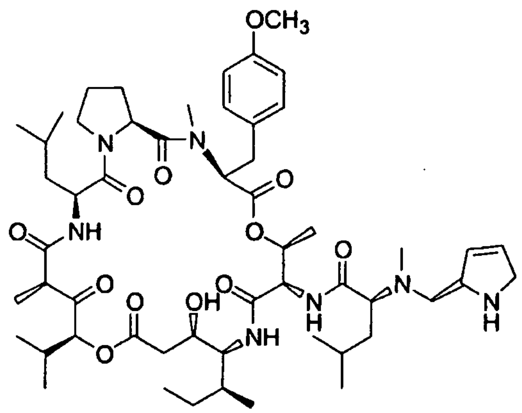

ist.

**32.** Die Zusammensetzung von Anspruch 26, wobei R -(N-Methyl)leucin-deoxo-(S)-prolin-lactat ist.

**33.** Die Zusammensetzung von Anspruch 26, wobei Y -H ist, und wobei $R^2$ die Struktur

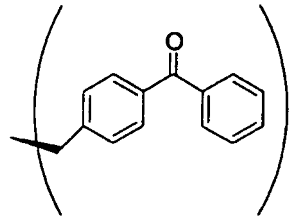

aufweist.

**34.** Die Zusammensetzung von Anspruch 26, wobei $R^2$ eine Lysin-Seitenkette ist und Y -H ist.

**35.** Die Zusammensetzung von Anspruch 26, wobei X -(NH)- Ist.

**36.** Die Zusammensetzung von Anspruch 26, desweiteren umfassend einen pharmazeutisch verträglichen Träger.

**37.** Eine Unterlage kovalent angebunden an das Didemnin-Analog von Anspruch 26.

**38.** Ein Verfahren zum Erzeugen eines Tamandarin-Analogs von Anspruch 1 oder eines Didemnin-Analogs von Anspruch 26, wobei die Verbesserung das Einbringen eines Deoxo-prolin-Restes an Stelle eines Prolin-Restes des Analogs umfasst, wobei $R^1$ einen Deoxo-prolin-Rest umfasst.

**39.** Das Verfahren von Anspruch 38, wobei das Analog eine (N-Methyl)leucin-prolin Gruppe umfasst und wobei die (N-Methyl)leucin-prolin-Gruppe ersetzt wird durch eine (N-Methyl) leucin-deoxo-prolin-Gruppe.

**40.** Das Verfahren von Anspruch 39, wobei die (N-Methyl)leucin-deoxoprolin-Gruppe erzeugt wird durch Reduzieren der Ester-Funktion von Prolin in eine Aldehyd-Funktion; und Koppeln des Prolins an die (N-Methyl)-leucin-Gruppe durch reduktive Aminierung, um in der (N-Methyl)-leucin-deoxo-prolin-Gruppe zu resultieren.

**41.** Das Verfahren von Anspruch 40, wobei die Amin-Gruppe des Prolins geschützt wird mit einer Amin-Schutzgruppe vor der reduktiven Aminierung.

**42.** Das Verfahren von Anspruch 40, wobei die Ester-Funktion des Prolin in eine Aldehyd-Funktion reduziert wird durch In-Kontakt-Bringen des Prolin mit einer starken Base und anschließendem In-Kontakt-Bringen des Prolin mit einem oxidierenden Agens.

**43.** Das Verfahren von Anspruch 40, wobei die reduktive Aminierung in einem nicht wässrigen Solvens in der Gegenwart einer starken Base und eines Carbonsäure-Katalysators durchgeführt wird.

**44.** Ein Verfahren zum Erzeugen eines Tamandarin-Analogs von Anspruch 1 oder eines Didemnin-Analogs von Anspruch 26, wobei die Verbesserung das Einbringen eines Dehydro-prolin-Restes an Stelle eines Prolin-Restes des Analogs umfasst, wobei $R^1$ einen Dehydro-prolin-Rest umfasst.

**45.** Das Verfahren von Anspruch 44, wobei das Analog eine (N-Methyl)leucin-prolin Gruppe umfasst und wobei die (N-Methyl)leucin-prolin-Gruppe ersetzt wird durch eine (N-Methyl) leucin-dehydro-prolin-Gruppe.

**46.** Das Verfahren von Anspruch 44, wobei der Dehydro-prolin-Rest erzeugt wird durch Schützen der Carboxyl- und Amino-Gruppen von 4-Hydroxyprolinat, Alkylsulfonylieren der 4-Hydroxyl-Gruppe, Ersetzen der Alkyl-sulfonat-Gruppe mit einer Aryl-selenyl-Gruppe, oxidatives Eliminieren der Aryl-selenyl- Gruppe, um zu einer Dehyro-prolin-Gruppe mit geschützten Carboxyl- und Amin-Gruppen zu gelangen, und Koppeln der Dehydro-prolin-Gruppe mit einer Amin-Gruppe des Analogs.

**47.** Das Verfahren von Anspruch 46, wobei die Alkyl-sulfonat-Gruppe eine Methylsulfonat-Gruppe ist.

**48.** Das Verfahren von Anspruch 46, wobei die Aryl-selenyl-Gruppe eine Phenylselenyl-Gruppe ist.

**49.** Das Verfahren von Anspruch 44, wobei das 4-Hydroxyprolinate trans-4-Hydroxyprolinate ist.

**50.** Eine Zusammensetzung umfassend ein Didemnin-Fragment mit der Struktur

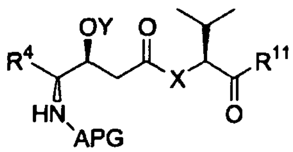

wobei:

i) Y ausgewählt ist aus der Gruppe von: -H und einer Hydroxyl-Schutzgruppe;
ii) X ausgewählt ist aus der Gruppe von: -O- und -(NH)-;
iii) $R^4$ ausgewählt ist aus der Gruppe von: einer Isoleucin-Seitenkette und einer Valin-Seitenkette;

iv) APG eine Amin-Schutzgruppe ist; und

v) $R^{11}$ ausgewählt ist aus der Gruppe von: -OH, $-NH_2$, -O(allyl), -O(pentafluorophenyl), und einem Substituenten mit der Struktur

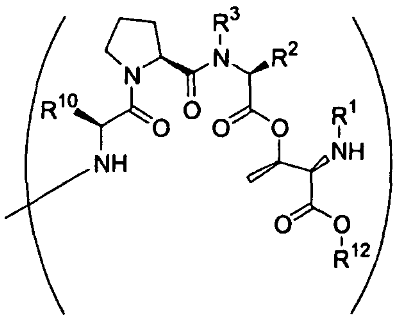

wobei:

a) $R^1$ ausgewählt ist aus der Gruppe von: -H, und einer Amin-Schutzgruppe;

b) entweder

(I) $R^3$ ausgewählt ist aus der Gruppe von: $-CH_3$ und -H; und $R^2$ ausgewählt ist aus der Gruppe von: einer Isoleucin-Seitenkette, einer Valin-Seitenkette, einer Alanin-Seitenkette, einer Norleucin-Seitenkette, einer Norvalin-Seitenkette, einer Prolin-Seitenkette, einer Leucin-Seitenkette, einer Histidin-Seitenkette, einer Tryptophan-Seitenkette, einer Arginin-Seitenkette, einer Lysine-Seitenkette, einem zweiten Fluorophor, und einem Substituenten mit der Struktur

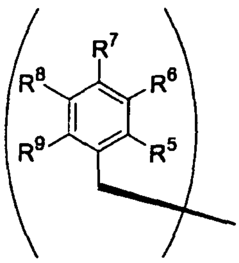

oder

(II) $R^2$ und $R^3$ zusammen einen Substituenten mit der Struktur

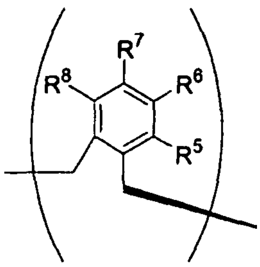

ausbilden,

c) jeder Rest von $R^6$, $R^6$, $R^7$, $R^8$, und $R^9$, wenn vorliegend, unabhängig ausgewählt ist aus der Gruppe bestehend aus: -H, -OH, $-OCH_3$, $-CO(C_6H_5)$, -Br, -I, -F, -Cl, $-CH_3$, und $-C_2H_5$;

d) $R^{10}$ ausgewählt ist aus der Gruppe von: einer Leucin-Seitenkette, einer Lysine-Seitenkette, und einer geschützten Lysin-Seitenkette; und

e) $R^{12}$ ausgewählt ist aus der Gruppe von: -H, und 2-(Trimethylsilyl)ethoxycarbonyl.

**51.** Ein Verfahren zum Herstellen eines Didemnin-Fragments, wobei das Verfahren das Koppeln eines ersten Reaktanten mit der Struktur

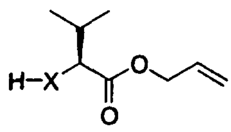

und eines zweiten Reaktanten mit der Struktur

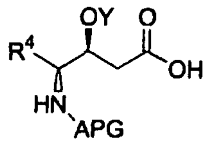

umfasst, um ein erstes Didemnin-Fragment mit der Struktur

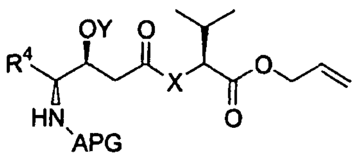

zu ergeben,
wobei X ausgewählt ist aus der Gruppe von: -O-, und -(NH)-; wobei APG eine Amin-Schutzgruppe ist; wobei Y eine Hydroxyl-Schutzgruppe ist; und wobei $R^4$ ausgewählt ist aus der Gruppe von: einer Isoleucin-Seitenkette und einer Valin-Seitenkette.

**52.** Das Verfahren von Anspruch 51, wobei Y -Triisopropylsilyl ist.

**53.** Das Verfahren von Anspruch 52, desweiteren umfassend Hydrolysieren des ersten Didemnin-Fragment, um ein zweites Didemnin-Fragment mit der Struktur

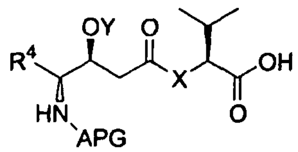

zu erhalten.

**54.** Das Verfahren von Anspruch 53, desweiteren umfassend Zugeben eines Aktivators zu der Carbonyl-Gruppe des zweiten Didemnin-Fragments, um ein drittes Didemnin-Fragment mit der Struktur

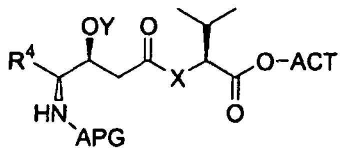

zu erhalten,
wobei -ACT ein Aktivator ist.

**55.** Das Verfahren von Anspruch 54, desweiteren umfassend Koppeln des dritten Didemnin-Fragments und eines dritten Reaktanten mit der Struktur

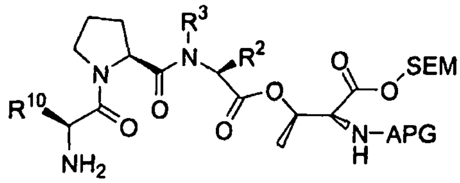

um ein viertes Didemnin-Fragment zu erhalten mit der Struktur

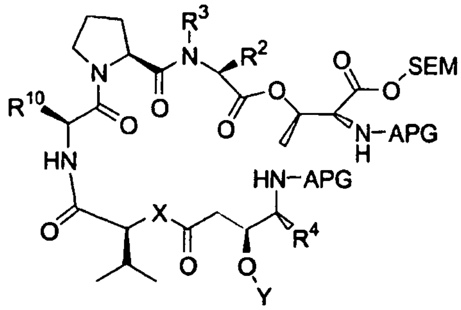

wobei:

i) entweder

(a) $R^3$ ausgewählt ist aus der Gruppe von: $-CH_3$ und -H; und $R^2$ ausgewählt ist aus der Gruppe von: einer Isoleucin-Seitenkette, einer Valin-Seitenkette, einer Alanin-Seitenkette, einer Norleucin-Seitenkette, einer Norvalin-Seitenkette, einer Prolin-Seitenkette, einer Leucin-Seitenkette, einer Histidin-Seitenkette, einer Tryptophan-Seitenkette, einer Arginin-Seitenkette, einer Lysine-Seitenkette, einem zweiten Fluorophor, und einem Substituenten mit der Struktur

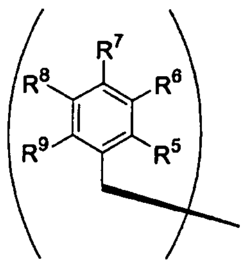

oder

(b) $R^2$ und $R^3$ zusammen einen Substituenten mit der Struktur

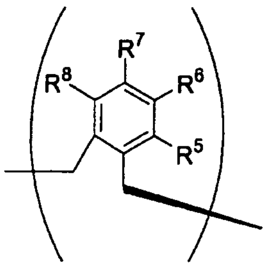

darstellen,

ii) jeder Rest von $R^5$, $R^6$, $R^7$, $R^8$, und $R^9$, wenn vorliegend, unabhängig ausgewählt ist aus der Gruppe bestehend aus -H, -OH, $-OCH_3$, $-CO(C_6H_5)$, -Br, -I, -F, -Cl, $-CH_3$, und $-C_2H_5$;

iii) APG eine Amin-Schutzgruppe ist;

iv) SEM 2-(Trimethylsilyl)ethoxycarbonyl ist und

iii) $R^{10}$ ausgewählt ist aus der Gruppe von: einer Leucin-Seitenkette und einer Lysine-Seitenkette.

**56.** Ein Verfahren zum Herstellen eines Didemnin-Analogs, wobei das Verfahren das Entfernen der SEM-Gruppe und der APG-Gruppe schützend die Amino-Gruppe angebunden an das C-Atom, an welches R4 gebunden ist, von dem vierten Didemnin-Fragment von Anspruch 55 einschließt und Zyklisieren des vierten Didemnin-Fragments, um ein erstes Didemnin-Analog mit der Struktur

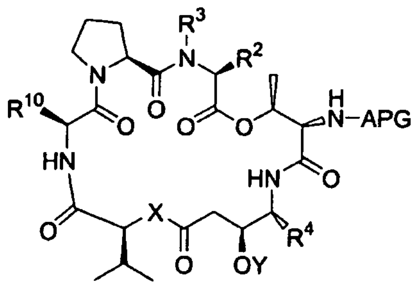

zu erhalten.

**57.** Das Verfahren von Anspruch 56, desweiteren umfassend Entfernen der APG Gruppe von dem ersten Ddidemnin-Analog, um ein zweites Didemnin-Analog mit der Struktur

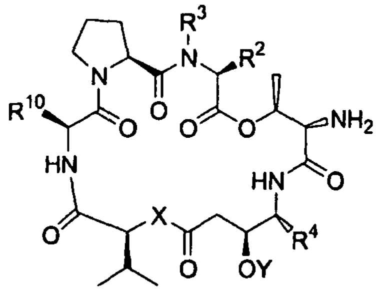

zu erhalten.

**58.** Das Verfahren von Anspruch 56, wobei the APG-Gruppe ausgewählt ist aus der Gruppe von: einer Carbobenzyloxy-Gruppe und einer tert-Butyloxycarbonyl-Gruppe.

**59.** Das Verfahren von Anspruch 57, desweiteren umfassend Koppeln des zweiten Didemnin-Analog und eines vierten Reagenzes mit der Struktur

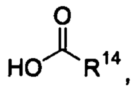

um ein drittes Didemnin-Analog mit der Struktur

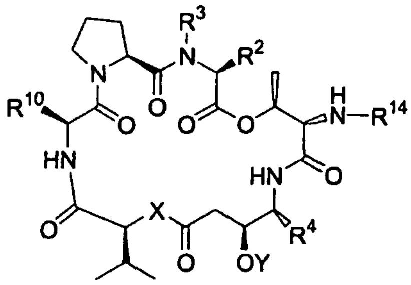

zu erhalten,
wobei $R^{14}$ ausgewählt ist aus der Gruppe von:

-Leucin,
-(N-Methyl)leucin,
-(N-Methyl)leucin-(ein erster Fluorophor),
-(N-Methyl)leucin-prolin,
-(N-Methyl)leucin-prolin-lactat,
-(N-Methyl)leucin-prolin-pyruvat,
-(N-Methyl)leucin-prolin-lactat-(ein erster Fluorophor),
-(N-Methyl)leucin-prolin-lactat-glutamin-pyroglutamat,
-(N-Methyl)leucin-prolin-lactat-glutamin-cyclopentanoat,
-(N-Methyl)leucin-prolin-alanin-leucin-pyroglutamat,
-(N-Methyl)leucin-prolin-(N-methyl-alanin)-leucin-pyroglutamat,
-(N-Methyl)leucin-deoxo-prolin,
-(N-Methyl)leucin-deoxo-prolin-lactat,
-(N-Methyl)leucin-deoxo-prolin-pyruvat,
-(N-Methyl)leucin-deoxo-prolin-lactat-(ein erster Fluorophor),
-(N-Methyl)leucin-deoxo-prolin-lactat-glutamin-pyroglutamat,
-(N-Methyl)leucin-deoxo-prolin-lactat-glutamin-cyclopentanoat,
-(N-Methyl)leucin-deoxo-prolin-alanin-leucin-pyroglutamat,
-(N-Methyl)leucin-deoxo-prolin-(N-methyl-alanin)-leucin-pyroglutamat,
-(N-Methyl)leucin-dehydro-prolin,
-(N-Methyl)leucin-dehydro-prolin-lactat,
-(N-Methyl)leucin-dehydro-prolin-pyruvat,
-(N-Methyl)leucin-dehydro-prolin-lactat-(ein erster Fluorophor),
-(N-Methyl)leucin-dehydro-prolin-lactat-glutamin-pyroglutamat,
-(N-Methyl)leucin-dehydro-prolin-lactat-glutamin-cyclopentanoat,
-(N-Methyl)leucin-dehydro-prolin-alanin-leucin-pyroglutamat,
-(N-Methyl)leucin-dehydro-prolin-(N-methyl-alanin)-leucin-pyroglutamat; und

und irgendeiner der oben genannten Gruppen, gekoppelt mit einer Enzymabspaltbaren Gruppe, die durch ein Enzym

abgespalten werden kann ausgewählt aus der Gruppe bestehend aus einer Carboxypeptidase, einer beta-Lactamase, einer beta-Galactosidase, einer Penicillin-V-amidase, einer Cytosine-Deaminase, einer Nitroreduktase, einer alkalischen Phosphatase, einer beta-Glucuronidase, und einem katalytischen Antikörper.

**60.** Das Verfahren von Anspruch 59, wobei Y eine Hydroxyl-Schutzgruppe ist, das Verfahren desweiteren umfassend Entfernen der Hydroxyl-Schutzgruppe von dem zweiten Didemnin-Analog, um ein viertes Didemnin-Analog mit der Struktur

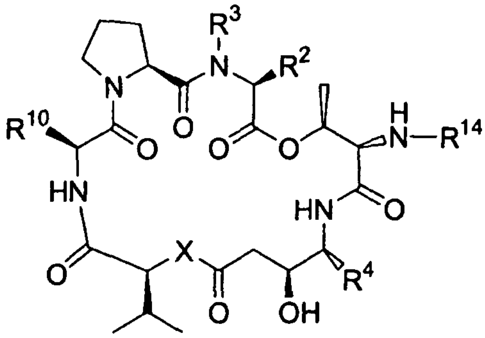

zu ergeben.

**61.** Verwendung von irgendeiner der Zusammensetzungen von Anspruch 1, 23, 26, oder 50 zum Herstellen eines Medikaments zum Inhibieren der Proteinsynthese in einer Zelle.

**62.** Verwendung von irgendeiner der Zusammensetzungen von Anspruch 1, 23, 26, oder 50 zum Herstellen eines Medikaments zum Inhibieren des Wachstums einer Zelle.

**63.** Verwendung von irgendeiner der Zusammensetzungen von Anspruch 1, 23, 26, oder 50 zum Herstellen eines Medikaments zum Inhibieren der Proliferation einer Zelle.

**64.** Verwendung von irgendeiner der Zusammensetzungen von Anspruch 1, 23, 26, oder 50 zum Herstellen eines Medikaments zum Inhibieren der Tumorgenese in einer Zelle.

**65.** Verwendung von irgendeiner der Zusammensetzungen von Anspruch 1, 23, 26, oder 50 zum Herstellen eines Medikaments zum Inhibieren des Verstärkens der Apoptose einer Zelle.

**66.** Eine Zusammensetzung umfassend ein Tamandarin-Analog mit der Struktur

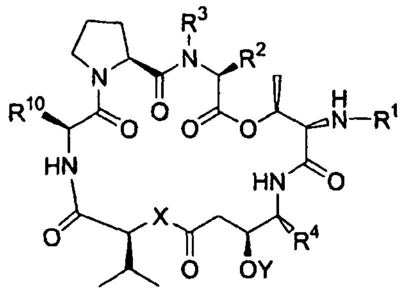

wobei:

i) $R^1$ ausgewählt ist aus der Gruppe von:

-H,
-(tert-Butyloxycarbonyl),

-Leucin,
-(N-Methyl)leucin,
-(N-Methyl)leucin-(ein erster Fluorophor),
-(N-Methyl)leucin-prolin,
-(N-Methyl)leucin-prolin-pyruvat,
-(N-Methyl)leucin-prolin-lactat-(ein erster Fluorophor),
-(N-Methyl)leucin-prolin-lactat-glutamin-pyroglutamat
-(N-Methyl)leucin-prolin-lactat-glutamin-cyclopentanoat,
-(N-Methyl)leucin-prolin-alanin-leucin-pyroglutamat,
-(N-Methyl)leucin-prolin-(N-methyl-alanin)-leucin-pyroglutamat,
-(N-Methyl)leucin-deoxo-prolin,
-(N-Methyl)leucin-deoxo-prolin-lactat,
-(N-Methyl)leucin-deoxo-prolin-pyruvat,
-(N-Methyl)leucin-deoxo-prolin-lactat-(ein erster Fluorophor),
-(N-Methyl)leucin-deoxo-prolin-lactat-glutamin-pyroglutamat,
-(N-Methyl)leucin-deoxo-prolin-lactat-glutamin-cyclopentanoat,
-(N-Methyl)leucin-deoxo-prolin-alanin-leucin-pyroglutamat,
-(N-Methyl)leucin-deoxo-prolin-(N-methyl-alanin)-leucin-pyroglutamat,
-(N-Methyl)leucin-dehydro-prolin,
-(N-Methyl)leucin-dehydro-prolin-lactat,
-(N-Methyl)leucin-dehydro-prolin-pyruvat,
-(N-Methyl)leucin-dehydro-prolin-lactat-(ein erster Fluorophor),
-(N-Methyl)leucin-dehydro-prolin-lactat-glutamin-pyroglutamat,
-(N-Methyl)leucin-dehydro-prolin-lactat-glutamin-cyclopentanoat,
-(N-Methyl)leucin-dehydro-prolin-alanin-leucin-pyroglutamat, und
-(N-Methyl)leucin-dehydro-prolin-(N-methyl-alanin)-leucin-pyroglutamat;

ii) entweder

(a) $R^3$ ausgewählt ist aus der Gruppe von: $-CH_3$ und -H; und $R^2$ ausgewählt ist aus der Gruppe von: einer Isoleucin-Seitenkette, einer Valin-Seitenkette, einer Alanin-Seitenkette, einer Norleucin-Seitenkette, einer Norvalin-Seitenkette, einer Leucin-Seitenkette, einer Histidin-Seitenkette, einer Tryptophan-Seitenkette, einer Arginin-Seitenkette, einer Lysine-Seitenkette, einem zweiten Fluorophor, und einem Substituenten mit der Struktur

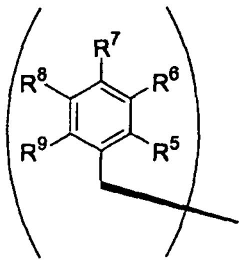

,

oder
(b) $R^2$ und $R^3$ zusammen einen Substituenten mit der Struktur

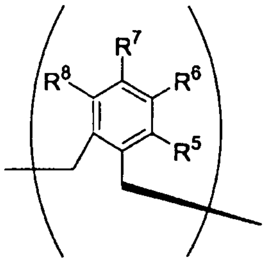

darstellen,

iii) jeder Rest von $R^5$, $R^6$, $R^7$, $R^8$, und $R^9$, wenn vorliegend, unabhängig ausgewählt ist aus der Gruppe bestehend aus -H, -OH, $-OCH_3$, $-CO(C_6H_5)$, -Br, -I, -F, -Cl, $-CH_3$, und $C_2H_5$;
iv) $R^4$ eine Valin-Seitenkette ist;
v) X ausgewählt ist aus der Gruppe von: -O- und -(NH)-;
vi) Y ausgewählt ist aus der Gruppe von: -H und einer Hydroxyl-Schutzgruppe;
vii) $R^{10}$ ausgewählt ist aus der Gruppe von: einer Leucin-Seitenkette und einer Lysine-Seitenkette; und
viii) das Molekül nicht Tamandarin B ist.

**67.** Die Zusammensetzung von Anspruch 66, wobei $R^4$ eine Valin-Seitenkette ist, Y -H ist, und X -O- ist.

**68.** Ein Tamandarin-Analog ausgewählt aus der Gruppe bestehend aus

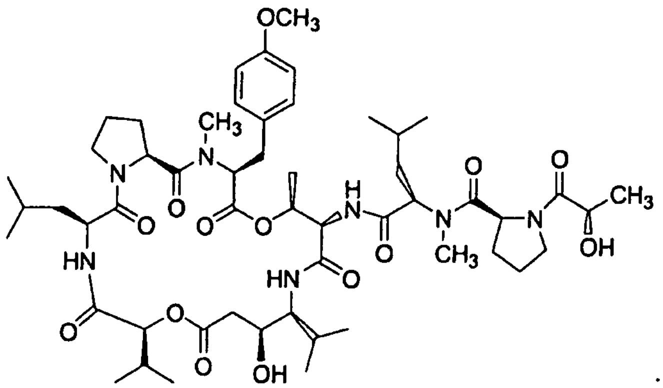

;

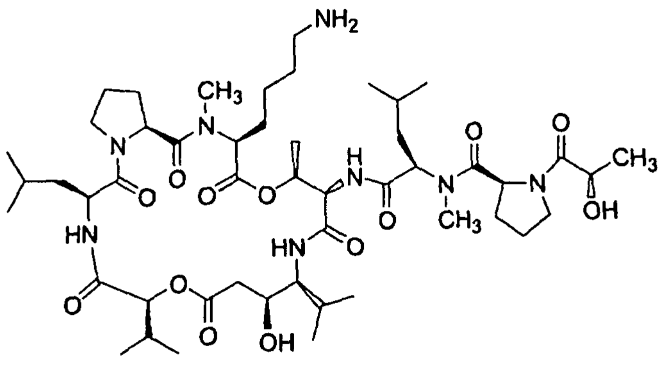

;

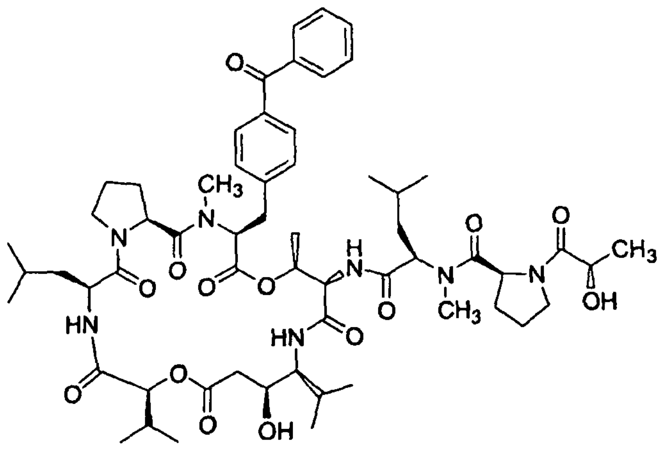

;

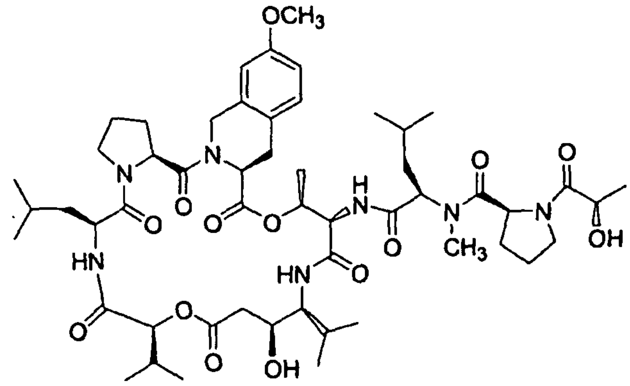

;

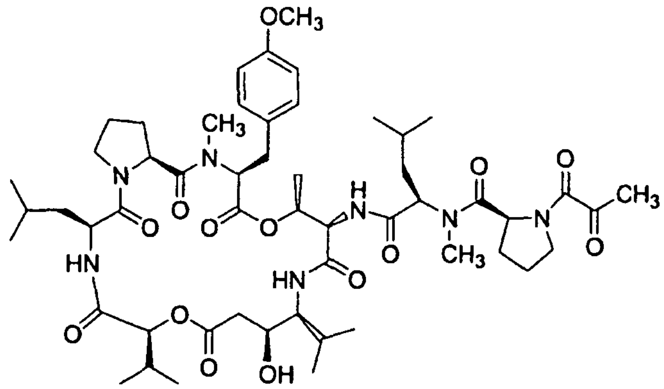

;

und

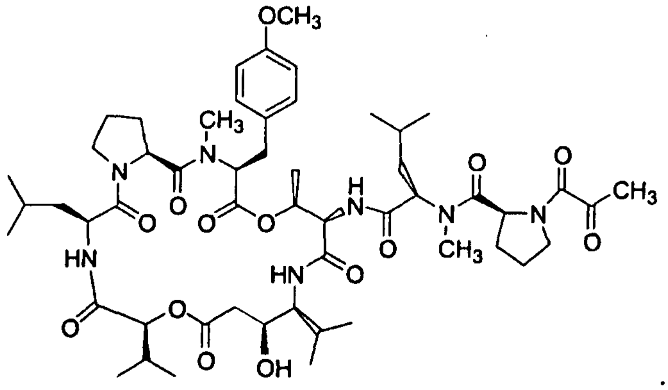

**69.** Die Zusammensetzung von Anspruch 67, wobei das Tamandarin-Analog

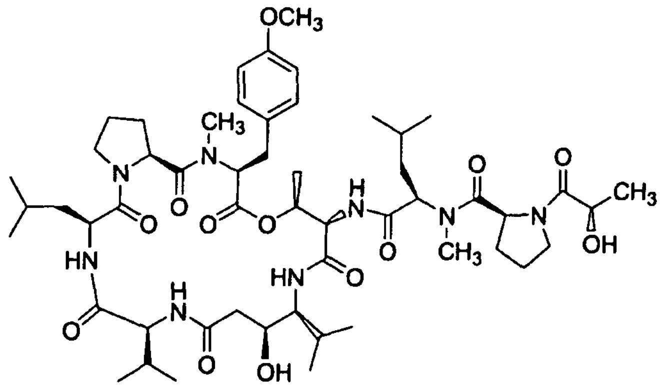

ist.

**70.** Eine Zusammensetzung umfassend ein Didemnin-Analog mit der Struktur

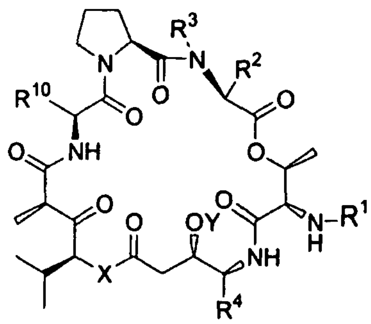

wobei:

i) $R^1$ ausgewählt ist aus der Gruppe von:

-H,
-(tert-Butyloxycarbonyl),
-Leucin,

-(N-Methyl)leucin,
-(N-Methyl)leucin-(ein erster Fluorophor),
-(N-Methyl)leucin-prolin,
-(N-Methyl)leucin-prolin-lactat,
-(N-Methyl)leucin-prolin-pyruvat,
-(N-Methyl)leucin-prolin-lactat-(ein erster Fluorophor),
-(N-Methyl)leucin-prolin-lactat-glutamin-pyroglutamat,
-(N-Methyl)leucin-prolin-lactat-glutamin-cyclopentanoat,
-(N-Methyl)leucin-prolin-alanin-leucin-pyroglutamat,
-(N-Methyl)leucin-prolin-(N-methyl-alanin)-leucin-pyroglutamat,
-(N-Methyl)leucin-deoxo-prolin,
-(N-Methyl)leucin-deoxo-prolin-lactat,
-(N-Methyl)leucin-deoxo-prolin-pyruvat,
-(N-Methyl)leucin-deoxo-prolin-lactat-(ein erster Fluorophor),
-(N-Methyl)leucin-deoxo-prolin-lactat-glutamin-pyroglutamat,
-(N-Methyl)leucin-deoxo-prolin-lactat-glutamin-cyclopentanoat,
-(N-Methyl)leucin-deoxo-prolin-alanin-leucin-pyroglutamat,
-(N-Methyl)leucin-deoxo-prolin-(N-methyl-alanin)-leucin-pyroglutamat,
-(N-Methyl)leucin-dehydro-prolin,
-(N-Methyl)leucin-dehydro-prolin-lactat,
-(N-Methyl)leucin-dehydro-prolin-pyruvat,
-(N-Methyl)leucin-dehydro-prolin-lactat-(ein erster Fluorophor),
-(N-Methyl)leucin-dehydro-prolin-lactat-glutamin-pyroglutamat,
-(N-Methyl)leucin-dehydro-prolin-lactat-glutamin-cyclopentanoat,
-(N-Methyl)leucin-dehydro-prolin-alanin-leucin-pyroglutamat, und
-(N-Methyl)leucin-dehydro-prolin-(N-methyl-alanin)-leucin-pyroglutamat;

ii) entweder

(a) $R^3$ ausgewählt ist aus der Gruppe von: $-CH_3$ und -H; und $R^2$ ausgewählt ist aus der Gruppe von: einer Isoleucin-Seitenkette, einer Valin-Seitenkette, einer Alanin-Seitenkette, einer Norleucin-Seitenkette, einer Norvalin-Seitenkette, einer Leucin-Seitenkette, einer Histidin-Seitenkette, einer Tryptophan-Seitenkette, einer Arginin-Seitenkette, einer Lysine-Seitenkette, einem zweiten Fluorophor, und einem Substituenten mit der Struktur

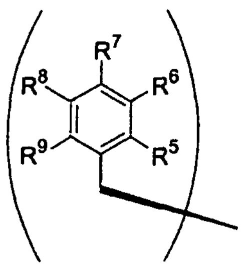

oder
(b) $R^2$ und $R^3$ zusammen einen Substituenten mit der Struktur

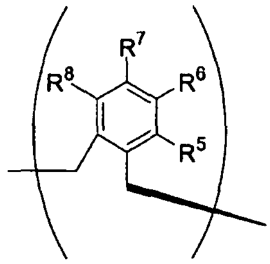

ausbilden

iii) jeder Rest von $R^5$, $R^6$, $R^7$, $R^8$, und $R^9$, wenn vorliegend, unabhängig ausgewählt ist aus der Gruppe bestehend aus -H, -OH, $-OCH_3$, -CO $(C_6H_5)$, -Br, -I, -F, -Cl, $-CH_3$, und $-C_2H_5$;
iv) $R^4$ einer Valin-Seitenkette ist;
v) X ausgewählt ist aus der Gruppe von: -O- und -(NH)-;
vi) Y ausgewählt ist aus der Gruppe von: -H und einer Hydroxyl-Schutzgruppe;
vii) $R^{10}$ ausgewählt ist aus der Gruppe von: einer Leucin-Seitenkette und einer Lysine- Seitenkette.

**71.** Die Zusammensetzung von Anspruch 70, wobei $R^1$ ausgewählt ist aus der Gruppe von:

-H,
-(tert-butyloxycarbonyl),
-leucin,
-(N-Methyl)leucin,
-(N-Methyl)leucin-(ein erster Fluorophor),
-(N-Methyl)leucin-(S)-prolin,
-(N-Methyl)leucin-(S)-prolin-(S)-lactat,
-(N-Methyl)leucin-(S)-prolin-pyruvat,
-(N-Methyl)leucin-(S)-prolin-(S)-lactat-(ein erster Fluorophor),
-(N-Methyl)leucin-(S)-prolin-(S)-lactat-glutamin-pyroglutamat,
-(N-Methyl)leucin-(S)-prolin-(S)-lactat-glutamin-cyclopentanoat,
-(N-Methyl)leucin-(S)-prolin-alanin-leucin-pyroglutamat,
-(N-Methyl)leucin-(S)-prolin-(N-methyl-alanin)-leucin-pyroglutamat,
-(N-Methyl)leucin-deoxo-(S)-prolin,
-(N-Methyl)leucin-deoxo-(S)-prolin-(S)-lactat,
-(N-Methyl)leucin-deoxo-(S)-prolin-pyruvat,
-(N-Methyl)leucin-deoxo-(S)-prolin-(S)-lactat-(ein erster Fluorophor),
-(N-Methyl)leucin-deoxo-(S)-prolin-(S)-lactat-glutamin-pyroglutamat,
-(N-Methyl)leucin-deoxo-(S)-prolin-(S)-lactat-glutamin-cyclopentanoat,
-(N-Methyl)leucin-deoxo-(S)-prolin-alanin-leucin-pyroglutamat,
-(N-Methyl)leucin-deoxo-(S)-prolin-(N-methyl-alanin)-leucin-pyroglutamat,
-(N-Methyl)leucin-dehydro-(S)-prolin,
-(N-Methyl)leucin-dehydro-(S)-prolin-(S)-lactat,
-(N-Methyl)leucin-dehydro-(S)prolin-pyruvat,
-(N-Methyl)leucin-dehydro-(S)-prolin-(S)-lactat-(ein erster Fluorophor),
-(N-Methyl)leucin-dehydro-(S)prolin-(S)-lactat-glutamin-pyroglutamat,
-(N-Methyl)leucin-dehydro-(S)-prolin-(S)-lactat-glutamin-cyclopentanoat,
-(N-Methyl)leucin-dehydro-(S)-prolin-alanin-leucin-pyroglutamat,
-(N-Methyl)leucin-dehydro-(S)-prolin-(N-methyl-alanin)-leucin-pyroglutamat,
-(N-Methyl)leucin-(S)-prolin-(S)-lactat-(S)-glutamin-(S)-pyroglutamat,
-(N-Methyl)leucin-(S)-prolin-(S)-lactat-(S)-glutamin-(S)-cyclopentanoat,
-(N-Methyl)leucin-(S)-prolin-(S)-alanin-(S)-leucin-(S)-pyroglutamat,
-(N-Methyl)leucin-(S)-prolin-(N-methyl-S-alanin)-(S)-leucin-(S)-pyroglutamat,
-(N-Methyl)leucin-deoxo-(S)-prolin-(S)-lactat-(S)-glutamin-(S)-pyroglutamat,
-(N-Methyl)leucin-deoxo-(S)-prolin-(S)-lactat-(S)-glutamin-(S)-cyclopentanoat,
-(N-Methyl)leucin-deoxo-(S)-prolin-(S)-alanin-(S)-leucin-(S)-pyroglutamat, und

-(N-Methyl)leucin-deoxo-(S)-prolin-(N-methyl-S-alanin)-(S)-leucin-(S)-pyroglutamat,
-(N-Methyl)leucin-deoxo-(S)-prolin-pyruvat,
-(N-Methyl)leucin-deoxo-(S)-prolin-(S)-lactat- (ein erster Fluorophor),
-(N-Methyl)leucin-deoxo-(S)-prolin-(S)-lactat-(S)-glutamin-(S)-pyroglutamat,
-(N-Methyl)leucin-deoxo-(S)-profin-(S)-lactat-(S)-glutamin-(S)-cyclopentanoat,
-(N-Methyl)leucin-deoxo-(S)-prolin-(S)-alanin-(S)-leucin-(S)-pyroglutamat, und
-(N-Methyl)leucin-deoxo-(S)-prolin-(N-methyl-S-alanin)-(S)-leucin-(S)-pyroglutamat.

**72.** Die Zusammensetzung von Anspruch 70, wobei $R^2$

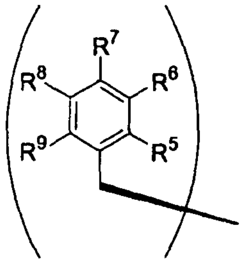

ist, $R^3$ Methyl ist, jeder Rest von $R^5$, $R^6$, $R^8$, und $R^9$ ein Hydrid Radikal ist, $R^7$ Methoxy ist, $R^{10}$ eine Leucin-Seitenkette ist, X -O- ist, und Y ein Hydrid Radikal ist.

**73.** Die Zusammensetzung von Anspruch 70, wobei R -(N-Methyl)leucin-deoxo-(S)-prolin-lactat ist.

**74.** Die Zusammensetzung von Anspruch 70, wobei Y-H ist, und wobei $R^2$ die Struktur

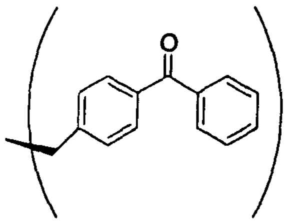

aufweist.

**75.** Die Zusammensetzung von Anspruch 70, wobei $R^2$ eine Lysin-Seitenkette ist und Y -H ist.

**76.** Die Zusammensetzung von Anspruch 70, wobei X -(NH)- ist.

**77.** Die Zusammensetzung von Anspruch 70, desweiteren umfassend einen pharmazeutisch verträglichen Träger.

**78.** Eine Unterlage kovalent angebunden an das Didemnin-Analog von Anspruch 70.

## Revendications

**1.** Composition comprenant un analogue de tamandarine de structure :

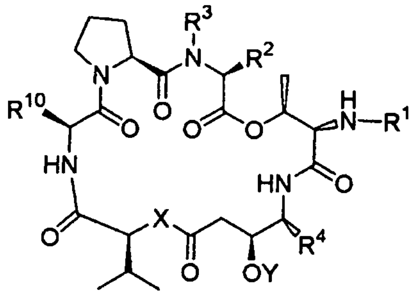

dans laquelle :

i) $R^1$ est choisi dans l'ensemble constitué par

-H,
-(tert-butyloxycarbonyle),
-leucine,
-(N-méthyl)leucine,
-(N-méthyl)leucine-(un premier fluorophore),
-(N-méthyl)leucine-proline,
-(N-méthyl)leucine-proline-lactate,
-(N-méthyl)leucine-proline-pyruvate,
-(N-méthyl)leucine-proline-lactate-(un premier fluorophore),
-(N-méthyl)leucine-proline-lactate-glutamine-pyroglutamate,
-(N-méthyl)leucine-proline-lactate-glutamine-cyclopentanoate,
-(N-méthyl)leucine-proline-alanine-leucine-pyroglutamate,
-(N-méthyl)leucine-proline-(N-méthyl-alanine)-leucine-pyroglutamate,
-(N-méthyl)leucine-désoxo-proline,
-(N-méthyl)leucine-désoxo-proline-lactate,
-(N-méthyl)leucine-désoxo-proline-pyruvate,
-(N-méthyl)leucine-désoxo-proline-lactate-(un premier fluorophore),
-(N-méthyl)leucine-désoxo-proline-lactate-glutamine-pyroglutamate,
-(N-méthyl)leucine-désoxo-proline-lactate-glutamine-cyclopentanoate,
-(N-méthyl)leucine-désoxo-proline-alanine-leucine-pyroglutamate,
-(N-méthyl)leucine-désoxo-proline-(N-méthyl-alanine)-leucine-pyroglutamate,
-(N-méthyl)leucine-déshydro-proline,
-(N-méthyl)leucine-déshydro-proline-lactate,
-(N-méthyl)leucine-déshydro-proline-pyruvate,
-(N-méthyl)leucine-déshydro-proline-lactate-(un premier fluorophore),
-(N-méthyl)leucine-déshydro-proline-lactate-glutamine-pyroglutamate,
-(N-méthyl)leucine-déshydro-proline-lactate-glutamine-cyclopentanoate,
-(N-méthyl)leucine-déshydro-proline-alanine-leucine-pyroglutamate, et
-(N-méthyl)leucine-déshydro-proline-(N-méthyl-alanine)-leucine-pyroglutamate ;

ii) soit

(a) $R^3$ est choisi dans l'ensemble constitué par $-CH_3$ et -H ; et $R^2$ est choisi dans l'ensemble constitué par une chaîne latérale d'isoleucine, une chaîne latérale de valine, une chaîne latérale d'alanine, une chaîne latérale de norleucine, une chaîne latérale de norvaline, une chaîne latérale de leucine, une chaîne latérale d'histidine, une chaîne latérale de tryptophane, une chaîne latérale d'arginine, une chaîne latérale de lysine, un deuxième fluorophore, et un substituant de structure :

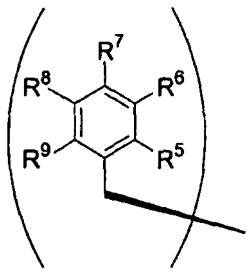

soit

(b) $R^2$ et $R^3$ forment ensemble un substituant de structure :

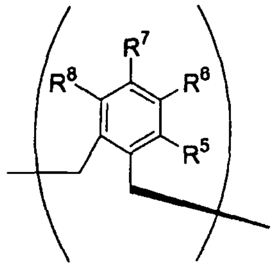

iii) chacun de $R^5$, $R^6$, $R^7$, $R^8$ et $R^9$, quand il est présent, est indépendamment choisi dans l'ensemble constitué par -H, -OH, $-OCH_3$, $-CO(C_6H_5)$, -Br, -I, -F, -Cl, $-CH_3$, et $-C_2H_5$ ;
iv) $R^4$ est une chaîne latérale d'isoleucine ;
v) X est choisi dans l'ensemble constitué par -O- et -(NH)- ;
vi) Y est choisi dans l'ensemble constitué par -H et un groupe protecteur d'hydroxyle ;
vii) $R^{10}$ est choisi dans l'ensemble constitué par une chaîne latérale de leucine et une chaîne latérale de lysine ; et
viii) la molécule n'est pas la tamandarine A.

**2.** Composition selon la revendication 1, dans laquelle $R^1$ est choisi dans l'ensemble constitué par :

-H,
-(tert-butyloxycarbonyle),
-leucine,
-(N-méthyl)leucine,
-(N-méthyl)leucine-(un premier fluorophore),
-(N-méthyl)leucine-(S)-proline,
-(N-méthyl)leucine-(S)-proline-(S)-lactate,
-(N-méthyl)leucine-(S)-proline -pyruvate,
-(N-méthyl)leucine-(S)-proline-(S)-lactate-(un premier fluorophore),
-(N-méthyl) leucine-(S)-proline-(S)-lactate-glutamine-pyroglutamate,
-(N-méthyl) leucine-(S)-proline-(S)-lactate-glutamine-cyclopentanoate,
-(N-méthyl) leucine-(S)-proline-alanine-leucine-pyroglutamate,
-(N-méthyl) leucine-(S)-proline-(N-méthyl-alanine)-leucine-pyroglutamate,
-(N-méthyl)leucine-désoxo-(S)-proline,
-(N-méthyl)leucine-désoxo-(S)-proline-(S)-lactate,
-(N-méthyl)leucine-désoxo-(S)-proline-pyruvate,
-(N-méthyl)leucine-désoxo-(S)-proline-(S)-lactate-(un premier fluorophore),
-(N-méthyl)leucine-désoxo-(S)-proline-(S)-lactate-glutamine-pyroglutamate,
-(N-méthyl)leucine-désoxo-(S)-proline-(S)-lactate-glutamine-cyclopentanoate,
-(N-méthyl)leucine-désoxo-(S)-proline-alanine-leucine-pyroglutamate,
-(N-méthyl)leucine-désoxo-(S)-proline-(N-méthyl-alanine)-leucine-pyroglutamate,
-(N-méthyl)leucine-déshydro-(S)-proline,

-(N-méthyl)leucine-déshydro-(S)-proline-(S)-lactate,
-(N-méthyl)leucine-déshydro-(S)-proline-pyruvate,
-(N-méthyl)leucine-déshydro-(S)-proline-(S)-lactate-(un premier fluorophore),
-(N-méthyl)leucine-déshydro-(S)-proline-(S)-lactate-glutamine-pyroglutamate,
-(N-méthyl)leucine-déshydro-(S)-proline-(S)-lactate-glutamine-cyclopentanoate,
-(N-méthyl)leucine-déshydro-(S)-proline-alanine-leucine-pyroglutamate, et
-(N-méthyl)leucine-déshydro-(S)-proline-(N-méthyl-alanine)-leucine-pyroglutamate.

**3.** Composition selon la revendication 1, dans lequel $R^1$ est choisi dans l'ensemble constitué par :

-H,
-(tert-butyloxycarbonyle),
-leucine,
-(N-méthyl)leucine,
-(N-méthyl)leucine-(un premier fluorophore),
-(N-méthyl)leucine-(S)-proline,
-(N-méthyl)leucine-(S)-proline-(S)-lactate,
-(N-méthyl)leucine-(S)-proline-pyruvate,
-(N-méthyl)leucine-(S)-proline-(S)-lactate-(un premier fluorophore),
-(N-méthyl)leucine-(S)-proline-(S)-lactate-(S)-glutamine-(S)-pyroglutamate,
-(N-méthyl)leucine-(S)-proline-(S)-lactate-(S)-glutamine-(S)-cyclopentanoate,
-(N-méthyl)leucine-(S)-proline-(S)-alanine-(S)-leucine-(S)-pyroglutamate,
-(N-méthyl)leucine-(S)-proline-(N-méthyl-S-alanine)-(S)-leucine-(S)-pyroglutamate,
-(N-méthyl)leucine-désoxo-(S)-proline,
-(N-méthyl)leucine-désoxo-(S)-proline-(S)-lactate,
-(N-méthyl)leucine-désoxo-(S)-proline-pyruvate,
-(N-méthyl)leucine-désoxo-(S)-proline-(S)-lactate-(un premier fluorophore),
-(N-méthyl)leucine-désoxo-(S)-proline-(S)-lactate-(S)-glutamine-(S)-pyroglutamate,
-(N-méthyl)leucine-désoxo-(S)-proline-(S)-lactate-(S)-glutamine-(S)-cyclopentanoate,
-(N-méthyl)leucine-désoxo-(S)-proline-(S)-alanine-(S)-leucine-(S)-pyroglutamate, et
-(N-méthyl)leucine-désoxo-(S)-proline-(N-méthyl-S-alanine)-(S)-leucine-(S)-pyroglutamate,
-(N-méthyl)leucine-déshydro-(S)-proline,
-(N-méthyl)leucine-déshydro-(S)-proline-(S)-lactate,
-(N-méthyl)leucine-déshydro-(S)-proline-pyruvate,
-(N-méthyl)leucine-déshydro-(S)-proline-(S)-lactate-(un premier fluorophore),
-(N-méthyl)leucine-déshydro-(S)-proline-(S)-lactate-(S)-glutamine-(S)-pyroglutamate,
-(N-méthyl)leucine-déshydro-(S)-proline-(S)-lactate-(S)-glutamine-(S)-cyclopentanoate,
-(N-méthyl)leucine-déshydro-(S)-proline-(S)-alanine-(S)-leucine-(S)-pyroglutamate, et
-(N-méthyl)leucine-déshydro-(S)-proline-(N-méthyl-S-alanine)-(S)-leucine-(S)-pyroglutamate.

**4.** Composition selon la revendication 1, dans laquelle $R^2$ est

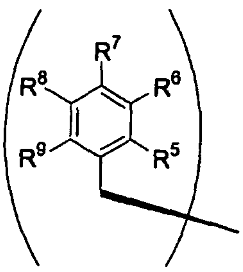

$R^3$ est méthyle, chacun de $R^5$, $R^6$, $R^8$ et $R^9$ est un radical hydrure, $R^7$ est méthoxy, $R^{10}$ est une chaîne latérale de leucine, X est -O-, et Y est un radical hydrure.

**5.** Composition selon la revendication 1, dans laquelle l'analogue de tamandarine est

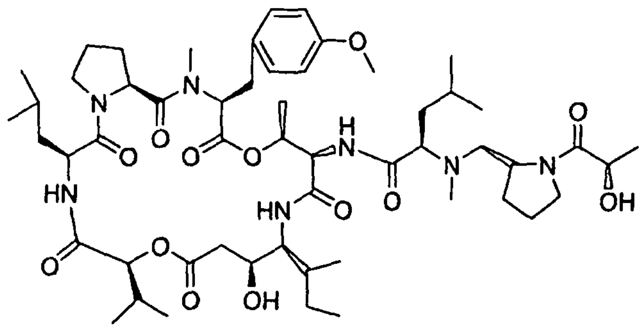

**6.** Composition selon la revendication 1, dans laquelle l'analogue de tamandarine est :

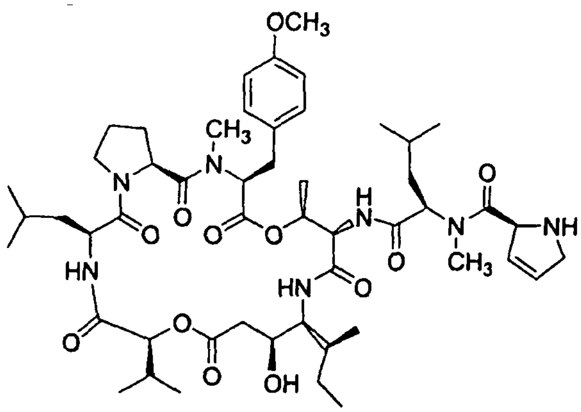

**7.** Composition selon la revendication 1, dans laquelle $R^1$ est -(N-méthyl)leucine-désoxo-(S)-proline-lactate.

**8.** Composition selon la revendication 1, dans laquelle $R^1$ est -(N-méthyl-R-leucine).

**9.** Composition selon la revendication 1, dans laquelle $R^1$ est -(N-méthyl-R-leucine)-(S)-proline-(S)-lactate-(S)-glutamine-(S)-pyroglutamate, Y est -H, et X est -O-.

**10.** Composition selon la revendication 9, dans laquelle l'analogue de tamandarine est

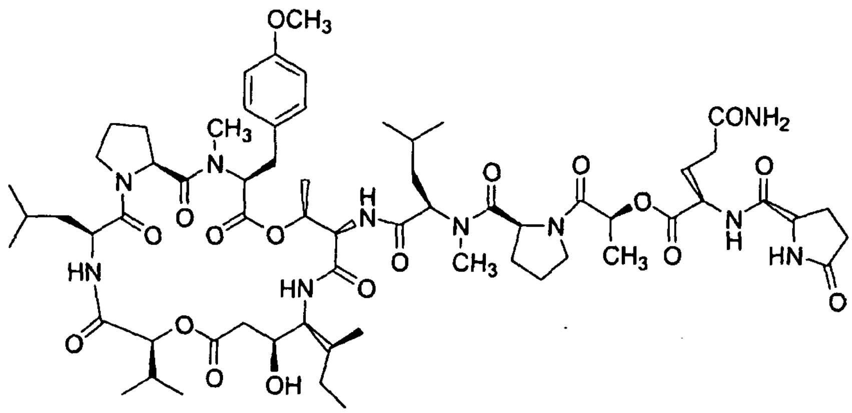

ou

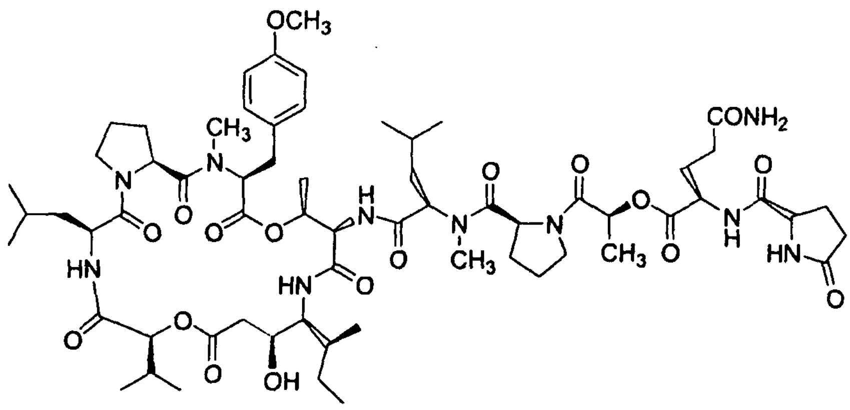

**11.** Composition selon la revendication 1, dans laquelle $R^1$ est -(N-méthyl)leucine-(S)-proline-(S)-lactate-(un premier fluorophore).

**12.** Composition selon la revendication 11, dans laquelle l'analogue de tamandarine est choisi dans l'ensemble constitué par

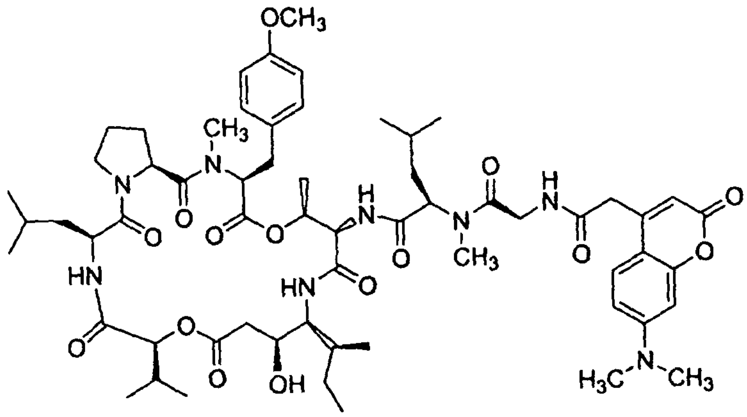

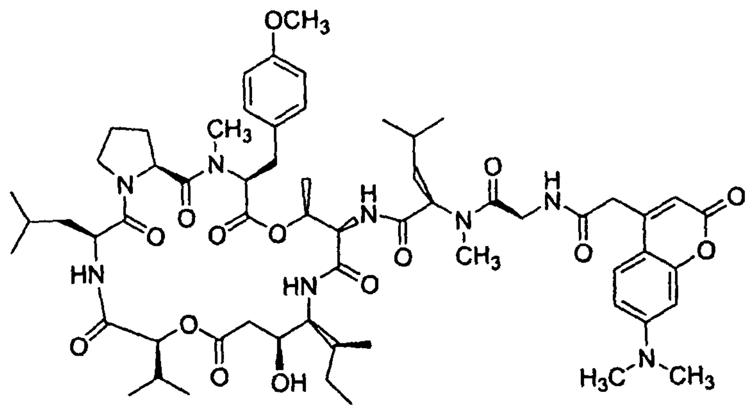

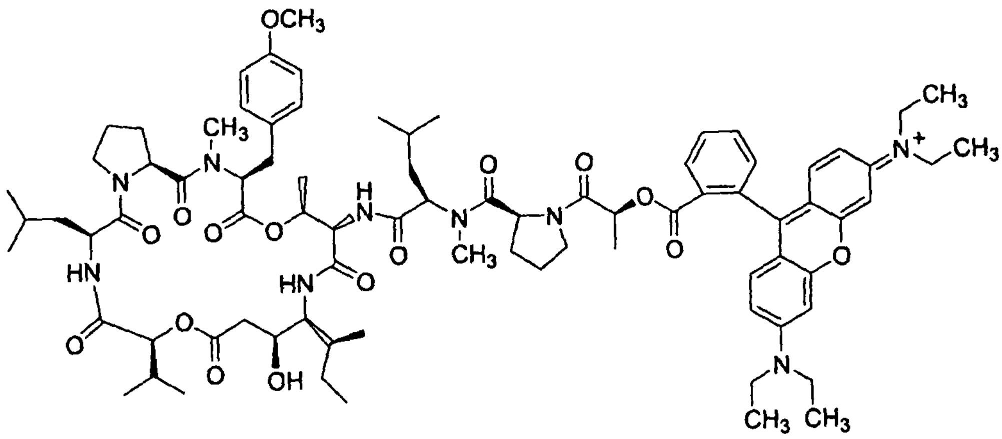

et

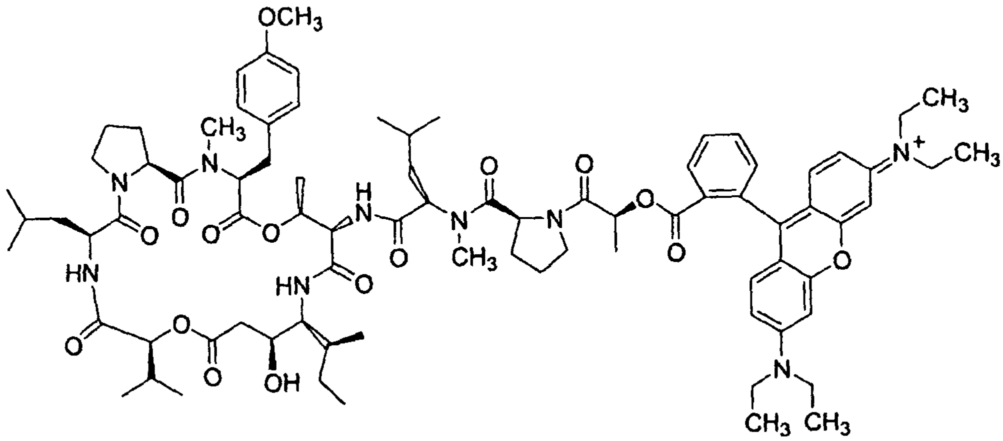

**13.** Composition selon la revendication 1, dans laquelle $R^1$ est -(N-méthyl)leucine-(S)-proline-pyruvate.

**14.** Composition selon la revendication 13, dans laquelle l'analogue de tamandarine est

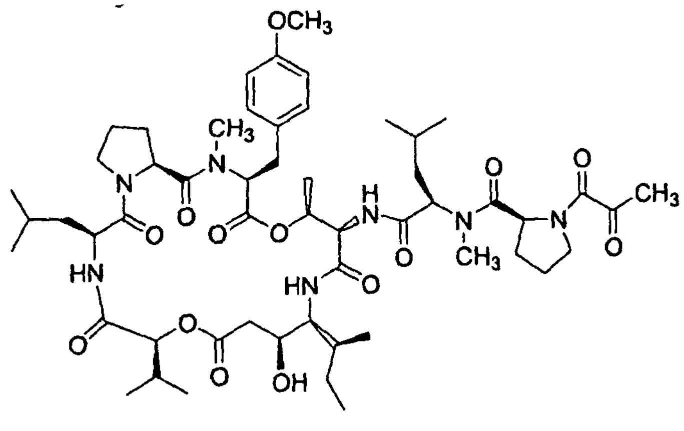

ou

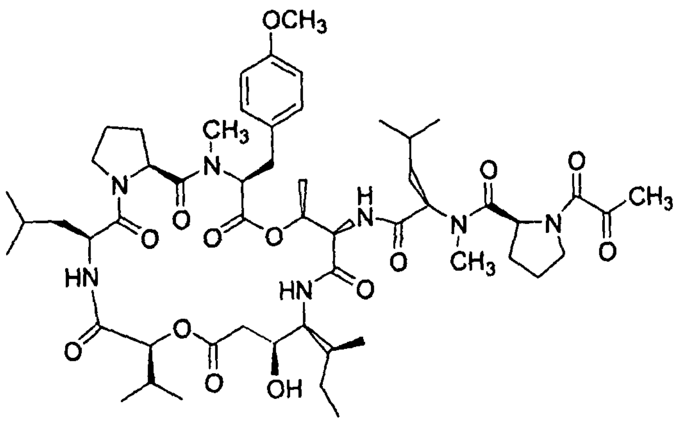

**15.** Composition selon la revendication 1, dans laquelle Y est -H, et dans laquelle $R^2$ a la structure

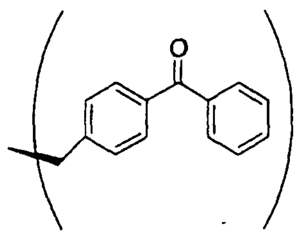

**16.** Composition selon la revendication 1, dans laquelle $R^2$ est une chaîne latérale de lysine et Y est -H.

**17.** Composition selon la revendication 1, dans laquelle l'analogue de tamandarine a la structure suivante :

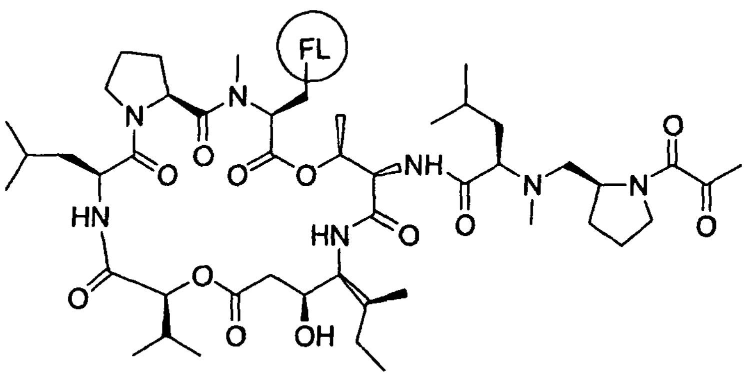

dans laquelle FL est un fluorophore.

**18.** Composition selon la revendication 1, dans laquelle X est -(NH)-.

**19.** Composition selon la revendication 1, dans laquelle l'analogue de tamandarine est choisi dans l'ensemble constitué par

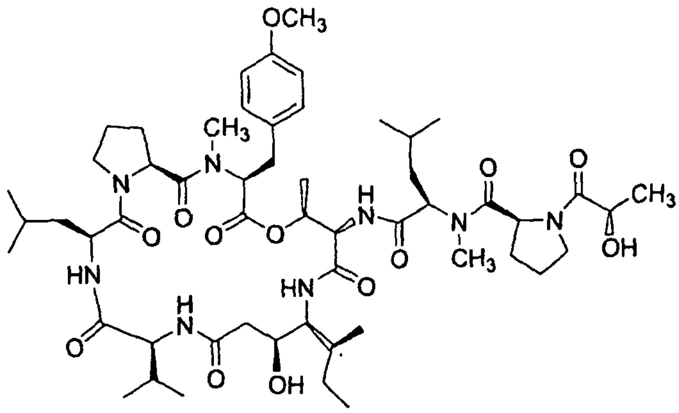

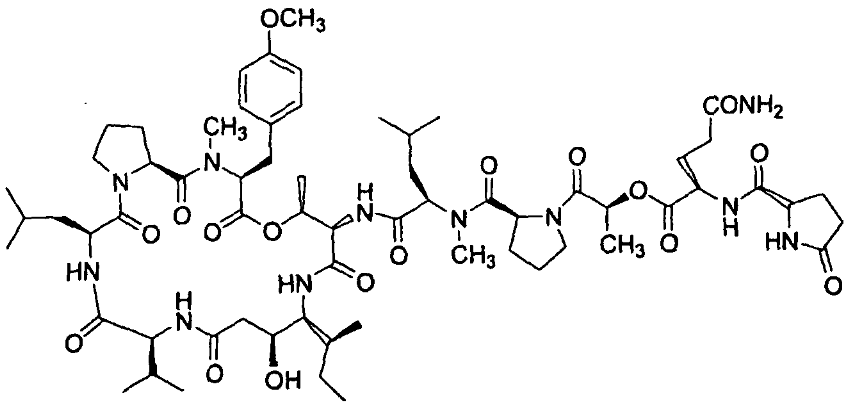

et

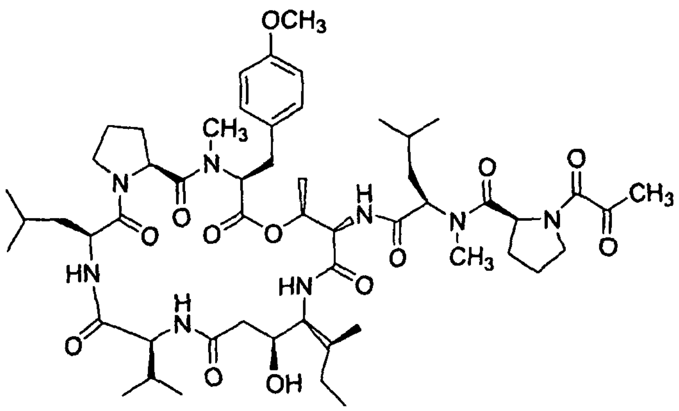

**20.** Composition selon la revendication 1, dans laquelle l'analogue de tamandarine est choisi dans l'ensemble constitué par

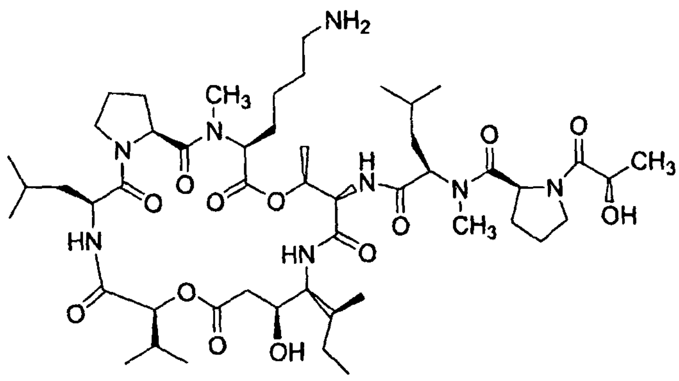

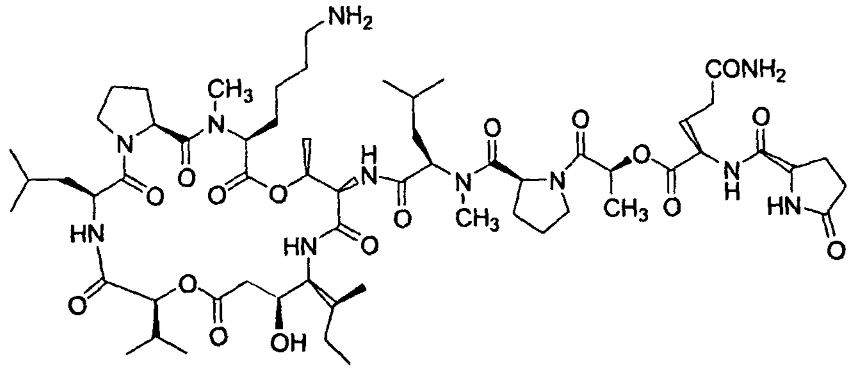

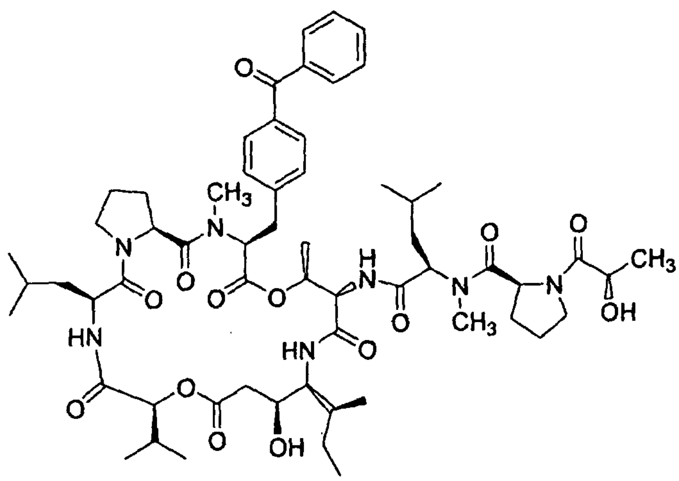

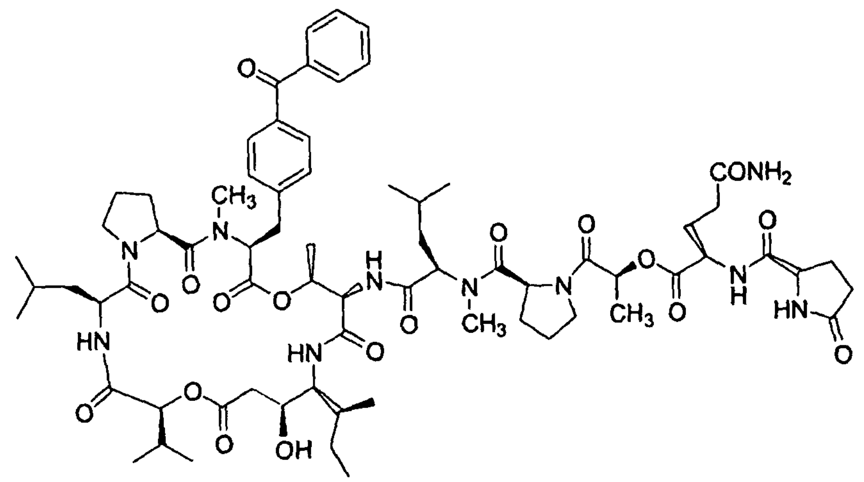

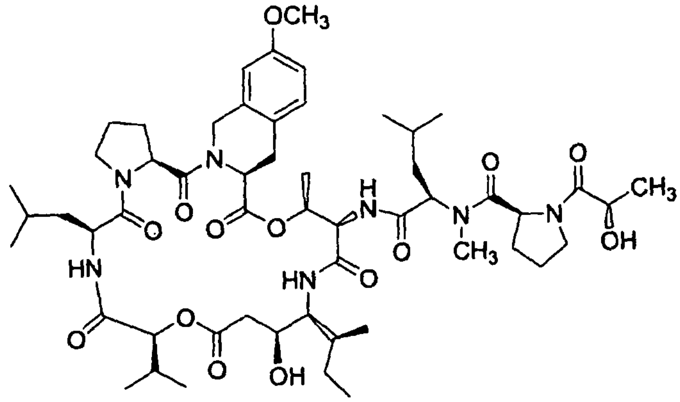

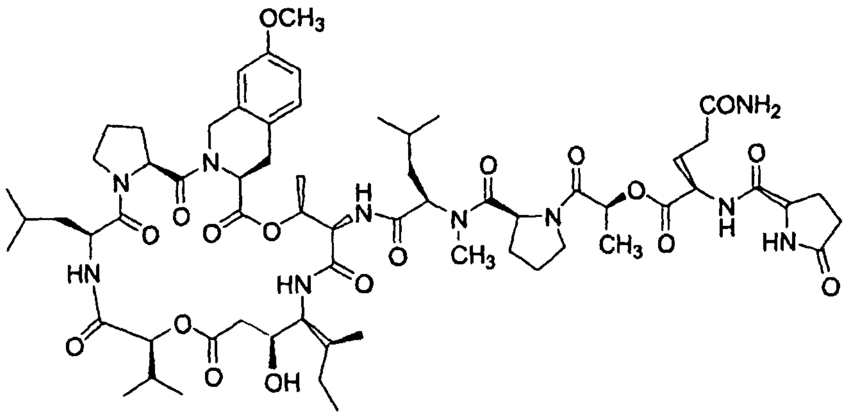

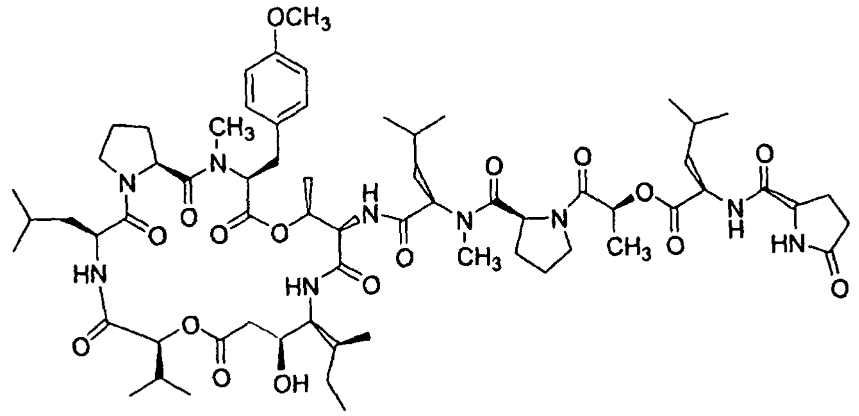

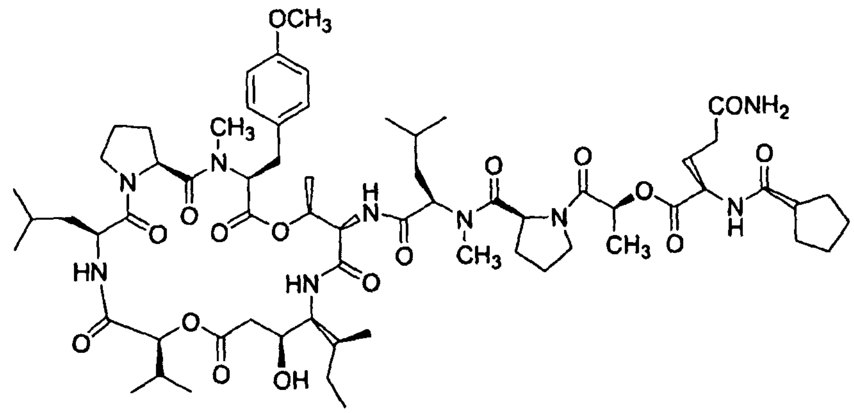

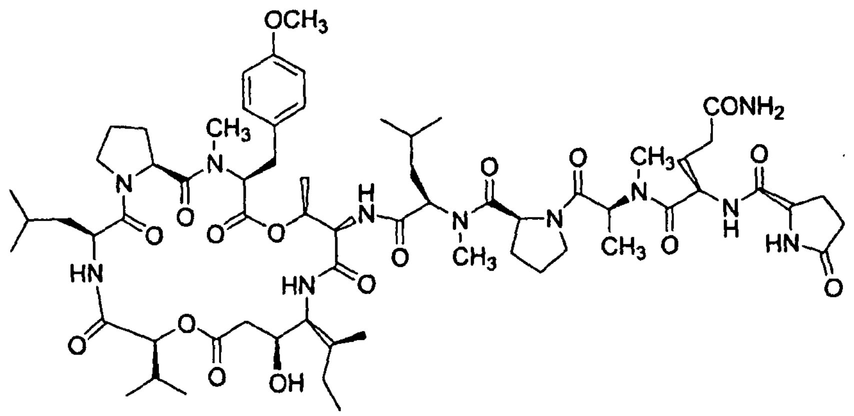

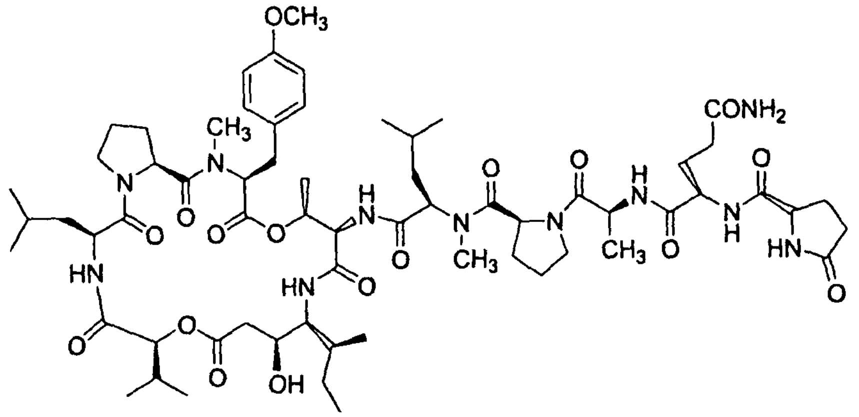

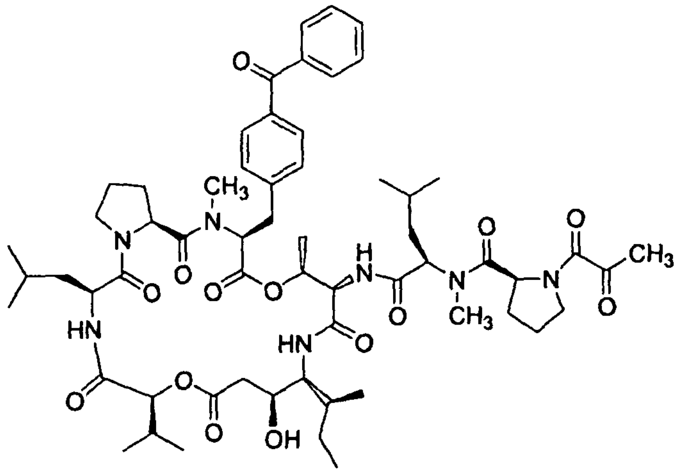

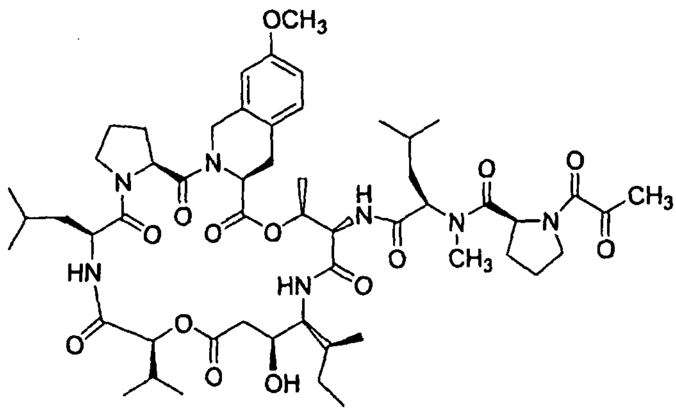

et

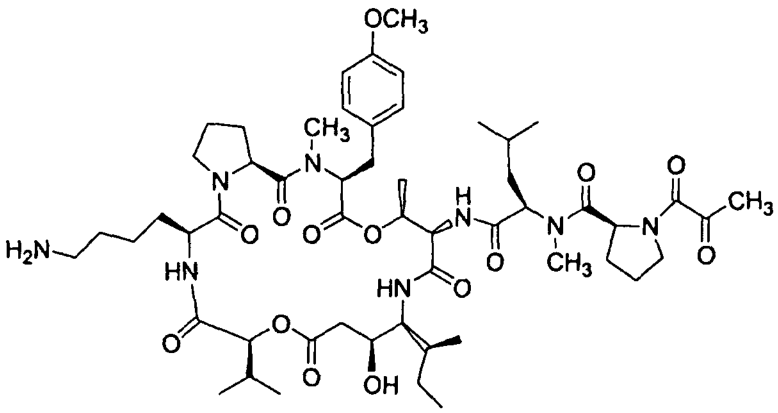

**21.** Composition selon la revendication 1, comprenant en outre un véhicule acceptable d'un point de vue pharmaceutique.

**22.** Support auquel est attaché de façon covalente l'analogue de tamandarine de la revendication 1.

**23.** Composition comprenant un composé ayant une structure choisie dans le groupe constitué par

(a)

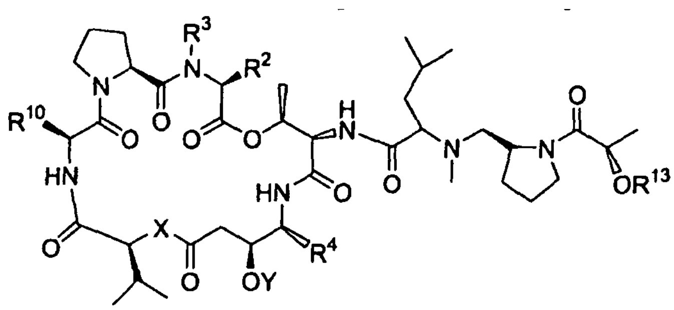

(b)

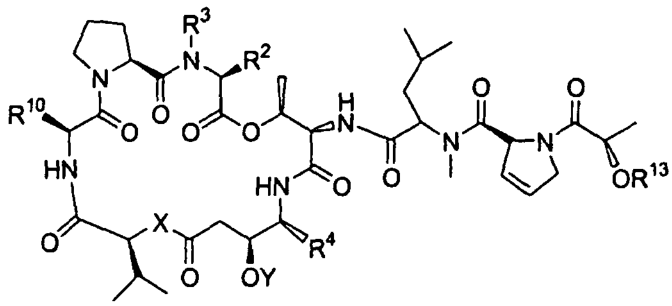

(c)

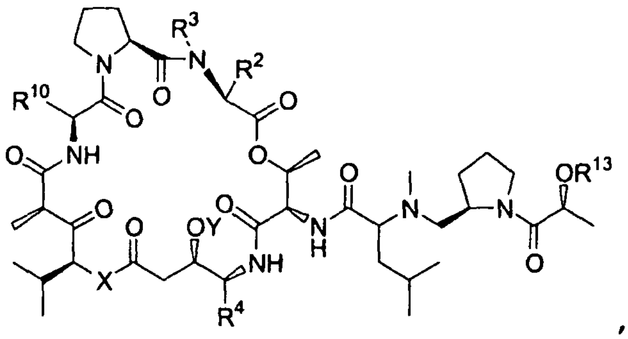

,

et

(d)

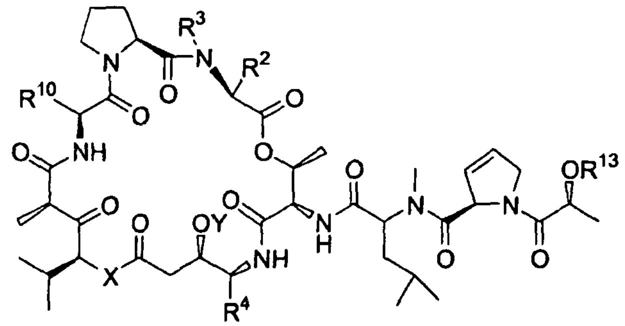

où :

i) soit

(a) $R^3$ est choisi dans l'ensemble constitué par $-CH_3$ et -H ; et $R^2$ est choisi dans l'ensemble constitué par une chaîne latérale d'isoleucine, une chaîne latérale de valine, une chaîne latérale d'alanine, une chaîne latérale de norleucine, une chaîne latérale de norvaline, une chaîne latérale de proline, une chaîne latérale de leucine, une chaîne latérale d'histidine, une chaîne latérale de tryptophane, une chaîne latérale d'arginine, une chaîne latérale de lysine, un deuxième fluorophore, et un substituant de structure :

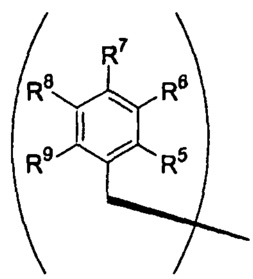

(b) $R^2$ et $R^3$ forment ensemble un substituant de structure :

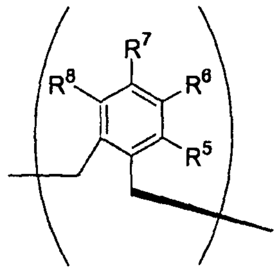

ii) chacun de $R^5$, $R^6$, $R^7$, $R^8$ et $R^9$, quand il est présent, est indépendamment choisi dans l'ensemble constitué par -H, -OH, $-OCH_3$, $-CO(C_6H_5)$, -Br, -I, -F, -Cl, $-CH_3$, et $-C_2H_5$ ; iii) $R^4$ est choisi dans l'ensemble constitué par une chaîne latérale d'isoleucine et une chaîne latérale de valine ;
iv) X est choisi dans l'ensemble constitué par -O- et -(NH)- ;
v) Y est choisi dans l'ensemble constitué par -H et un groupe protecteur d'hydroxyle ;
vi) $R^{10}$ est choisi dans l'ensemble constitué par une chaîne latérale de leucine et une chaîne latérale de lysine ; et
vii) $R^{13}$ est un fragment pouvant être coupé par une enzyme, qui peut être coupé par une enzyme choisie dans le groupe constitué par une carboxypeptidase, une β-lactamase, une β-galactosidase, une pénicilline V-amidase, une cytosine désaminase, une nitroréductase, une phosphatase alcaline, une β-glucuronidase, et un anticorps catalytique.

**24.** Composition selon la revendication 25, dans laquelle $R^{13}$ a la structure :

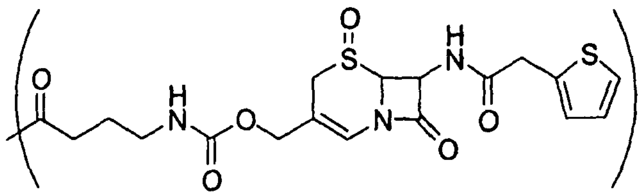

**25.** Composition selon la revendication 25, dans laquelle $R^{13}$ a la structure :

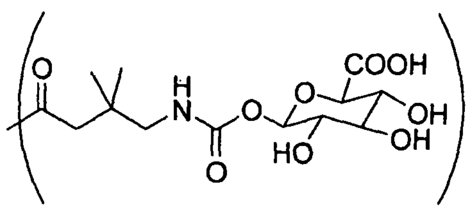

**26.** Composition comprenant un analogue de didemnine de structure :

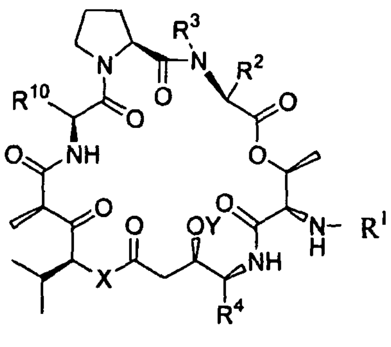

dans laquelle :

i) $R^1$ est choisi dans l'ensemble constitué par :

-(tert-butyloxycarbonyle),
-leucine,
-(N-méthyl)leucine-(un premier fluorophore),
-(N-méthyl)leucine-proline,
-(N-méthyl)leucine-proline-lactate-(un premier fluorophore),
-(N-méthyl)leucine-proline-lactate-glutamine-pyroglutamate,
-(N-méthyl)leucine-proline-lactate-glutamine-cyclopentanoate,
-(N-méthyl)leucine-proline-alanine-leucine-pyroglutamate,
-(N-méthyl)leucine-proline-(N-méthyl-alanine)-leucine-pyroglutamate,
-(N-méthyl)leucine-désoxo-proline,
-(N-méthyl)leucine-désoxo-proline-lactate,
-(N-méthyl)leucine-désoxo-proline-pyruvate,
-(N-méthyl)leucine-désoxo-proline-lactate-(un premier fluorophore),
-(N-méthyl)leucine-désoxo-proline-lactate-glutamine-pyroglutamate,
-(N-méthyl)leucine-désoxo-proline-lactate-glutamine-cyclopentanoate,
-(N-méthyl)leucine-désoxo-proline-alanine-leucine-pyroglutamate,
-(N-méthyl)leucine-désoxo-proline-(N-méthyl-alanine)-leucine-pyroglutamate,
-(N-méthyl)leucine-déshydro-proline,
-(N-méthyl)leucine-déshydro-proline-lactate,
-(N-méthyl)leucine-déshydro-proline-pyruvate,
-(N-méthyl)leucine-déshydro-proline-lactate-(un premier fluorophore),
-(N-méthyl)leucine-déshydro-proline-lactate-glutamine-pyroglutamate,
-(N-méthyl)leucine-déshydro-proline-lactate-glutamine-cyclopentanoate,
-(N-méthyl)leucine-déshydro-proline-alanine-leucine-pyroglutamate, et
-(N-méthyl)leucine-déshydro-proline-(N-méthyl-alanine)-leucine-pyroglutamate ;

ii) soit

(a) $R^3$ est choisi dans l'ensemble constitué par $-CH_3$ et -H ; et $R^2$ est choisi dans l'ensemble constitué par une chaîne latérale d'isoleucine, une chaîne latérale de valine, une chaîne latérale d'alanine, une chaîne latérale de norleucine, une chaîne latérale de norvaline, une chaîne latérale de leucine, une chaîne latérale d'histidine, une chaîne latérale de tryptophane, une chaîne latérale d'arginine, une chaîne latérale de lysine, un deuxième fluorophore, et un substituant de structure :

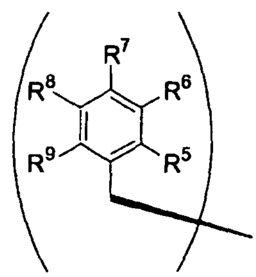

(b) $R^2$ et $R^3$ forment ensemble un substituant de structure :

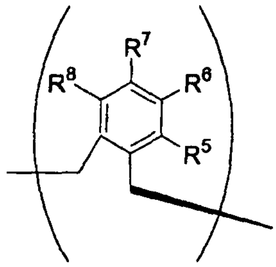

iii) chacun de $R^5$, $R^6$, $R^7$, $R^8$ et $R^9$, quand il est présent, est indépendamment choisi dans l'ensemble constitué par -H, -OH, $-OCH_3$, $-CO(C_6H_5)$, -Br, -I, -F, -Cl, $-CH_3$, et $-C_2H_5$ ;
iv) $R^4$ est choisi dans l'ensemble constitué par une chaîne latérale d'isoleucine et une chaîne latérale de valine ;
v) X est choisi dans l'ensemble constitué par -O- et -(NH)- ;
vi) Y est choisi dans l'ensemble constitué par -H et un groupe protecteur d'hydroxyle ; et
vii) $R^{10}$ est choisi dans l'ensemble constitué par une chaîne latérale de leucine et une chaîne latérale de lysine.

**27.** Composition selon la revendication 26, dans laquelle $R^1$ est choisi dans l'ensemble constitué par :

-(tert-butyloxycarbonyle),
-leucine,
-(N-méthyl)leucine-(un premier fluorophore),
-(N-méthyl)leucine-(S)-proline,
-(N-méthyl)leucine-(S)-proline-(S)-lactate-(un premier fluorophore),
-(N-méthyl)leucine-(S)-proline-(S)-lactate-glutamine-pyroglutamate,
-(N-méthyl)leucine-(S)-proline-(S)-lactate-glutamine-cyclopentanoate,
-(N-méthyl)leucine-(S)-proline-alanine-leucine-pyroglutamate,
-(N-méthyl)leucine-(S)-proline-(N-méthyl-alanine)-leucine-pyroglutamate,
-(N-méthyl)leucine-désoxo-(S)-proline,
-(N-méthyl)leucine-désoxo-(S)-proline-(S)-lactate,
-(N-méthyl)leucine-désoxo-(S)-proline-pyruvate,
-(N-méthyl)leucine-désoxo-(S)-proline-(S)-lactate-(un premier fluorophore),
-(N-méthyl)leucine-désoxo-(S)-proline-(S)-lactate-glutamine-pyroglutamate,
-(N-méthyl)leucine-désoxo-(S)-proline-(S)-lactate-glutamine-cyclopentanoate,
-(N-méthyl)leucine-désoxo-(S)-proline-alanine-leucine-pyroglutamate,
-(N-méthyl)leucine-désoxo-(S)-proline-(N-méthyl-alanine)-leucine-pyroglutamate,
-(N-méthyl)leucine-déshydro-(S)-proline,
-(N-méthyl) leucine-déshydro-(S)-proline-(S)-lactate,
-(N-méthyl) leucine-déshydro-(S)-proline-pyruvate,
-(N-méthyl) leucine-déshydro-(S)-proline-(S)-lactate-(un premier fluorophore),
-(N-méthyl)leucine-déshydro-(S)-proline-(S)-lactate-glutamine-pyroglutamate,
-(N-méthyl)leucine-déshydro-(S)-proline-(S)-lactate-glutamine-cyclopentanoate,
-(N-méthyl)leucine-déshydro-(S)-proline-alanine-leucine-pyroglutamate, et
-(N-méthyl)leucine-déshydro-(S)-proline-(N-méthyl-alanine)-leucine-pyroglutamate.

**28.** Composition selon la revendication 26, dans lequel $R^1$ est choisi dans l'ensemble constitué par :

-(tert-butyloxycarbonyle),
-leucine,
-(N-méthyl)leucine-(un premier fluorophore),
-(N-méthyl)leucine-(S)-proline,
-(N-méthyl)leucine-(S)-proline-(S)-lactate-(un premier fluorophore),
-(N-méthyl)leucine-(S)-proline-(S)-lactate-(S)-glutamine-(S)-pyroglutamate,
-(N-méthyl)leucine-(S)-proline-(S)-lactate-(S)-glutamine-(S)-cyclopentanoate,
-(N-méthyl)leucine-(S)-proline-(S)-alanine-(S)-leucine-(S)-pyroglutamate,
-(N-méthyl)leucine-(S)-proline-(N-méthyl-S-alanine)-(S)-leucine-(S)-pyroglutamate,
-(N-méthyl)leucine-désoxo-(S)-proline,
-(N-méthyl)leucine-désoxo-(S)-proline-(S)-lactate,

-(N-méthyl)leucine-désoxo-(S)-proline-pyruvate,
-(N-méthyl)leucine-désoxo-(S)-proline-(S)-lactate-(un premier fluorophore),
-(N-méthyl)leucine-désoxo-(S)-proline-(S)-lactate-(S)-glutamine-(S)-pyroglutamate,
-(N-méthyl)leucine-désoxo-(S)-proline-(S)-lactate-(S)-glutamine-(S)-cyclopentanoate,
-(N-méthyl)leucine-désoxo-(S)-proline-(S)-alanine-(S)-leucine-(S)-pyroglutamate, et
-(N-méthyl)leucine-désoxo-(S)-proline-(N-méthyl-S-alanine)-(S)-leucine-(S)-pyroglutamate,
-(N-méthyl)leucine-déshydro-(S)-proline,
-(N-méthyl)leucine-déshydro-(S)-proline-(S)-lactate,
-(N-méthyl)leucine-désoxo-(S)-proline-pyruvate,
-(N-méthyl)leucine-désoxo-(S)-proline-(S)-lactate-(un premier fluorophore),
-(N-méthyl)leucine-désoxo-(S)-proline-(S)-lactate-(S)-glutamine-(S)-pyroglutamate,
-(N-méthyl) leucine-désoxo-(S)-proline-(S)-lactate-(S)-glutamine-(S)-cyclopentanoate,
-(N-méthyl) leucine-désoxo-(S)-proline-(S)-alanine-(S)-leucine-(S)-pyroglutamate, et
-(N-méthyl)leucine-désoxo-(S)-proline-(N-méthyl-S-alanine)-(S)-leucine-(S)-pyroglutamate.

**29.** Composition selon la revendication 26, dans laquelle $R^2$ est

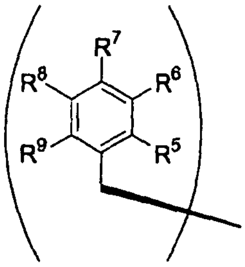

$R^3$ est méthyle, $R^4$ est une chaîne latérale d'isoleucine, chacun de $R^5$, $R^6$ $R^8$ et $R^9$ est un radical hydrure, $R^7$ est méthoxy, $R^{10}$ est une chaîne latérale de leucine, X est -O-, et Y est un radical hydrure.

**30.** Composition selon la revendication 26, dans laquelle l'analogue de didemnine est

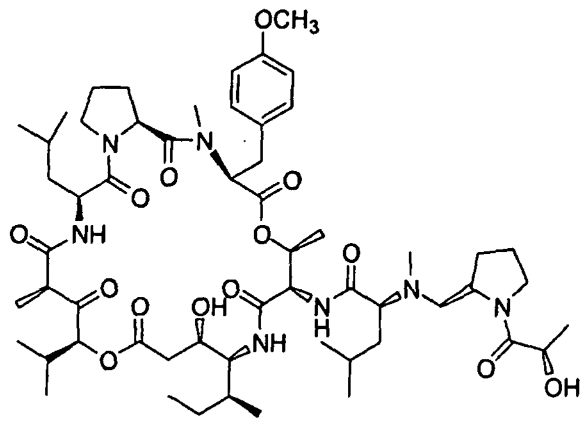

**31.** Composition selon la revendication 26, dans laquelle l'analogue de didemnine est

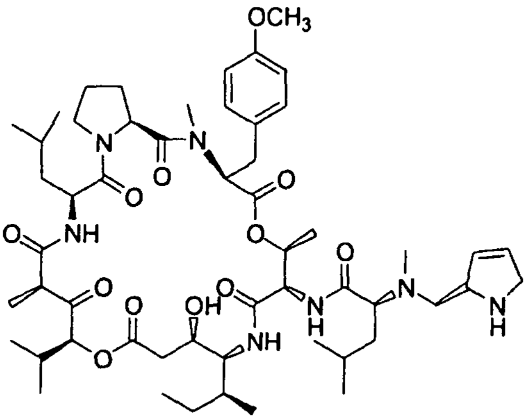

**32.** Composition selon la revendication 26, dans laquelle R est -(N-méthyl)leucine-désoxo-(S)-proline-lactate.

**33.** Composition selon la revendication 26, dans laquelle Y est -H, et dans laquelle $R^2$ a la structure

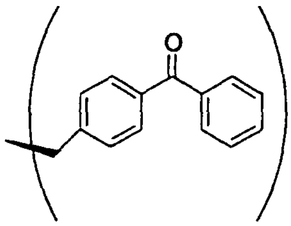

**34.** Composition selon la revendication 26, dans laquelle $R^2$ est une chaîne latérale de lysine et Y est -H.

**35.** Composition selon la revendication 26, dans laquelle X est -(NH)-.

**36.** Composition selon la revendication 26, comprenant en outre un véhicule acceptable d'un point de vue pharmaceutique.

**37.** Support attaché de façon covalente à l'analogue de didemnine de la revendication 26.

**38.** Procédé de préparation d'un analogue de tamandarine de la revendication 1 ou d'un analogue de didemnine de la revendication 26, l'amélioration comprenant l'incorporation d'un résidu désoxo-proline à la place d'un résidu proline de l'analogue, $R^1$ comprenant un résidu désoxo-proline.

**39.** Procédé selon la revendication 38, dans lequel l'analogue comprend un fragment (N-méthyl)leucine-proline et dans lequel le fragment (N-méthyl)leucine-proline est remplacé par un fragment (N-méthyl)leucine-désoxo-proline.

**40.** Procédé selon la revendication 39, dans lequel le fragment (N-méthyl)leucine-désoxoproline est produit par réduction de la fonction ester de la proline en une fonction aldéhyde ; et couplage de la proline avec le fragment (N-méthyl) leucine par amination réductrice pour obtenir le fragment (N-méthyl)leucine-désoxo-proline.

**41.** Procédé selon la revendication 40, dans lequel le fragment amine de la proline est protégé par un groupe protecteur d'amine avant l'amination réductrice.

**42.** Procédé selon la revendication 40, dans lequel la fonction ester de la proline est réduite en une fonction aldéhyde par mise en contact de la proline avec une base forte et ensuite mise en contact de la proline avec un agent oxydant.

**43.** Procédé selon la revendication 40, dans lequel l'amination réductrice est réalisée dans un solvant non aqueux en présence d'une base forte et d'un catalyseur acide carboxylique.

**44.** Procédé de préparation d'un analogue de tamandarine de la revendication 1 ou d'un analogue de didemnine de la revendication 26, l'amélioration comprenant l'incorporation d'un résidu déshydro-proline à la place d'un résidu proline de l'analogue, $R^1$ comprenant un résidu déshydro-proline.

**45.** Procédé selon la revendication 44, dans lequel l'analogue comprend un fragment (N-méthyl)leucine-proline et dans lequel le fragment (N-méthyl)leucine-proline est remplacé par un fragment (N-méthyl)leucine-déshydro-proline.

**46.** Procédé selon la revendication 44, dans lequel le résidu déshydro-proline est produit par protection des fragments carboxyle et amino du 4-hydroxyprolinate, alkylsulfonation du fragment 4-hydroxyle, déplacement du fragment alkylsulfonate avec un fragment arylsélényle, élimination par oxydation du fragment arylsélényle pour engendrer un fragment déshydro-proline ayant des fragments carboxyle et amine protégés, et couplage du fragment déshydro-proline avec un fragment amine de l'analogue.

**47.** Procédé selon la revendication 46, dans lequel le fragment alkylsulfonate est un fragment méthylsulfonate.

**48.** Procédé selon la revendication 46, dans lequel le fragment arylsélényle est un fragment phénylsélényle.

**49.** Procédé selon la revendication 44, dans lequel le 4-hydroxyprolinate est le trans-4-hydroxyprolinate.

**50.** Composition comprenant un fragment de didemnine de structure :

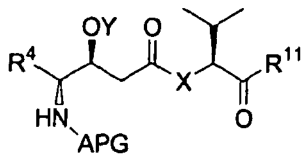

dans laquelle :

i) Y est choisi dans l'ensemble constitué par -H et un groupe protecteur d'hydroxyle ;
ii) X est choisi dans l'ensemble constitué par -O- et -(NH)- ;
iii) $R^4$ est choisi dans l'ensemble constitué par une chaîne latérale d'isoleucine et une chaîne latérale de valine ;
iv) APG est un groupe protecteur d'amine ; et
v) $R^{11}$ est choisi dans l'ensemble constitué par -OH, $-NH_2$, -O(allyle), -O(pentafluorophényle), et un substituant de structure

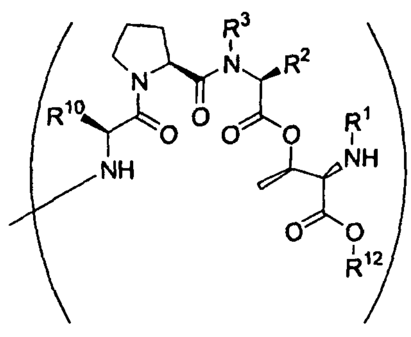

dans laquelle

a) $R^1$ est choisi dans l'ensemble constitué par -H, et un groupe protecteur d'amine ;
b) soit

(I) $R^3$ est choisi dans l'ensemble constitué par $-CH_3$ et -H ; et $R^2$ est choisi dans l'ensemble constitué par une chaîne latérale d'isoleucine, une chaîne latérale de valine, une chaîne latérale d'alanine, une chaîne latérale de norleucine, une chaîne latérale de norvaline, une chaîne latérale de proline, une chaîne latérale

de leucine, une chaîne latérale d'histidine, une chaîne latérale de tryptophane, une chaîne latérale d'arginine, une chaîne latérale de lysine, un deuxième fluorophore, et un substituant de structure :

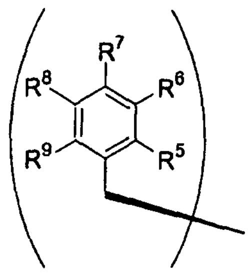

ou

(II) $R^2$ et $R^3$ forment ensemble un substituant de structure :

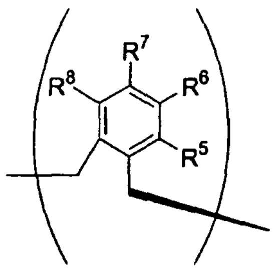

c) chacun de $R^5$, $R^6$, $R^7$, $R^8$ et $R^9$, quand il est présent, est indépendamment choisi dans l'ensemble constitué par -H, -OH, $-OCH_3$, -CO $(C_6H_5)$, -Br, -I, -F, -Cl, $-CH_3$, et $-C_2H_5$ ; d) $R^{10}$ est choisi dans l'ensemble constitué par une chaîne latérale de leucine, une chaîne latérale de lysine, et une chaîne latérale lysine protégée ; et e) $R^{12}$ est choisi dans l'ensemble constitué par -H, et 2-(triméthylsilyl)éthoxycarbonyle.

**51.** Procédé de préparation d'un fragment de didemnine, le procédé comprenant le couplage d'un premier réactant de structure :

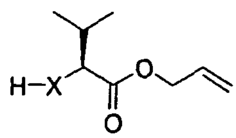

et d'un deuxième réactant de structure :

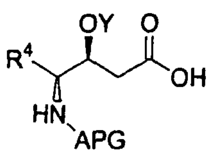

pour engendrer un premier fragment de didemnine de structure

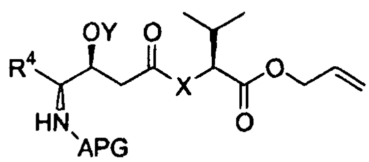

dans laquelle X est choisi dans l'ensemble constitué par -O- et -(NH)- ; dans laquelle APG est un groupe protecteur d'amine ; dans laquelle Y est un groupe protecteur d'hydroxyle ; et dans laquelle $R^4$ est choisi dans l'ensemble constitué par une chaîne latérale d'isoleucine et une chaîne latérale de valine.

**52.** Procédé selon la revendication 51, dans lequel Y est -triisopropylsilyle.

**53.** Procédé selon la revendication 52, comprenant en outre l'hydrolyse du premier fragment de didemnine pour engendrer un deuxième fragment de didemnine de structure :

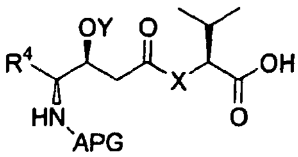

**54.** Procédé selon la revendication 53, comprenant en outre l'addition d'un activateur au fragment carbonyle du deuxième fragment de didemnine pour engendrer un troisième fragment de didemnine de structure

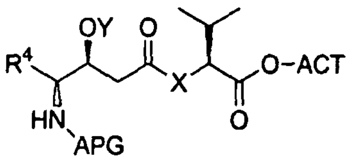

dans laquelle -ACT est un activateur.

**55.** Procédé selon la revendication 54, comprenant en outre le couplage du troisième fragment de didemnine et d'un troisième réactant de structure

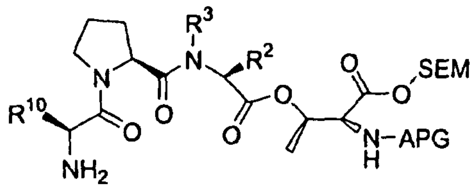

pour engendrer un quatrième fragment de didemnine de structure

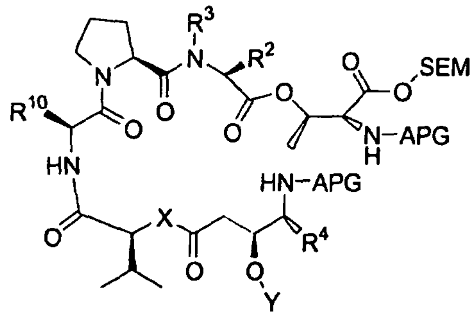

dans laquelle :

i) soit

(a) $R^3$ est choisi dans l'ensemble constitué par $-CH_3$ et -H ; et $R^2$ est choisi dans l'ensemble constitué par

une chaîne latérale d'isoleucine, une chaîne latérale de valine, une chaîne latérale d'alanine, une chaîne latérale de norleucine, une chaîne latérale de norvaline, une chaîne latérale de proline, une chaîne latérale de leucine, une chaîne latérale d'histidine, une chaîne latérale de tryptophane, une chaîne latérale d'arginine, une chaîne latérale de lysine, un deuxième fluorophore, et un substituant de structure :

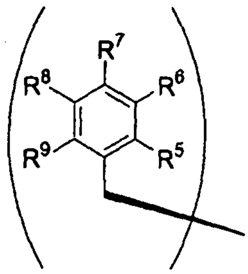

soit

(b) $R^2$ et $R^3$ forment ensemble un substituant de structure :

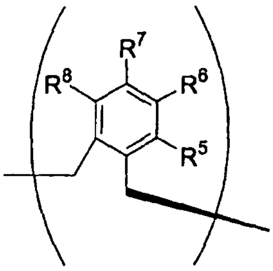

ii) chacun de $R^5$, $R^6$, $R^7$, $R^8$ et $R^9$, quand il est présent, est indépendamment choisi dans l'ensemble constitué par -H, -OH, $-OCH_3$, $-CO(C_6H_5)$, -Br, -I, -F, -Cl, $-CH_3$, et $-C_2H_5$ ;

iii) APG est un groupe protecteur d'aminé ;

iv) SEM est 2-(triméthylsilyl)éthoxycarbonyle et

iii) $R^{10}$ est choisi dans l'ensemble constitué par une chaîne latérale de leucine et une chaîne latérale de lysine.

**56.** Procédé pour produire un analogue de didemnine, le procédé comprenant l'élimination du fragment SEM et du fragment APG protégeant le groupe amino attaché à l'atome de carbone auquel R4 est lié, à partir du quatrième fragment de didemnine de la revendication 55, et la cyclisation du quatrième fragment de didemnine pour engendrer un premier analogue de didemnine de structure

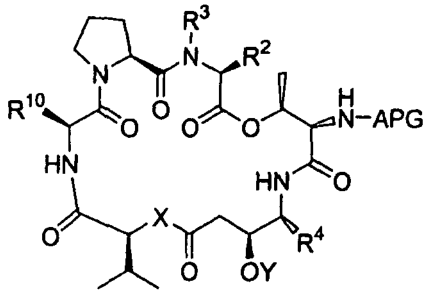

**57.** Procédé selon la revendication 56, comprenant en outre l'élimination du groupe APG à partir du premier analogue de didemnine pour engendrer un deuxième analogue de didemnine de structure

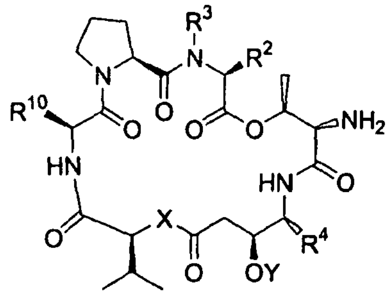

**58.** Procédé selon la revendication 56, dans lequel le groupe APG est choisi dans l'ensemble constitué par un fragment carbobenzyloxy et un fragment tert-butyloxycarbonyle.

**59.** Procédé selon la revendication 57, comprenant en outre le couplage du deuxième analogue de didemnine et d'un quatrième réactant de structure

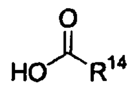

pour engendrer un troisième analogue de didemnine de structure

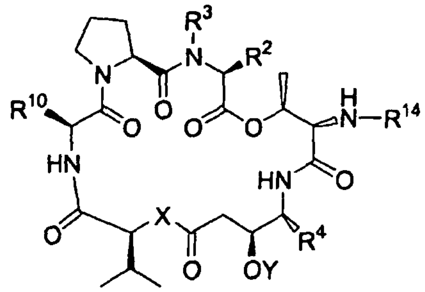

dans laquelle $R^{14}$ est choisi dans l'ensemble constitué par :

-leucine,
-(N-méthyl)leucine,
-(N-méthyl)leucine-(un premier fluorophore),
-(N-méthyl)leucine-proline,
-(N-méthyl)leucine-proline-lactate,
-(N-méthyl)leucine-proline-pyruvate,
-(N-méthyl)leucine-proline-lactate-(un premier fluorophore),
-(N-méthyl)leucine-proline-lactate-glutamine-pyroglutamate,
-(N-méthyl)leucine-proline-lactate-glutamine-cyclopentanoate,
-(N-méthyl)leucine-proline-alanine-leucine-pyroglutamate,
-(N-méthyl)leucine-proline-(N-méthyl-alanine)-leucine-pyroglutamate,
-(N-méthyl)leucine-désoxo-proline,
-(N-méthyl)leucine-désoxo-proline-lactate,
-(N-méthyl)leucine-désoxo-proline-pyruvate,
-(N-méthyl) leucine-désoxo-proline-lactate-(un premier fluorophore),
-(N-méthyl)leucine-désoxo-proline-lactate-glutamine-pyroglutamate,
-(N-méthyl)leucine-désoxo-proline-lactate-glutamine-cyclopentanoate,
-(N-méthyl)leucine-désoxo-proline-alanine-leucine-pyroglutamate,
-(N-méthyl)leucine-désoxo-proline (N-méthyl-alanine)-leucine-pyroglutamate,
-(N-méthyl)leucine-déshydro-proline,

-(N-méthyl)leucine-déshydro-proline-lactate,
-(N-méthyl)leucine-déshydro-proline-pyruvate,
-(N-méthyl)leucine-déshydro-proline-lactate-(un premier fluorophore),
-(N-méthyl)leucine-déshydro-proline-lactate-glutamine-pyroglutamate,
-(N-méthyl)leucine-déshydro-proline-lactate-glutamine-cyclopentanoate,
-(N-méthyl)leucine-déshydro-proline-alanine-leucine-pyroglutamate,
-(N-méthyl)leucine-déshydro-proline-(N-méthyl-alanine)-leucine-pyroglutamate ; et

l'un quelconque des fragments ci-dessus, couplé avec un fragment pouvant être coupé par une enzyme, qui peut être coupé par une enzyme choisie dans l'ensemble constitué par une carboxypeptidase, une β-lactamase, une β-galactosidase, une pénicilline V-amidase, une cytosine désaminase, une nitroréductase, une phosphatase alcaline, une β-glucuronidase, et un anticorps catalytique.

**60.** Procédé selon la revendication 59, dans lequel Y est un groupe protecteur d'hydroxyle, le procédé comprenant en outre l'élimination du groupe protecteur d'hydroxyle à partir du deuxième analogue de didemnine pour engendrer un quatrième analogue de didemnine de structure

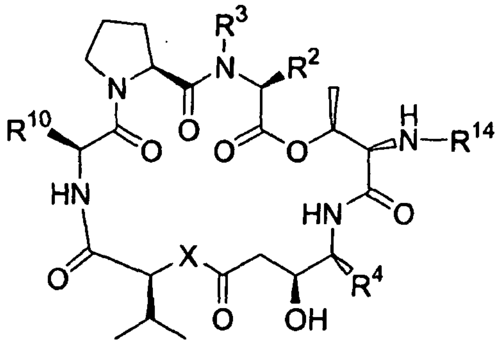

**61.** Utilisation de l'une quelconque des compositions de la revendication 1, 23, 26 ou 50, pour préparer un médicament destiné à inhiber la synthèse protéique dans une cellule.

**62.** Utilisation de l'une quelconque des compositions de la revendication 1, 23, 26 ou 50, pour préparer un médicament destiné à inhiber la croissance d'une cellule.

**63.** Utilisation de l'une quelconque des compositions de la revendication 1, 23, 26 ou 50, pour préparer un médicament destiné à inhiber la prolifération d'une cellule.

**64.** Utilisation de l'une quelconque des compositions de la revendication 1, 23, 26 ou 50, pour préparer un médicament destiné à inhiber l'oncogenèse dans une cellule.

**65.** Utilisation de l'une quelconque des compositions de la revendication 1, 23, 26 ou 50, pour préparer un médicament destiné à inhiber un accroissement de l'apoptose d'une cellule.

**66.** Composition comprenant un analogue de tamandarine de structure

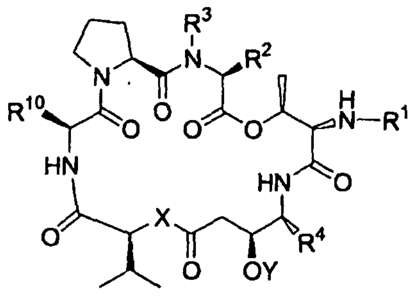

dans laquelle :

i) $R^1$ est choisi dans l'ensemble constitué par

-H,
-(tert-butyloxycarbonyle),
-leucine,
-(N-méthyl)leucine,
-(N-méthyl)leucine-(un premier fluorophore),
-(N-méthyl)leucine-proline,
-(N-méthyl)leucine-proline-pyruvate,
-(N-méthyl)leucine-proline-lactate-(un premier fluorophore),
-(N-méthyl)leucine-proline-lactate-glutamine-pyroglutamate,
-(N-méthyl)leucine-proline-lactate-glutamine-cyclopentanoate,
-(N-méthyl)leucine-proline-alanine-leucine-pyroglutamate,
-(N-méthyl)leucine-proline-(N-méthyl-alanine)-leucine-pyroglutamate,
-(N-méthyl)leucine-désoxo-proline,
-(N-méthyl)leucine-désoxo-proline-lactate,
-(N-méthyl)leucine-désoxo-proline-pyruvate,
-(N-méthyl)leucine-désoxo-proline-lactate-(un premier fluorophore),
-(N-méthyl)leucine-désoxo-proline-lactate-glutamine-pyroglutamate,
-(N-méthyl)leucine-désoxo-proline-lactate-glutamine-cyclopentanoate,
-(N-méthyl)leucine-désoxo-proline-alanine-leucine-pyroglutamate,
-(N-méthyl)leucine-désoxo-proline-(N-méthyl-alanine)-leucine-pyroglutamate,
-(N-méthyl)leucine-déshydro-proline,
-(N-méthyl)leucine-déshydro-proline-lactate,
-(N-méthyl)leucine-déshydro-proline-pyruvate,
-(N-méthyl)leucine-déshydro-proline-lactate-(un premier fluorophore),
-(N-méthyl)leucine-déshydro-proline-lactate-glutamine-pyroglutamate,
-(N-méthyl)leucine-déshydro-proline-lactate-glutamine-cyclopentanoate,
-(N-méthyl)leucine-déshydro-proline-alanine-leucine-pyroglutamate, et
-(N-méthyl) leucine-déshydro-proline-(N-méthyl-alanine)-leucine-pyroglutamate ;

ii) soit

(a) $R^3$ est choisi dans l'ensemble constitué par $-CH_3$ et -H ; et $R^2$ est choisi dans l'ensemble constitué par une chaîne latérale d'isoleucine, une chaîne latérale de valine, une chaîne latérale d'alanine, une chaîne latérale de norleucine, une chaîne latérale de norvaline, une chaîne latérale de leucine, une chaîne latérale d'histidine, une chaîne latérale de tryptophane, une chaîne latérale d'arginine, une chaîne latérale de lysine, un deuxième fluorophore, et un substituant de structure :

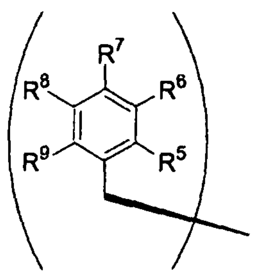

soit
(b) $R^2$ et $R^3$ forment ensemble un substituant de structure :

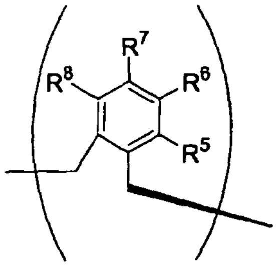

iii) chacun de $R^5$, $R^6$, $R^7$, $R^8$ et $R^9$, quand il est présent, est indépendamment choisi dans l'ensemble constitué par -H, -OH, $-OCH_3$, $-CO(C_6H_5)$, -Br, -I, -F, -Cl, $-CH_3$, et $-C_2H_5$ ;
iv) $R^4$ est une chaîne latérale de valine ;
v) X est choisi dans l'ensemble constitué par -O- et (NH) - ;
vi) Y est choisi dans l'ensemble constitué par -H et un groupe protecteur d'hydroxyle ;
vii) $R^{10}$ est choisi dans l'ensemble constitué par une chaîne latérale de leucine et une chaîne latérale de lysine ; et
viii) la molécule n'est pas la tamandarine B.

**67.** Composition selon la revendication 66, dans laquelle $R^4$ est une chaîne latérale de valine, Y est -H, et X est -O-.

**68.** Analogue de tamandarine choisi dans le groupe constitué par

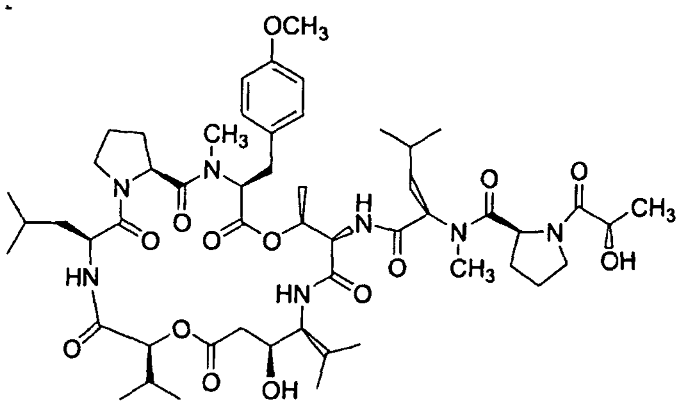

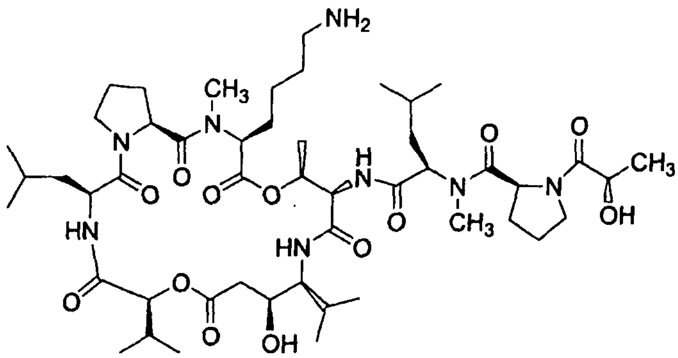

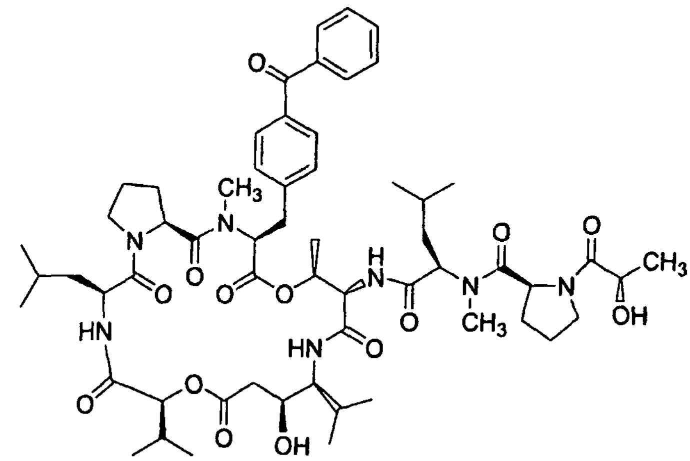

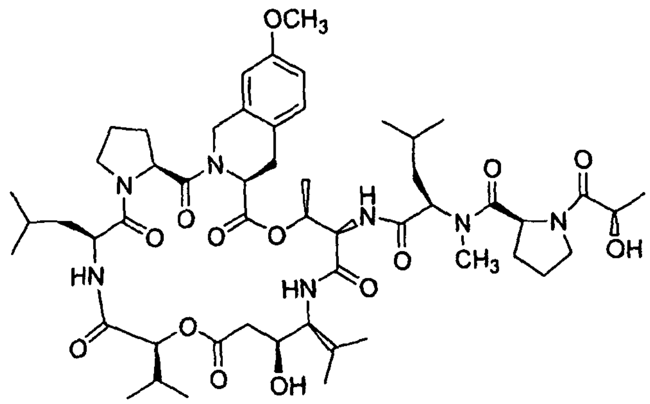

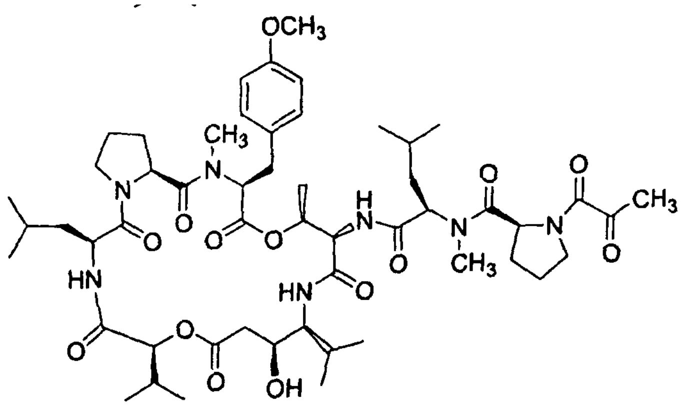

et

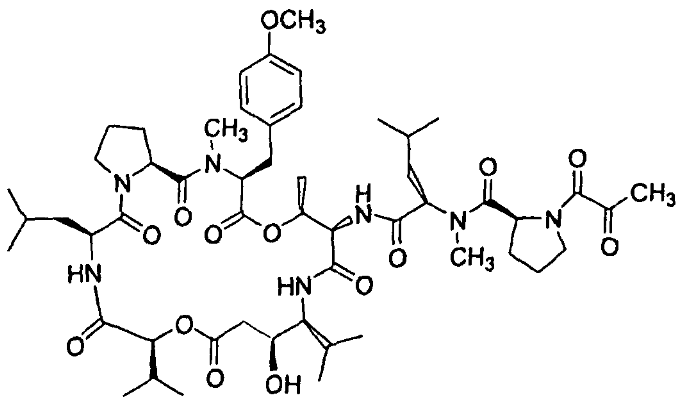

**69.** Composition selon la revendication 67, dans laquelle l'analogue de tamandarine est

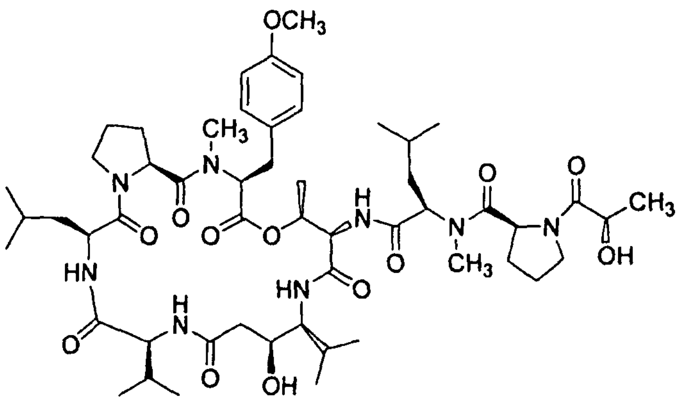

**70.** Composition comprenant un analogue de didemnine de structure

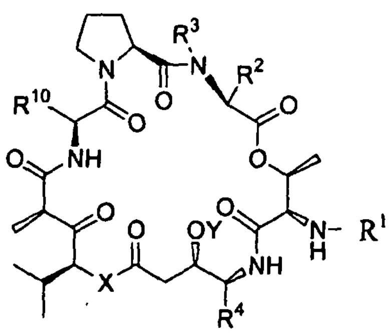

dans laquelle :

i) $R^1$ est choisi dans l'ensemble constitué par :

-H,
-(tert-butyloxycarbonyle),
-leucine,
-(N-méthyl)leucine,
-(N-méthyl)leucine-(un premier fluorophore),
-(N-méthyl)leucine-proline,
-(N-méthyl)leucine-proline-lactate,

-(N-méthyl)leucine-proline-pyruvate,
-(N-méthyl)leucine-proline-lactate-(un premier fluorophore),
-(N-méthyl)leucine-proline-lactate-glutamine-pyroglutamate,
-(N-méthyl)leucine-proline-lactate-glutamine-cyclopentanoate,
-(N-méthyl) leucine-proline-alanine-leucine-pyroglutamate,
-(N-méthyl) leucine-proline- (N-méthyl-alanine) -leucine-pyroglutamate,
-(N-méthyl)leucine-désoxo-proline,
-(N-méthyl)leucine-désoxo-proline-lactate,
-(N-méthyl)leucine-désoxo-proline-pyruvate,
-(N-méthyl)leucine-désoxo-proline-lactate-(un premier fluorophore),
-(N-méthyl)leucine-désoxo-proline-lactate-glutamine-pyroglutamate,
-(N-méthyl)leucine-désoxo-proline-lactate-glutamine-cyclopentanoate,
-(N-méthyl)leucine-désoxo-proline-alanine-leucine-pyroglutamate,
-(N-méthyl)leucine-désoxo-proline-(N-méthyl-alanine)-leucine-pyroglutamate,
-(N-méthyl)leucine-déshydro-proline,
-(N-méthyl)leucine-déshydro-proline-lactate,
-(N-méthyl)leucine-déshydro-proline-pyruvate,
-(N-méthyl)leucine-déshydro-proline-lactate-(un premier fluorophore),
-(N-méthyl)leucine-déshydro-proline-lactate-glutamine-pyroglutamate,
-(N-méthyl)leucine-déshydro-proline-lactate-glutamine-cyclopentanoate,
-(N-méthyl)leucine-déshydro-proline-alanine-leucine-pyroglutamate, et
-(N-méthyl)leucine-déshydro-proline-(N-méthyl-alanine)-leucine-pyroglutamate ;

ii) soit

(a) $R^3$ est choisi dans l'ensemble constitué par $-CH_3$ et -H ; et $R^2$ est choisi dans l'ensemble constitué par une chaîne latérale d'isoleucine, une chaîne latérale de valine, une chaîne latérale d'alanine, une chaîne latérale de norleucine, une chaîne latérale de norvaline, une chaîne latérale de leucine, une chaîne latérale d'histidine, une chaîne latérale de tryptophane, une chaîne latérale d'arginine, une chaîne latérale de lysine, un deuxième fluorophore, et un substituant de structure :

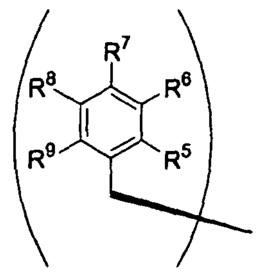

(b) $R^2$ et $R^3$ forment ensemble un substituant de structure :

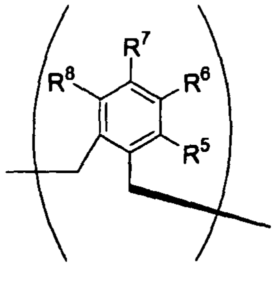

iii) chacun de $R^5$, $R^6$, $R^7$, $R^8$ et $R^9$, quand il est présent, est indépendamment choisi dans l'ensemble constitué par -H, -OH, $-OCH_3$, $-CO(C_6H_5)$, -Br, -I, -F, -Cl, $-CH_3$, et $-C_2H_5$ ;
iv) $R^4$ est une chaîne latérale de valine ;
v) X est choisi dans l'ensemble constitué par -O- et -(NH)- ;

vi) Y est choisi dans l'ensemble constitué par -H et un groupe protecteur d'hydroxyle ; et
vii) $R^{10}$ est choisi dans l'ensemble constitué par une chaîne latérale de leucine et une chaîne latérale de lysine.

**71.** Composition selon la revendication 70, dans laquelle $R^1$ est choisi dans l'ensemble constitué par :

-H,
-(tert-butyloxycarbonyle),
-leucine,
-(N-méthyl)leucine,
-(N-méthyl)leucine-(un premier fluorophore),
-(N-méthyl)leucine-(S)-proline,
-(N-méthyl)leucine-(S)-proline-(S)-lactate,
-(N-méthyl)leucine-(S)-proline-pyruvate,
-(N-méthyl)leucine-(S)-proline-(S)-lactate-(un premier fluorophore),
-(N-méthyl) leucine-(S)-proline-(S)-lactate-glutamine-pyroglutamate,
-(N-méthyl) leucine-(S)-proline-(S)-lactate-glutamine-cyclopentanoate,
-(N-méthyl) leucine-(S)-proline-alanine-leucine-pyroglutamate,
-(N-méthyl) leucine-(S)-proline-(N-méthyl-alanine)-leucine-pyroglutamate,
-(N-méthyl) leucine-désoxo-(S)-proline,
-(N-méthyl) leucine-désoxo-(S)-proline-(S)-lactate,
-(N-méthyl)leucine-désoxo-(S)-proline-pyruvate,
-(N-méthyl)leucine-désoxo-(S)-proline-(S)-lactate-(un premier fluorophore),
-(N-méthyl)leucine-désoxo-(S)-proline-(S)-lactate-glutamine-pyroglutamate,
-(N-méthyl)leucine-désoxo-(S)-proline-(S)-lactate-glutamine-cyclopentanoate,
-(N-méthyl)leucine-désoxo-(S)-proline-alanine-leucine-pyroglutamate,
-(N-méthyl)leucine-désoxo-(S)-proline-(N-méthyl-alanine)-leucine-pyroglutamate,
-(N-méthyl)leucine-déshydro-(S)-proline,
-(N-méthyl)leucine-déshydro-(S)-proline-(S)-lactate,
-(N-méthyl)leucine-déshydro-(S)-proline-pyruvate,
-(N-méthyl)leucine-déshydro-(S)-proline-(S)-lactate-(un premier fluorophore),
-(N-méthyl)leucine-déshydro-(S)-proline-(S)-lactate-glutamine-pyroglutamate,
-(N-méthyl)leucine-déshydro-(S)-proline-(S)-lactate-glutamine-cyclopentanoate,
-(N-méthyl)leucine-déshydro-(S)-proline-alanine-leucine-pyroglutamate, et
-(N-méthyl)leucine-déshydro-(S)-proline-(N-méthyl-alanine)-leucine-pyroglutamate,
-(N-méthyl) leucine-(S)-proline-(S)-lactate-(S)-glutamine-(S)-pyroglutamate,
-(N-méthyl) leucine-(S)-proline-(S)-lactate-(S)-glutamine-(S)-cyclopentanoate,
-(N-méthyl) leucine-(S)-proline-(S)-alanine-(S)-leucine-(S)-pyroglutamate,
-(N-méthyl) leucine-(S)-proline-(N-méthyl-S-alanine)-(S)-leucine-(S)-pyroglutamate,
-(N-méthyl)leucine-désoxo-(S)-proline-(S)-lactate-(S)-glutamine-(S)-pyroglutamate,
-(N-méthyl)leucine-désoxo-(S)-proline-(S)-lactate-(S)-glutamine-(S)-cyclopentanoate,
-(N-méthyl)leucine-désoxo-(S)-proline-(S)-alanine-(S)-leucine-(S)-pyroglutamate,
-(N-méthyl)leucine-désoxo-(S)-proline-(N-méthyl-S-alanine)-(S)-leucine-(S)-pyroglutamate,
-(N-méthyl)leucine-désoxo-(S)-proline-pyruvate,
-(N-méthyl)leucine-désoxo-(S)-proline-(S)-lactate-(un premier fluorophore),
-(N-méthyl)leucine-désoxo-(S)-proline-(S)-lactate-(S)-glutamine-(S)-pyroglutamate,
-(N-méthyl)leucine-désoxo-(S)-proline-(S)-lactate-(S)-glutamine-(S)-cyclopentanoate,
-(N-méthyl)leucine-désoxo-(S)-proline-(S)-alanine-(S)-leucine-(S)-pyroglutamate, et
-(N-méthyl)leucine-désoxo-(S)-proline-(N-méthyl-S-alanine)-(S)-leucine-(S)-pyroglutamate.

**72.** Composition selon la revendication 70, dans laquelle $R^2$ est

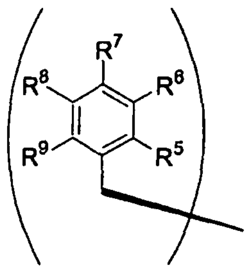

$R^3$ est méthyle, chacun de $R^5$, $R^6$, $R^8$ et $R^9$ est un radical hydrure, $R^7$ est méthoxy, $R^{10}$ est une chaîne latérale de leucine, X est -O-, et Y est un radical hydrure.

**73.** Composition selon la revendication 70, dans laquelle R est -(N-méthyl)leucine-désoxo-(S)-proline-lactate.

**74.** Composition selon la revendication 70, dans laquelle Y est -H, et dans laquelle $R^2$ a la structure

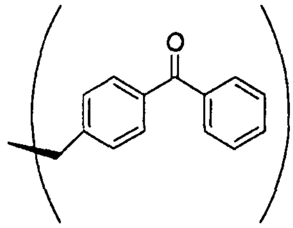

**75.** Composition selon la revendication 70, dans laquelle $R^2$ est une chaîne latérale de lysine et Y est -H.

**76.** Composition selon la revendication 70, dans laquelle X est -(NH)-.

**77.** Composition selon la revendication 70, comprenant en outre un véhicule acceptable du point de vue pharmaceutique.

**78.** Support auquel est attaché de façon covalente l'analogue de didemnine de la revendication 70.

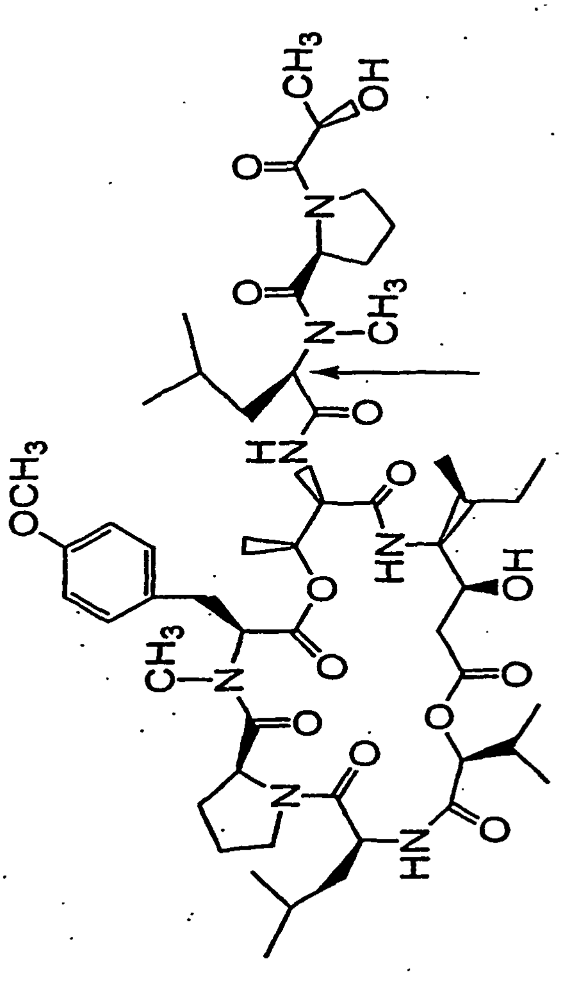

Fig. 1A

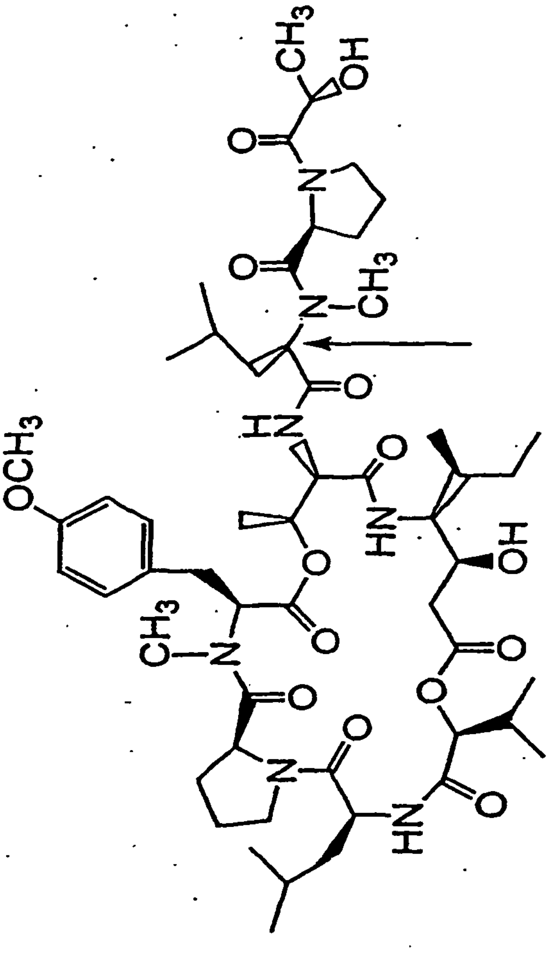

Fig. 1B

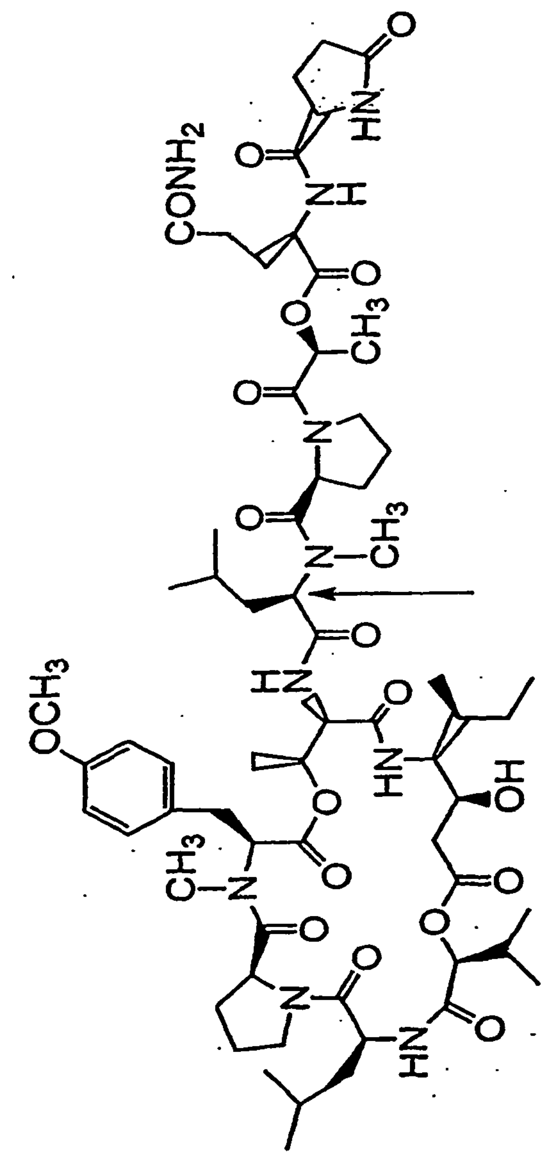

Fig. 2A

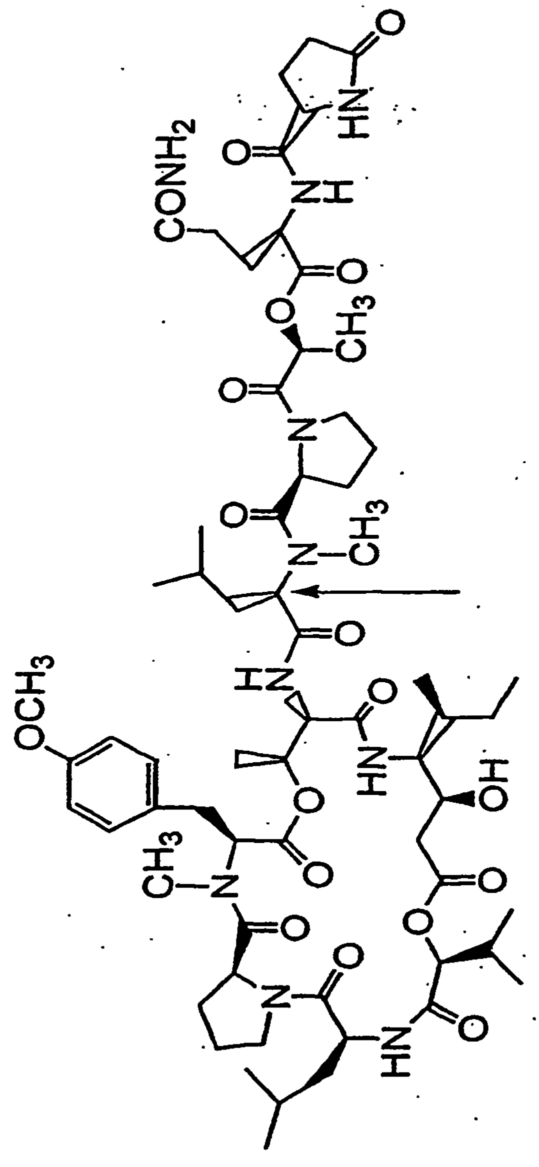

Fig. 2B

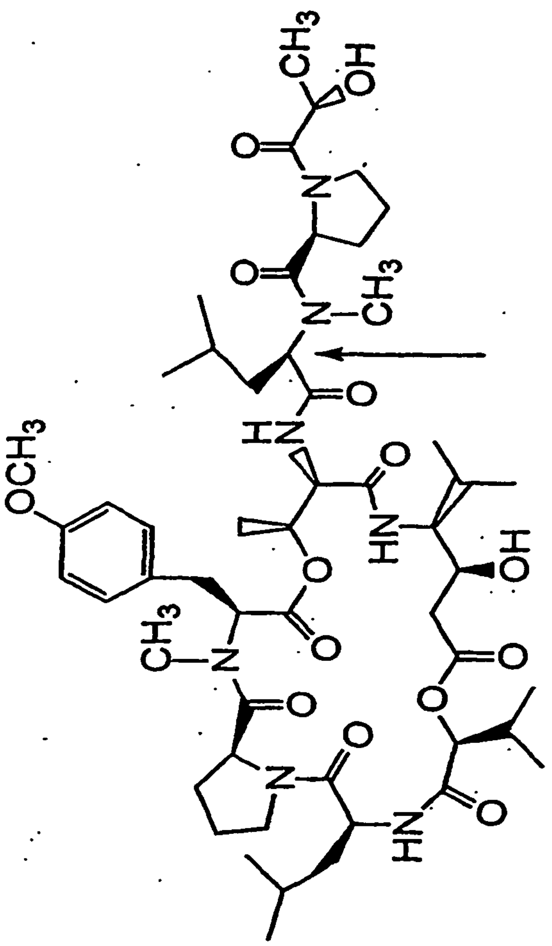

Fig. 3A

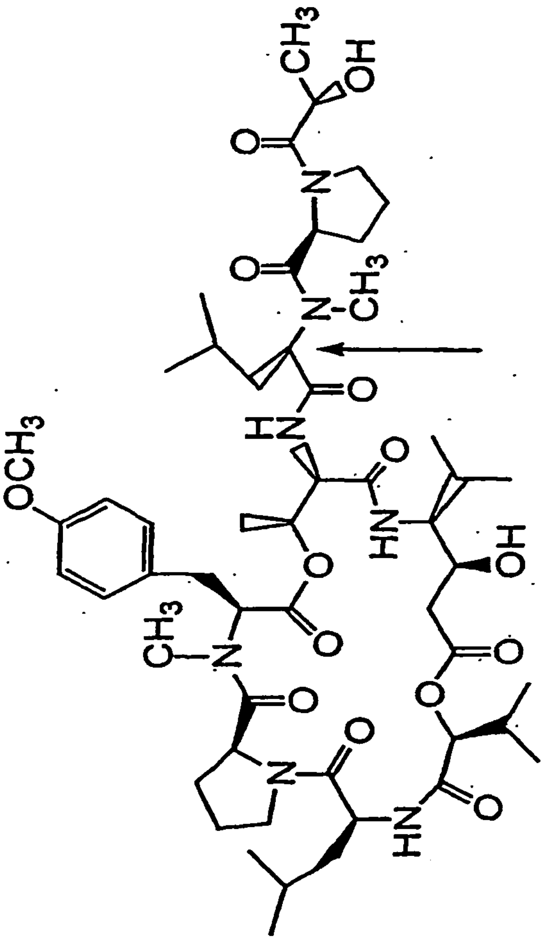

Fig. 3B

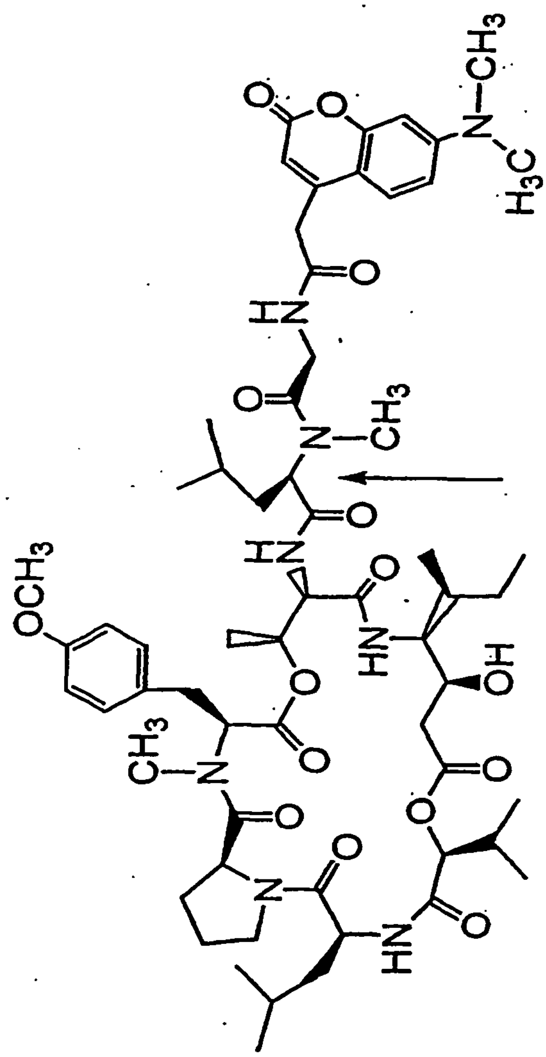

Fig. 4A

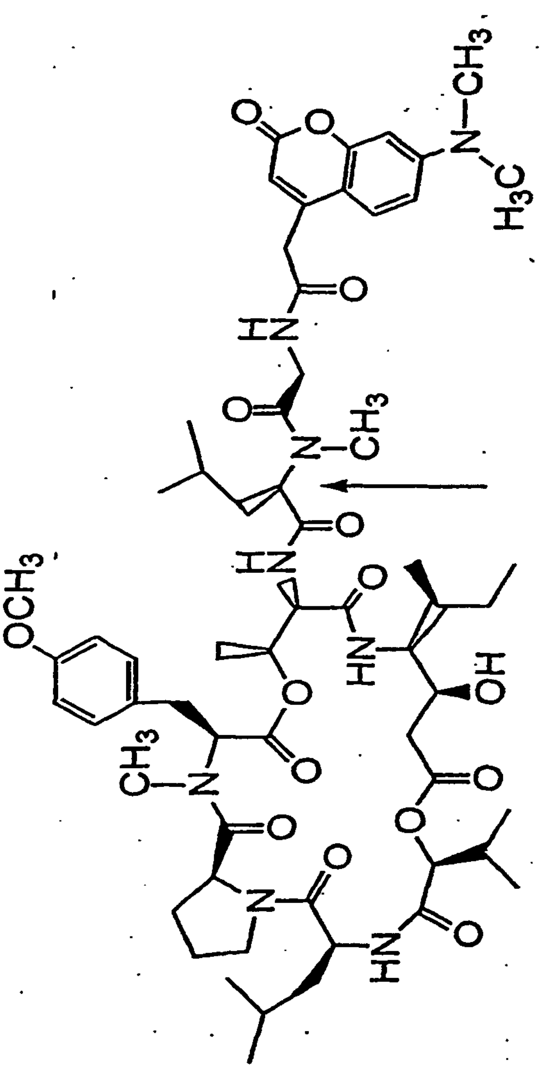

Fig. 4B

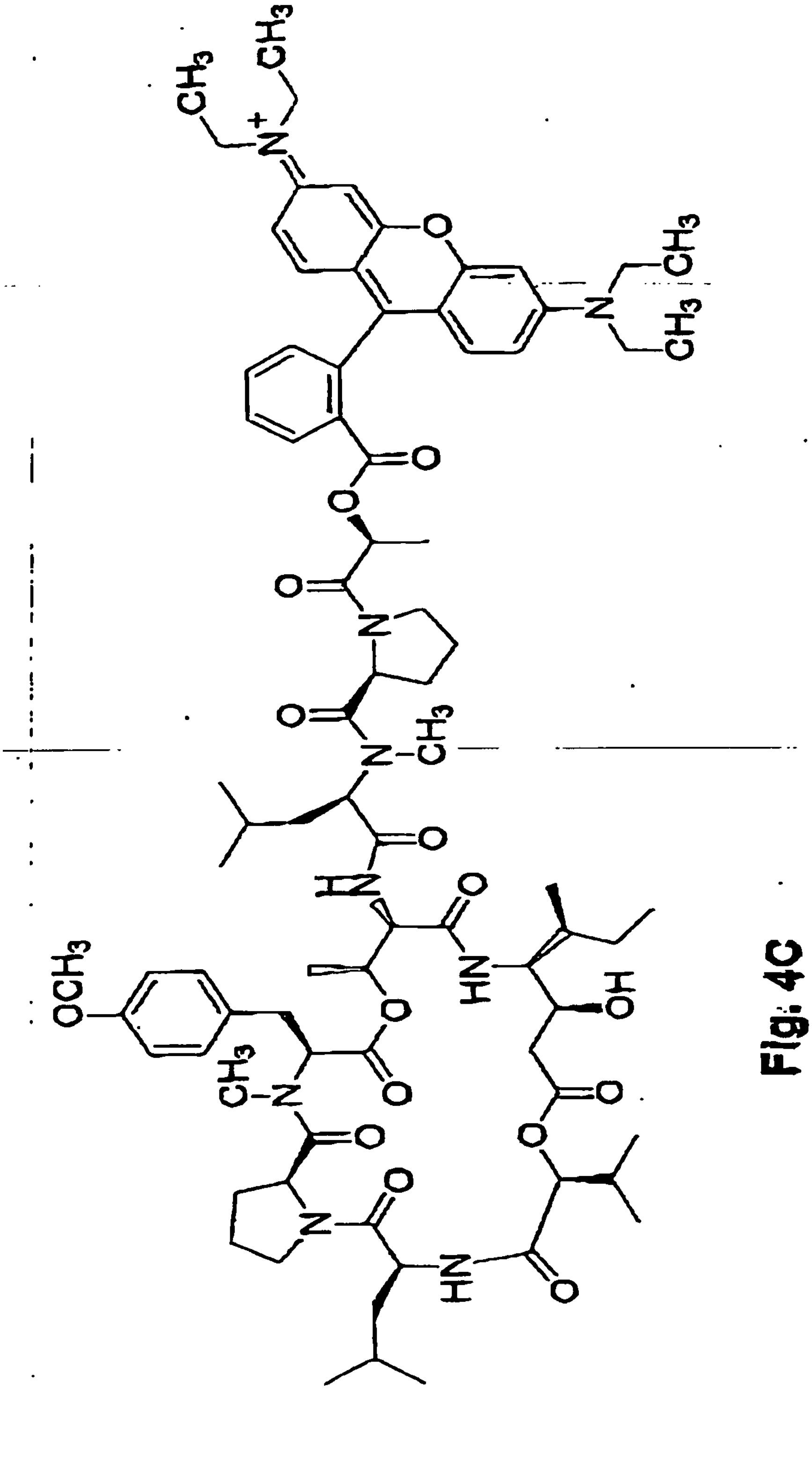

Fig. 4C

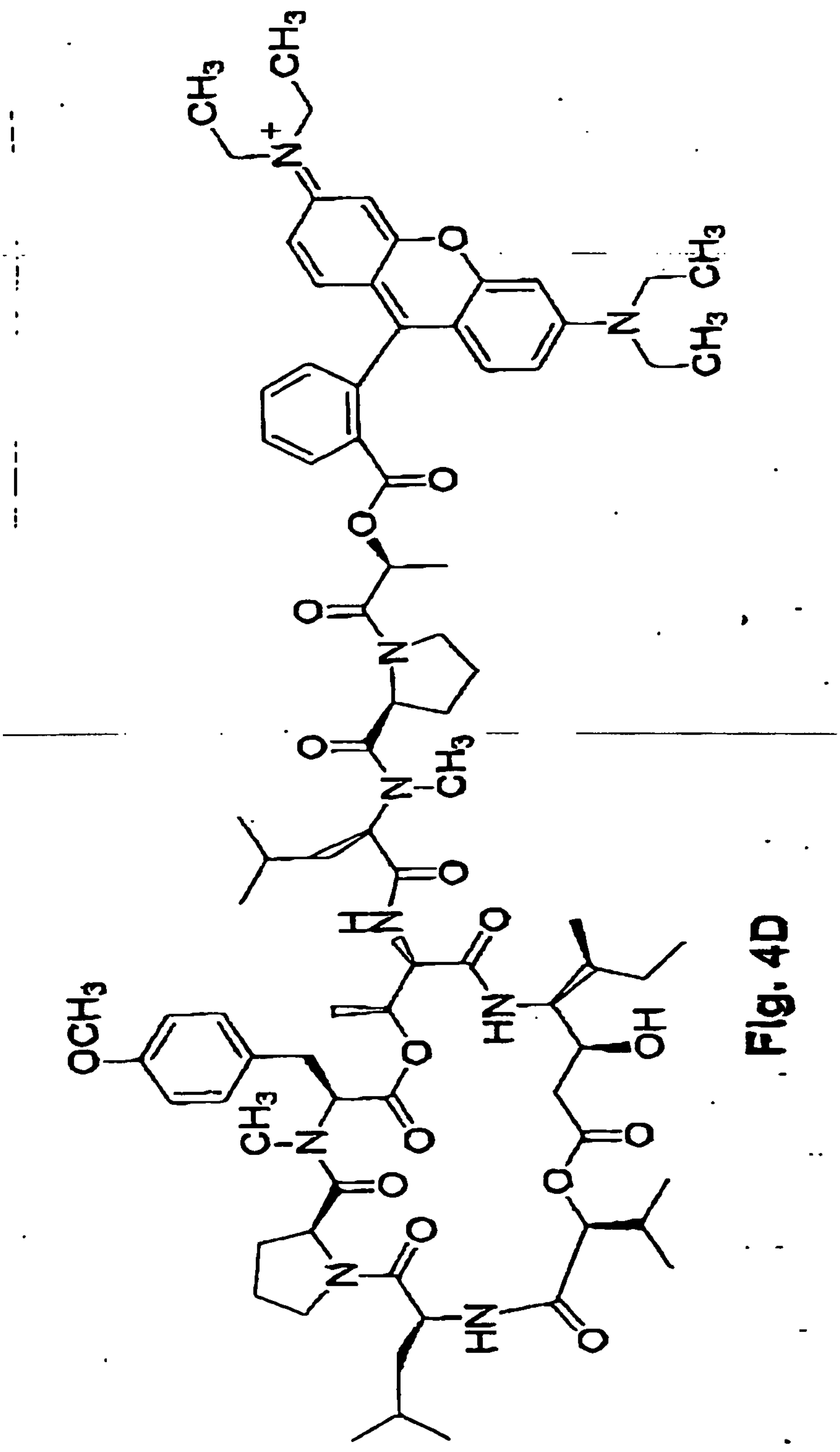

Fig. 4D

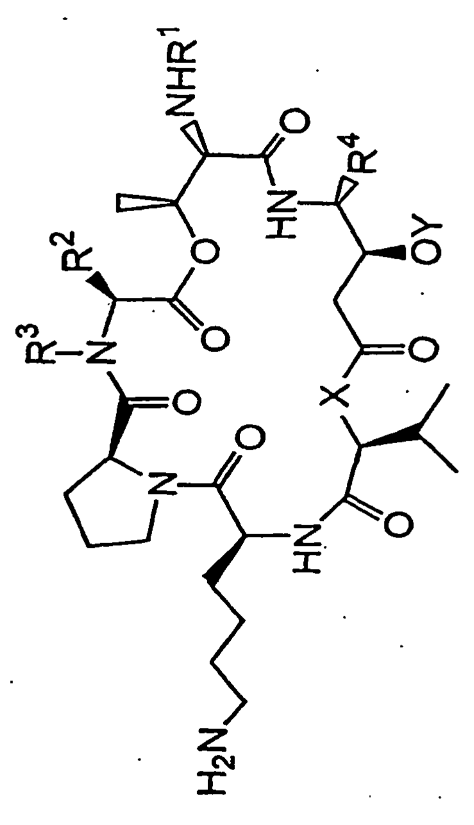

Fig. 5A

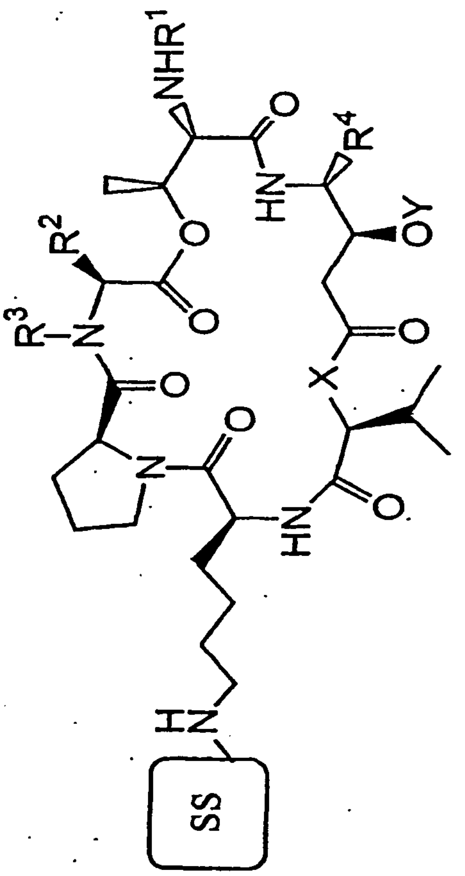

Fig. 5B

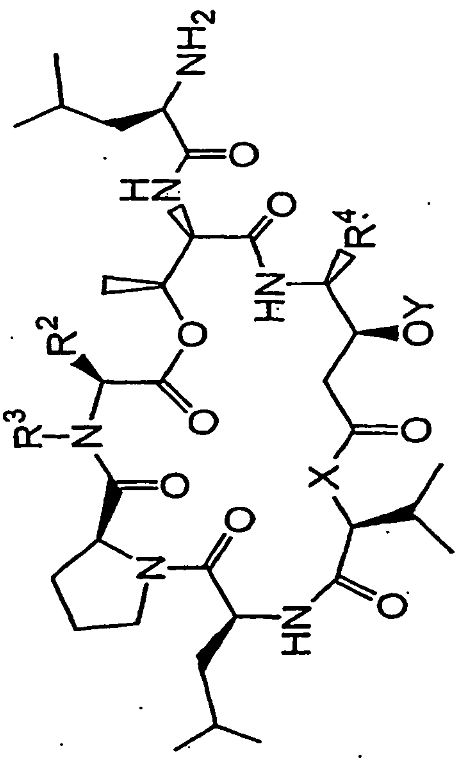

Fig. 6A

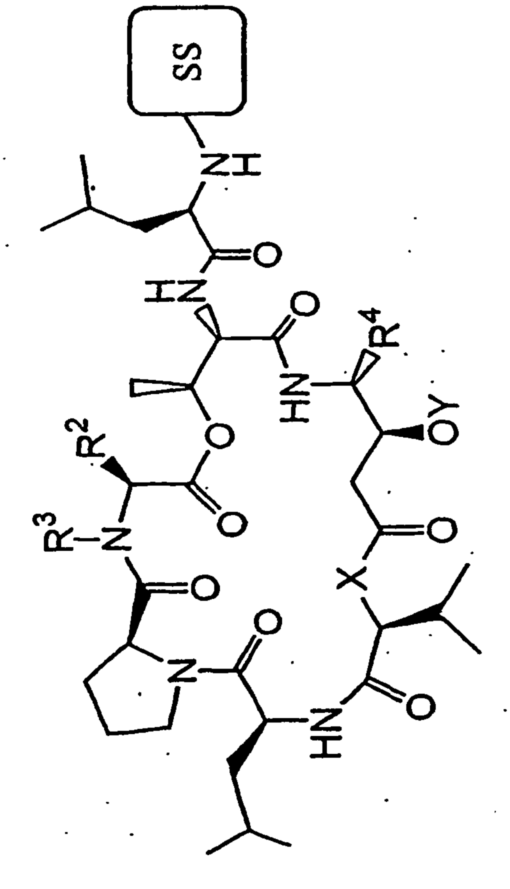

Fig. 6B

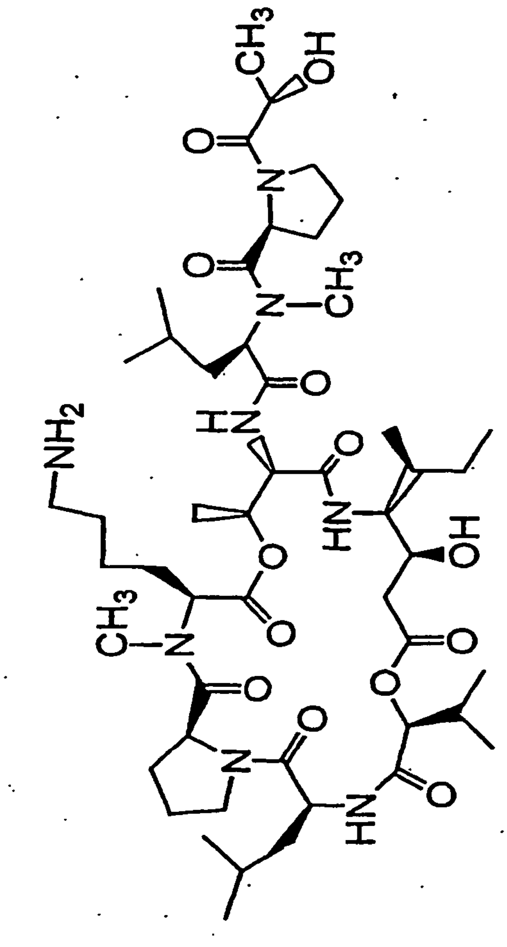

Fig. 7

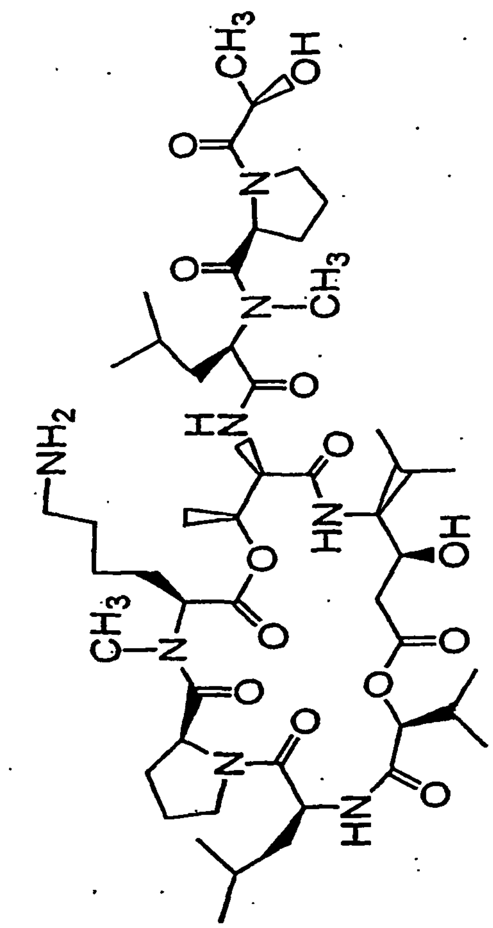

Fig. 8

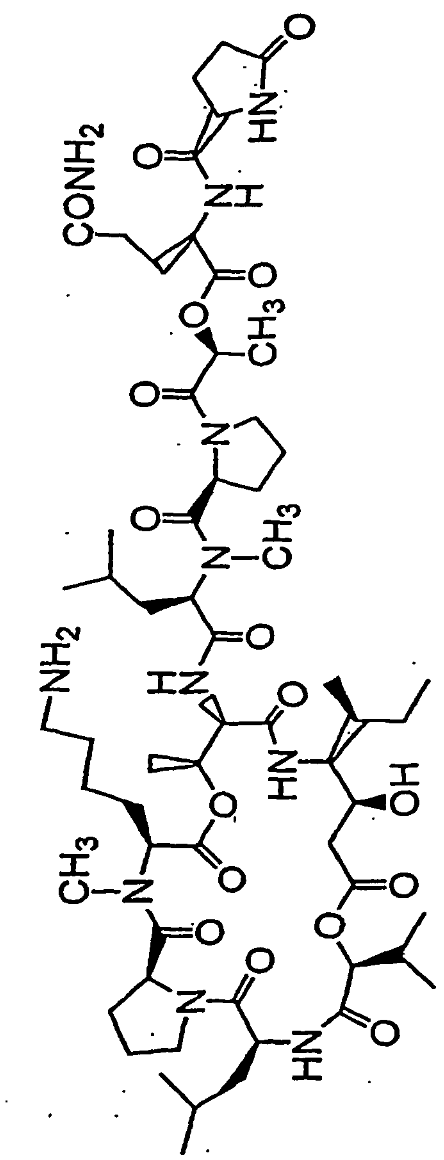

Fig. 9

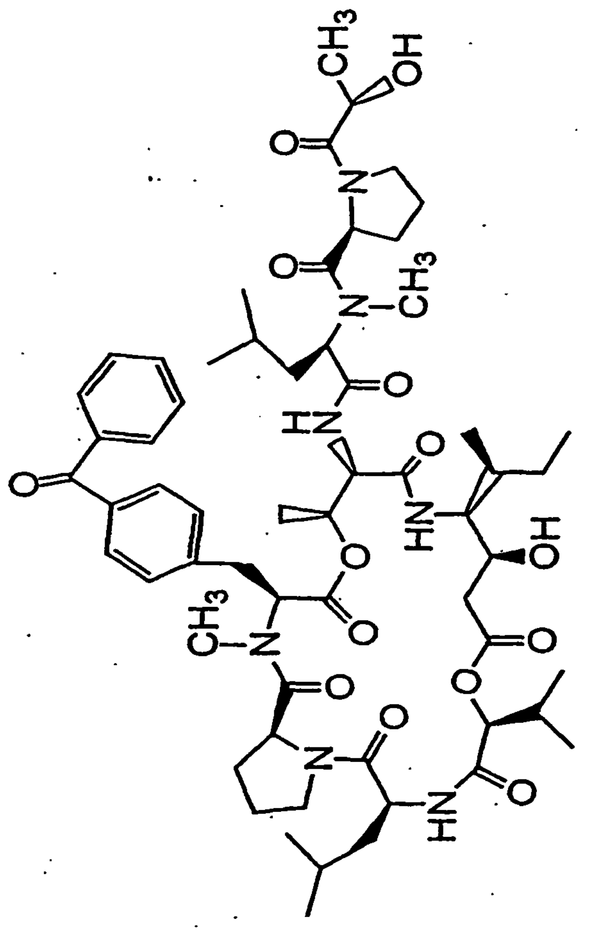

Fig. 10

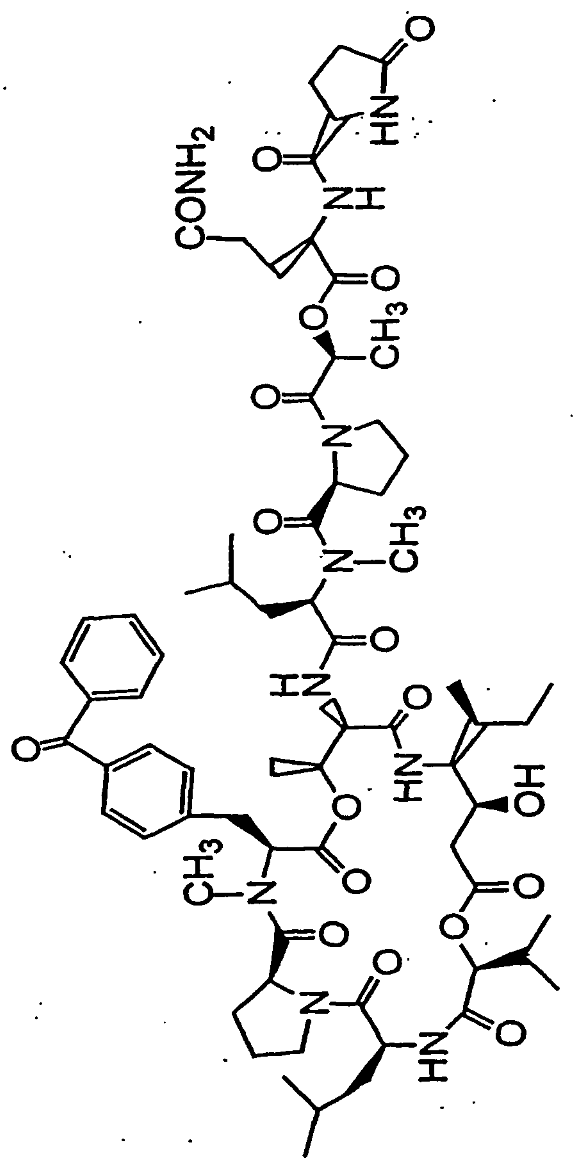

Fig. 11

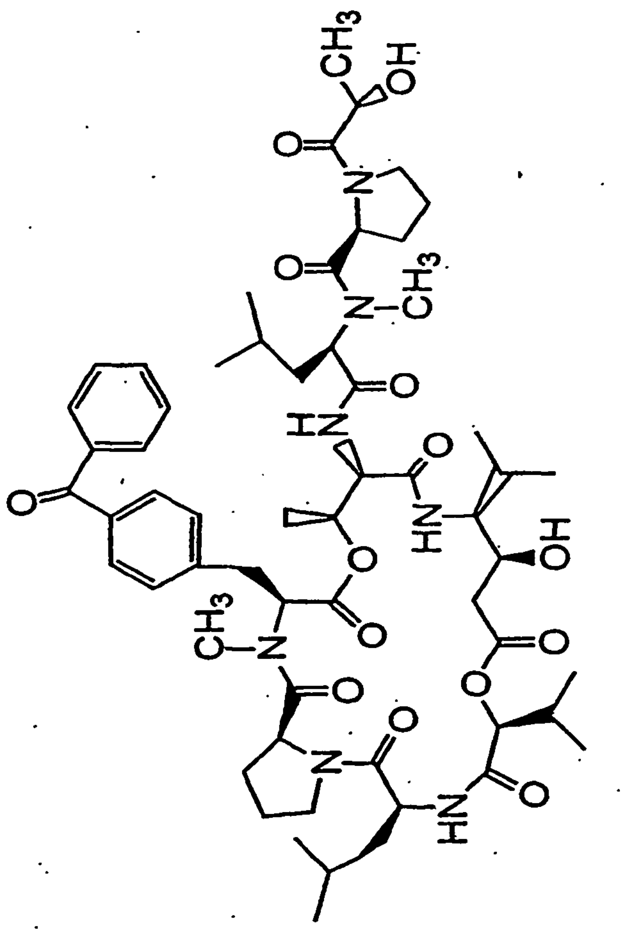

Fig. 12

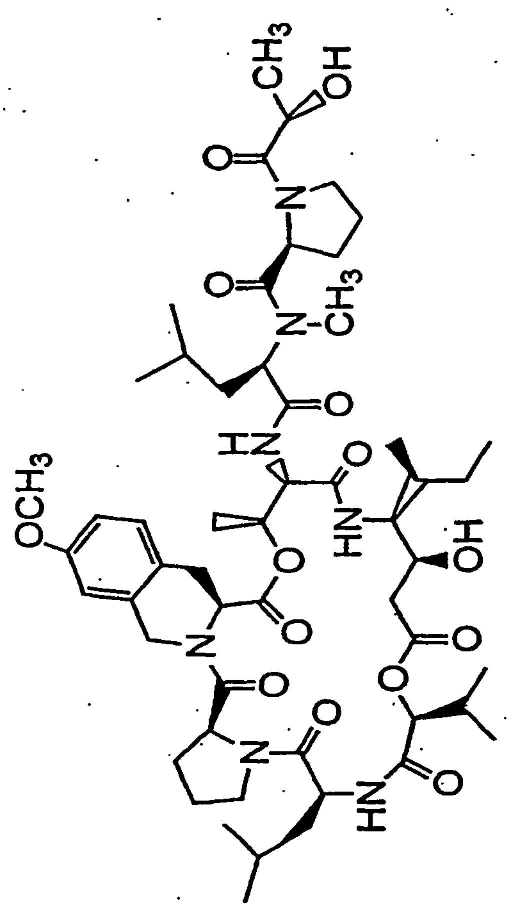

Fig. 13

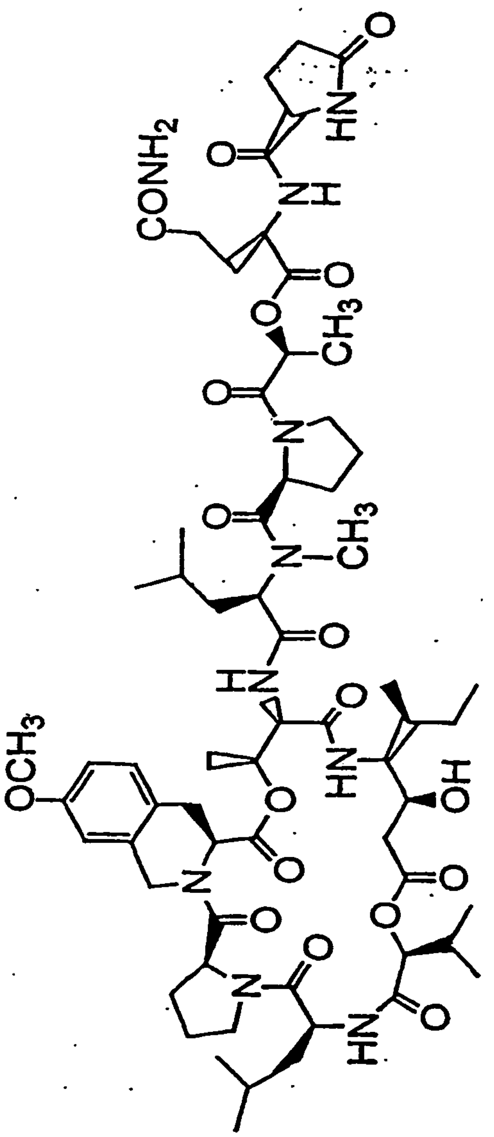

Fig. 14

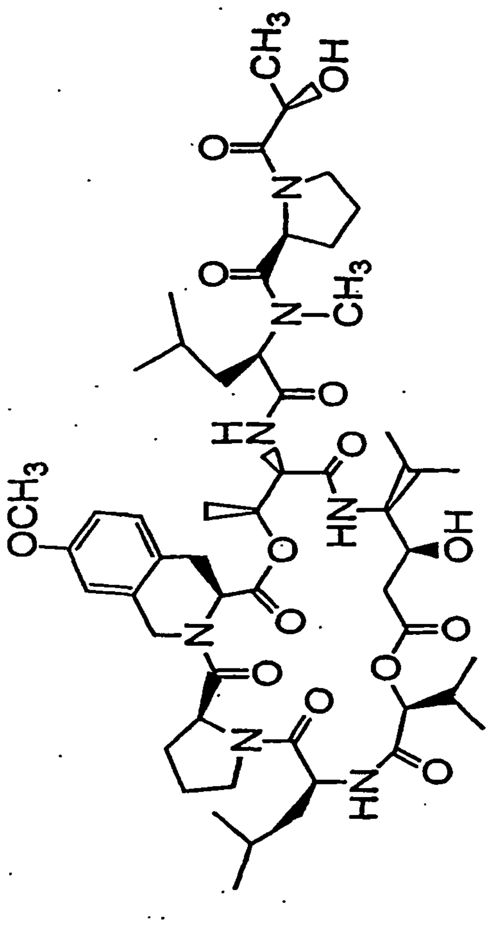

Fig. 15

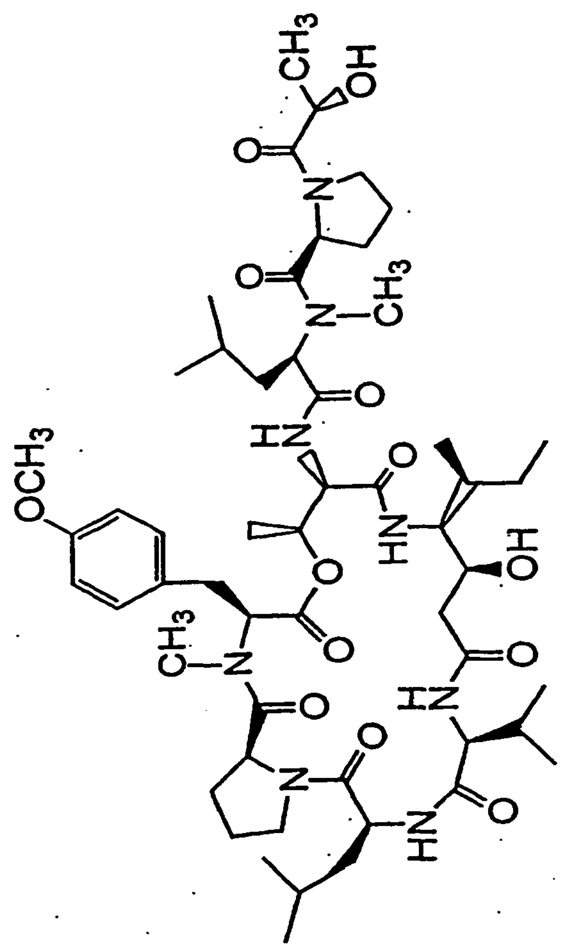

Fig. 16

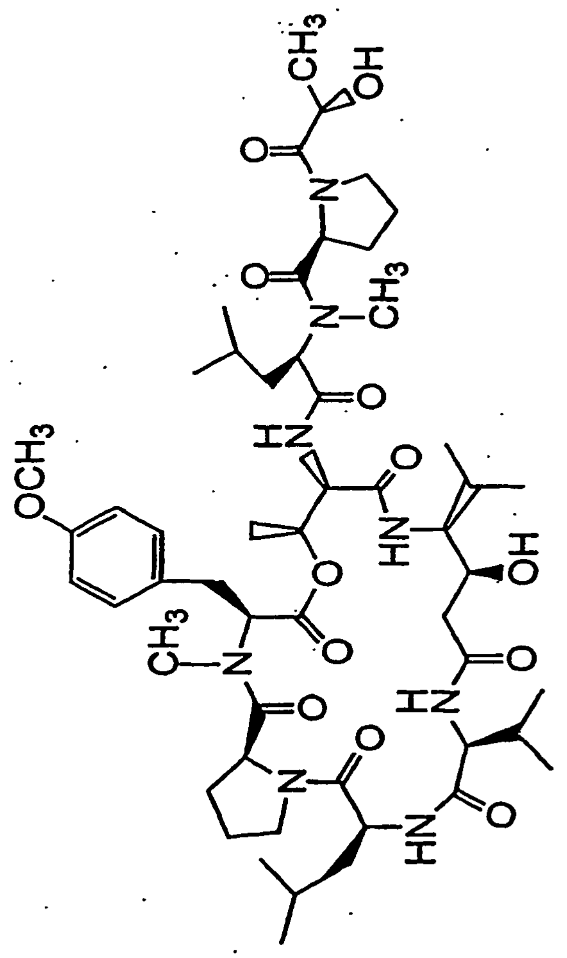

Fig. 17

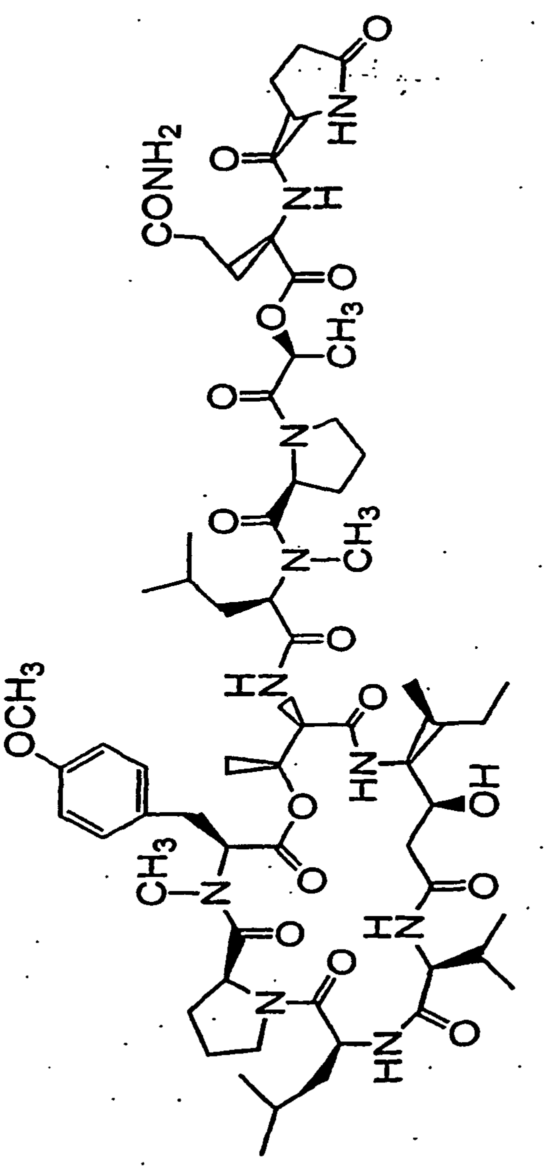

Fig. 18

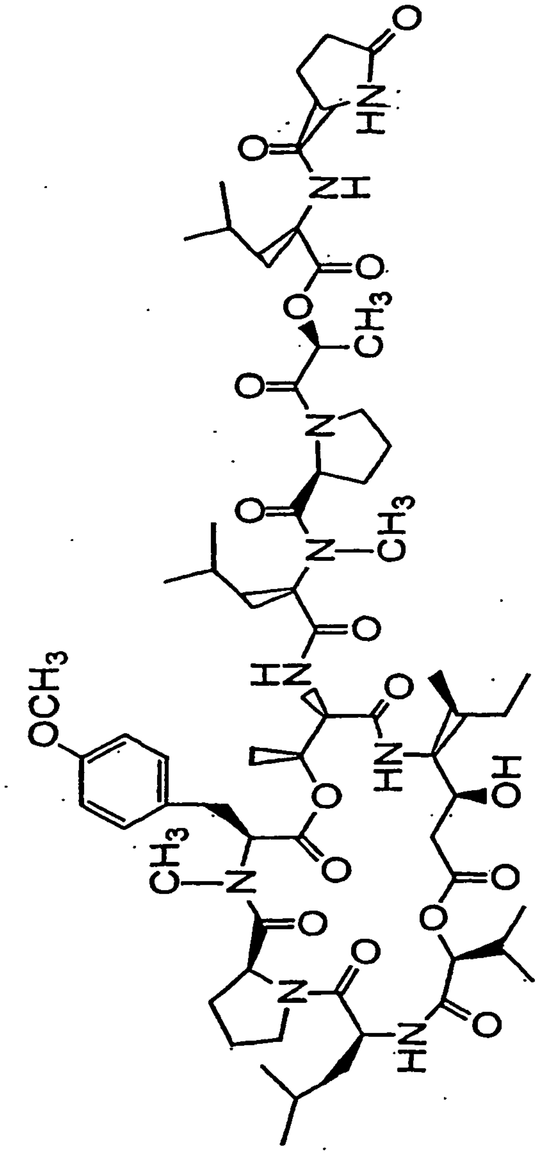

Fig. 19

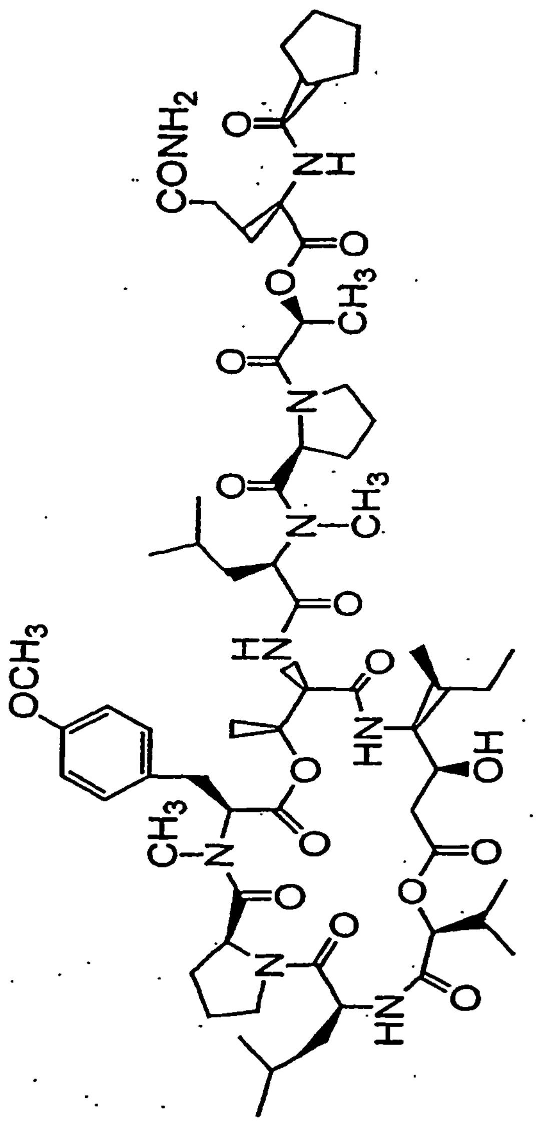

Fig. 20

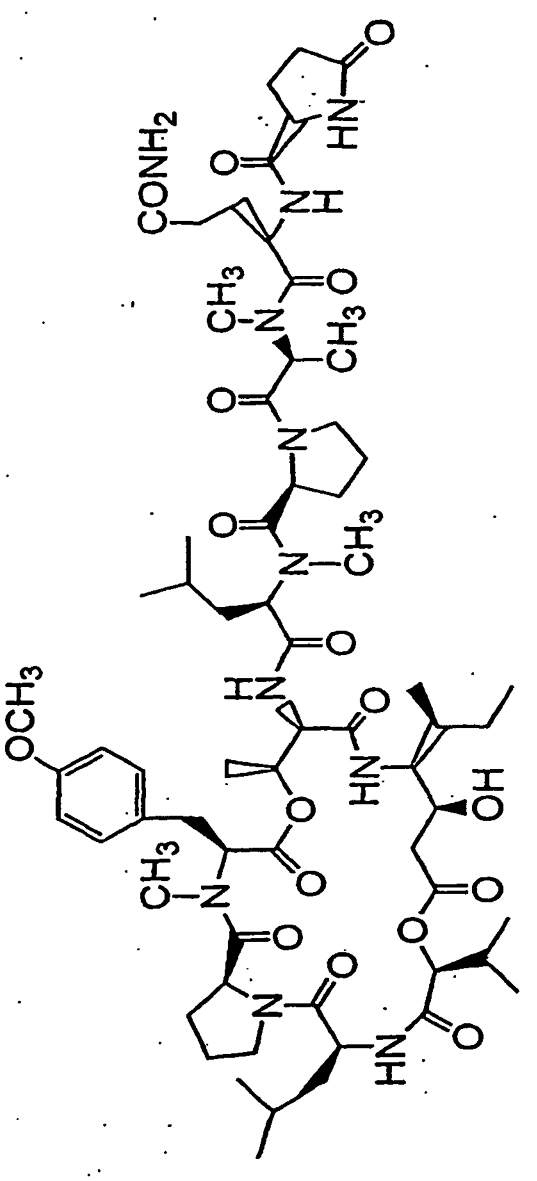

Fig. 21

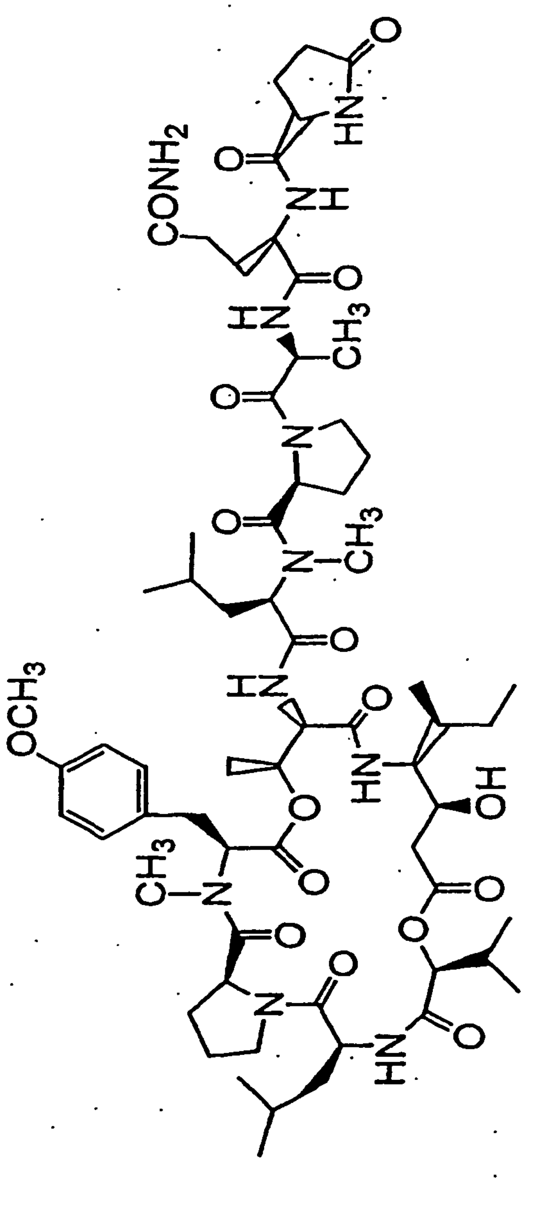

Fig. 22

Fig. 23

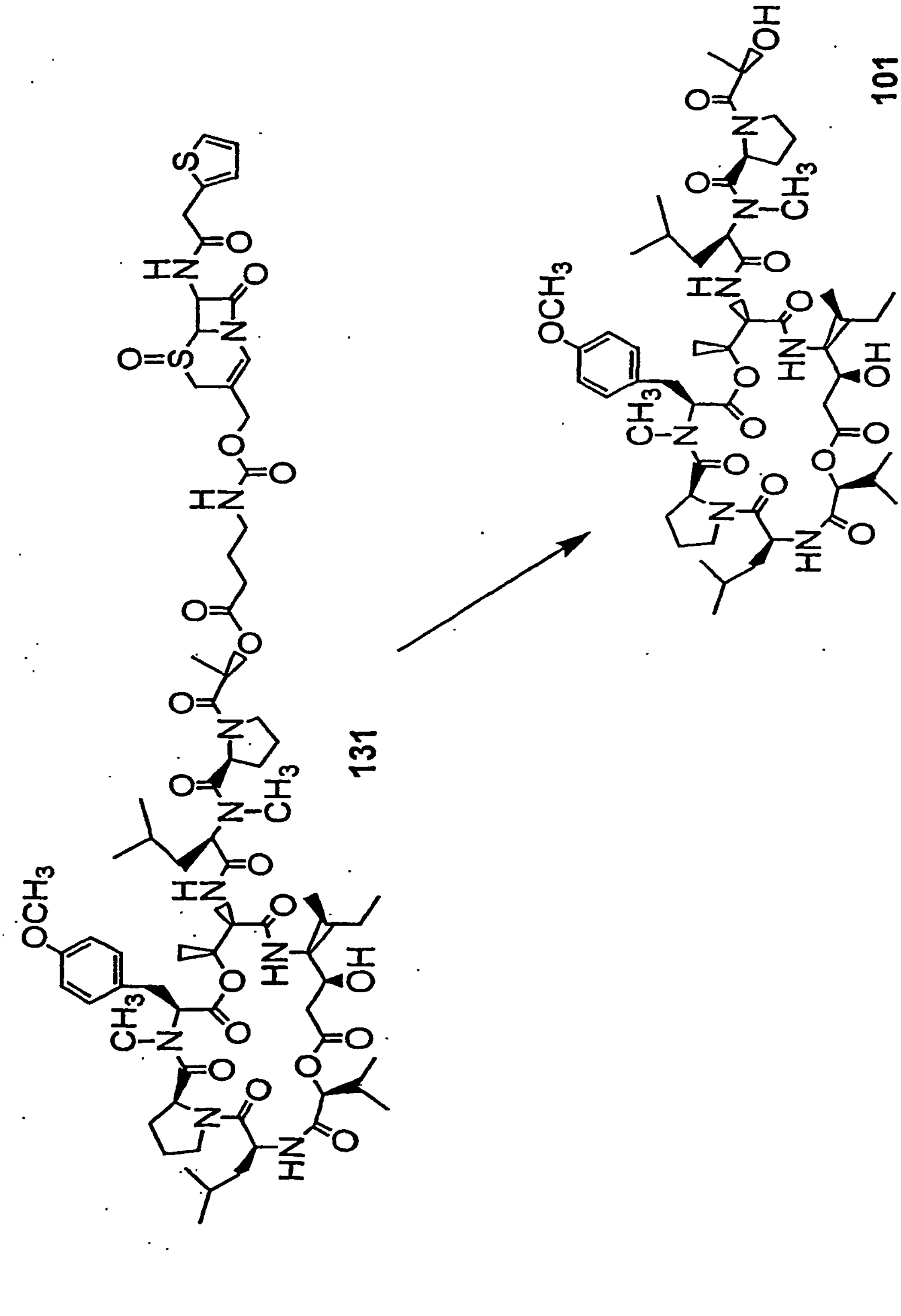

131

101

Fig. 24

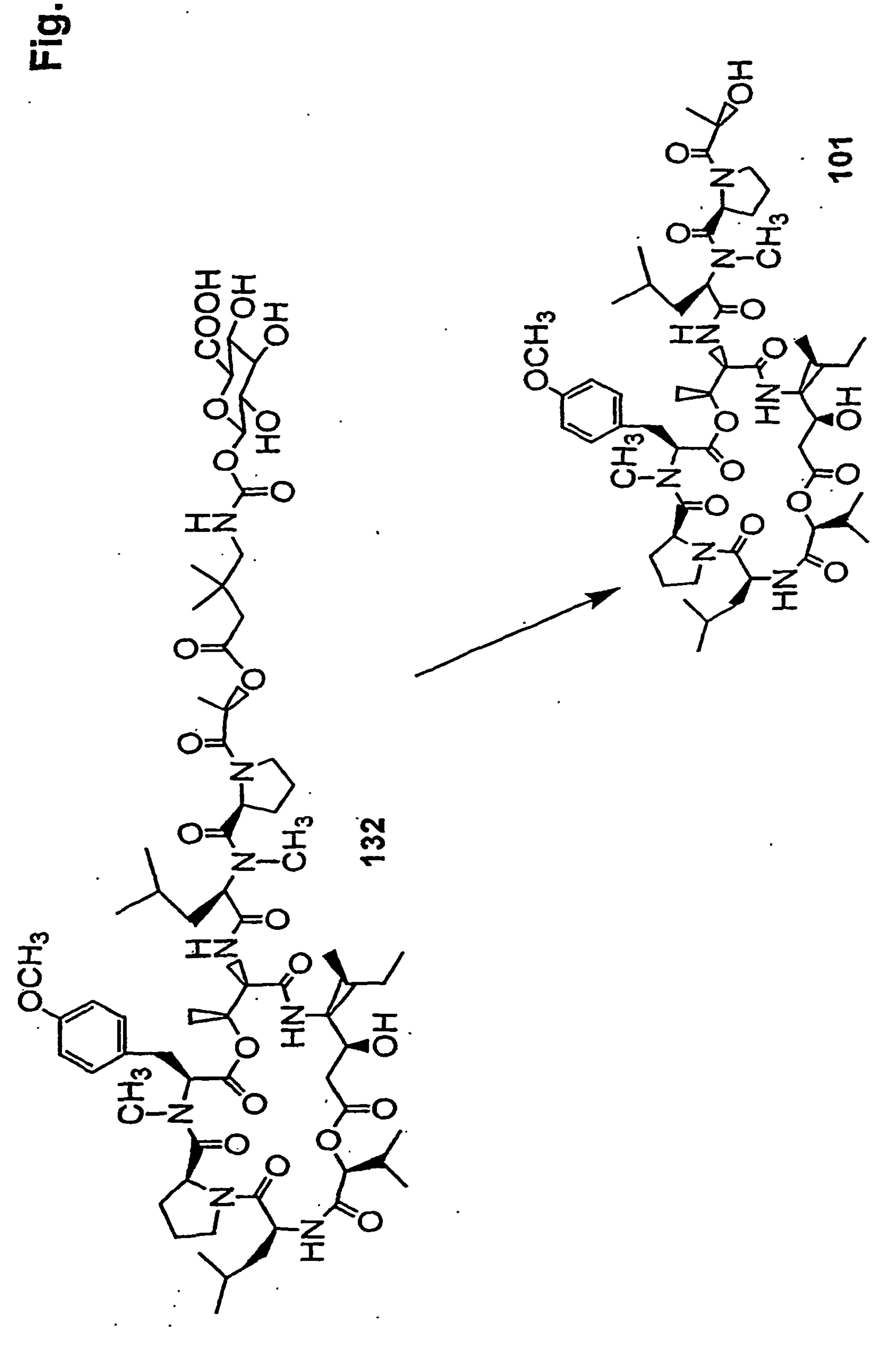

132

101

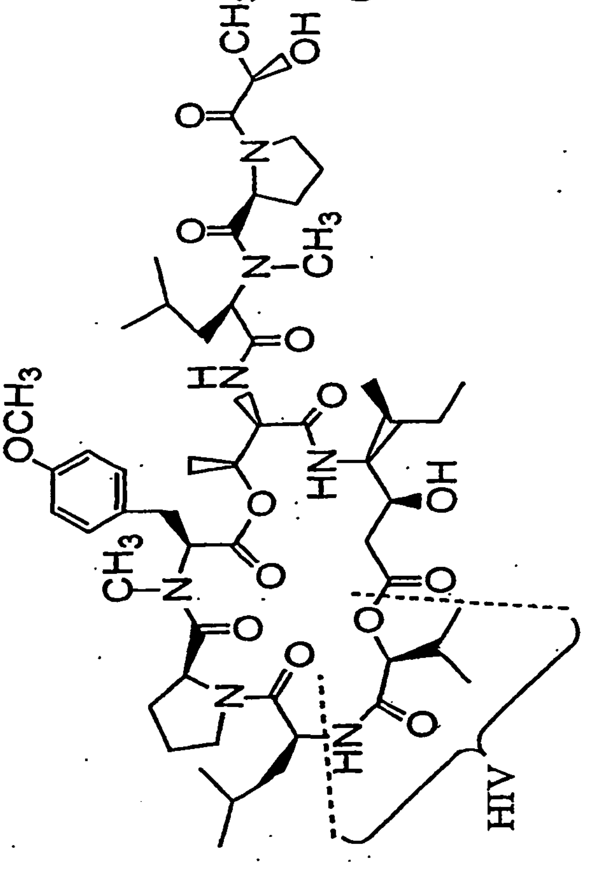


PRIOR ART
**Fig. 25A**

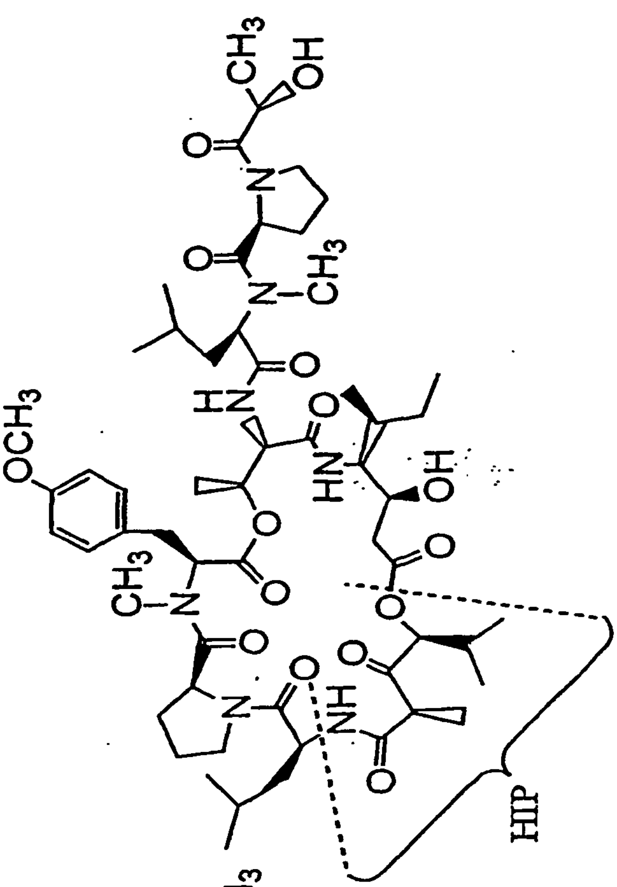


PRIOR ART
**Fig. 25B**

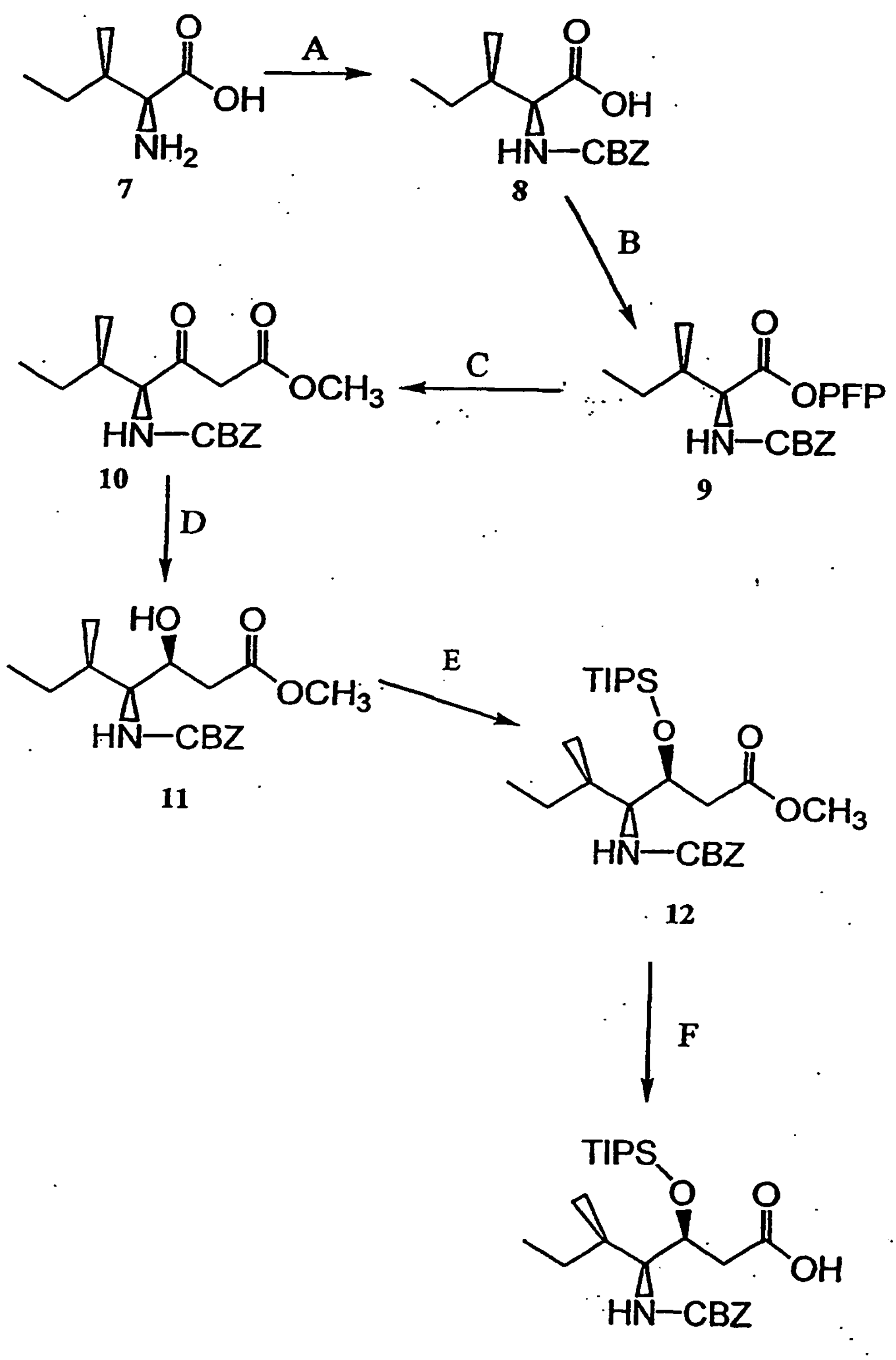


7, 8, 9, 10, 11, 12, 13

Fig. 26A

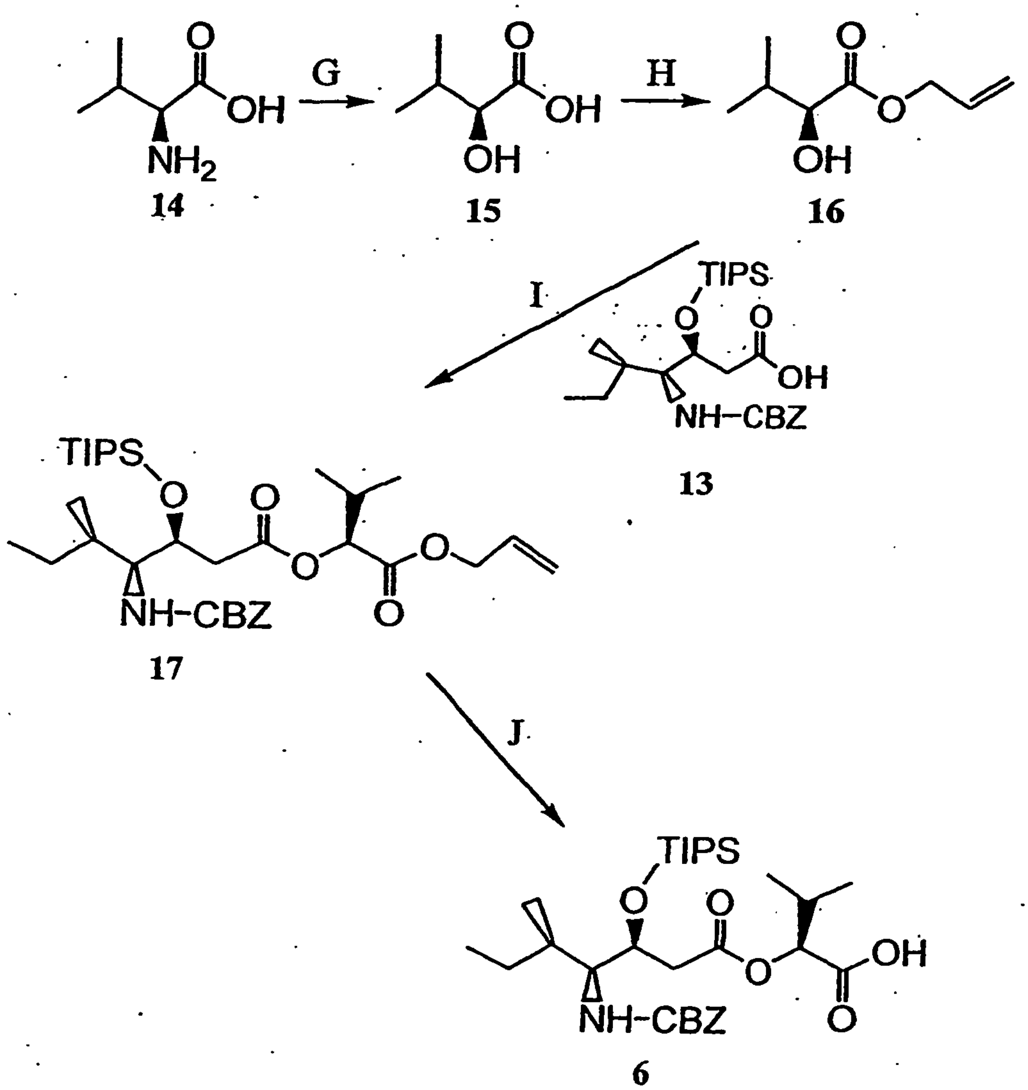


14 15 16 13 17 6

Fig. 26B

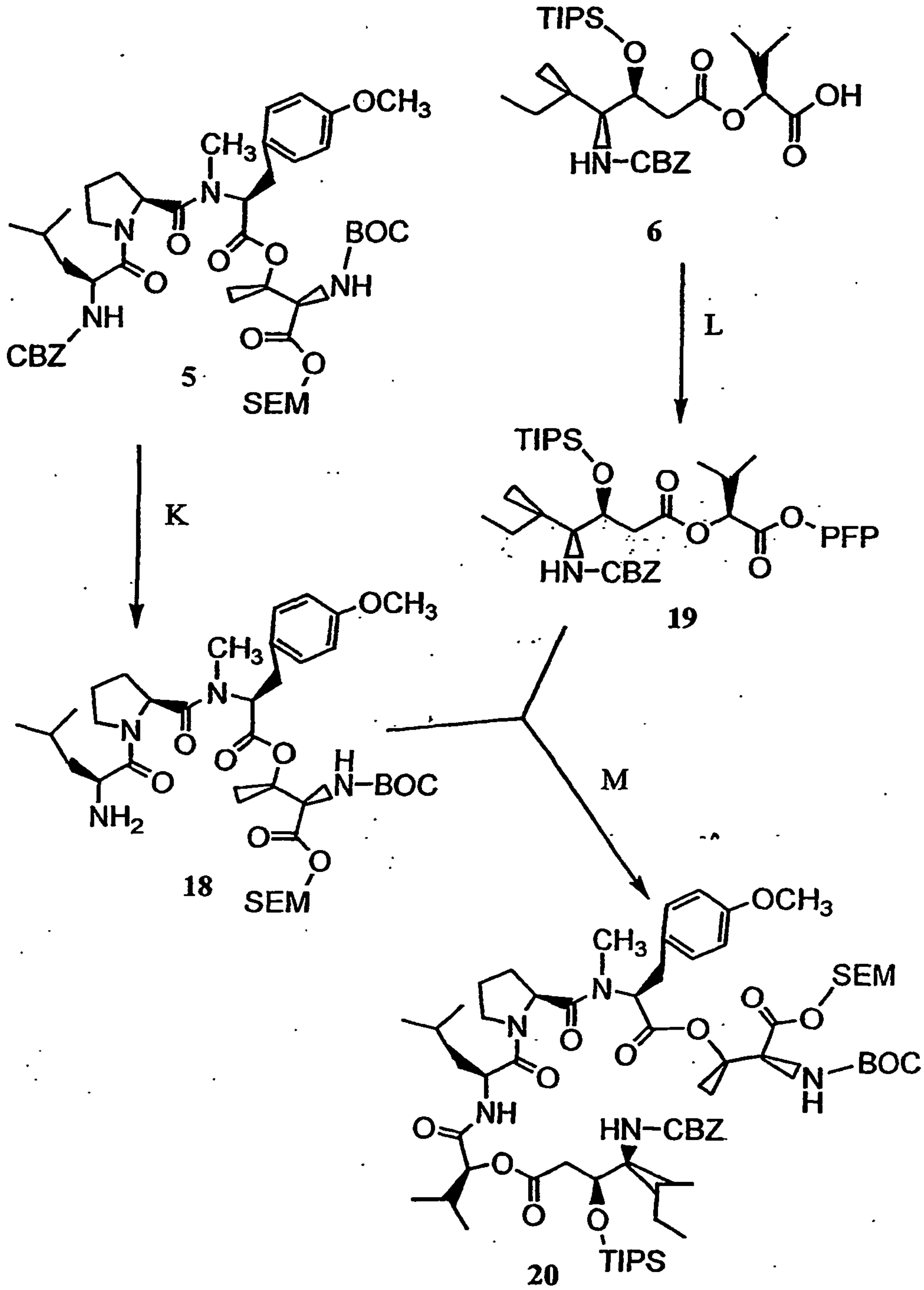


5

6

19

18

20

**Fig. 26C**

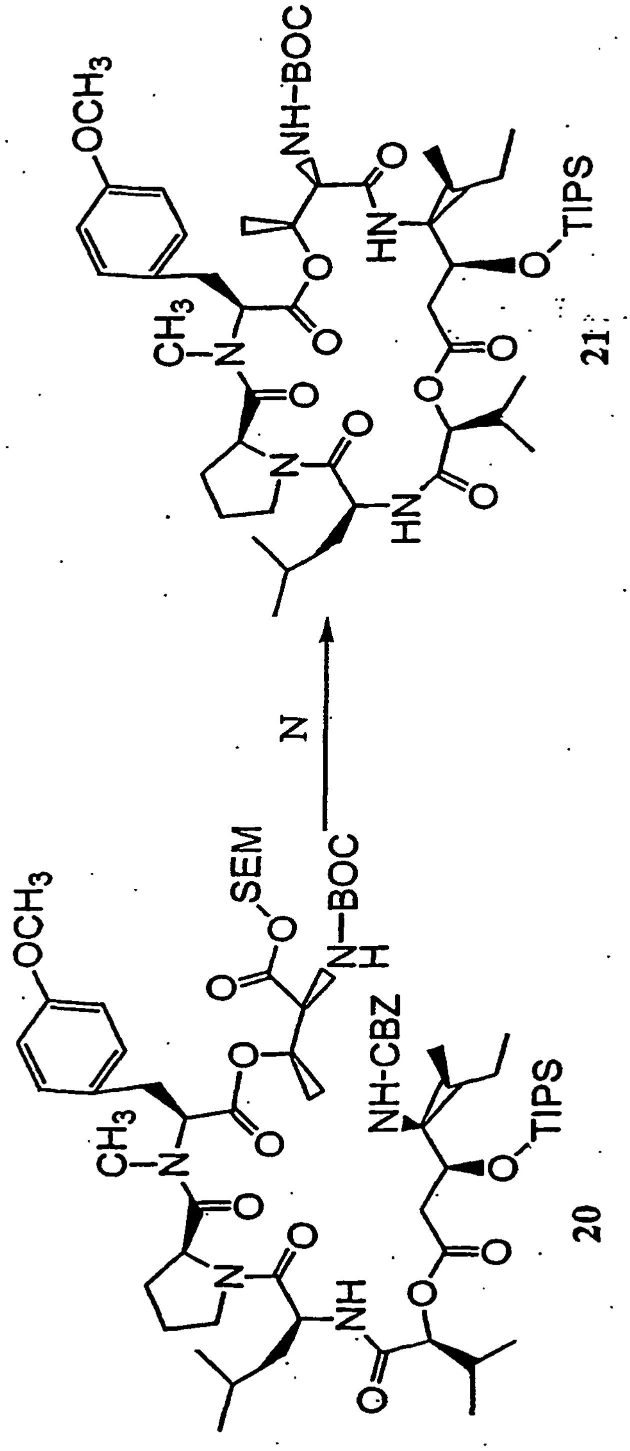


20

21

Fig. 26D

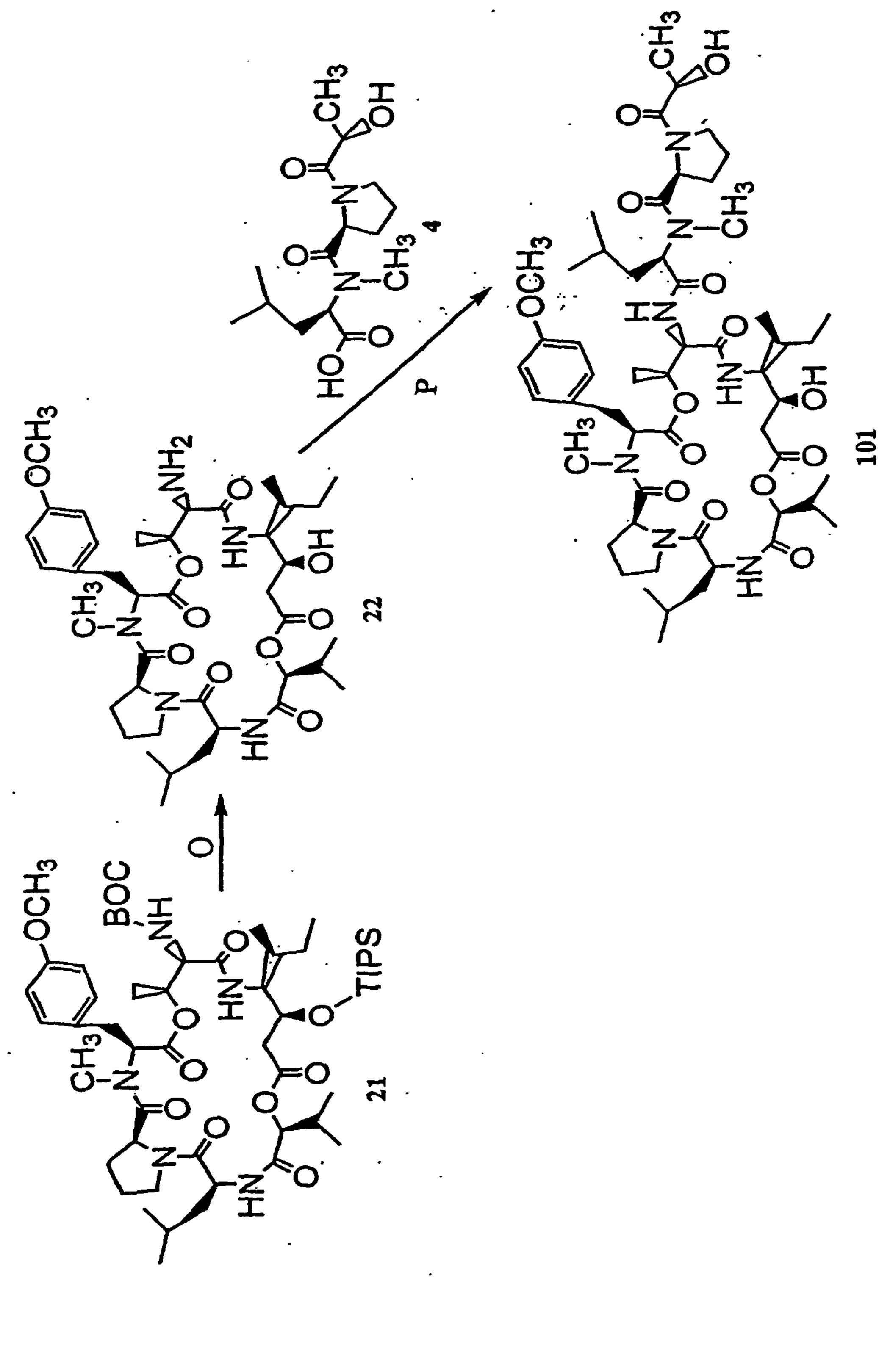


21 22 4 101

Fig. 26E

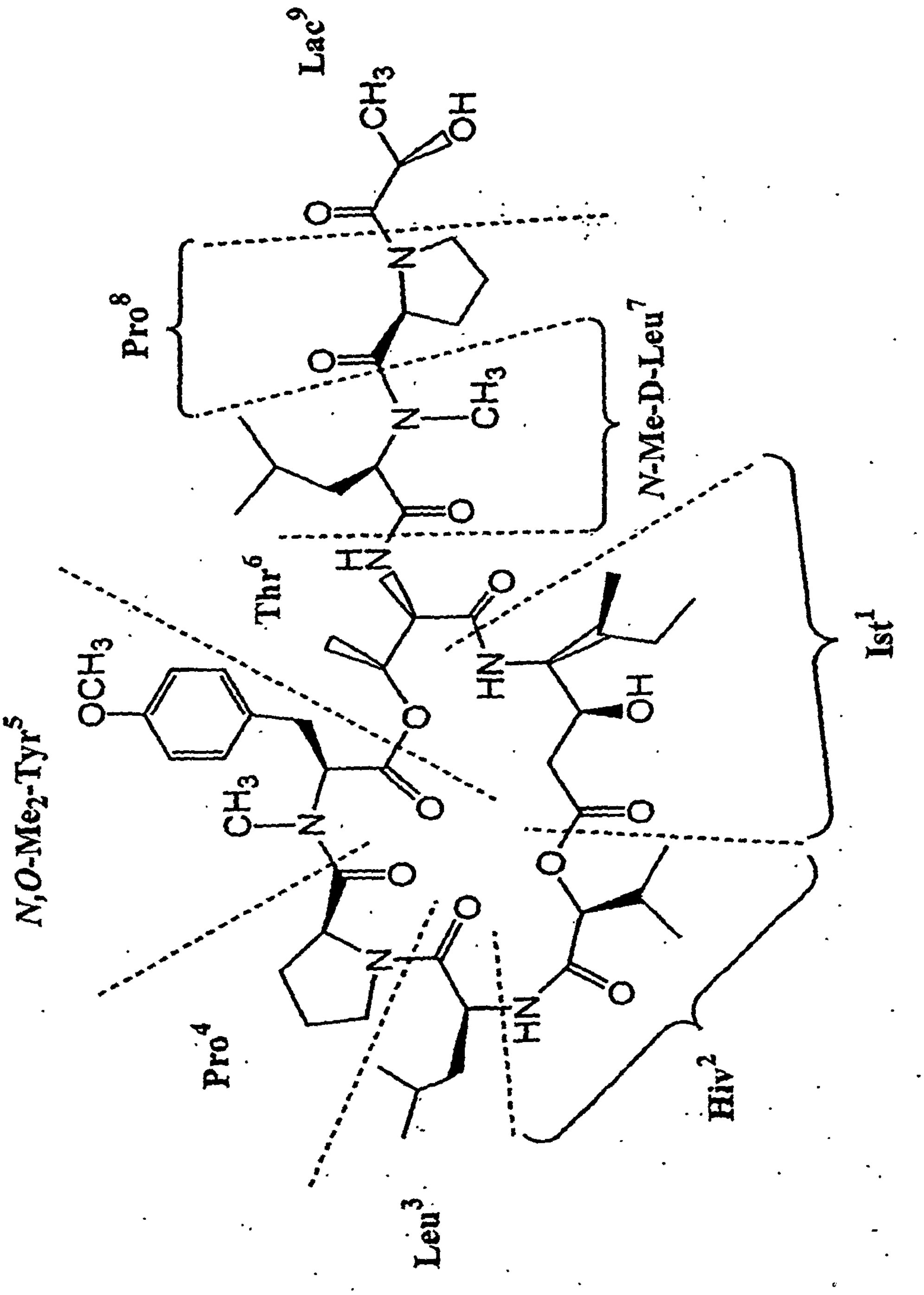

Fig. 27

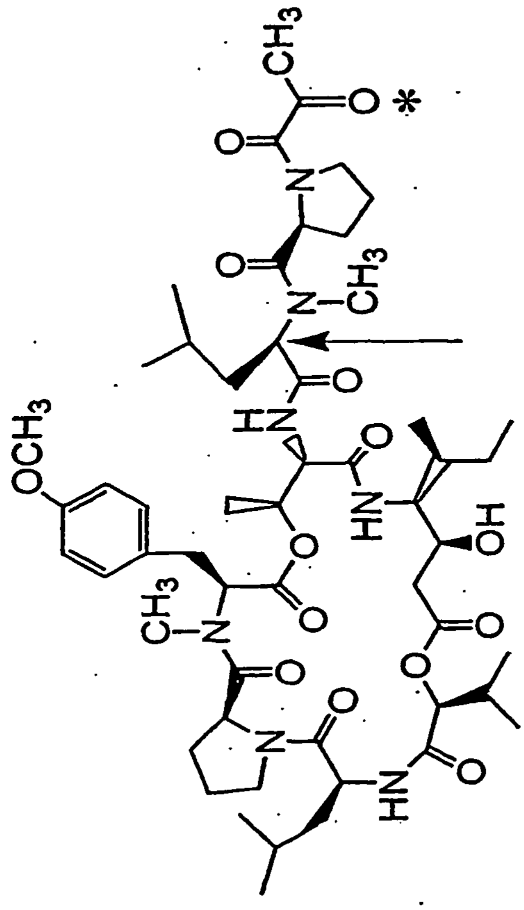

Fig. 28A

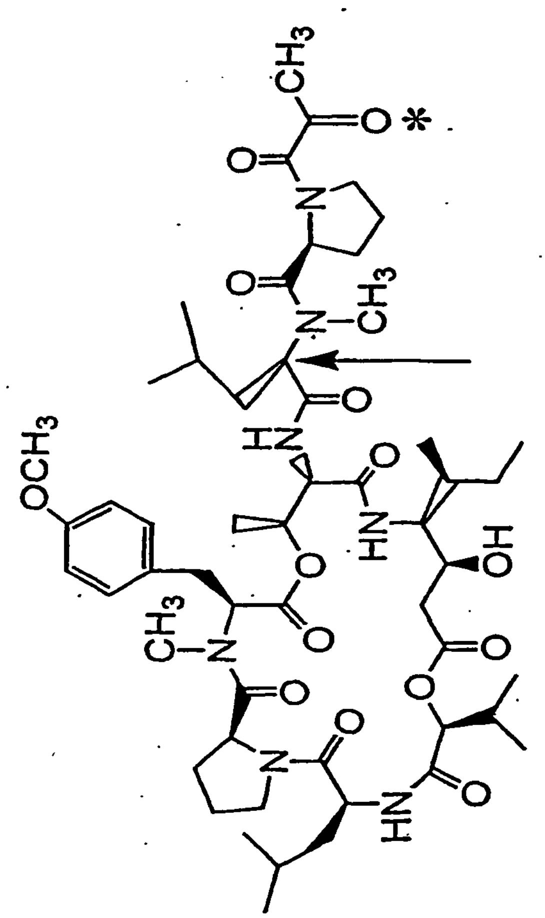

Fig. 28B

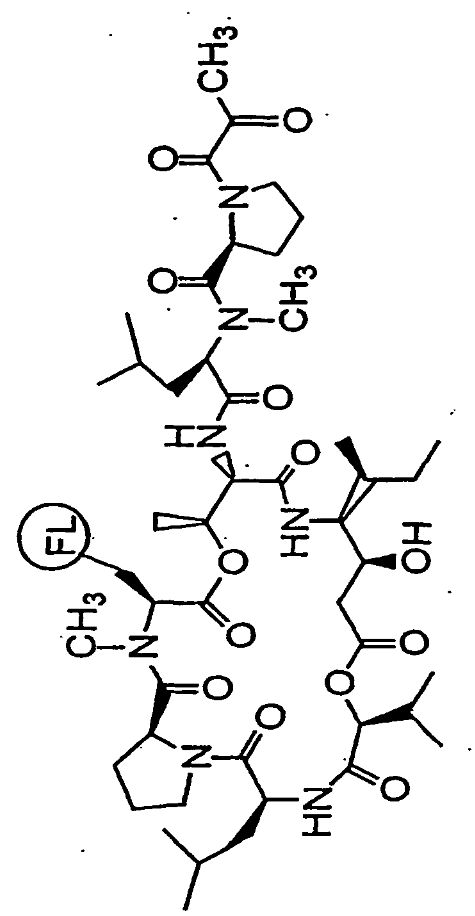

Fig. 29

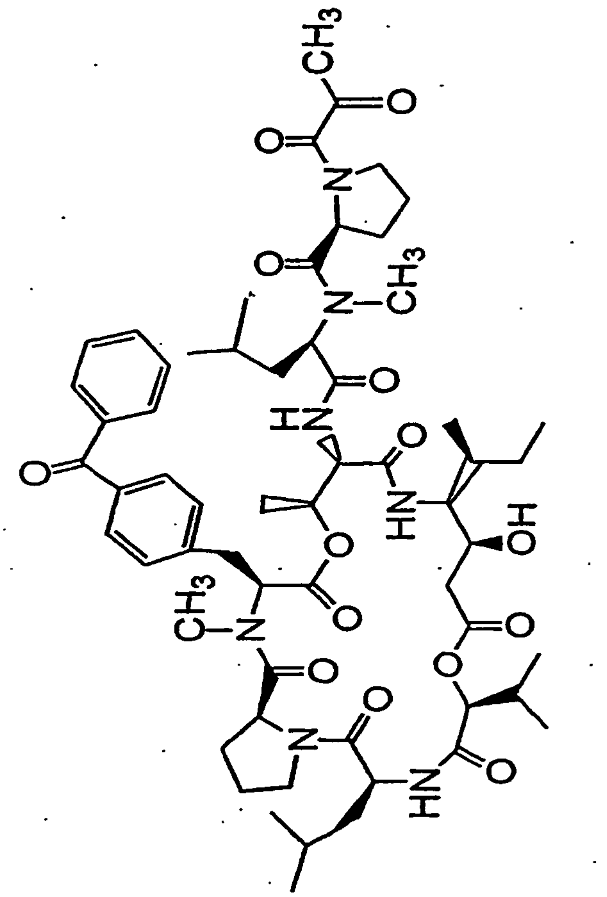

Fig. 30

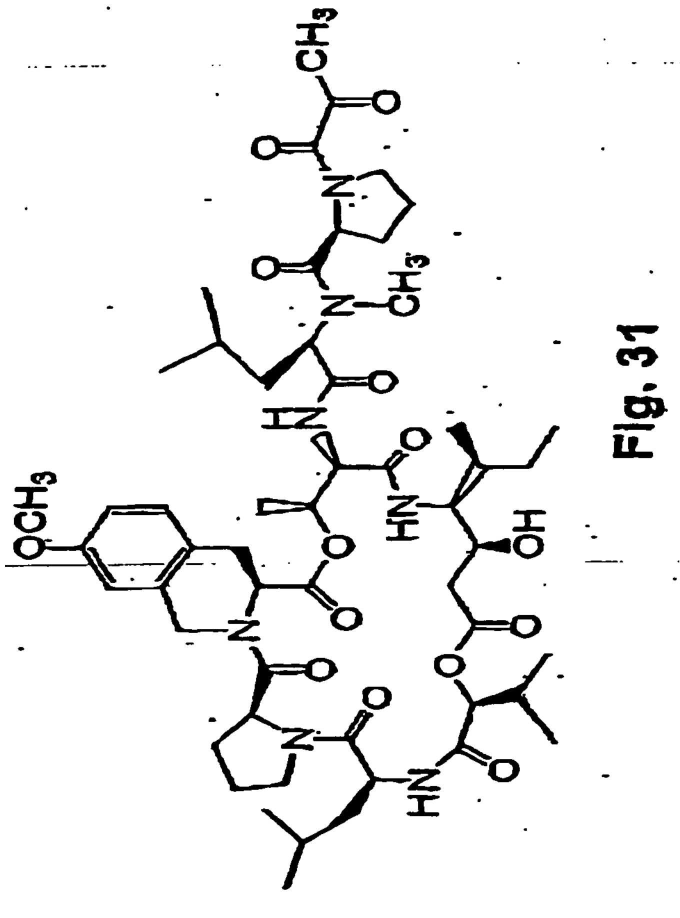

Fig. 31

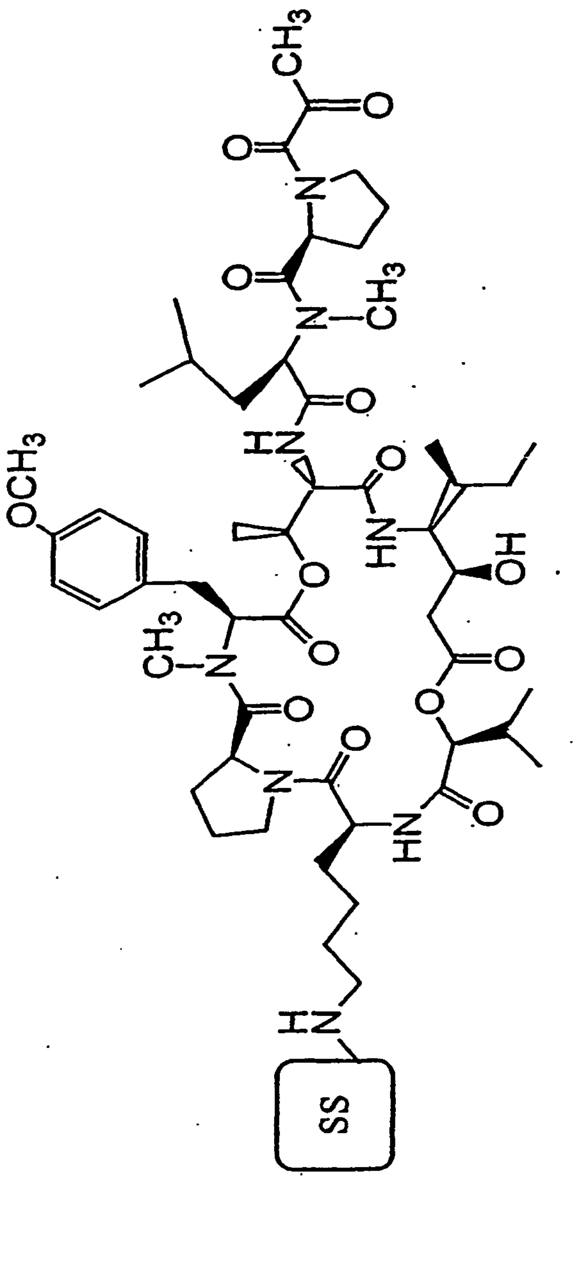

Fig. 32

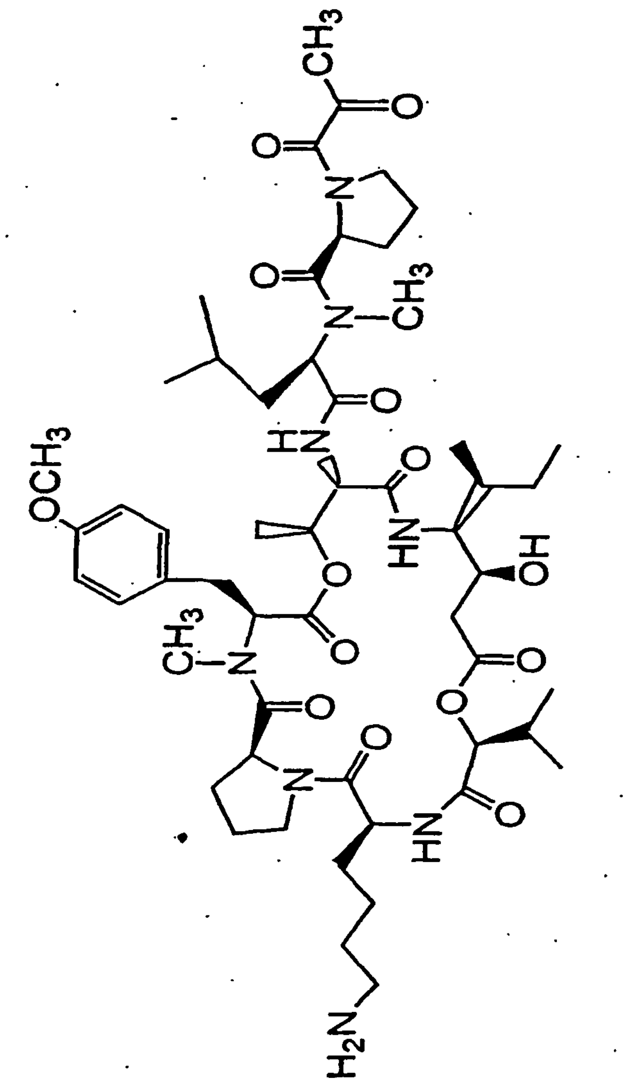

Fig. 33

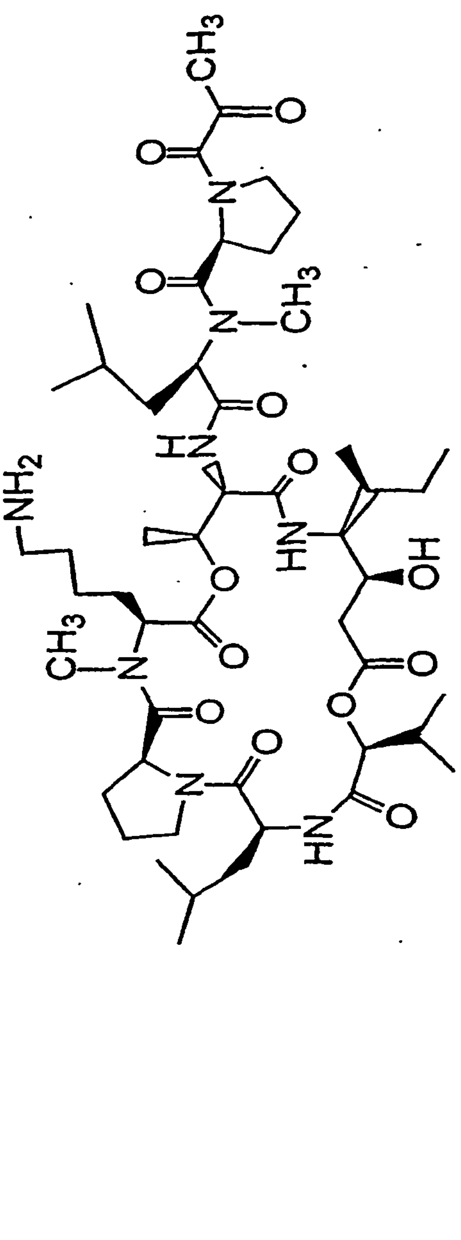

Fig. 34

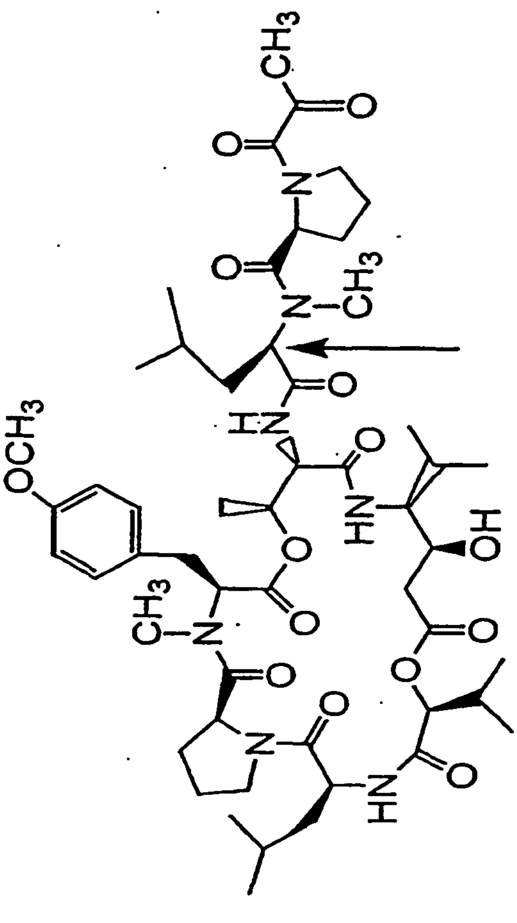

Fig. 35A

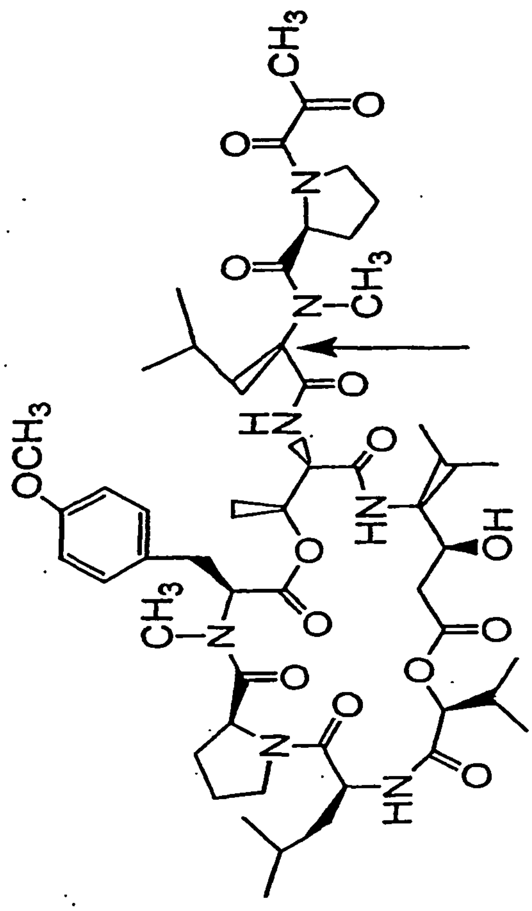

Fig. 35B

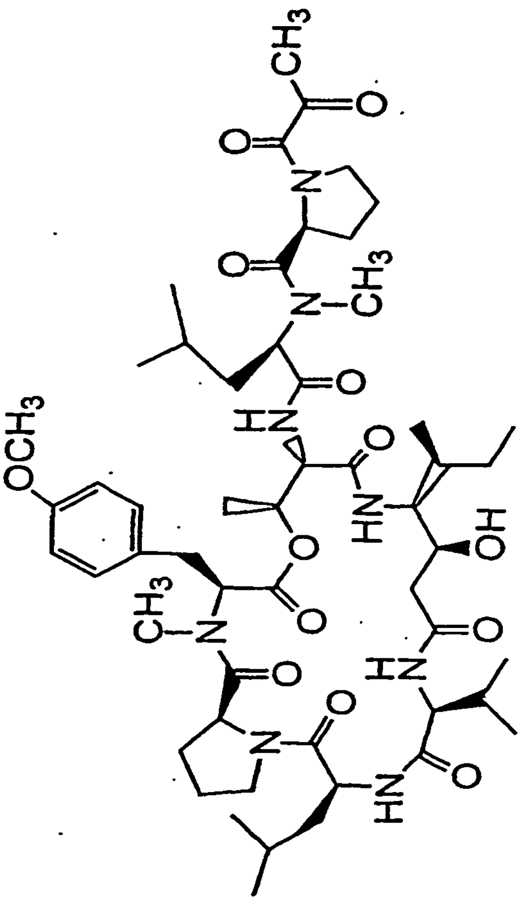

Fig. 36

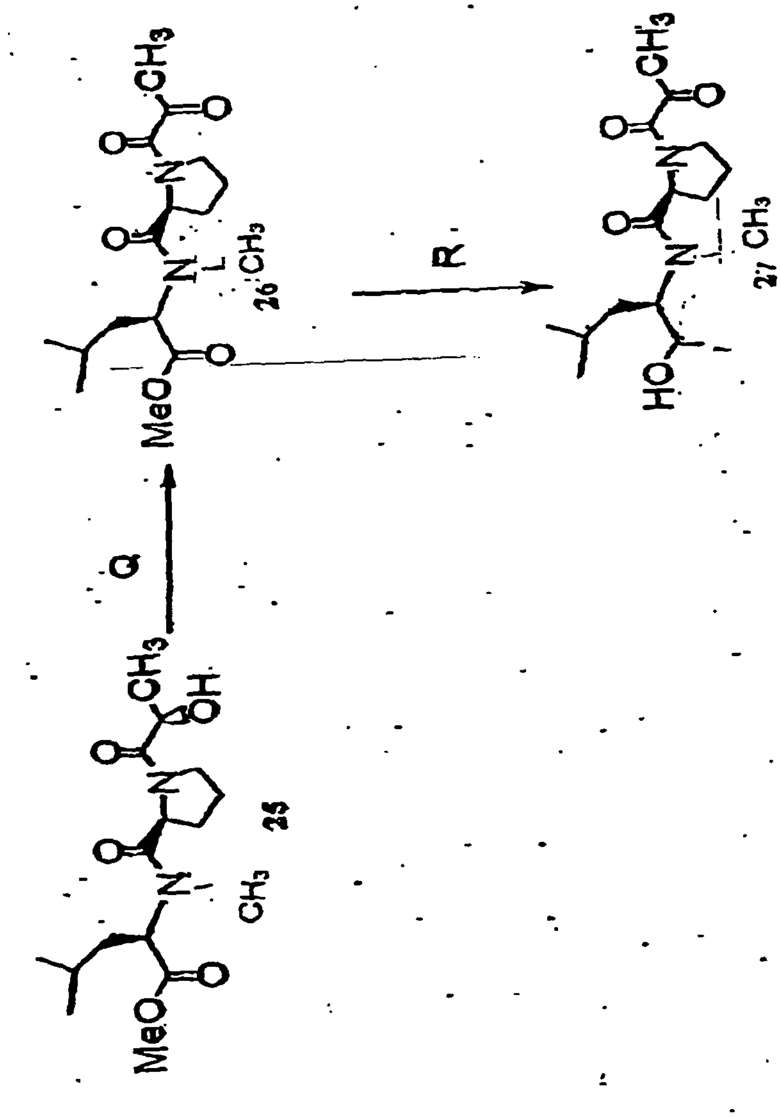


25

26

27

Fig. 37A

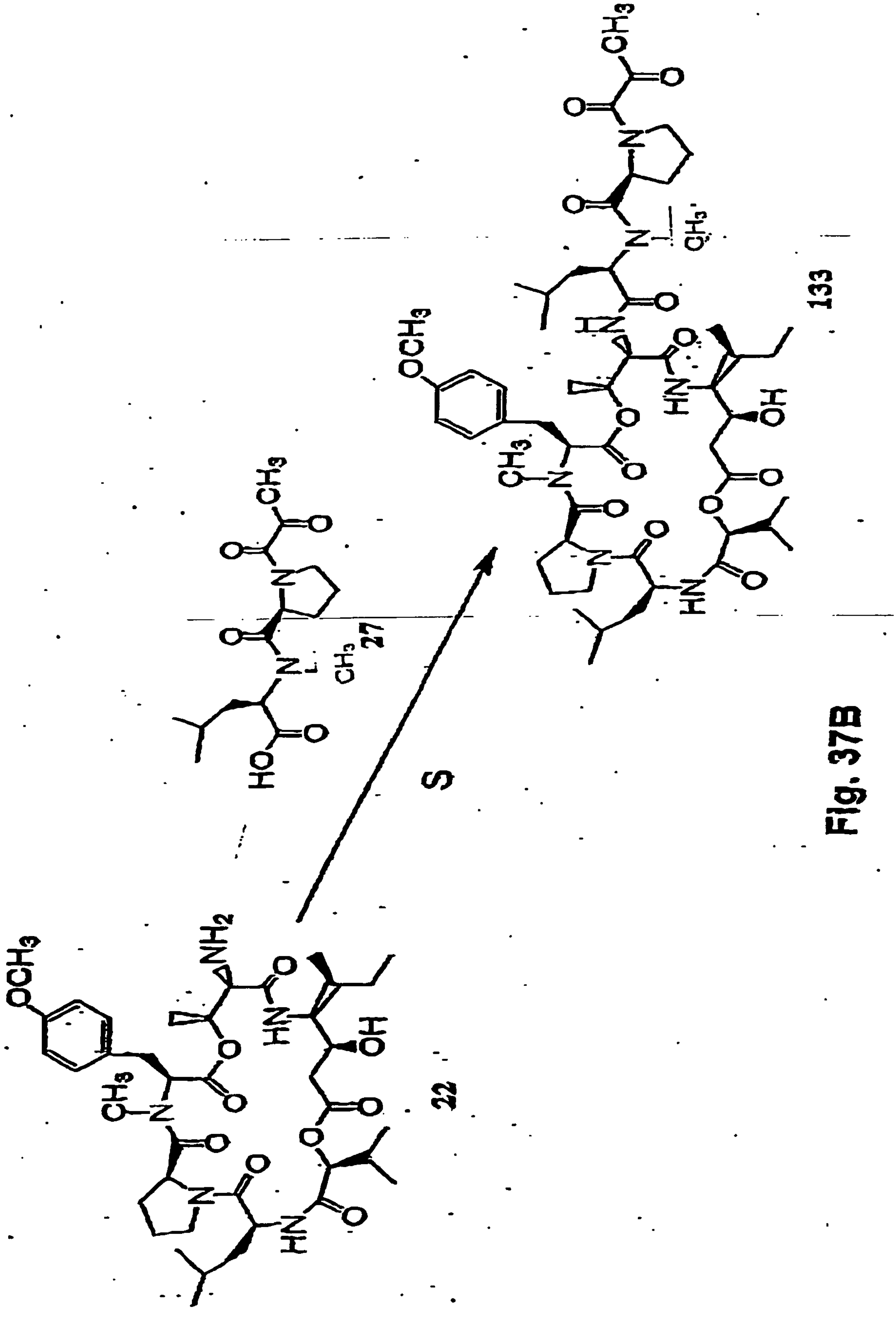


22

27

133

Fig. 37B

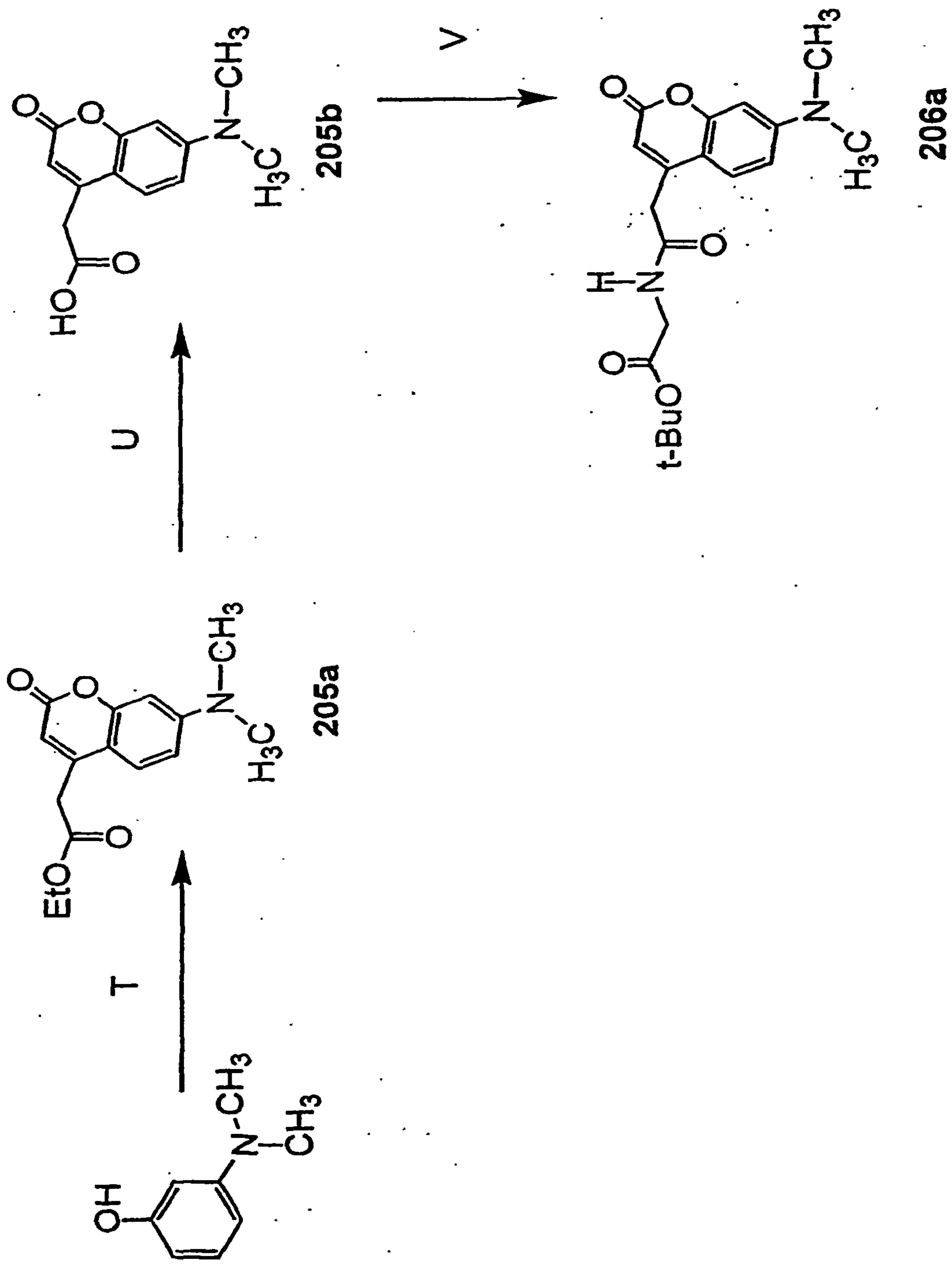


205a

205b

206a

Fig. 38A

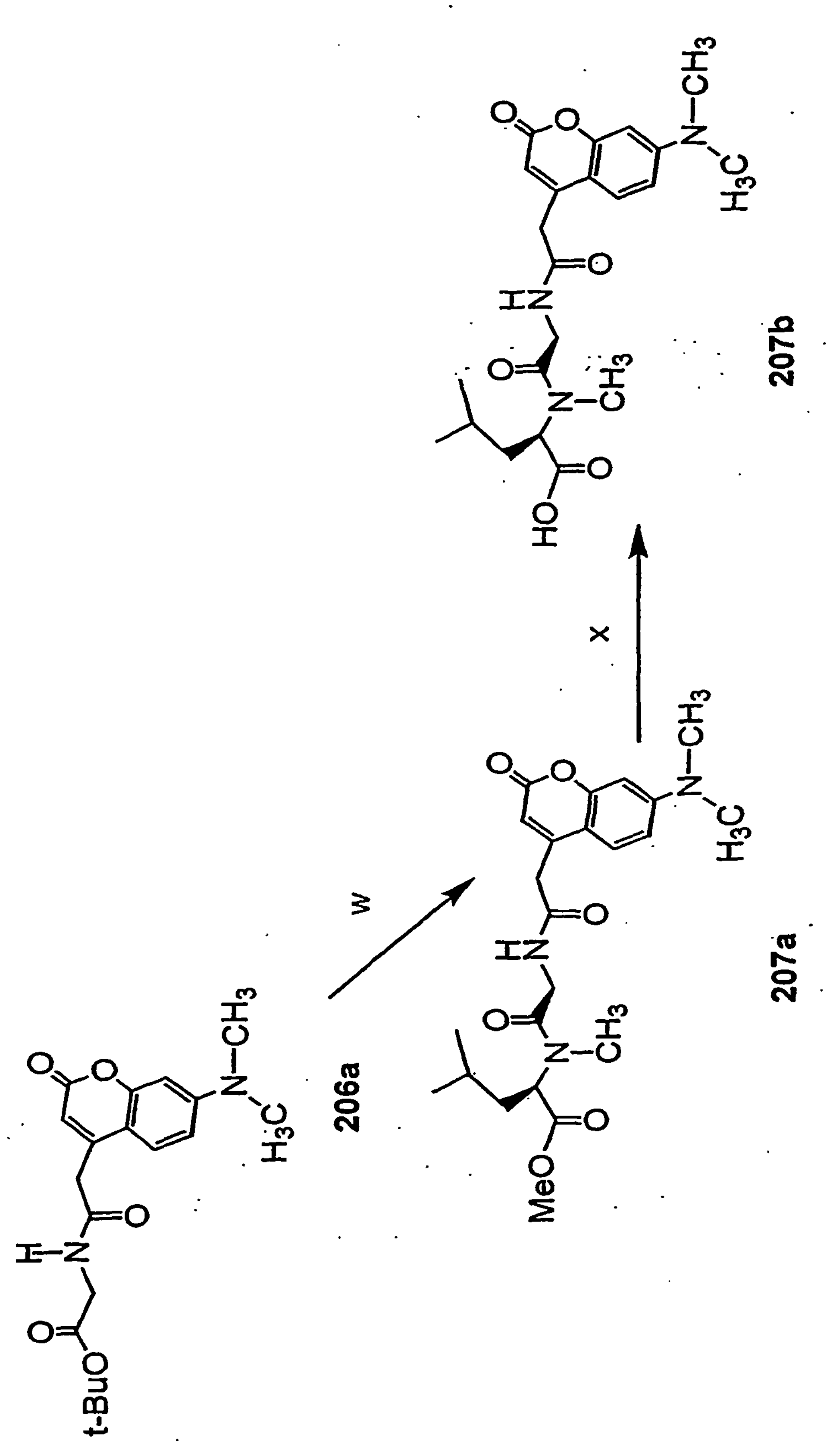


206a

207a

207b

Fig. 38B

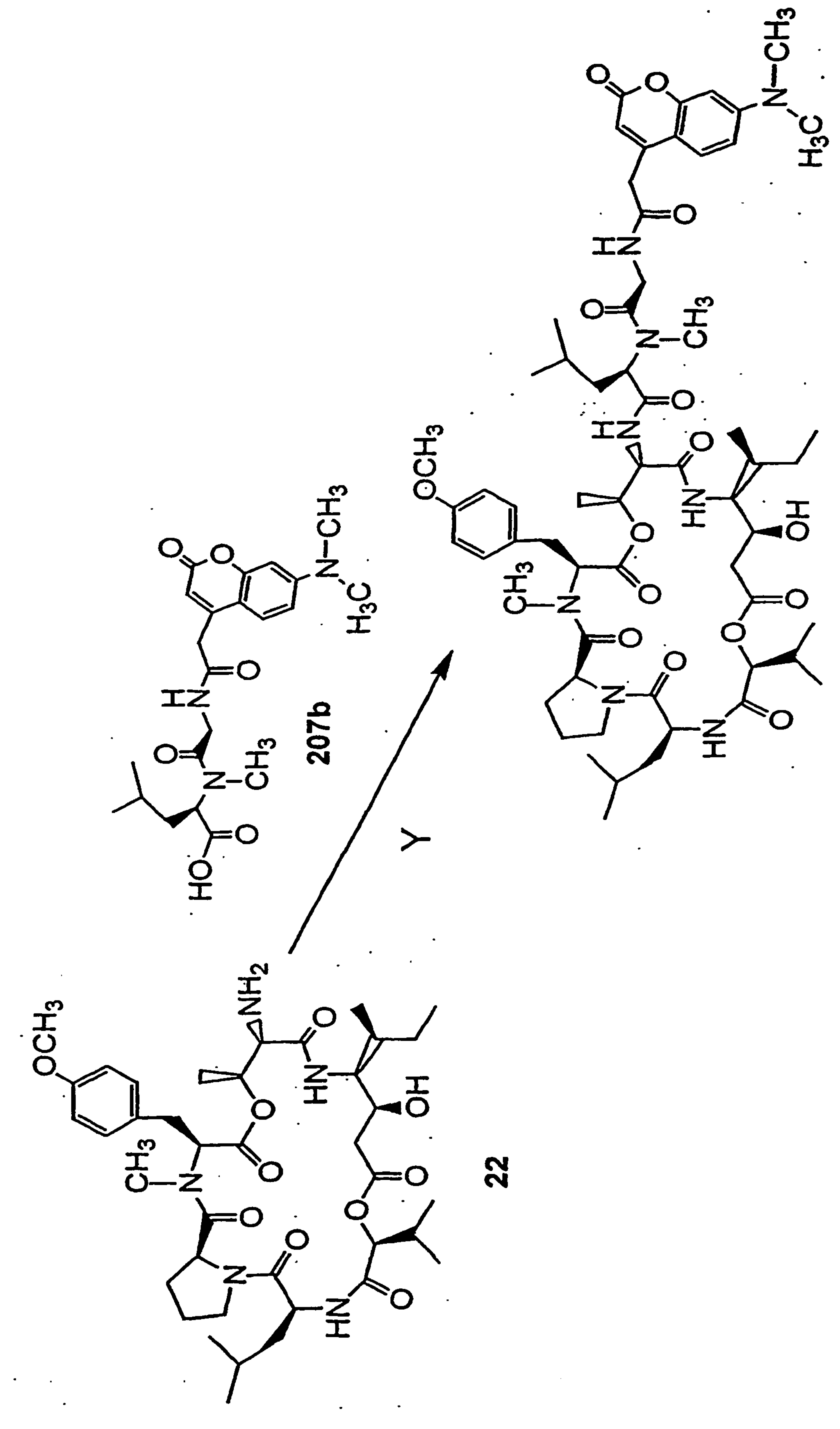


22

207b

107

Fig. 38C

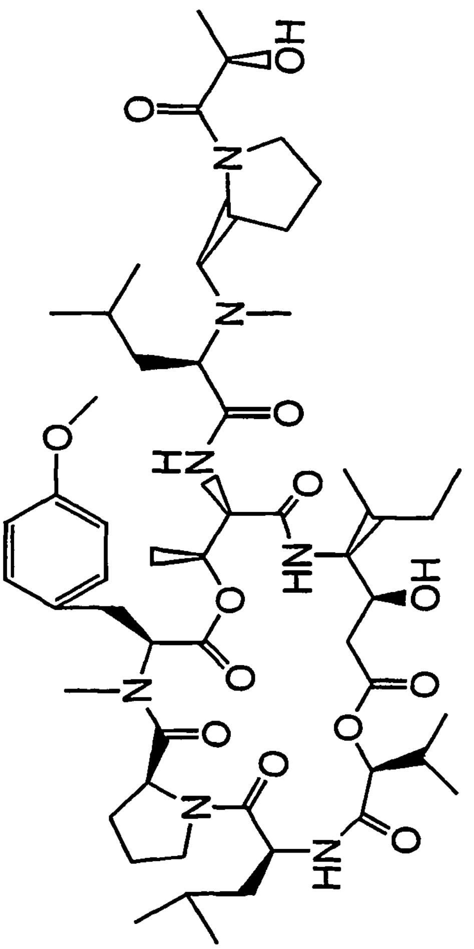

Fig. 39

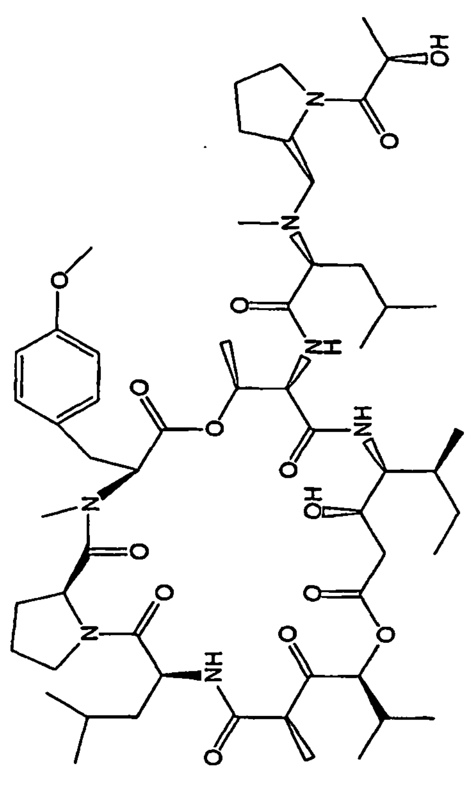

Fig. 40

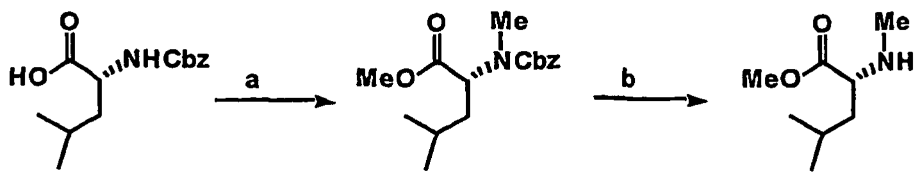


39 40

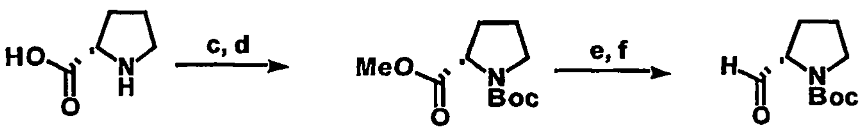


41 42

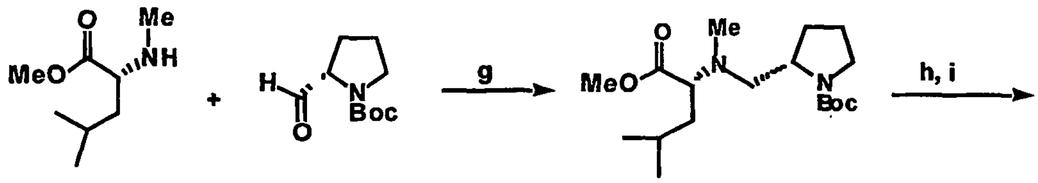


40 + 42 43

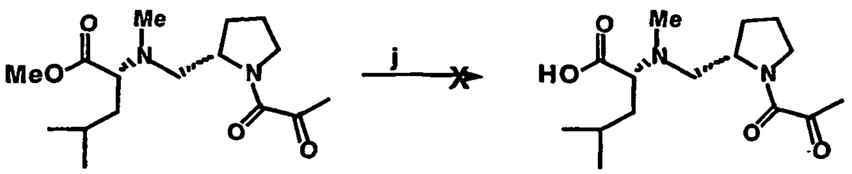


44 45

a. $Me_2SO_4$, KOH, $Bu_4N^+HSO_4^-$, THF, 78%; b. $H_2$, Pd/C, 99%; c. MeOH, $SOCl_2$ 95%; d. $Boc_2O$, $Et_3N$, $CH_2Cl_2$, 85%; e. $NaBH_4$, LiCl, THF/EtOH, 85%; f. $SO_3$.Pyr complex, DMSO, $Et_3N$, $CH_2Cl_2$; g. $Na(AcO)_3BH$, AcOH, $CH_2Cl_2$, 88%; h. HCl in dioxane, 90%; i. pyruvic acid, BOP, NMM, $CH_2Cl_2$, 70%; j. $LiOH.H_2O$, $THF/H_2O$

**Fig. 41**

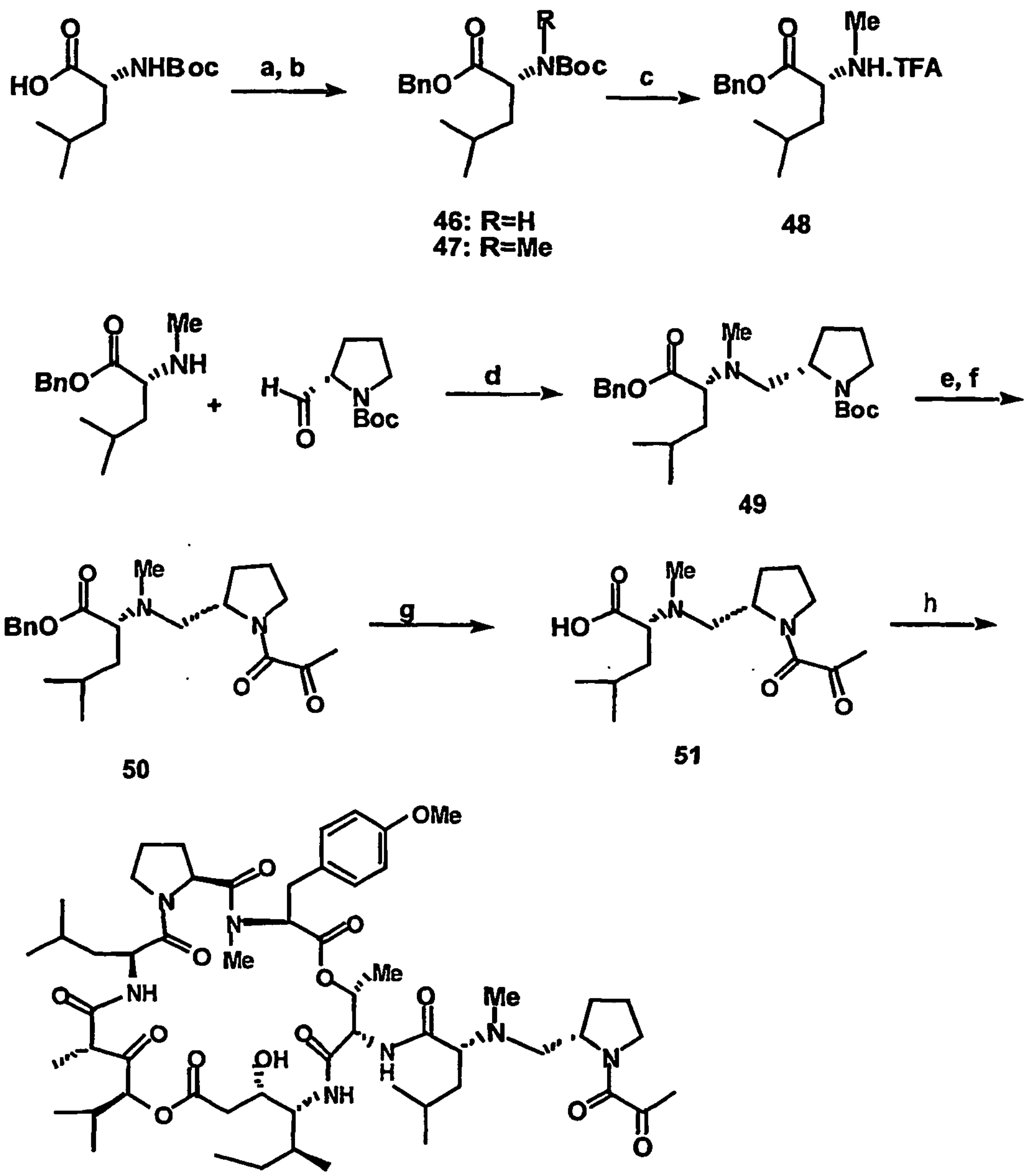


46: R=H
47: R=Me
48
49
50
51
12

a. $Li_2CO_3$, BnBr, DMF, 85%; b. MeI, NaHMDS, $CH_2Cl_2$ 78%; c. TFA/$CH_2Cl_2$, 90%
d. $Na(AcO)_3BH$, AcOH, $CH_2Cl_2$, 88%; e. HCl in dioxane, 90%; f. pyruvic acid, BOP, NMM, $CH_2Cl_2$, 70%; g. $H_2$, Pd/C, 99%; h. didemnin macrocycle salt, DIEA, HATU, $CH_2Cl_2$, 72%

**Fig. 42**

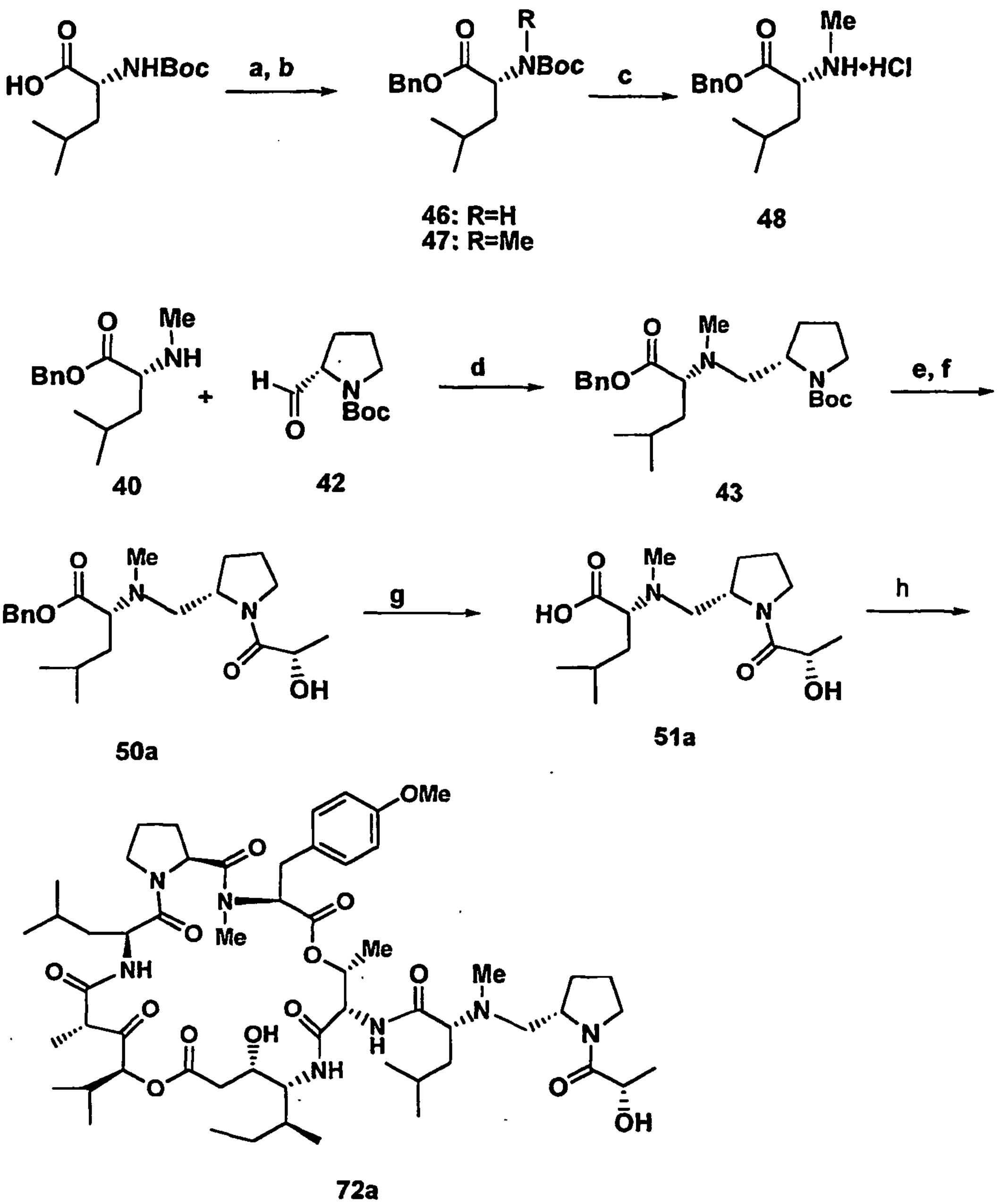


46: R=H
47: R=Me
48
40
42
43
50a
51a
72a

a. $Li_2CO_3$, BnBr, DMF, 85%; b. MeI, NaHMDS, $CH_2Cl_2$ 78%; c. HCl in dioxane, 98%
d. $Na(AcO)_3BH$, AcOH, $CH_2Cl_2$, 88%; e. HCl in dioxane, 98%; f. lactic acid, BOP, NMM, $CH_2Cl_2$,61%; g. $H_2$, Pd/C; h. didemnin macrocycle salt, DIEA, HATU, $CH_2Cl_2$, 72%

**Fig. 43**

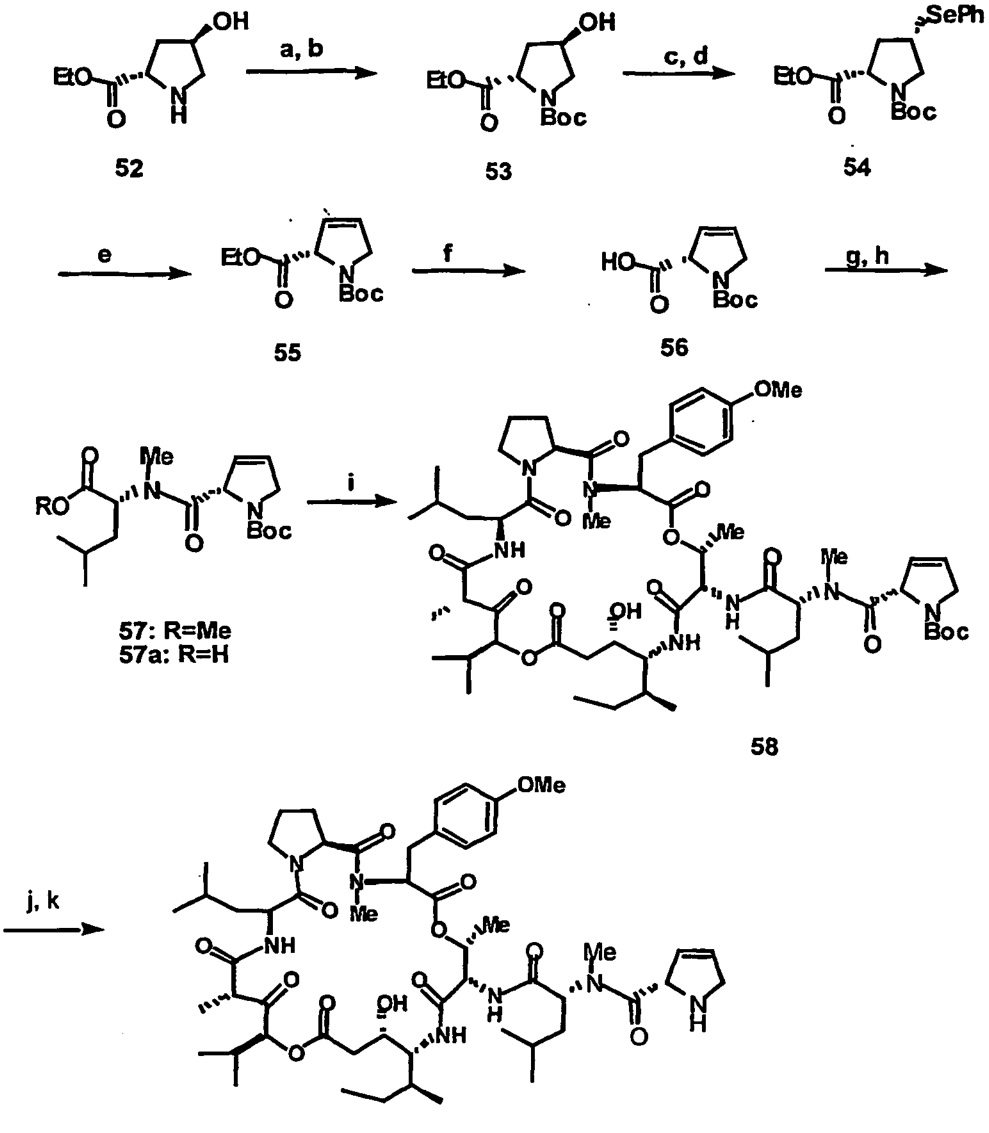


52 53 54 55 56 58

59

a. EtOH, $SOCl_2$, 95%; b. $Boc_2O$, $Et_3N$, $CH_2Cl_2$, 75%; c. MsCl, pyr. $CH_2Cl_2$, 86%; d. $Se_2Ph_2$, $NaBH_4$, EtOH, 86%; e. Pyr., $H_2O_2$, $CH_2Cl_2$, 82%; f. LiOH.$H_2O$, THF/$H_2O$, 95%; g. *N*-Me-D-Leucine methyl ester, BOP, NMM, $CH_2Cl_2$, 75%; h. HCl.dioxane; i. DB macrocycle salt, DIEA, HATU, $CH_2Cl_2$, 72%; j. HCl gas; k. $NaHCO_3$, ethyl acetate

**Fig. 44**

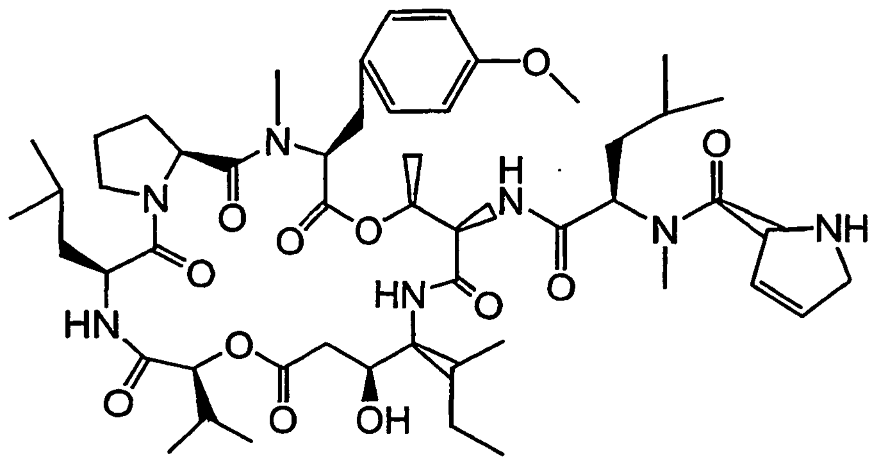

Fig. 45

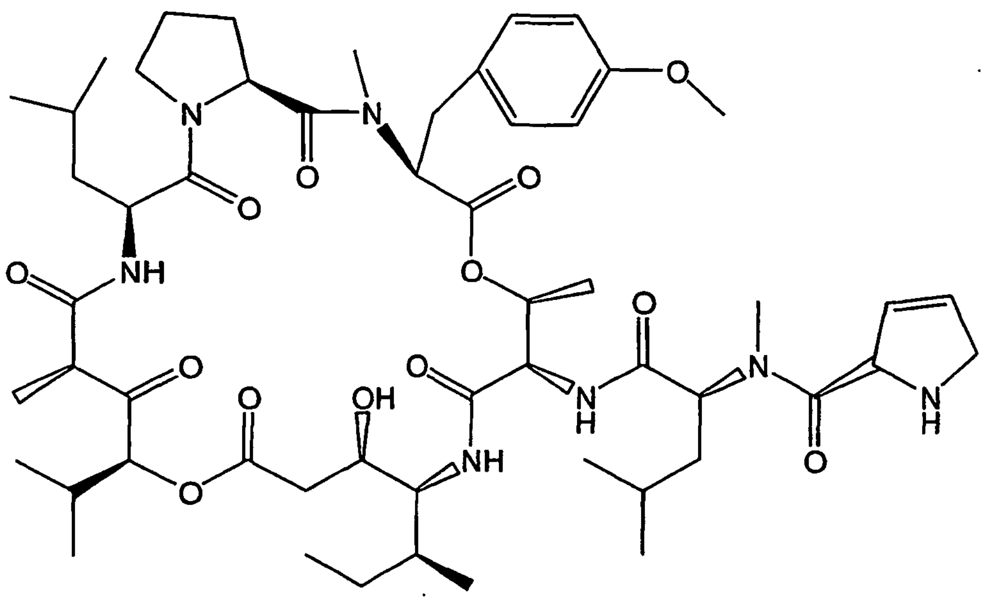

Fig. 46

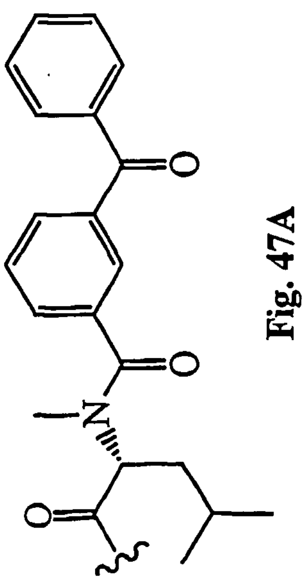

Fig. 47A

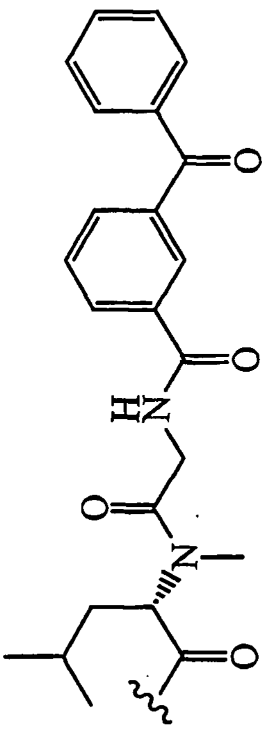

Fig. 47B

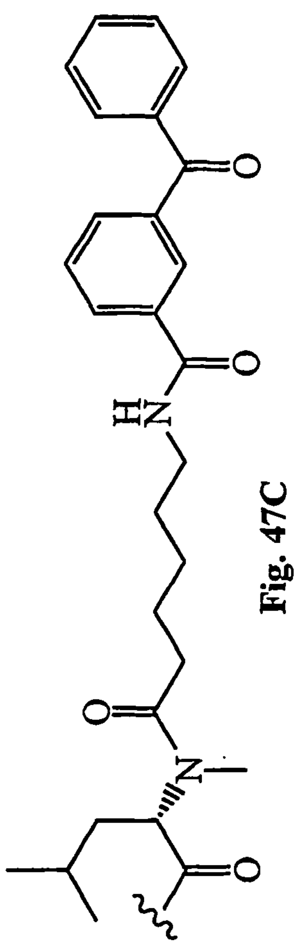

Fig. 47C

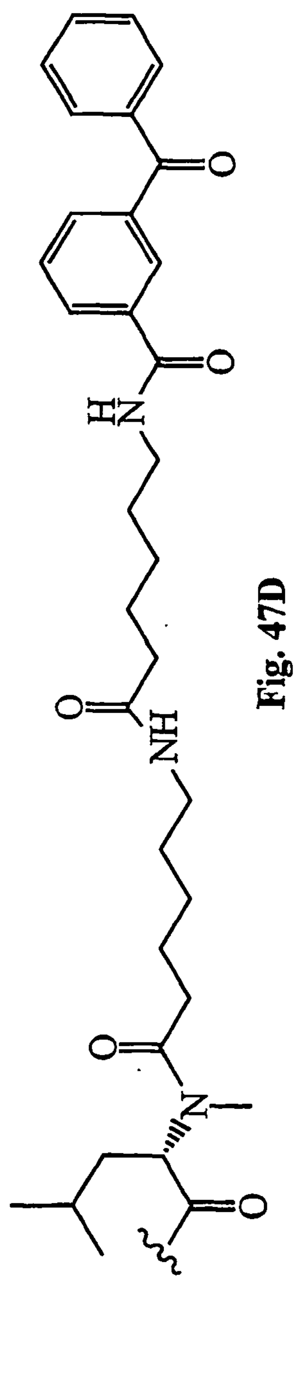

Fig. 47D

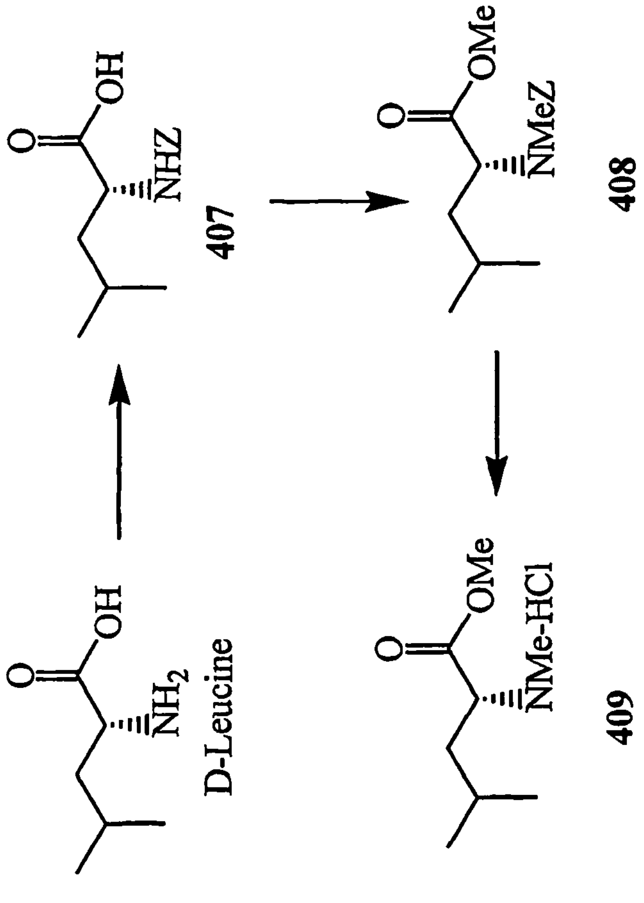

D-Leucine

407

408

409

Fig. 48

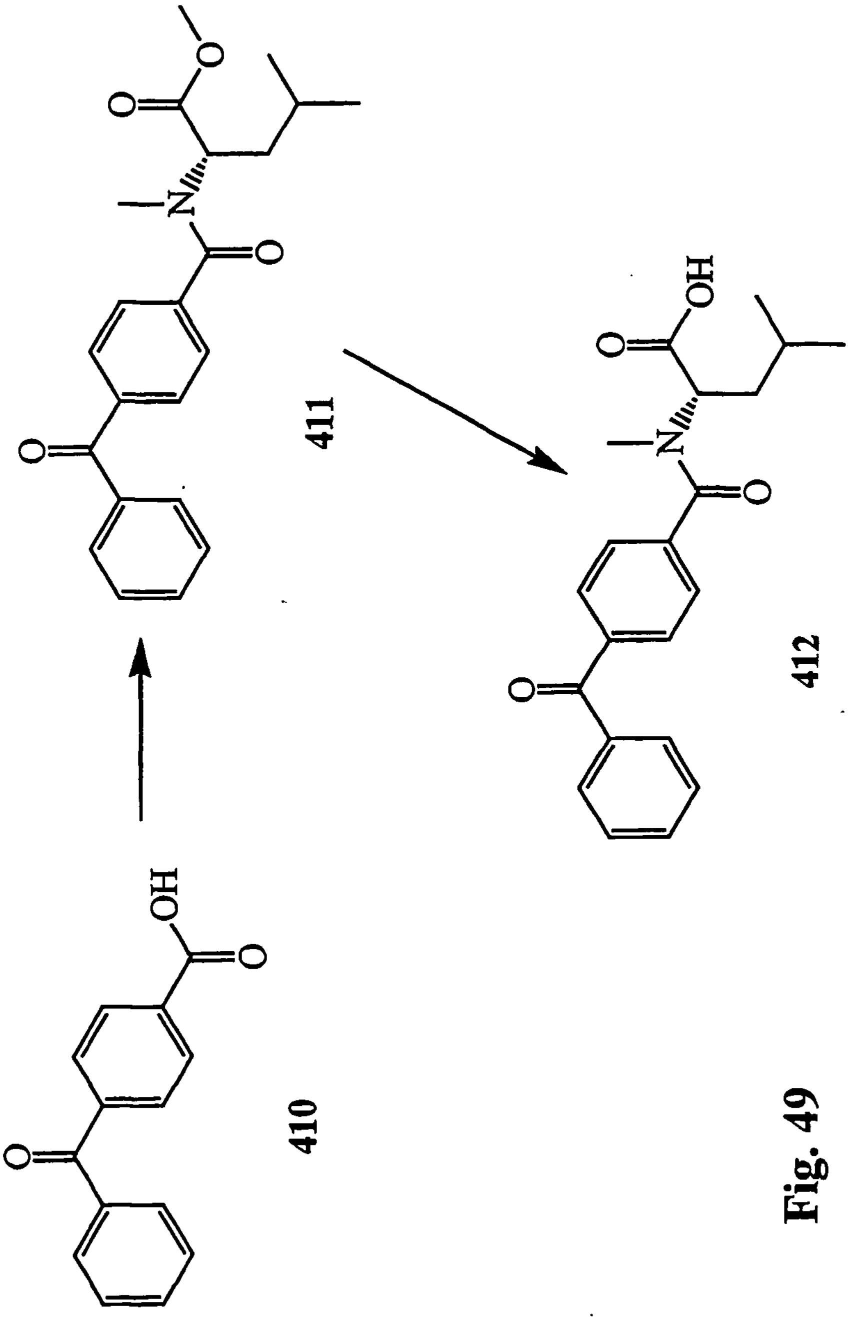

410

411

412

Fig. 49

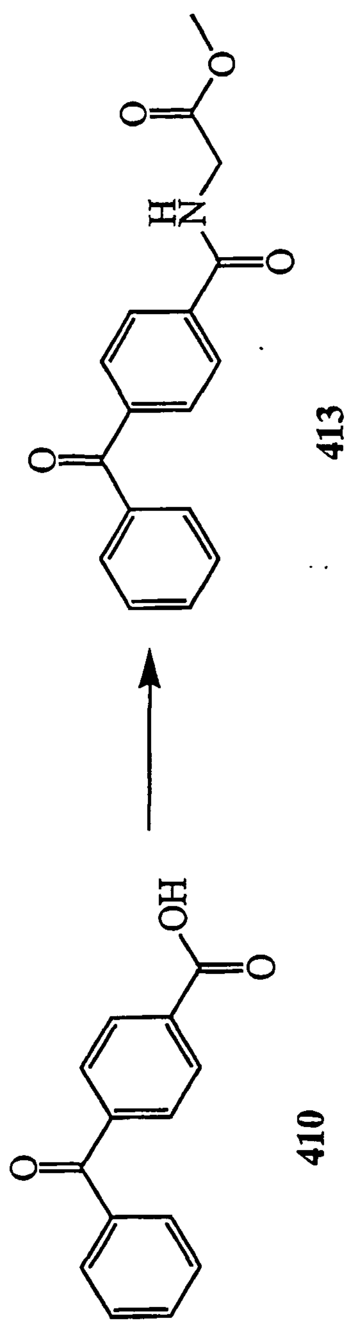

410

413

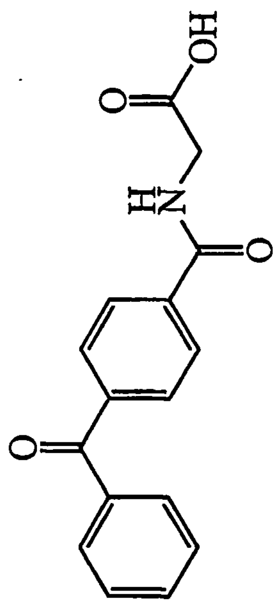

414

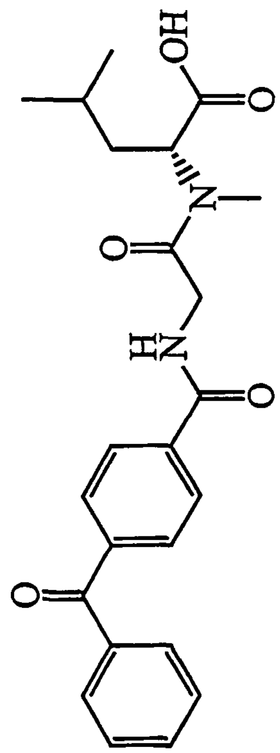

415

Fig. 50